(19) Europäisches Patentamt
European Patent Office
Office européen des brevets

(11) Publication number: 0 030 835 B1

(12) EUROPEAN PATENT SPECIFICATION

(45) Date of publication of patent specification: 11.05.83

(51) Int. Cl.³: C 07 D 237/14, C 07 D 237/04, A 61 K 31/50

(21) Application number: 80304409.8

(22) Date of filing: 05.12.80

(54) **Derivatives of 2-substituted-6-phenyl-3(2H)pyridazinone, their production and pharmaceutical compositions containing them.**

(30) Priority: 05.12.79 JP 158996/79
29.07.80 JP 105359/80
29.07.80 JP 105360/80
29.07.80 JP 105361/80
29.07.80 JP 105362/80
29.07.80 JP 105363/80

(43) Date of publication of application:
24.06.81 Bulletin 81/25

(45) Publication of the grant of the patent:
11.05.83 Bulletin 83/19

(84) Designated Contracting States:
AT BE CH DE FR GB IT LI NL

(56) References cited:
FR - A - 2 416 887

THE MERCK INDEX NINTH EDITION (1976) RAHWAY, N.J. USA Appendix APP—1* APP—1, 2nd column, A3 Cimetidine*

(73) Proprietor: MORISHITA PHARMACEUTICAL CO. LTD.
29 Doshomachi 4-chome Higashi-ku
Osaka (JP)

(72) Inventor: Yamada, Toshihiro
280-43 Harimada-cho
Moriyama-shi Shiga-ken (JP)

(74) Representative: Myerscough, Philip Boyd et al,
J.A.Kemp & Co. 14, South Square Gray's Inn
London, WC1R 5EU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 030 835 B1

Derivatives of 2-substituted-6-phenyl-3(2H)pyridazinone, their production and pharmaceutical compositions containing them

The present invention relates to derivatives of 2-substituted-6-phenyl-3(2H)-pyridazinone to their production and to pharmaceutical compositions containing these derivatives.

The etiology of peptic ulcers has not yet been completely eludicated. It is considered that various important factors combine together to cause peptic ulcers. Nowadays, an antacid is used to neutralise the acidity of gastric juices or an anticholinergic drug is used to inhibit the secretion of the gastric juices in order to treat peptic ulcers. However, sufficiently lengthy activity is not available by the former method (refer to Avery-Jones, J.E. and Lennard-Jones, J.E. "Clinical Gastroenterology", 2nd. Ed., Blackwell Scientific Publications, Oxford, 1968, Chapter 14.). On the other hand, it is known that the latter method causes side effects involving the parasympathetic nerve system such as the inhibition of movement of the stomach at doses at which the anticholinergic drug is effective (refer to Kaye, M.D., Rhodes, J. and Sweetman, P.M., "Gut", *9*,590 (1968)).

Compounds having a thioamide group on a pyridazinone ring and their anti-peptic ulcer activity are disclosed in our Japanese Patent Applications Laying-Open Nos. 21180/1978 and 118469/1980. These compounds inhibit the secretion of gastric juices, and possess a strong anti-ulcer activity because they contain the key 6-phenyl-3(2H)-pyridazinone grouping. However, problems concerning the length of time over which anti-peptic ulcer activity is exhibited and of unfavourable side effects still remain.

It has now been discovered that these problems can be overcome by derivatives of 2-substituted-6-phenyl-3(2H)-pyridazinone represented by the general formula (I):

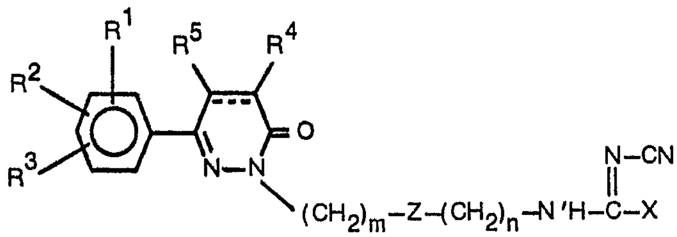

(I)

wherein $R^1$, $R^2$ and $R^3$ independently represents a hydrogen atom, a halogen atom, a lower alkyl group or a lower alkoxy group; $R^4$ and $R^5$ independently represent a hydrogen atom or an alkyl group; *m* is an integer of from 1 to 3; *n* is 0 or an integer of from 1 to 3; Z represents a $-CH_2-$, $-O-$ or $-S-$; X represents $-S-R^6$ or $-N''H-R^7$, wherein $R^6$ represents a lower alkyl group and $R^7$ represents a hydrogen atom or a lower alkyl group or propargyl group; and the two carbon atoms at positions 4 and 5 of the heterocyclic ring of the formula (I) to which $R^4$ and $R^5$ are bonded respectively, are joined by either a single or a double carbon-carbon bond; and acid addition salts thereof; with the proviso that when Z represents $-O-$ or $-S-$, the carbon atoms at positions 4 and 5 are joined by a double bond and *n* represents an integer of from 1 to 3. The apostrophes on the nitrogen atoms N′ and N″ are merely for ease in identifying these nitrogen atoms. The compounds of the invention show a strong anti-peptic ulcer activity with fewer unfavourable side effects. A lower alkyl group or a lower alkoxy group is a group having up to 6, preferably up to 4, more preferably up to 3, carbon atoms.

$R^1$, $R^2$ and $R^3$ in the formula (I) can represent chlorine, methyl or methoxy. Preferably, the group of formula:

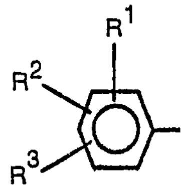

is a phenyl,4-chlorophenyl,3,4-dichlorophenyl,4-methoxyphenyl,3,4-dimethoxyphenyl,3,4,5-trimethoxyphenyl,4-methylphenyl,5-methylphenyl or 2,4-dimethylphenyl group. Preferably one of $R^4$ and $R^5$ is a methyl group and the other is hydrogen, or both $R^4$ and $R^5$ represent hydrogen. $R^6$ can be a methyl group and $R^7$ may be a methyl, ethyl or propyl group.

The compounds represented by the general formula (I) can be easily prepared in accordance with the invention in high yield by any process shown in the following Charts Nos. 1 to 3, wherein $R^8$ represents a lower alkyl group:

CHART No. 1

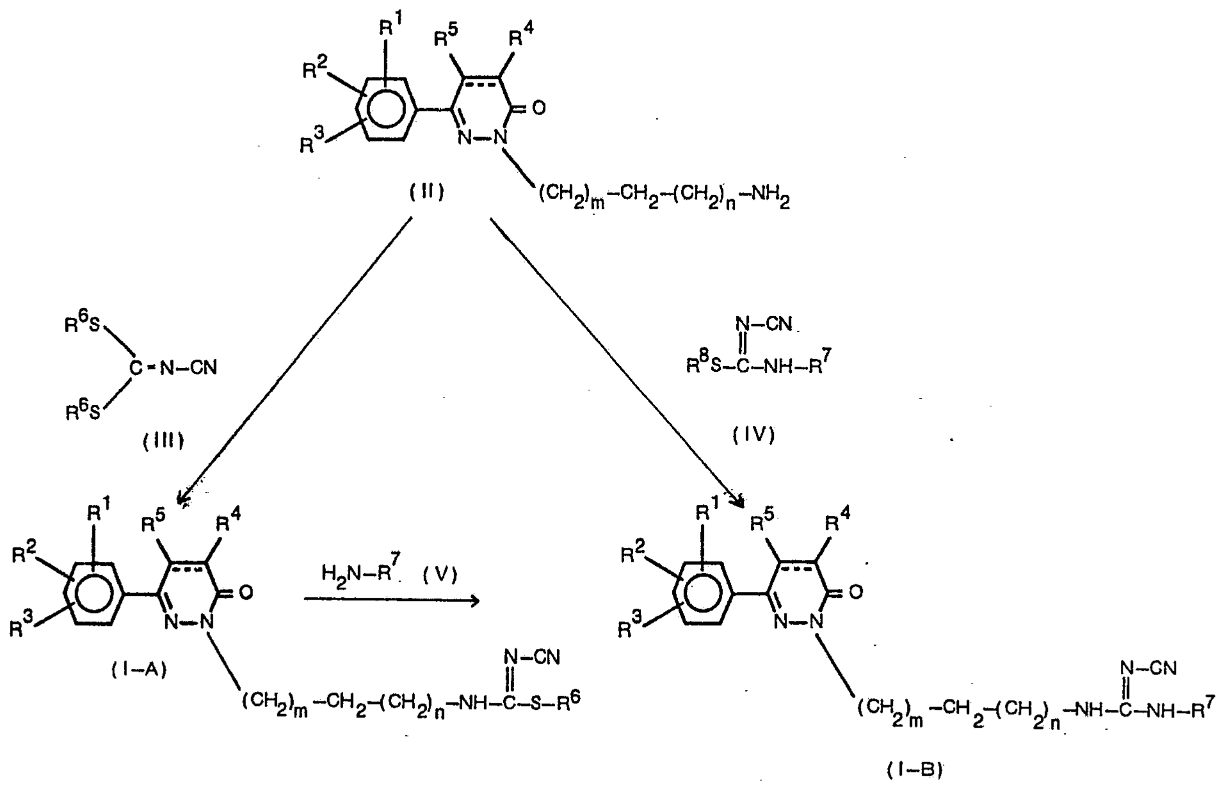


CHART No. 2

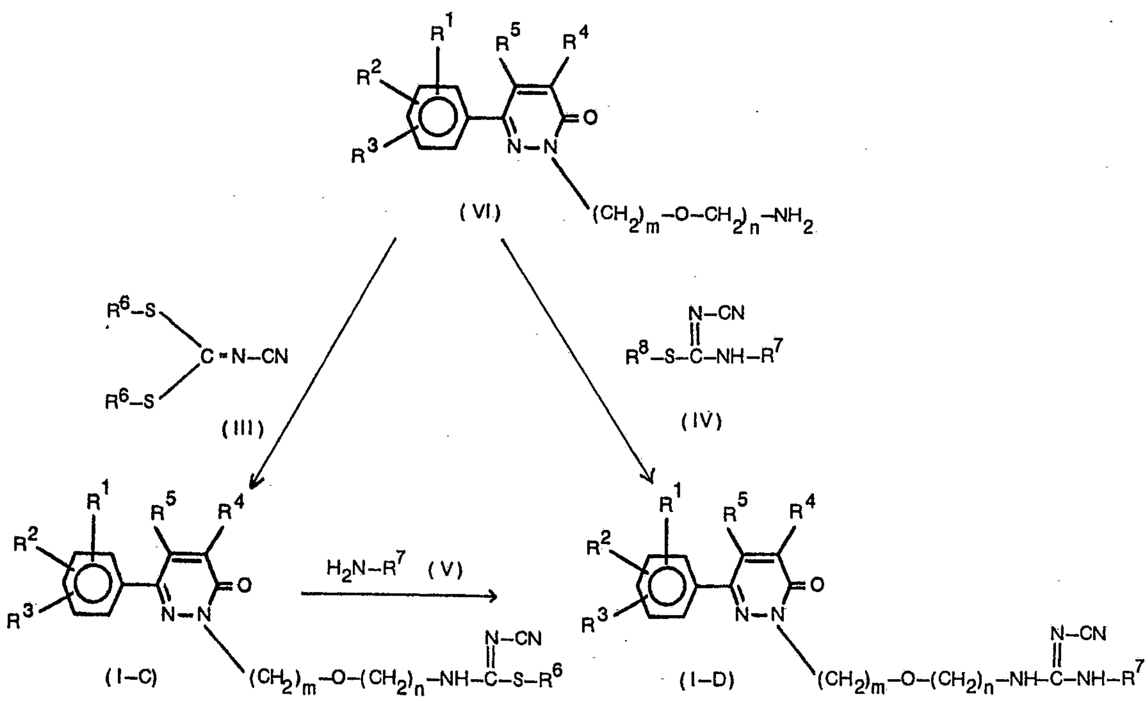

CHART No. 3

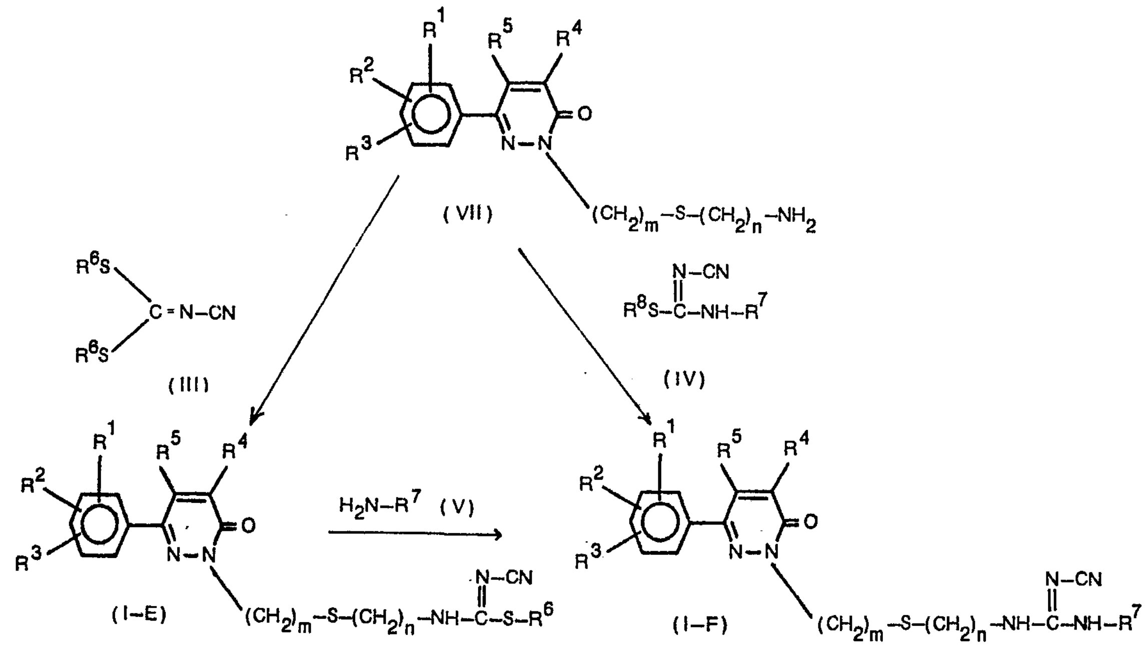


(VII) (III) (IV) (I–E) (I–F)

The compounds of the invention are prepared from a compound (II) having an omega-aminoalkyl group on the nitrogen atom at the 2- position of the pyridazinone ring in Chart No. 1, from a compound (VI) having an omega-aminoalkylene-oxo-alkyl group on the 2-nitrogen atom in Chart No. 2, and from a compound (VII) having an omega-aminoalkylene-thioalkyl group on the 2-nitrogen atom in Chart No. 3. These starting compounds can themselves be prepared by using a compound having an omega-halogenoalkyl group or an omega-phthalimidoalkyl group on the nitrogen atom at the 2- position of the pyridazinone ring as follows:

The starting compound (II) wherein Z is —$CH_2$— is obtained by reaction of the corresponding compound having an omega-halogenoalkyl group or an omega-phthalimidoalkyl group with an aqueous ammoniacal solution or with hydrazine hydrate. The compound (II) can also be prepared by dissolving the corresponding compound having an omega-halogenoalkyl group or an omega-phthalimidoalkyl group in an appropriate solvent and passing gaseous ammonia into the thus prepared solution to carry out amination, etc.

The starting compounds (VI) and (VII) wherein Z is —O— or is —S— respectively are obtained by reacting a compound having an omega-halogeno $C_1$ to $C_3$-alkyl group (which means *m* is 1 to 3 in the general formula (I)) with an equimolar amount or more of an aminoalkyl alcohol or aminoalkylmercaptan (aminoalkanethiol) in an inert solvent in the presence of metallic sodium, sodium hydroxide, etc. at an elevated temperature or room temperature for a few hours.

The isothiourea-derivatives of the present invention represented by formulae (I—A), (I—C) and (I—E) can be prepared by bringing the starting compound (II), (VI) or (VII) into reaction with a corresponding amount of a compound (III) as is shown in Charts Nos. 1 to 3. For instance, dimethyl cyanodithioimidocarbonate or diethyl cyanodithioimidocarbonate can be used as the compound (III). In this reaction, a solvent is not always necessary although an inert solvent such as ethanol, acetonitrile or chloroform is suitable. The reaction is carried out at room temperature or under cooling. However, it is possible to effect the reaction under reflux while using a solvent.

The derivatives of isothiourea of the invention e.g. the compounds (I—A), (I—C) and (I—E), are also important as intermediate compounds for preparing derivatives of guanidine of the invention, e.g. compounds (I—B), (I—D) and (I—F), respectively. As is shown in Charts Nos. 1 to 3, the compounds (I—B), (I—D) and (I—F) are obtained by bringing the respective derivatives of isothiourea, e.g. the compounds (I—A), (I—C) and (I—E), and ammonia or a lower alkylamine, $R^7$—$NH_2$ wherein $R^7$ denotes a hydrogen atom or a lower alkyl, into reaction in an inert solvent such as ethanol, acetonitrile or benzene at an elevated temperature or at room temperature for a few hours. The group —$SR^6$ in the isothiourea derivatives is substituted by the group —NH—$R^7$ in the ammonia or the alkylamine.

In the case where Z represents —$CH_2$—, —O— or —S—, the compounds (I—B), (I—D) and (I—F) may be prepared directly from the starting compounds (II), (VI) and (VII). In such a case, for instance, a nearly equimolar mixture of a compound (II) and a derivative of isothiourea (IV) are brought into reaction in an inert solvent such as acetonitrile, chloroform, etc. at a raised temperature or room

temperature for from a few hours to a few days to give the desired compound of the present invention. The compounds of the general formula (I) can be converted into the appropriate acid addition salts.

The pharmacological and toxicological properties of the compounds of the present invention are explained as follows:

1. *Pharmacological properties:*

Anti-peptic ulcer activity of the compounds was evaluated on Shay's ulcer, stress ulcer and aspirin-induced ulcer, as well as the effects of the compounds on gastric secretion, by the following methods using rats as experimental animals:

1—1) *Shay's ulder:*

Two groups of rats of body weight of from $2 \times 10^{-1}$ to $2.5 \times 10^{-1}$ kg (200 to 250 g), each group consisting of 10 animals, were treated under ether-anaesthesia by ligature of the pylorus after 48 hour-fasting (during which water was taken ad lib.) and, just after the operation, an aqueous suspension of one of the compounds of the invention in a 1% solution of carboxymethyl-cellulose (hereinafter referred to as CMC) was administered intraduodenally to the rats in one group at a dose of $10^{-4}$ kg/kg (100 mg/kg). The other group (control) was given an aqueous 1% solution of CMC intraduodenally. After keeping these animals for 18 hours without giving them any food and water, their stomachs were extirpated under ether anaesthesia and the area ($m^2 \times 10^{-6}$) ($mm^2$) of the ulcer generated on the forestomach part of the rats was determined under an anatomical microscope. The total area of the ulcers of one group was represented by an ulcer coefficient. The rate of inhibition of the occurrence of ulcers (%) was obtained by calculation according to the following formula:

$$\text{Rate of inhibition of the occurrence of ulcers (\%)} = 100 \times \frac{\text{(Ulcer coeff. of control gp)} - \text{(Ulcer coeff. of test gp)}}{\text{(ulcer coeff. of control gp)}}$$

The compounds tested in this experiment and their rate of inhibition of the occurrence of ulcers are shown in Table 1 below.

1—2) *Stress ulcer:*

One group of 10 rats of body weight of from $2 \times 10^{-1}$ to $2.5 \times 10^{-1}$ kg (200 to 250 g) after oral administration of an aqueous suspension of the compound of the invention being tested in a 1% CMC solution at a dose of $10^{-4}$ kg/kg (100 mg/kg) were kept in stress cages and immersed in water at a temperature of 23°C to the depth of their thorax for 20 hours to give a stress of restriction in water. Another group of 10 rats of the same strain (a control group) were also treated with the same stress, but after administration of an aqueous 1% CMC solution.

Just after removing the rats from the water, they were clubbed to death and their stomachs were extirpated. Then, about $1.5 \times 10^{-5}$ $m^3$ (15 ml) of an aqueous 1% formaldehyde solution was injected into each stomach. After maintaining the formaldehyde solution in the stomachs for 10 minutes to enable the solution to contact to the inner stomach wall, each stomach was cut open along the greater curvature to examine the length of erosion (in $10^{-3}$ m (mm)) generated on the mucosa of the glandular stomach region under an anatomical microscope. The sum of the length of erosion for rats of one group was represented by the ulcer coefficient. The rate of inhibition of the occurrence of the ulcer by each compound was obtained by the same method as in 1—1), the results being shown also in Table 1.

1—3) *Aspirin-induced ulcer:*

One group of 10 rats of body weight of from $2 \times 10^{-1}$ to $2.5 \times 10^{-1}$ kg (200 to 250 g) was subjected to laparotomy under ether-anaesthesia, after 24 hour-fasting with ad lib. taking water, to have ligature of their pylorus (according to Okabe *et al.,* 1974). Just after the ligature, an aqueous suspension of one of the compounds of the invention in a 1% CMC solution was intraduodenally administered to the rats at a dose of $10^{-4}$ kg/kg (100 mg/kg). After closing the abdomen, an aqueous dispersion of asprin in a 1% CMC solution was orally administered at a dose of $10^{-4}$ kg/kg (100 mg/kg). After 7 hours of the aspirin administration, the stomach was extirpated under ether-anaesthesia, and the length (in $10^{-3}$ m (mm)) of erosions generated on the mucosa of the glandular stomach region was measured. The sum of the length of erosion on rats of one group was represented by the ulcer coefficient. The same procedures were carried out on another group of 10 rats of the same strain (a control group) except that an aqueous 1% CMC solution was administered instead of the aqueous dispersion of one of the compounds of the present invention. The rate of inhibition of the occurrence of ulcers was obtained by calculation as in 1—2), the results being also shown in Table 1.

1—4) *Effects on gastric secretion:*

One group of 10 rats of body weight of from $1.5 \times 10^{-1}$ to $1.8 \times 10^{-1}$ kg (150 to 180 g) was subjected to ligature of the pylorus after 24-hours fasting, under ether-anaesthesia, and one of the

compounds of the present invention or the reference compound was administered as a dispersion in an aqueous 1% CMC solution into the duodenum. Only an aqueous 1% CMC solution was administered into the duodenum of a control group of 10 rats. After 7 hours from closing the abdomen, the stomach was extirpated under ether-anaesthesia to collect the gastric juice that had accumulated in it. The inhibiting effect of each drug on gastric secretion was calculated by the following formula:

$$\text{Inhibiting effect on gastric secretion (\%)} = 100 \times \frac{A-B}{A}$$

wherein Amount of gastric juice of control = A ml
Amount of gastric juice of test group = B ml
The results are also shown in Table 1.

*Table 1:* Toxicological and Pharmacological Properties of the Present Compounds

| Compound No. | Structure of Tested compound* | | | | | | Acute oral toxicity ($LD_{50}$, kg × $10^{-6}$/kg) | Rate of inhibition** | | | |
|---|---|---|---|---|---|---|---|---|---|---|---|
| | $R^1$ | $R^4$ | $R^7$ | m | n | Z | | Gastric secretion (%) | Shay's ulcer (%) | Stress ulcer (%) | Aspirin-ulcer (%) |
| No. 44 | H | H | $CH_3$ | 1 | 2 | $CH_2$ | over 5,000 | 77.6 | 97.8 | 71.2 | 97.5 |
| No. 48 | H | H | $C_2H_5$ | 1 | 1 | $CH_2$ | over 5,000 | 51.9 | 76.4 | 70.5 | 93.8 |
| No. 50 | H | H | $C_2H_5$ | 1 | 2 | $CH_2$ | over 5,000 | 59.2 | 73.4 | 77.2 | 86.2 |
| No. 53 | H | H | $C_2H_5$ | 1 | 3 | $CH_2$ | over 5,000 | 68.5 | 93.2 | 79.0 | 90.6 |
| No. 138 | H | H | $CH_3$ | 1 | 2 | S | 4,800 | 70.2 | 88.3 | 68.2 | 90.2 |
| No. 81 | H | $CH_3$ | $CH_3$ | 1 | 2 | $CH_2$ | over 5,000 | 54.2 | 72.4 | 63.4 | 90.8 |
| No. 59 | 4—Cl | H | $CH_3$ | 1 | 2 | $CH_2$ | 4,800 | 63.1 | 61.5 | 72.1 | 89.5 |
| No. 65 | 4—$CH_3$ | H | $CH_3$ | 1 | 2 | $CH_2$ | over 5,000 | 58.5 | 70.3 | 70.5 | 91.4 |

Note: * The structure of the compounds of the invention is expressed by the formula:

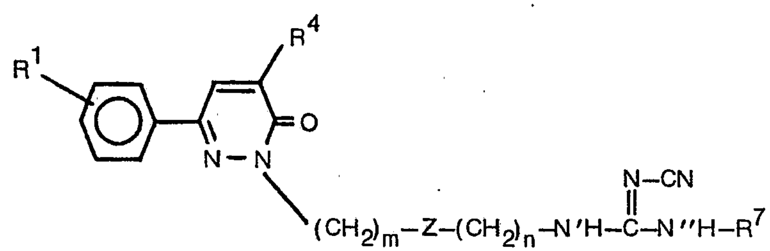

** Experiments were carried out on rats and the rate of inhibition was obtained at a dose of $10^{-4}$ kg/kg (100 mg/kg) orally or intraduodenally.

2. *Toxicological properties:*

The toxicological test data obtained by orally administering compounds of the invention to rats showed that $LD_{50}$ p.o. is $4.8 \times 10^{-3}$ kg/kg (4,800 mg/kg) or larger than $5.0 \times 10^{-3}$ kg/kg (5,000 mg/kg) as is seen in Table 1.

Furthermore, in a subacute oral toxicological test, compounds of the invention were administered to rats at a dose of $3 \times 10^{-3}$ kg/day (3,000 mg/day) for one month. The general symptoms attributable to the compound, the weight gain of the animal and the amount of feed taken by the animal were determined. These results were compared to those determined on control animals. After one month, the rats were sacrificed to examine their major organs, blood and urine. No abnormal findings were observed on specimens treated with a compound of the invention.

Since the safety of compounds of the present invention is high enough, they can be effectively and safely used as an active ingredient in a pharmaceutical composition also comprising a pharmaceutically acceptable carrier or adjuvant. These compositions can be used in treating peptic ulcers. The pharmaceutical composition may take the form of tablets, sugar-coated tablets, capsules and granules. The content of a compound of the invention in such a pharmaceutical composition may be more than 50% by weight, preferably 70% by weight.

The clinical dose of a compound of the invention can be $7.5 \times 10^{-5}$ to $1.2 \times 10^{-3}$ kg/60 kg body weight/day (75 to 1,200 mg/60 kg body weight/day, preferably $1.5 \times 10^{-4}$ to $6.0 \times 10^{-4}$ kg/60 kg body weight/day (150 to 600 mg/60 kg body weight/day).

The following Examples illustrate the present invention. Figures 1 to 143 of the accompanying drawings correspond respectively to the infrared absorption spectra of Compounds Nos. 1 to 143 prepared in Examples 1 to 17. Percentages are by weight.

Example 1

(a) Synthesis of 2-(4-aminobutyl)-6-phenyl-3(2H)-pyridazinone:

$1.5 \times 10^{-2}$ Kg (15 g, 0.3 mol) of hydrazine hydrate was added into a suspension of $10^{-1}$ kg (100 g, 0.27 mol) of 6-phenyl-2 (4-phthalimidobutyl)-3(2H)-pyridazinone in $5 \times 10^{-4}$ $m^3$ (500 ml) of ethanol. The mixture was heated in an oil bath at 100°C for 4 hours under reflux. Then, after cooling the reaction mixture, $3.5 \times 10^{-5}$ $m^3$ (35 ml) of concentrated hydrochloric acid and $5 \times 10^{-5}$ $cm^3$ (50 ml) of water were added to the reaction mixture, and the mixture was heated at 100°C for 2 hours under reflux. The white crystals which deposited after cooling the mixture were separated by filtration. The filtrate was concentrated under reduced pressure to obtain a further deposit of white crystals by filtering the condensed filtrate containing the crystals. After making the filtrate alkaline with the addition of an aqueous 30% solution of sodium hydroxide, the filtrate was extracted with chloroform. The chloroform-extract was washed with water and dried on anhydrous sodium sulphate. On distilling the solvent from the dried extract under reduced pressure, an oily matter yellow in colour was obtained in an amount of $6.2 \times 10^{-2}$ kg (62 g) corresponding to a yield of 95%. This was 2-(4-aminobutyl)-6-phenyl-3(2H)-pyridazinone. The hydrochloride of this compound was white powdery crystals melting at 206 to 207°C.

b) Synthesis of 2-(4-bromobutyl)-6-phenyl-3(2H)-pyridazinone:

$4.3 \times 10^{-2}$ kg (43 g, 0.25 mol) of 6-phenyl-3-(2H)-pyridazinone was dissolved in $5 \times 10^{-4}$ $m^3$ (500 ml) of ethyl methyl ketone. $1.62 \times 10^{-1}$ kg (162 g, 0.75 mol) of 1,4-dibromobutane and $3.5 \times 10^{-2}$ kg (35 g, 0.25 mol) of potassium carbonate were added to this solution. The mixture was heated at 100°C for 10 hours under reflux. After the matter that had deposited had been separated by filtration, the organic layer was distilled under reduced pressure, and the residue was extracted with chloroform. The extract was washed with water and dried over anhydrous sodium sulphate. The solvent was removed from the extract by distillation. The residue was further distilled under reduced pressure to obtain an oily material showing a boiling point of 222 to 225°C/$5.33 \times 10^2$ Pa (4 mmHg) pale yellow in colour in an amount of $5.5 \times 10^{-2}$ kg (55 g) corresponding to a yield of 72%. After leaving at room temperature, the oil turned into powdery crystals pale yellow in colour melting at 42 to 43°C.

c) Synthesis of 2-(4-aminobutyl)-6-phenyl-3(2H)-pyridazinone:

$4.5 \times 10^{-2}$ kg (45 g, 0.15 mol) of 2-(4-bromobutyl)-6-phenyl-3(2H)-pyridazinone was dissolved in $3 \times 10^{-5}$ $m^3$ (300 ml) of ethanol. $3 \times 10^{-5}$ $m^3$ (300 ml) of an aqueous 28% solution of ammonium hydroxide was added to the solution and the mixture was stirred at room temperature for 24 hours. The reaction mixture was then subjected to distillation under reduced pressure, and the residue was made acidic with the addition of an aqueous 10% solution of hydrochloric acid and extracted with chloroform to remove the unreacted starting material. The aqueous layer, after having been made alkaline with an aqueous 30% solution of sodium hydroxide, was extracted with benzene. The benzene layer was washed with water, dried over anhydrous sodium sulphate and distilled under reduced pressure to give an oily matter pale yellow in colour in an amount of $2.7 \times 10^{-2}$ kg (27 g) corresponding to a yield of 77%.

d) Synthesis of N-cyano-S-methyl-N′-[4-(3-oxo-6-phenyl-2-pyridazinyl)butyl] isothiourea (Compound No. 1):

$5 \times 10^{-5}$ $M^3$ (50 ml) of an ethanolic solution of $8.8 \times 10^{-3}$ kg (8.8 g, 0.06 mol) of 2-(4-aminobutyl)-6-phenyl-3(2H)-pyridazinone was slowly added dropwise over 30 minutes into $5 \times 10^{-5}$ $m^3$ (50 ml) of an ethanolic solution of $1.46 \times 10^{-2}$ kg (14.6 g, 0.06 mol) of dimethyl cyanodithioimidocarbonate while cooling in an ice bath. After stirring for 8 hours at room temperature, the mixture was condensed to one-half its volume under reduced pressure. The crystals that deposited were collected by filtration and recrystallised from ethanol to give pale yellow crystals melting at 171 to 172°C in an amount of $1.78 \times 10^{-2}$ kg (17.8 g) corresponding to a yield of 87%.

The physical properties of these crystals were:

Infrared absorption spectrum (hereinafter referred to as IR) (Nujol method) ("Nujol" is a Trade Mark)

$\nu_{max}^{Nujol}$ 3280 (NH), 2170 (CN) and 1670 (C=O),

its chart being shown in Figure 1 of the accompanying drawings. Nuclear magnetic resonance spectrum (hereinafter referred to as NMR) in DMSO-$d_6$) $\delta$:

1.7 (4H, multiplet, N—$CH_2$—$CH_2$—$CH_2$—$CH_2$—NH)
2.58 (3H, singlet, $SCH_3$)
3.38 (2H, triplet, J = 7 Hz, $CH_2NH$)
4.19 (2H, triplet, J = 7 Hz, CO—N—$CH_2$)
7.06 (1H, doublet, J = 10 Hz, $C_4$—H)
7.5 and 7.9 (5H, multiplet, aromatic H)
8.06 (1H, doublet, J = 10 Hz, $C_5$—H)
8.3 (1H, broad, NH)

Mass spectrum m/e: 341

Elementary analysis:

found: 59.81% of C, 5.89% of H and 20.46% of N
calcd. as $C_{17}H_{19}N_5OS$: 59.79% of C, 5.62% of H and 20.51% of N.

## Example 2

The following compounds of the present invention were synthesized while using the respective corresponding derivatives of 3(2H)-pyridazinone:

Compound No. 2: N-cyano-S-methyl-N'-[2-(3-oxo-6-phenyl-2-pyridazinyl) ethyl] isothiourea, m.pt. 155—156°C.

Compound No. 3: N-cyano-S-methyl-N'-[3-(3-oxo-6-phenyl-2-pyridazinyl) propyl] isothiourea, m.pt. 159—160°C.

Compound No. 4: N-cyano-S-methyl-N'-[5-(3-oxo-6-phenyl-2-pyridazinyl) pentyl] isothiourea, m.pt. 103—105°C.

Compound No. 5: N-cyano-S-methyl-N'-[6-(3-oxo-6-phenyl-2-pyridazinyl) hexyl] isothiourea, m.pt. 117—118°C.

Compound No. 6: N-cyano-S-methyl-N'-[3-{6-(4-chlorophenyl)-3-oxo-2-pyridazinyl} propyl] isothiourea, m.pt. 153—154°C.

Compound No. 7: N-cyano-S-methyl-N'-[4-{6-(4-chlorophenyl)-3-oxo-2-pyridazinyl} butyl] isothiourea, m.pt. 80—82°C.

Compound No. 8: N-cyano-S-methyl-N'-[5-{6-(4-chlorophenyl)-3-oxo-2-pyridazinyl} pentyl] isothiourea, m.pt. 124—125.

Compound No. 9: N-cyano-S-methyl-N'-[3-{6-(4-methylphenyl)-3-oxo-2-pyridazinyl} propyl] isothiourea, m.pt. 156—159°C.

Compound No. 10: N-cyano-S-methyl-N'-[4-{6-(4-methylphenyl)-3-oxo-2-pyridazinyl} butyl] isothiourea, m.pt. 171—174°C.

Compound No. 11: N-cyano-S-methyl-N'-[5-{6-(4-methylphenyl)-3-oxo-2-pyridazinyl} pentyl] isothiourea, m.pt. 146—149°C.

Compound No. 12: N-cyano-S-methyl-N'-[4-{6-(2,4-dimethylphenyl)-3-oxo-2-pyridazinyl} butyl] isothiourea, m.pt. 129—131°C.

Compound No. 13: N-cyano-S-methyl-N'-[3-{6-(4-methoxyphenyl)-3-oxo-2-pyridazinyl} propyl] isothiourea, m.pt. 97—99°C.

Compound No. 14: N-cyano-S-methyl-N'-[3-{6-(3,4-dimethoxyphenyl)-3-oxo-2-pyridazinyl} propyl] isothiourea, m.pt. 149—151°C.

Compound No. 15: N-cyano-S-methyl-N'-[4-{6-(3,4-dimethoxyphenyl)-3-oxo-2-pyridazinyl} butyl] isothiourea, m.pt. 146—147°C.

Compound No. 16: N-cyano-S-methyl-N'-[5-{6-(3,4-dimethoxyphenyl)-3-oxo-2-pyridazinyl} pentyl] isothiourea, m.pt. 129—132°C.

Compound No. 17: N-cyano-S-methyl-N'-[4-{3-oxo-6-(3,4,5-trimethoxyphenyl)-2-pyridazinyl}butyl] isothiourea, m.pt. 132—135°C.

Compound No. 18: N-cyano-S-methyl-N'-[3-(4-methyl-3-oxo-6-phenyl-2-pyridazinyl) propyl] isothiourea, m.pt. 177—179°C.

Compound No. 19: N-cyano-S-methyl-N'-[4-(4-methyl-3-oxo-6-phenyl-2-pyridazinyl) butyl] isothiourea, m.pt. 146—148°C.

Compound No. 20: N-cyano-S-methyl-N'-[5-(4-methyl-3-oxo-6-phenyl-2-pyridazinyl) pentyl] isothiourea, m.pt. 102—103°C.

Compound No. 21: N-cyano-S-methyl-N'-[3-(5-methyl-3-oxo-6-phenyl-2-pyridazinyl) propyl] isothiourea, m.pt. 171—173°C.

Compound No. 22: N-cyano-S-methyl-N'-[4-(5-methyl-3-oxo-6-phenyl-2-pyridazinyl) butyl] isothiourea, m.pt. 140—143°C.

Compound No. 23: N-cyano-S-methyl-N'-[5-(5-methyl-3-oxo-6-phenyl-2-pyridazinyl) pentyl] isothiourea, m.pt. 129—131°C.

IR of the Compound Nos. 2 to 23 are shown in Figures 2 to 23 of the accompanying drawings, respectively.

Example 3

(a) Synthesis of 2-(4-aminobutyl)-4,5-dihydro-6-phenyl-3(2H)-pyridazinone:

$4.5 \times 10^{-2}$ kg (45 g, 0.12 mol) of 4,5-dihydro-6-phenyl-2-(4-phthalimidobutyl)-3(2H)-pyridazinone was suspended in $3 \times 10^{-5}$ $m^3$ (300 ml) of ethanol. $8 \times 10^{-3}$ kg (8 g, 0.16 mol) of hydrazine hydrate was added to the suspension. This mixture was heated on an oil bath at 100°C for 3 hours under reflux. After cooling, $3 \times 10^{-5}$ $m^3$ (30 ml) of concentrated hydrochloric acid and $5 \times 10^{-5}$ $m^3$ (50 ml) of water were added to the reaction mixture, and the mixture was further heated at 100°C for 2 hours. After cooling, the white crystals that had deposited were removed by filtration. The filtrate was concentrated under reduced pressure and the white crystals that deposited were removed by filtration. After making the filtrate alkaline with the addition of an aqueous 30% solution of sodium hydroxide, the filtrate was extracted with chloroform. The extract was washed with water, dried over anhydrous sodium sulphate and subjected to distillation under reduced pressure to obtain the objective compound as a pale yellow oily substance in an amount of $2 \times 10^{-2}$ kg (20 g) corresponding to a yield of 68%.

(b) Synthesis of N-cyano-S-methyl-N'-[4-(4,5-dihydro-3-oxo-phenyl-2-pyridazinyl) butyl] isothiourea, Compound No. 24:

$1.84 \times 10^{-2}$ kg (18.4 g, 0.075 mol) of the 2-(4-aminobutyl)-4,5-dihydro-6-phenyl-3(2H)-pyridazinone obtained in (a) above was dissolved in $1.5 \times 10^{-5}$ $m^3$ (15 ml) of ethanol. This solution was slowly added dropwise into $1.5 \times 10^{-5}$ $m^3$ (15 ml) of an ethanolic solution containing $1.09 \times 10^{-2}$ kg (10.9 g, 0.0075 mol) of dimethyl cyanodithioimidocarbonate in an ice bath, which was cooled in iced water. Crystals slowly appeared in the mixture while the mixture was stirred at room temperature for 24 hours as it was. The reaction mixture was concentrated thereafter, and the crystals that had deposited were collected by filtration. Recrystallisation from acetonitrile gave pale yellow crystals melting at 133 to 134°C in an amount of $2.1 \times 10^{-2}$ kg (21 g) corresponding to 82% of the theoretical yield.

The physical properties of this compound were as follows:

IR (Nujol method): shown in Figure 24 of the accompanying drawings,
$\nu_{max}^{Nujol}$ 3300 (NH), 2160 (CN) and 1650 (C=O).
NMR (in DMSO-$d_6$):
1.6 (4H, multiplet, N—$CH_2$—$CH_2CH_2$—$CH_2$—NH)
2.50 (2H, triplet, J = 8Hz, $C_4$—H)
2.54 (3H, singlet, S—$CH_3$)
2.96 (2H, triplet, J = 8Hz, $C_5$—H)
3.32 (2H, triplet, J = 7Hz, $CH_2$—NH)
3.76 (2H, triplet, J = 7Hz, CO—N—$CH_2$)
7.4 (3H, multiplet, aromatic H)
7.7 (2H, multiplet, aromatic H)
8.2 (1H, broad, NH)
Mass spectrum m/e: 343
Elementary analysis:
found: 59.56% of C, 6.03% of H and 20.35% of N
calcd. as $C_{17}H_{21}N_5OS$: 59.47% of C, 6.12% of H and 20.41% of N.

Example 4

The following compounds of the present invention were synthesized while using the respective corresponding derivatives of 4,5-dihydro-3(2H)-pyridazinone in accordance with Example 3:

Compound No. 25: N-cyano-S-methyl-N'-[3-(4,5-dihydro-3-oxo-6-phenyl-2-pyridazinyl) propyl] isothiourea, m.pt. 117—120°C.

Compound No. 26: N-cyano-S-methyl-N'-[5-(4,5-dihydro-3-oxo-6-phenyl-2-pyridazinyl) pentyl] isothiourea, m.pt. 137—138°C.

Compound No. 27: N-cyano-S-methyl-N'-[3-{6-(4-chlorophenyl)-4,5-dihydro-3-oxo-2-pyridazinyl} propyl] isothiourea, m.pt. 156—157°C.

Compound No. 28: N-cyano-S-methyl-N'-[4-{6-(4-chlorophenyl)-4,5-dihydro-3-oxo-2-pyridazinyl} butyl] isothiourea, m.pt. 153—155°C.

Compound No. 29: N-cyano-S-methyl-N'-[5-{6-(4-chlorophenyl)-4,5-dihydro-3-oxo-2-pyridazinyl} pentyl] isothiourea, m.pt. 122—124°C.

Compound No. 30: N-cyano-S-methyl-N'-[3-{6-(3,4-dichlorophenyl)-4,5-dihydro-3-oxo-2-pyridazinyl} propyl] isothiourea, m.pt. 113—114°C.

Compound No. 31: N-cyano-S-methyl-N'-[4-{6-(3,4-dichlorophenyl)-4,5-dihydro-3-oxo-2-pyridazinyl} butyl] isothiourea, m.pt. 135—136°C.

Compound No. 32: N-cyano-S-methyl-N'-[5-{6-(3,4-dichlorophenyl)-4,5-dihydro-3-oxo-2-pyridazinyl}pentyl] isothiourea, m.pt. 132—134°C.

Compound No. 33: N-cyano-S-methyl-N'-[3-{4,5-dihydro-6-(4-methoxyphenyl)-3-oxo-2-pyridazinyl} propyl] isothiourea, m.pt. 168—169°C.

Compound No. 34: N-cyano-S-methyl-N'-[4-{4,5-dihydro-6-(4-methoxyphenyl)-3-oxo-2-pyridazinyl} butyl] isothiourea, m.pt. 157—158°C.

Compound No. 35: N-cyano-S-methyl-N'-[5-{4,5-dihydro-6-(4-methoxyphenyl-3-oxo-2-pyridazinyl}pentyl] isothiourea, m.pt. 117—118°C.

Compound No. 36: N-cyano-S-methyl-N'-[4-{4,5-dihydro-6-(3,4-dimethoxyphenyl)-3-oxo-2-pyridazinyl} butyl] isothiourea, m.pt. 141—143°C.

Compound No. 37: N-cyano-S-methyl-N'-[4-{4,5-dihydro-3-oxo-6-(3,4,5-trimethoxyphenyl)-2-pyridazinyl} butyl] isothiourea, m.pt. 140—141°C.

Compound No. 38: N-cyano-S-methyl-N'-[3-{4,5-dihydro-6-(4-methylphenyl)-3-oxo-2-pyridazinyl} propyl] isothiourea, m.pt. 145—147°C.

Compound No. 39: N-cyano-S-methyl-N'-[4-{4,5-dihydro-6-(4-methylphenyl)-3-oxo-2-pyridazinyl} butyl] isothiourea, m.pt. 154—156°C.

Compound No. 40: N-cyano-S-methyl-N'-[5-{4,5-dihydro-6-(4-methylphenyl)-3-oxo-2-pyridazinyl} pentyl] isothiourea, m.pt. 144—146°C.

Compound No. 41: N-cyano-S-methyl-N'-[4-{4,5-dihydro-6-(2,4-dimethylphenyl)-3-oxo-2-pyridazinyl} butyl] isothiourea, m.pt. 129—131°C.

Compound No. 42: N-cyano-S-methyl-N'-[4-(4,5-dihydro-4-methyl-3-oxo-6-phenyl-2-pyridazinyl) butyl] isothiourea, m.pt. 113—115°C.

Compound No. 43: N-cyano-S-methyl-N'-[4-(4,5-dihydro-5-methyl-3-oxo-6-phenyl-2-pyridazinyl) butyl] isothiourea, m.pt. 98—99°C.

IR of Compound Nos. 25 to 43 are shown in Figures 25 to 43 respectively of the accompanying drawings.

Example 5

Synthesis of N-cyano-S-methyl-N'-[4-(3-oxo-6-phenyl-2-pyridazinyl) butyl]-N"-methylguanidine, Compound No. 44 of the present invention:

$5 \times 10^{-1}$ kg (500 g) of a methanolic 30% solution of methylamine was added to $1.30 \times 10^{-1}$ kg (130 g, 0.53 mol) of N-cyano-S-methyl-N'-[4-(3-oxo-6-phenyl-2-pyridazinyl) butyl] isothiourea. This mixture was heated at around 60°C for 8 hours. After distilling off the solvent, the residue obtained was recrystallised from a mixture of water and ethanol to obtain colourless crystals melting at 184 to 185°C in an amount of $8.8 \times 10^{-2}$ kg (88 g) corresponding to a yield of 72%.

The physical properties of the product, N-cyano-N'-[4-(3-oxo-6-phenyl-2-pyridazinyl) butyl-N''-methylguanidine, were as follows:

IR shown in Figure 44 of the accompanying drawings:

$\nu_{max}^{Nujol}$ 3330,3330 and 3300 (NH), 2160 (CN) and 1660 (C=O).

NMR (in DMSO-$d_6$) $\delta$:

1.7 (4H, multiplet, N—$CH_2$—$CH_2CH_2$—$CH_2$—NH)

2.74 (3H, singlet, NH—$CH_3$)

3.22 (2H, triplet, J = 7Hz, $CH_2$—NH)

4.20 (2H, triplet, J = 7Hz, CO—N—$CH_2$)

7.0 (2H, broad, NH × 2)

7.08 (1H, doublet, J = 10Hz, $C_4$—H)

7.5 (3H, multiplet, aromatic H)

7.9 (2H, multiplet, aromatic H)

8.05 (1H, doublet, J = 10Hz, $C_5$—H)

Mass spectrum m/e: 324

Elementary analysis:

found: 62.76% of C, 6.28% of H and 25.88% of N.

calcd. as $C_{17}H_{20}N_6O$: 62.95% of C, 6.21% of H and 25.91% of N.

Example 6

The following compounds of the present invention were synthesised similarly to and in

accordance with Example 5 while using the respective corresponding derivative of isothiourea:

Compound No. 45: N-cyano-N'-[2-(3-oxo-6-phenyl-2-pyridazinyl)ethyl]-N"-methylguanidine, m.pt. 228—229°C.

Compound No. 46: N-cyano-N'-[2-(3-oxo-6-phenyl-2-pyridazinyl)ethyl]-N"-ethylguanidine, m.pt. 199—200°C.

Compound No. 47: N-cyano-N'-[3-(3-oxo-6-phenyl-2-pyridazinyl)propyl]-N"-methylguanidine, m.pt. 185—186°C.

Compound No. 48: N-cyano-N'-[3-(3-oxo-6-phenyl-2-pyridazinyl)propyl]-N"-ethylguanidine, m.pt. 156—158°C.

Compound No. 49: N-cyano-N'-[3-(3-oxo-6-phenyl-2-pyridazinyl)propyl]-N"-propylguanidine, m.pt. 136—137°C.

Compound No. 50: N-cyano-N'-[4-(3-oxo-6-phenyl-2-pyridazinyl)butyl]-N"-ethylguanidine, m.pt. 163—166°C.

Compound No. 51: N-cyano-N'-[4-(3-oxo-6-phenyl-2-pyridazinylbutyl]-N"-propylguanidine, m.pt. 122—123°C.

Compound No. 52: N-cyano-N'-[5-(3-oxo-6-phenyl-2-pyridazinyl)pentyl]-N"-methylguanidine, m.pt. 148—149°C.

Compound No. 53: N-cyano-N'-[5-(3-oxo-6-phenyl-2-pyridazinyl)pentyl]-N"-ethylguanidine, m.pt. 145—148°C.

Compound No. 54: N-cyano-N'-[5-(3-oxo-6-phenyl-2-pyridazinyl)pentyl]-N"-propylguanidine, m.pt. 126—127°C.

Compound No. 55: N-cyano-N'-[6-(3-oxo-6-phenyl-2-pyridazinyl)hexyl]-N"-methylguanidine, m.pt. 73—74°C.

Compound No. 56: N-cyano-N'-[6-(3-oxo-6-phenyl-2-pyridazinyl)hexyl]-N"-ethylguanidine, m.pt. 76—78°C.

Compound No. 57: N-cyano-N'-[3-{6-(4-chlorophenyl)-3-oxo-2-pyridazinyl} propyl]-N"-methylguanidine, m.pt. 220—221°C.

Compound No. 58: N-cyano-N'-[3-{6-(4-chlorophenyl)-3-oxo-2-pyridazinyl} propyl]-N"-ethylguanidine, m.pt. 169—170°C.

Compound No. 59: N-cyano-N'-[4-{6-(4-chlorophenyl)-3-oxo-2-pyridazinyl} butyl]-N"-methylguanidine, m.pt. 208—209°C.

Compound No. 60: N-cyano-N'-[4-{6-(4-chlorophenyl)-3-oxo-2-pyridazinyl} butyl]-N"-ethylguanidine, m.pt. 170—171°C.

Compound No. 61: N-cyano-N'-[5-{6-(4-chlorophenyl)-3-oxo-2-pyridazinyl} pentyl]-N"-methylguanidine, m.pt. 155—157°C.

Compound No. 62: N-cyano-N'-[5-{6-(4-chlorophenyl)-3-oxo-2-pyridazinyl} pentyl]-N"-ethylguanidine, m.pt. 95—97°C.

Compound No. 63: N-cyano-N'-[3-{6-(4-methylphenyl)-3-oxo-2-pyridazinyl} propyl]-N"-methylguanidine, m.pt. 223—225°C.

Compound No. 64: N-cyano-N'-[3-{6-(4-methylphenyl)-3-oxo-2-pyridazinyl} propyl]-N"-ethylguanidine, m.pt. 184—185°C.

Compound No. 65: N-cyano-N'-[4-{6-(4-methylphenyl)-3-oxo-2-pyridazinyl} butyl]-N"-methylguanidine, m.pt. 169—170°C.

Compound No. 66: N-cyano-N'-[4-{6-(4-methylphenyl)-3-oxo-2-pyridazinyl} butyl]-N"-ethylguanidine, m.pt. 136—139°C.

Compound No. 67: N-cyano-N'-[5-{6-(4-methylphenyl)-3-oxo-2-pyridazinyl} pentyl]-N"-methylguanidine, m.pt. 151—153°C.

Compound No. 68: N-cyano-N'-[5-{6-(4-methylphenyl)-3-oxo-2-pyridazinyl} pentyl]-N"-ethylguanidine, m.pt. 73—75°C.

Compound No. 69: N-cyano-N'-[3-{6-(4-methoxyphenyl)-3-oxo-2-pyridazinyl} propyl]-N"-methylguanidine, m.pt. 103—105°C.

Compound No. 70: N-cyano-N'-[3-{6-(4-methoxyphenyl)-3-oxo-2-pyridazinyl} propyl]-N"-ethylguanidine, m.pt. 95—96°C.

Compound No. 71: N-cyano-N'-[4-{6-(2,4-dimethylphenyl)-3-oxo-2-pyridazinyl} butyl]-N"-methylguanidine, m.pt. 126—128°C.

Compound No. 72: N-cyano-N'-[4-{6-(2,4-dimethylphenyl)-3-oxo-2-pyridazinyl} butyl]-N"-ethylguanidine, m.pt. 175—176°C.

Compound No. 73: N-cyano-N'-[4-{6-(3,4-dimethoxyphenyl)-3-oxo-2-pyridazinyl} butyl]-N"-methylguanidine, m.pt. 181—182°C.

Compound No. 74: N-cyano-N'-[4-{6-(3,4-dimethoxyphenyl-3-oxo-2-pyridazinyl} butyl-N"-ethylguanidine, m.pt. 173—174°C.

Compound No. 75: N-cyano-N'-[5-{6-(3,4-dimethoxyphenyl)-3-oxo-2-pyridazinyl} pentyl]-N"-methylguanidine, m.pt. 150—151°C.

Compound No. 76: N-cyano-N'-[5-{6-(3,4-dimethoxyphenyl)-3-oxo-2-pyridazinyl} pentyl]-N''-ethylguanidine, m.pt. 136—139°C.

Compound No. 77: N-cyano-N'-[4-{3-oxo-6-(3,4,5-trimethoxyphenyl)-2-pyridazinyl} butyl]-N''-methylguanidine, m.pt. 125—128°C.

Compound No. 78: N-cyano-N'-[4-(3-oxo-6-phenyl-2-pyridazinyl) butyl]-N''-guanidine, m.pt. 145—147°C.

Compound No. 79: N-cyano-N'-[3-(4-methyl-3-oxo-6-phenyl-2-pyridazinyl) propyl]-N''-methylguanidine, m.pt. 193—195°C.

Compound No. 80: N-cyano-N'-[3-(4-methyl-3-oxo-6-phenyl-2-pyridazinyl) propyl]-N''-ethylguanidine, m.pt. 165—166°C.

Compound No. 81: N-cyano-N'-[4-(4-methyl-3-oxo-6-phenyl-2-pyridazinyl) butyl]-N''-methylguanidine, m.pt. 158—159°C.

Compound No. 82: N-cyano-N'-[4-(4-methyl-3-oxo-6-phenyl-2-pyridazinyl) butyl]-N''-ethylguanidine, m.pt. 128—129°C.

Compound No. 83: N-cyano-N'-[5-(4-methyl-3-oxo-6-phenyl-2-pyridazinyl) pentyl]-N''-methylguanidine, m.pt. 138—140°C.

Compound No. 84: N-cyano-N'-[5-(4-methyl-3-oxo-6-phenyl-2-pyridazinyl) pentyl]-N''-ethylguanidine, m.pt. 125—126°C.

Compound No. 85: N-cyano-N'-[3-(5-methyl-3-oxo-6-phenyl-2-pyridazinyl) propyl]-N''-methylguanidine, m.pt. 207—208°C.

Compound No. 86: N-cyano-N'-[3-(5-methyl-3-oxo-6-phenyl-2-pyridazinyl) propyl]-N''-ethylguanidine, m.pt. 192—193°C.

Compound No. 87: N-cyano-N'-[4-(5-methyl-3-oxo-6-phenyl-2-pyridazinyl) butyl]-N''-methylguanidine, m.pt. 168—170°C.

Compound No. 88: N-cyano-N'-[4-(5-methyl-3-oxo-6-phenyl-2-pyridazinyl) butyl]-N''-ethylguanidine, m.pt. 165—167°C.

Compound No. 89: N-cyano-N'-[5-(5-methyl-3-oxo-6-phenyl-2-pyridazinyl) pentyl]-N''-methylguanidine, m.pt. 106—107°C.)

Compound No. 90: N-cyano-N'-[5-(5-methyl-3-oxo-6-phenyl-2-pyridazinyl) pentyl]-N''-ethylguanidine, m.pt. 138—139°C.

The respective infrared absorption spectra of Compounds Nos. 45 to 90 are shown in Figures 45 to 90 respectively of the accompanying drawings.

## Example 7

Synthesis of N-cyano-S—N'-[4,5-dihydro-3-oxo-6-phenyl-2-pyridazinyl) butyl]-N''-methylguanidine, Compound No. 91:

$4 \times 10^{-5}$ $m^3$ (40 ml) of a 30% methanolic solution of methylamine was added to $2 \times 10^{-3}$ kg (2 g, 0.006 mol) of N-cyano-S-methyl-N'-[4-(4,5-dihydro-3-oxo-6-phenyl-2-pyridazinyl) butyl] isothiourea. This mixture was heated at a reflux temperature for 8 hours. The residue obtained by distilling off the solvent from the reaction mixture was recrystallised from a mixture of ethanol and isopropyl ether to give colourless crystals melting at 187 to 189°C in an amount of $1.8 \times 10^{-3}$ kg (1.8 g) corresponding to a yield of 95%.

The physical properties of the thus obtained Compound No. 91 were as follows:

IR (Nujol method) shown in Figure 91 of the accompanying drawings:
$\nu_{max}^{Nujol}$ 3290 (NH), 2150 (CN) and 1655 (C=O).

NMR (in DMSO-$d_6$) $\delta$:
1.6 (4H, multiplet, N—$CH_2$—$\mathit{CH_2CH_2}$—$CH_2$—NH
2.50 (2H, triplet, J = 8Hz, $C_4$—H)
2.68 (3H, doublet, J = 4Hz, NH—$\mathit{CH_3}$)
2.96 (2H, triplet, J = 8Hz, $C_5$—H)
3.2 (2H, multiplet, $CH_2$—$\mathit{CH_2}$NH)
3.78 (2H, triplet, J = 7Hz, CO—N—$\mathit{CH_2}$)
6.6 (2H, broad, NH × 2)
7.4 (3H, multiplet, aromatic H)
7.8 (2H, multiplet, aromatic H)

Mass spectrum m/e: 326

Elementary analysis:
found: 62.43% of C, 6.88% of H and 25.97% of N.
calcd. as $C_{17}H_{22}N_6O$: 62.58% of C, 6.75% of H and 25.77% of N.

## Example 8

The following compounds of the present invention were synthesised in accordance with Example 7 while using the respective corresponding derivative of isothiourea:

Compound No. 92: N-cyano-N'-[4-(4,5-dihydro-3-oxo-6-phenyl-2-pyridazinyl) butyl]-N"-ethylguanidine, m.pt. 98—101°C.

Compound No. 93: N-cyano-N'-[5-(4,5-dihydro-3-oxo-6-phenyl-2-pyridazinyl) pentyl]-N"-methylguanidine, m.pt. 137—139°C.

Compound No. 94: N-cyano-N'-[5-(4,5-dihydro-3-oxo-6-phenyl-2-pyridazinyl) pentyl]-N"-ethylguanidine, m.pt. 130—132°C.

Compound No. 95: N-cyano-N'-[3-{6-(4-chlorophenyl)-4,5-dihydro-3-oxo-2-pyridazinyl} propyl]-N"-methylguanidine, m.pt. 187—188°C.

Compound No. 96: N-cyano-N'-[3-{6-(4-chlorophenyl)-4,5-dihydro-3-oxo-2-pyridazinyl} propyl]-N"-ethylguanidine, m.pt. 146—148°C.

Compound No. 97: N-cyano-N'-[4-{6-(4-chlorophenyl)-4,5-dihydro-3-oxo-2-pyridazinyl} butyl]-N"-methylguanidine, m.pt. 174—175°C.

Compound No. 98: N-cyano-N'-[4-{6-(4-chlorophenyl)-4,5-dihydro-3-oxo-2-pyridazinyl} butyl]-N"-ethylguanidine, m.pt. 138—139°C.

Compound No. 99: N-cyano-N'-[5-{6-(4-chlorophenyl)-4,5-dihydro-3-oxo-2-pyridazinyl} pentyl]-N"-methylguanidine, m.pt. 131—133°C.

Compound No. 100: N-cyano-N'-[5-{6-(4-chlorophenyl)-4,5-dihydro-3-oxo-2-pyridazinyl} pentyl]-N"-ethylguanidine, m.pt. 91—93°C.

Compound No. 101: N-cyano-N'-[3-{6-(3,4-dichlorophenyl)-4,5-dihydro-3-oxo-2-pyridazinyl} propyl]-N"-methylguanidine, m.pt. 201—203°C.

Compound No. 102: N-cyano-N'-[3-{6-(3,4-dichlorophenyl)-4,5-dihydro-3-oxo-2-pyridazinyl} propyl]-N"-ethylguanidine, m.pt. 157—158°C.

Compound No. 103: N-cyano-N'-[4-{6-(3,4-dichlorophenyl)-4,5-dihydro-3-oxo-2-pyridazinyl} butyl]-N"-methylguanidine, m.pt. 202—204°C.

Compound No. 104: N-cyano-N'-[4-{6-(3,4-dichlorophenyl)-4,5-dihydro-3-oxo-2-pyridazinyl} butyl]-N"-ethylguanidine, m.pt. 150—151°C.

Compound No. 105: N-cyano-N'-[5-{6-(3,4-dichlorophenyl)-4,5-dihydro-3-oxo-2-pyridazinyl} pentyl]-N"-methylguanidine, m.pt. 128—130°C.

Compound No. 106: N-cyano-N'-[5-{6-(3,4-dichlorophenyl)-4,5-dihydro-3-oxo-2-pyridazinyl} pentyl]-N"-ethylguanidine, m.pt. 82—85°C.

Compound No. 107: N-cyano-N'-[3-{4,5-dihydro-6-(4-methylphenyl)-3-oxo-2-pyridazinyl} propyl]-N"-methylguanidine, m.pt. 182—184°C.

Compound No. 108: N-cyano-N'-[3-{4,5dihydro-6-(4-methylphenyl)-3-oxo-2-pyridazinyl} propyl]-N"-ethylguanidine, m.pt. 152—155°C.

Compound No. 109: N-cyano-N'-[4-{4,5-dihydro-6-(4-methylphenyl)-3-oxo-2-pyridazinyl} butyl]-N"-methylguanidine, m.pt. 176—177°C.

Compound No. 110: N-cyano-N'-[4-{4,5-dihydro-6-(4-methylphenyl)-3-oxo-2-pyridazinyl} butyl]-N"-ethylguanidine, m.pt. 137—138°C.

Compound No. 111: N-cyano-N'-[5-{4,5-dihydro-6-(4-methylphenyl)-3-oxo-2-pyridazinyl} pentyl]-N"-methylguanidine, m.pt. 170—172°C.

Compound No. 112: N-cyano-N'-[5-{4,5-dihydro-6-(4-methylphenyl)-3-oxo-2-pyridazinyl} pentyl]-N"-ethylguanidine, m.pt. 125—128°C.

Compound No. 113: N-cyano-N'-[4-{4,5-dihydro-6-(2,4-dimethylphenyl)-3-oxo-2-pyridazinyl} butyl]-N"-methylguanidine, m.pt. 135—137°C.

Compound No. 114: N-cyano-N'-[4-{4,5-dihydro-6-(2,4-dimethylphenyl)-3-oxo-2-pyridazinyl} butyl]-N''-ethylguanidine, m.pt. 129—130°C.

Compound No. 115: N-cyano-N'-[3-{4,5-dihydro-6-(4-methoxyphenyl)-3-oxo-2-pyridazinyl} propyl]-N"-methylguanidine, m.pt. 142—144°C.

Compound No. 116: N-cyano-N'-[3-{4,5-dihydro-6-(4-methoxyphenyl)-3-oxo-2-pyridazinyl} propyl]-N"-ethylguanidine, m.pt. 105—106°C.

Compound No. 117: N-cyano-N'-[4-{4,5-dihydro-6-(4-methoxyphenyl)-3-oxo-2-pyridazinyl} butyl]-N"-methylguanidine, m.pt. 173—174°C.

Compound No. 118: N-cyano-N'-[4-{4,5-dihydro-6-(4-methoxyphenyl)-3-oxo-2-pyridazinyl} butyl]-N"-ethylguanidine, m.pt. 139—140°C.

Compound No. 119: N-cyano-N'-[4-{4,5-dihydro-6-(3,4-dimethoxyphenyl)-3-oxo-2-pyridazinyl} butyl]-N"-methylguanidine, m.pt. 131—134°C.

Compound No. 120: N-cyano-N'-[4-{4,5-dihydro-6-(3,4-dimethoxyphenyl)-3-oxo-2-pyridazinyl} butyl]-N"-ethylguanidine, m.pt. 148—151°C.

Compound No. 121: N-cyano-N'-[4-{4,5-dihydro-3-oxo-6-(3,4,5-trimethoxyphenyl)-2-pyridazinyl} butyl]-N"-methylguanidine, m.pt. 123—125°C.

Compound No. 122: N-cyano-N'-[4-{4,5-dihydro-4-methyl-3-oxo-6-phenyl-2-pyridazinyl} butyl]-N''-methylguanidine, m.pt. 134—135°C.

Compound No. 123: N-cyano-N'-[4-{4,5-dihydro-4-methyl-3-oxo-6-phenyl-2-pyridazinyl} butyl]-N"-ethylguanidine, m.pt. 130—132°C.

Compound No. 124: N-cyano-N'-[4-{4,5-dihydro-5-methyl-3-oxo-6-phenyl-2-pyridazinyl} butyl]-N″-methylguanidine, m.pt. 108—109°C.

Compound No. 125: N-cyano-N′-[4-{4,5-dihydro-5-methyl-3-oxo-6-phenyl-2-pyridazinyl} butyl]-N″-ethylguanidine, m.pt. 125—126°C.

IR of Compounds Nos. 92 to 125 are shown in Figures 92 to 125 respectively of the accompanying drawings.

Example 9

Synthesis of N-cyano-N′-[2-(3-oxo-6-phenyl-2-pyridazinyl)ethyl]-N″-propargylguanidine, Compound No. 126:

$2.2 \times 10^{-3}$ Kg (2.2 g, 0.01 mol) of 2-(2-aminoethyl)-6-phenyl-3(2H)-pyridazinone and $1.5 \times 10^{-3}$ Kg (1.5 g, 0.01 mol) of N-cyano-S-methyl-N′-propargylisothiourea were dissolved in $2 \times 10^{-5}$ $m^3$ (20 ml) of acetonitrile. This solution was heated at a reflux temperature for 24 hours. After treating the reaction mixture, while hot, with active charcoal, the solvent was distilled off. The residue was recrystallized from acetonitrile to obtain $2.0 \times 10^{-3}$ Kg (2.0 g) of pale yellow crystals melting at 192 to 194°C, corresponding to a yield of 63% of product.

The physical properties of the product, Compound No. 126 were:

IR (Nujol method) shown in Fig. 126 of the accompanying drawings:
$\nu_{max}^{Nujol}$ 3300 and 3220 (NH), 2160 (CN) and 1650 (C=O).
NMR (in DMSO-$d_6$) $\delta$:
3.04 (1H, singlet, C ≡ CH)
3.64 (2H, triplet, J = 7Hz, $CH_2$—NH
3.91 (2H, singlet, NH—$CH_2$—C ≡ CH)
4.28 (2H, triplet, J = 7Hz, CO—N—$CH_2$)
7.00 (1H, doublet, J = 10Hz, $C_4$—H)
7.0 (1H, broad, NH)
7.4 (1H, broad, NH)
7.4 (3H, multiplet, aromatic H)
7.9 (2H, multiplet, aromatic H)
7.94 (1H, doublet, J = 10Hz, $C_5$—H
Mass spectrum m/e: 320
Elementary analysis:
found: 63.66% of C, 5.32% of H and 26.35% of N
calcd. as $C_{19}H_{22}N_6O$: 63.78% of C, 5.08% of H and 26.25% of N.

Example 10

The following compounds of the present invention were synthesized in accordance with Example 9 while using the respective corresponding derivatives of 3(2H)-pyridazinone.

Compound No. 127: N-cyano-N′-[3-(3-oxo-6-phenyl-2-pyridazinyl)propyl]-N″-propargylguanidine, m.pt. 180—182°C.

Compound No. 128: N-cyano-N′-[4-(3-oxo-6-phenyl-2-pyridazinyl)butyl]-N″-propargylguanidine, m.pt. 137—138°C.

Compound No. 129: N-cyano-N′-[4-(4,5-dihydro-3-oxo-6-phenyl-2-pyridazinyl)butyl]-N″-propargylguanidine, m.pt. 143—144°C.

IR of Compounds Nos. 127 to 129 are respectively shown in Figs. 127 to 129 of the accompanying drawings.

Example 11

(a) Synthesis of 2-(2-aminoethoxymethyl)-6-phenyl-3(2H)-pyridazinone:

A mixture of $1.3 \times 10^{-2}$ kg (13 g, 0.05 mol) of 2-bromomethyl-6-phenyl-3(2H)-pyridazinone and $10^{-4}$ $m^3$ (100 ml) of 2-bromoethanol was heated on a water bath at 100°C for 24 hours. After the reaction was over, excess 2-bromoethanol was distilled off under reduced pressure. An aqueous saturated solution of sodium hydrogen carbonate and chloroform were added to the residue and the organic layer was dried to solids under reduced pressure. $3 \times 10^{-4}$ $M^3$ (300 ml) of an aqueous 28% solution of ammonium hydroxide was added to the residue. This mixture was stirred at room temperature for 24 hours. The mixture was subjected to distillation under reduced pressure, and aqueous 10% solution of hydrochloric acid was added to make the residue acidic and extracted with chloroform. The aqueous layer was made alkaline with an aqueous 30% solution of sodium hydroxide and extracted with benzene, washed with water and dried over anhydrous sodium sulphate. The dried extract was condensed under reduced pressure to obtain an oily product pale yellow in colour in an amount of $6 \times 10^{-3}$ kg (6 g) corresponding to a yield of 49%.

(b) Synthesis of 2-(2-aminoethoxymethyl)-6-phenyl-3(2H)-pyridazinone by another route:

$3 \times 10^{-3}$ kg (3 g, 0.05 mol) of ethanolamine and $1.2 \times 10^{-3}$ kg (1.2 g, 0.05 mol) of metallic sodium were added to $5.0 \times 10^{-5}$ $m^3$ (50 ml) of toluene. This mixture was heated at a reflux temperature for 5 hours until the metallic sodium disappeared. A solution of $1.1 \times 10^{-2}$ kg (11 g, 0.05 mol) of 2-chloro-methyl-6-phenyl-3(2H)-pyridazinone dissolved in $2 \times 10^{-5}$ $m^3$ (20 ml) of toluene was slowly added dropwise into the above-mentioned solution. Refluxing was continued for 2 hours. The reaction mixture was cooled to room temperature. After removing the sodium chloride that had deposited by filtration, the solid obtained was washed with $5 \times 10^{-5}$ $m^3$ (50 ml) of toluene. A mixture of the filtrate and the washings was made acidic with an aqueous 10% solution of hydrochloric acid. The aqueous layer was made alkaline with an aqueous 30% solution of sodium hydroxide and extracted with benzene. The extract was washed with water, dried over anhydrous sodium sulphate and condensed under reduced pressure to obtain the objective product as oily substance pale yellow in colour in an amount of $7 \times 10^{-3}$ kg (7 g) corresponding to a yield of 57%.

(c) Synthesis of N-cyano-S-methyl-N'-[2-{3-oxo-6-phenyl-2-pyridazinyl)methoxy}ethyl] isothiourea, Compound No. 130:

$10^{-2}$ Kg (10 g, 0.04 mol) of 2-(2-amino-ethoxymethyl)-6-phenyl-3(2H)-pyridazinone and $6 \times 10^{-3}$ kg (6 g, 0.04 mol) of dimethyl cyanodithioimidocarbonate were dissolved in $5 \times 10^{-5}$ $m^3$ (50 ml) of ethanol. This solution was stirred at room temperature for about 6 hours. On recrystallizing the deposited crystals from a mixture of ethanol and isopropyl ether, pale yellow crystals melting at 101 to 104°C were obtained in an amount of $10^{-2}$ kg (10 g) corresponding to 72% of the theoretical yield of the objective product, Compound No. 130.

The physical properties of Compound No. 130 were:

IR (Nujol method) shown in Figure 130 of the accompanying drawings:
$\nu_{max}^{Nujol}$ 3320 (NH), 2150 (CN) and 1650 (C=O).
NMR (in DMSO-$d_6$) $\delta$:
2.66 (3H, singlet, S—$CH_3$)
3.70 (2H, triplet, J = 6Hz, O—$CH_2$—$\mathit{CH_2}$—NH
3.82 (2H, triplet, J = 6Hz, O—$\mathit{CH_2}$—$CH_2$—NH)
4.92 (2H, singlet, N—$CH_2$—O)
7.30 (1H, doublet, J = 10Hz, $C_4$—H)
7.6 (3H, multiplet, aromatic H)
8.0 (2H, multiplet, aromatic H)
8.25 (1H, doublet, J = 10Hz, $C_5$—H)
8.5 (1H, broad, NH)
Elementary analysis:
found: 55.78% of C, 4.79% of H and 20.51% of N.
calcd. as $C_{16}H_{17}N_5O_2S$: 55.96% of C, 4.99% of H and 20.39% of N.

## Example 12

Synthesis of N-cyano-N'-[2-{(3-oxo-6-phenyl-2-pyridazinyl)methoxy}ethyl]-N''-methylguanidine, Compound No. 131:

$2 \times 10^{-3}$ Kg (2 g, 0.006 mol) of N-cyano-S-methyl-N'-[2-{3-oxo-6-phenyl-2-pyridazinyl)-methoxy}ethyl] isothiourea was dissolved in $2 \times 10^{-5}$ $m^3$ (20 ml) of a 30% methanolic solution of methylamine and heated at about 50°C under agitation for 6 hours. After cooling the reaction mixture, the solvent was distilled off. The residue was recrystallized from acetonitrile to obtain the desired product as colourless crystals melting at 110 to 113°C in an amount of $1.6 \times 10^{-3}$ kg (1.6 g) corresponding to 82% of the theoretical yield.

According to another route of synthesis:

$2.5 \times 10^{-3}$ kg (2.5 g, 0.01 mol) of 2-(2-aminoethoxymethyl)-6-phenyl-3(2H)-pyridazinone and $1.3 \times 10^{-3}$ kg (1.3 g, 0.01 mol) of N-cyano-S-methyl-N'-methylisothiourea were dissolved in $5 \times 10^{-5}$ $m^3$ (50 ml) of ethanol. The solution was heated at about 60°C for 8 hours under agitation. After cooling the reaction mixture, the solvent was distilled off. The residue was recrystallized from acetonitrile to obtain the desired product as colourless crystals melting at 110 to 113°C in an amount of $2.1 \times 10^{-3}$ kg (2.1 g) corresponding to 65% of the theoretical yield.

The physical properties of this Compound No. 131 were:

IR (Nujol method) shown in Figure 131 of the accompanying drawings.
$\nu_{max}^{Nujol}$ 3240 $cm^{-1}$ (NH), 2170 $cm^{-1}$ (CN) and 1670 $cm^{-1}$ (C=O).
NMR (in DMSO-$d_6$) $\delta$:
2.66 (3H, doublet, J = 4Hz, NH—$CH_3$)
3.47 (2H, triplet, J = 6Hz, O—$CH_2$—$\mathit{CH_2}$·NH)
3.85 (2H, triplet, J = 6Hz, O—$\mathit{CH_2}$—$CH_2$·NH)

5.25 (2H, singlet, N—$CH_2$—O)
7.1 (2H, broad, NH x 2)
7.15 (1H, doublet, J = 10Hz, $C_4$—H)
7.4 (3H, multiplet, aromatic H)
7.9 (2H, multiplet, aromatic H)
8.15 (1H, doublet, J = 10Hz, $C_5$—H)
Mass spectrum m/e: 326
Elementary analysis:
found: 58.93% of C, 5.73% of H and 25.63% of N
calcd. as $C_{16}H_{18}N_6O_2$: 58.92% of C, 5.51% of H and 25.77% of N.

Example 13

The following compounds Nos. 132 to 134 of the present invention were synthesized in similar manner as compound No. 131 in Example 12 while using the respective corresponding derivatives of isothiourea. The infrared absorption spectra of compounds Nos. 132 to 134 are shown in Figures 132 to 134 respectively of the accompanying drawings:

Compound No. 132: N-cyano-N'-[2-{(3-oxo-6-phenyl-2-pyridazinyl)methoxy}ethyl]-N''-ethylguanidine, m.pt. 100—103°C.
Compound No. 133: N-cyano-N'-[2-{(3-oxo-6-phenyl-2-pyridazinyl)ethoxy}ethyl]-N''-methylguanidine, m.pt. 90—93°C.
Compound No. 134: N-cyano-N'-[2-{(6-(4-methoxyphenyl)-3-oxo-2-pyridazinyl)methoxy}ethyl]-N''-methylguanidine, m.pt. 123—126°C.

Example 14

(a) Synthesis of 2-[(2-aminoethyl)thiomethyl-6-phenyl]-3(2H)-pyridazinone.

8.5 x $10^{-3}$ Kg (8.5 g, 0.075 mol) of cysteamine hydrochloride was added to $10^{-4}$ $m^3$ (100 ml) of an ethanolic solution containing 3.5 x $10^{-3}$ kg (3.5 g, 0.15 mol) of metallic sodium. This mixture was stirred at 70°C for 30 min. Then, 1.1 x $10^{-2}$ kg (11 g, 0.05 mol) of 2-chloromethyl-6-phenyl-3(2H)-pyridazinone dissolved in $10^{-4}$ $m^3$ (100 ml) of ethanol was added to the above-mentioned mixture, and the whole was heated for 5 hours under reflux. After cooling, the solvent was distilled off and the residue was extracted with chloroform. The organic extract was thoroughly washed with water and dried over anhydrous sodium sulphate. After distilling chloroform from the extract, the residue was treated with active carbon again in ethanol, and the solvent was distilled off to leave a brown-coloured oily substance in an amount of $10^{-2}$ kg (10 g) corresponding to 77% of the theoretical yield.

The physical properties of the product were:

IR (Neat method):
$\nu_{max}^{neat}$ 3350 (NH) and 1670 (C=O).
NMR (in DMSO-$d_6$) $\delta$:
2.53 (2H, singlet, $NH_2$)
2.86 (4H, singlet, S—$CH_2CH_2$—NH
5.28 (2H, singlet, N—$CH_2$—S)
7.12 (1H, doublet, J = 10Hz, $C_4$—H)
7.5 (3H, multiplet, aromatic H)
7.9 (2H, multiplet, aromatic H)
8.08 (1H, doublet, J = 10Hz, $C_5$—H)

(b) Synthesis of N-cyano-S-methyl-N'-[2-{(3-oxo-6-phenyl-2-pyridazinyl)methylthio}ethyl] isothiourea, Compound No. 135:

Into 60 ml of ethanol, 9.7 x $10^{-3}$ kg (9.7 g, 0.037 mol) of 2-[(2-amino-ethyl) thiomethyl]-6-phenyl-3(2H)-pyridazinone and 5.4 x $10^{-3}$ kg (5.4 g, 0.037 mol) of dimethyl cyanodithioimidocarbonate were added to 6 x $10^{-5}$ $m^3$ (60 ml) of ethanol. This reaction mixture was stirred at room temperature for about 8 hours. The crystals that deposited were recrystallized from a mixture of ethanol and isopropyl ether to obtain the desired product, Compound No. 135, as pale yellow crystals melting at 136 to 138°C in an amount of 9.7 x $10^{-3}$ kg (9.7 g) corresponding to 73% of the theoretical yield.

The physical properties of Compound No. 135 were as follows:

IR (Nujol method) shown in Figure 135 of the accompanying drawings:
$\nu_{max}^{Nujol}$ 3240 (NH), 2180 (CN), and 1670 (C=O).
NMR (in DMSO-$d_6$) $\delta$:
2.62 (3H, singlet, S—$CH_3$)
3.03 (2H, triplet, J = 6Hz, S—$CH_2CH_2$NH)
3.68 (2H, triplet, J = 6Hz, S—$CH_2CH_2$NH)
5.36 (2H, singlet, N—$CH_2$—S)

7.20 (1H, doublet, J = 10Hz, $C_4$—H)
7.6 (3H, multiplet, aromatic H)
8.0 (2H, multiplet, aromatic H)
8.22 (1H, doublet, J = 10Hz, $C_5$—H)
8.6 (1H, broad, NH)

Mass spectrum m/e: 359

Elementary analysis:
found: 53.21% of C, 4.59% of H and 19.60% of N
calcd. as $C_{16}H_{17}N_5OS_2$: 53.45% of C, 4.78% of H and 19.48% of N.

Example 15

Compounds Nos. 136 and 137 below were synthesized in accordance with Example 14 while using the respective corresponding derivatives of 3(2H)-pyridazinone. The infrared absorption spectra of compounds Nos. 136 and 137 are shown in Figures 136 and 137 respectively of the accompanying drawings:

Compound No. 136: N-cyano-S-methyl-N'-[2-{(6-(4-chlorophenyl-3-oxo-2-pyridazinyl) methylthio}ethyl] isothiourea, m.pt. 159—162°C.

Compound No. 137: N-cyano-S-methyl-N'-[2-{(6-(4-methylphenyl)-3-oxo-2-pyridazinyl) methylthio}ethyl] isothiourea, m.pt. 59—61°C.

Example 16

Synthesis of N-cyano-N'-[2-{(3-oxo-6-phenyl-2-pyridazinyl) methylthio}ethyl]-N"-methylguanidine, Compound No. 138:

$2.5 \times 10^{-3}$ kg (2.5 g. 0.007 mol) of N-cyano-S-methyl-N'-[2-{(3-oxo-6-phenyl-2-pyridazinyl)-methylthio}ethyl] isothiourea was dissolved in $3 \times 10^{-5}$ $m^3$ (30 ml) of a 30% methanolic solution of methylamine. This solution was heated at about 50°C for 7 hours. After cooling, the solvent was distilled off. The residue was recrystallized from ethanol to obtain the desired compound as colourless crystals melting at 157 to 158°C in an amount of $2.0 \times 10^{-3}$ kg (2.0 g) corresponding to 84% of the theoretical yield.

According to anotdher route of synthesis:

$2.6 \times 10^{-3}$ kg (2.6 g, 0.01 mol) of 2-(2-aminoethylthiomethyl)-6-phenyl-3(2H)-pyridazinone and $1.3 \times 10^{-3}$ kg (1.3 g, 0.01 mol) of N-cyano-S-methyl-N'-methylisothiourea were dissolved in $5 \times 10^{-5}$ $m^3$ (50 ml) of ethanol. This solution was heated at about 60°C for 4 hours. After cooling the reaction mixture, the solvent was distilled off. The resiude was recrystallized from ethanol to obtain the desired compound as colourless crystals melting at 157 to 158°C in an amount of $3.0 \times 10^{-3}$ kg (3.0 g) corresponding to 88% of the theoretical yield. The physical properties of Compound No. 138 were as follows:

IR (Nujol method) shown in Figure 138 of the accompanying drawings:
$\nu_{max}^{Nujol}$ 3300 $cm^{-1}$ (NH), 2180 (CN) and 1650 (C=O).

NMR (in DMSO-$d_6$) $\delta$:
2.68 (3H, doublet, J = 4Hz, NH—$CH_3$)
2.90 (2H, triplet, J = 6Hz, S—$CH_2$—$CH_2$—NH)
3.46 (2H, triplet, J = 6Hz, S—$CH_2$—$CH_2$—NH)
5.27 (2H, singlet, CO—N—$CH_2$—S)
7.1 (2H, broad, NH x 2)
7.10 (1H, doublet, J = 10Hz, $C_4$—H)
7.5 (3H, multiplet, aromatic H)
7.9 (2H, multiplet, aromatic H)
8.10 (1H, doublet, J = 10Hz, $C_5$—H)

Mass spectrum m/e: 342

Elementary analysis:
found: 55.92% of C, 5.50% of H and 24.48% of N
calcd. as $C_{16}H_{18}N_6OS$: 56.11% of C, 5.31% of H and 24.55% of N.

Example 17

The following compounds Nos. 139 to 143 were synthesized similarly to and in accordance with Example 16 while using the respective corresponding derivatives of isothiourea. The infrared absorption spectra of compounds Nos. 139 to 143 are shown in Figures 139 to 143 respectively of the accompanying drawings:

Compound No. 139: N-cyano-N'-[2-{(3-oxo-6-phenyl-2-pyridazinyl)-methylthio} ethyl]-N"-ethylguanidine, m.pt. 171—172°C.

Compound No. 140: N-cyano-N′-[2-{(6-(4-chlorophenyl)-3-oxo-2-pyridazinyl)methylthio} ethyl]-N″-methylguanidine, m.pt. 217—218°C.

Compound No. 141: N-cyano-N′-[2-{(6-(4-chlorophenyl)-3-oxo-2-pyridazinyl)methylthio} ethyl]-N″-ethylguanidine, m.pt. 165—168°C.

Compound No. 142: N-cyano-N′-[2-{(6-(4-methylphenyl)-3-oxo-2-pyridazinyl)methylthio} ethyl]-N″-methylguanidine, m.pt. 168—171°C.

Compound No. 143: N-cyano-N′-[2-{(6-(4-methylphenyl)-3-oxo-2-pyridazinyl)methylthio} ethyl]-N″-ethylguanidine, m.pt. 179—180°C.

Examples 18 and 19 illustrate the formulation of pharmaceutical compositions incorporating compounds of the invention.

Example 18

A pharmaceutical composition in the form of capsules was formulated in accordance with the following recipe:

| Ingredient | Amount (kg) |
|---|---|
| Compound No. 53 (refer to Example 5) | $1.5 \times 10^{-4}$ (150 mg) |
| Lactose | $7.5 \times 10^{-5}$ (75 mg) |
| Starch | $2.5 \times 10^{-5}$ (25 mg |
| Talc | $5 \times 10^{-6}$ (5 mg) |
| Magnesium stearate | $2 \times 10^{-6}$ (2 mg) |

After sifting the ingredients to adjust their grain size they were mixed well, and formed into hard capsules.

Example 19

A pharmaceutical composition in the form of tablets was formulated in accordance with the following recipe:

| Ingredient | Amount (kg) |
|---|---|
| Compound No 44 (refer to Example 5) | $1.5 \times 10^{-4}$ (150 mg) |
| Lactose | $4.0 \times 10^{-5}$ (40 mg) |
| Starch | $1.5 \times 10^{-5}$ (15 mg) |
| Carboxymethylcellulose | $10^{-5}$ (10 mg) |
| Methylcellulose | $8 \times 10^{-6}$ (8 mg) |
| Talc | $10^{-6}$ (1 mg) |
| Magnesium stearate | $10^{-6}$ (1 mg) |

After sifting the ingredients to adjust their grain size, they were mixed well and then formulated into tablets.

**Claims for the Contracting States: BE CH DE FR GB IT LI NL**

1. A compound represented by the general formula (I):

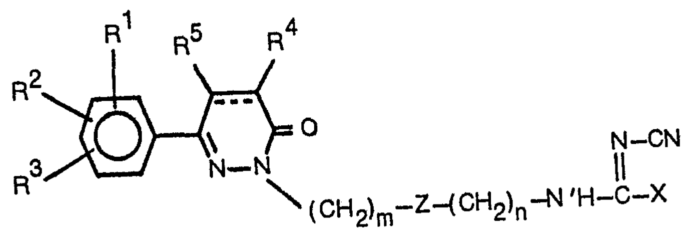

(I)

wherein $R^1$, $R^2$ and $R^3$ independently represent a hydrogen atom, halogen atom, lower alkyl group or lower alkoxy group; $R^4$ and $R^5$ independently represent a hydrogen atom or a lower alkyl group; *m* represents an integer of from 1 to 3; *n* represents O or an integer of from 1 to 3; Z represents $—CH_2—$, —O— or —S—; X represents $—S—R^6$ or $—N''H—R^7$, wherein $R^6$ represents a lower alkyl group and $R^7$ represents a hydrogen atom, a lower alkyl or a propargyl group; and the carbon atoms at positions 4 and 5 of the heterocyclic ring of the formula (I), to which $R^4$ and $R^5$ are bonded respectively, are joined by either a single or a double carbon-carbon bond; and acid addition salts thereof; with the proviso that when Z represents —O— or —S—, said carbon atoms at positions 4 and 5 are joined by a double bond and *n* represents an integer of 1 to 3; wherein the lower alkyl and lower alkoxy groups contain up to 6 carbon atoms.

2. A compound according to Claim 1, wherein $R^1$ $R^2$ and $R^3$ independently represent chlorine, methyl or methoxy.

3. A compound according to Claim 1 or 2, wherein the group of formula:

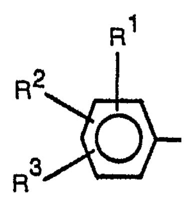

is a phenyl, 4-chlorophenyl, 3,4-dichlorophenyl, 4-methoxyphenyl, 3,4-dimethoxyphenyl, 3,4,5-trimethoxyphenyl, 4-methylphenyl, 5-methylphenyl, or 2,4-dimethylphenyl group.

4. A compound according to any one of the preceding claims, wherein one of $R^4$ and $R^5$ is a methyl group and the other is hydrogen, or both $R^4$ and $R^5$ represents hydrogen.

5. A compound according to any one of the preceding claims, wherein $R^6$ is a methyl group.

6. A compound according to any one of Claims 1 to 4, wherein $R^7$ is a methyl, ethyl or propyl group.

7. A compound according to Claim 1, which is N-cyano-N'-[4-(3-oxo-6-phenyl-2-pyridazinyl)-butyl]-N"-methylguanidine.

8. A compound according to Claim 1, which is N-cyano-N'-[3-(3-oxo-6-phenyl-2-pyridazinyl)-propyl]-N"-ethylguanidine.

9. A compound according to Claim 1, which is N-cyano-N'-[4-(3-oxo-6-phenyl-2-pyridazinyl)-butyl]-N"-ethylguanidine.

10. A compound according to Claim 1, which is N-cyano-N'-[5-(3-oxo-6-phenyl-2-pyridazinyl)-pentyl]-N"-ethylguanidine.

11. A compound according to Claim 1, which is N-cyano-N'-[2-{(3-oxo-6-phenyl-2-pyridazinyl)-methylthio} ethyl-N"-methylguanidine.

12. A compound according to Claim 1, which is N-cyano-N'-[4-(4-methyl-3-oxo-6-phenyl-2-pyridazinyl) butyl]-N"-methylguanidine.

13. A compound according to Claim 1, which is N-cyano-N'-[4-{6-(4-chlorophenyl)-3-oxo-2-pyridazinyl} butyl]-N"-methylguanidine.

14. A compound according to Claim 1, which is N-cyano-N'-[4-{6-(4-methylphenyl)-3-oxo-2-pyridazinyl} butyl]-N"-methylguanidine.

15. A process for preparing a compound of formula (I) as defined in Claim 1 wherein Z represents $—CH_2—$ and X represents $—S—R^6$, $R^6$ being as defined in Claim 1, which process comprises reacting a compound represented by the formula (II):

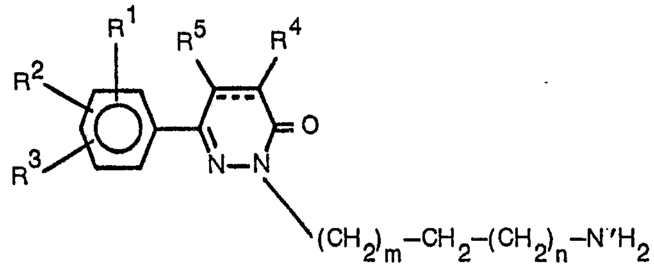

(II)

with dialkylcyano-dithioimido carbonate.

16. A process for preparing a compound of formula (I) as defined in Claim 1, wherein Z represents $-CH_2-$ and X represents —N″H—$R^7$, $R^7$ being as defined in Claim 1, which process comprises reacting a compound represented by the formula (II):

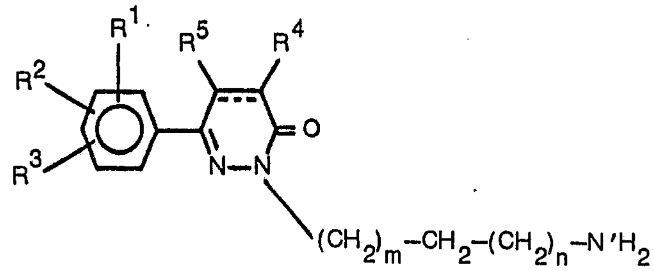

(II)

with S-alkyl-N-cyano-N″-alkyl-isothiourea.

17. A process for preparing a compound of formula (I) as defined in Claim 1, wherein Z represents $-CH_2-$ and X represents —N″H—$R^7$, $R^7$ being as defined in Claim 1, which process comprises reacting a compound of formula (I) as defined in Claim 1, wherein Z represents $-CH_2-$ and X represents —S—$R^6$, $R^6$ being as defined in Claim 1, with ammonia or an alkyl amine containing up to 6 carbon atoms.

18. A process for preparing a compound of formula (I) as defined in Claim 1, wherein Z represents —O— and X represents —S—$R^6$, $R^6$ being as defined in Claim 1, which process comprises reacting a compound represented by the formula (VI):

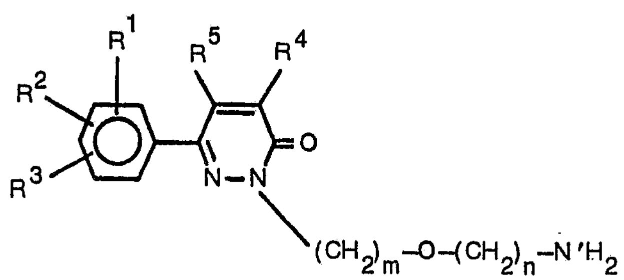

(VI)

with dialkylcyano-dithioimido-carbonate.

19. A process for preparing a compound of formula (I) as defined in Claim 1, wherein Z represents —O— and X represents —N″H—$R^7$, $R^7$ being as defined in Claim 1, which process comprises reacting a compound represented by the formula (VI):

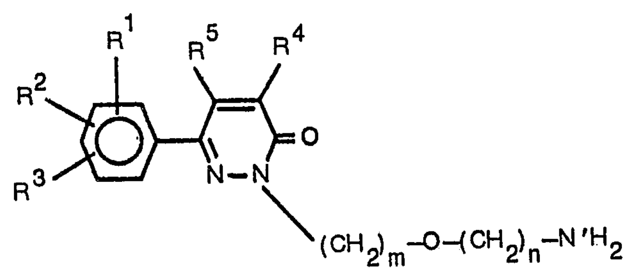

(VI)

with S-alkyl-N-cyano-N″-alkyl-isothiourea.

20. A process for preparing a compound of formula (I) as defined in Claim 1, wherein Z represents —O— and X represents —N″H—$R^7$, $R^7$ being as defined in Claim 1, which process comprises reacting

a compound of formula (I) as defined in Claim 1, wherein Z represents —O— and X represents —S—$R^6$, $R^6$ being as defined in Claim 1, with ammonia or an alkyl amine containing up to 6 carbon atoms.

21. A process for preparing a compound of formula (I) as defined in Claim 1, wherein Z represents —S— and X represents —S—$R^6$, $R^6$ being as defined in Claim 1, which process comprises reacting a compound represented by the formula (VII):

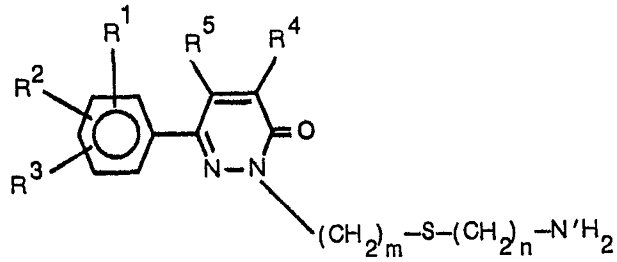

(VII)

with dialkylcyano-dithioimido carbonate.

22. A process for preparing a compound of formula (I) as defined in Claim 1, wherein Z represents —S— and X represents —N″H—$R^7$, $R^7$ being as defined in Claim 1, which process comprises reacting a compound represented by the formula (VII):

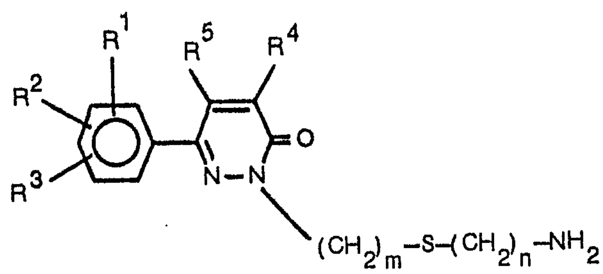

(VII)

with S-alkyl-N-cyano-N″-alkyl-isothiourea.

23. A process for preparing a compound of formula (I) as defined in Claim 1, wherein Z represents —S— and X represents —N″H—$R^7$, $R^7$ being as defined in Claim 1, which process comprises reacting a compound of formula (I) as defined in Claim 1, wherein Z represents —S— and X represents —S—$R^6$, $R^6$ being as defined in Claim 1, with ammonia or an alkyl amine containing up to 6 carbon atoms.

24. A pharmaceutical composition comprising as active ingredient a compound as claimed in any one of claims 1 to 14 or which has been prepared by a process as claimed in any one of Claims 15 to 23, together with a pharmaceutically acceptable carrier or adjuvant.

**Claims for the Contracting State: AT**

1. A process for preparing a compound represented by the general formula (I-a):

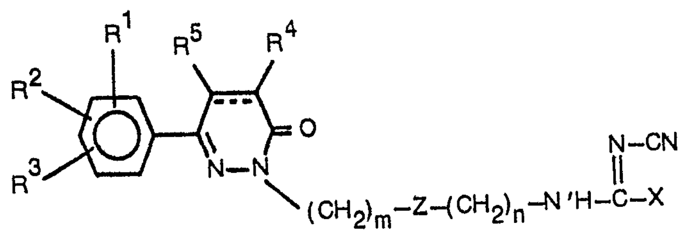

(I-a)

wherein $R^1$, $R^2$ and $R^3$ independently represent a hydrogen atom, halogen atom, lower alkyl group or lower alkoxy group; $R^4$ and $R^5$ independently represent a hydrogen atom or a lower alkyl group; *m* represents an integer of from 1 to 3; *n* represents 0 or an integer of from 1 to 3; Z represents —$CH_2$—; X represents —S—$R^6$ wherein $R^6$ represents a lower alkyl group; and the carbon atoms at positions 4 and 5 of the heterocyclic ring of the formula (I-a), to which $R^4$ and $R^5$ are bonded respectively, are joined by either a single or a double carbon-carbon bond; wherein the lower alkyl and lower alkoxy groups contain up to 6 carbon atoms; which process comprises reacting a compound represented by the formula (II):

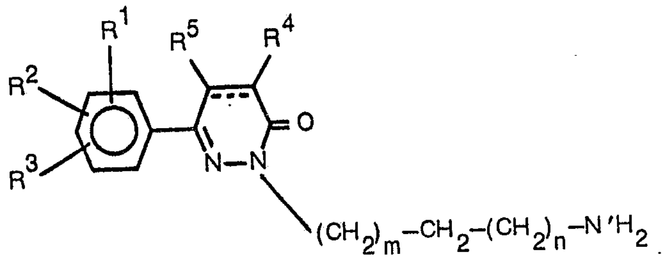

(II)

with dialkylcyclo-dithioimido carbonate.

2. A process for preparing a compound of formula (I-a) as defined in claim 1 except that X represents —N″H—$R^7$ wherein $R^7$ represents a hydrogen atom, an alkyl group of up to 6 carbon atoms or a propargyl group, which process comprises reacting a compound represented by the formula (II):

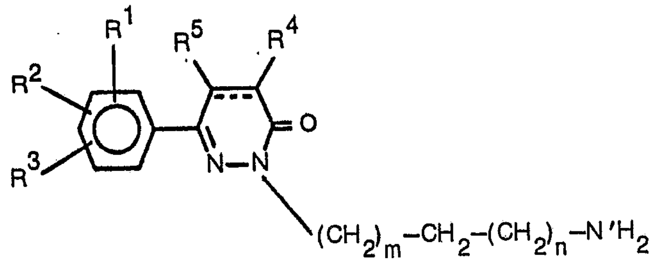

(II)

with S-alkyl-N-cyano-N″-alkyl-isothiourea.

3. A process for preparing a compound of formula (I-a) as defined in claim 1 except that X represents —N″H—$R^7$, $R^7$ being as defined in claim 2, which process comprises reacting a compound of formula (I-a) as defined in claim 1 with ammonia or an alkyl amine containing up to 6 carbon atoms.

4. A process for preparing a compound represented by the general formula (I-c):

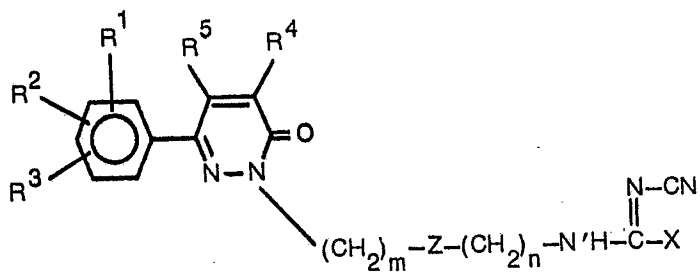

(I-c)

wherein $R^1$, $R^2$ and $R^3$ independently represent a hydrogen atom, halogen atom, lower alkyl group or lower alkoxy group; $R^4$ and $R^5$ independently represent a hydrogen atom or a lower alkyl group; *m* represents an integer of from 1 to 3; *n* represents an integer of from 1 to 3; Z represents —O—; X represents —S—$R^6$ wherein $R^6$ represents a lower alkyl group, the lower alkyl and lower alkoxy groups containing up to 6 carbon atoms; which process comprises reacting a compound represented by the formula (VI):

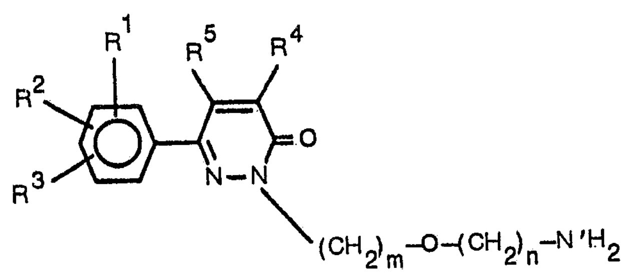

(VI)

with dialkylcyclo-dithioimido-carbonate.

5. A process for preparing a compound of formula (I-c) as defined in claim 4 except that X represents —N″H—$R^7$, $R^7$ being as defined in claim 2, which process comprises reacting a compound represented by the formula (VI):

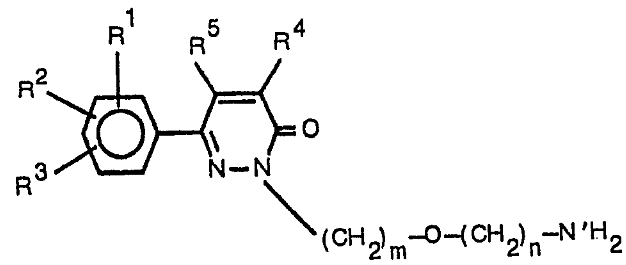

(VI)

with S-alkyl-N-cyano-N″-alkyl-isothiourea.

6. A process for preparing a compound of formula (I-c) as defined in claim 4 except that X represents —N″H—$R^7$, $R^7$ being as defined in claim 2, which process comprises reacting a compound of formula (I-c) as defined in claim 4 with ammonia or an alkyl amine containing up to 6 carbon atoms.

7. A process for preparing a compound represented by the general formula (I-e):

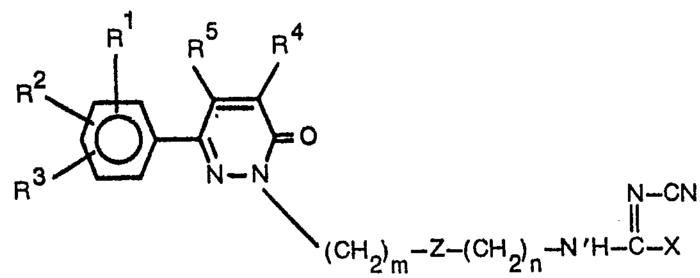

(I-e)

wherein $R^1$, $R^2$ and $R^3$ independently represent a hydrogen atom, halogen atom, lower alkyl group or lower alkoxy group; $R^4$ and $R^5$ independently represent a hydrogen atom or a lower alkyl group; *m* represents an integer of from 1 to 3; *n* represents an integer of from 1 to 3; Z represents —S—; X represents —S—$R^6$ wherein $R^6$ represents a lower alkyl group, the lower alkyl and lower alkoxy groups containing up to 6 carbon atoms; which process comprises reacting a compound represented by the formula (VII):

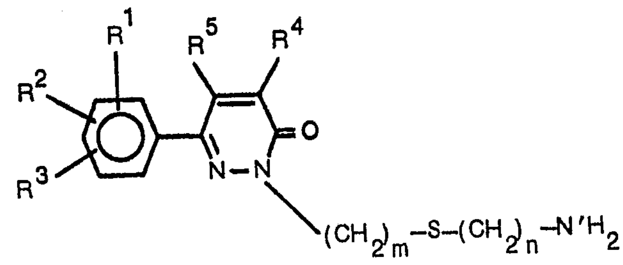

(VII)

with dialkylcyano-dithioimido carbonate.

8. A process for preparing a compound of formula (I-e) as defined in claim 7 except that X represents —N″H—$R^7$, $R^7$ being as defined in claim 2, which process comprises reacting a compound represented by the formula (VII):

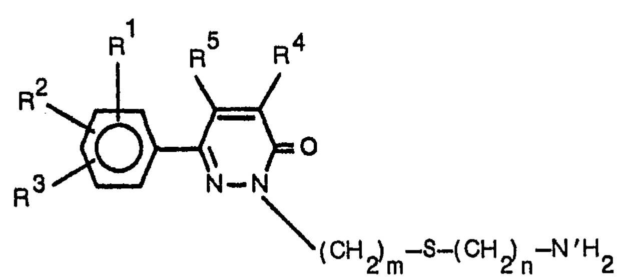

(VII)

with S-alkyl-N-cyano-N″-alkyl-isothiourea.

9. A process for preparing a compound of formula (I-e) as defined in claim 7 except that X represents —N″H—$R^7$, $R^7$ being as defined in claim 2, which process comprises reacting a compound of formula (I-e) as defined in claim 7 with ammonia or an alkyl amine containing up to 6 carbon atoms.

**Patentansprüche für die Vertragsstaaten: BE CH DE FR GB IT LI NL**

1. Eine Verbindung der allgemeinen Formel (I)

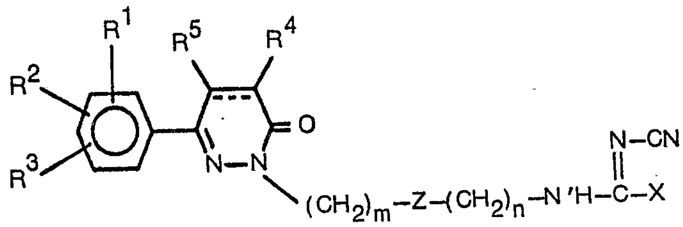

(I)

in der bedeuten:

$R^1$, $R^2$ und $R^3$ unabhängig voneinander ein Wasserstoffatom, ein Halogenatom, eine niedere Alkylgruppe oder eine niedere Alkoxygruppe;

$R^4$ und $R^5$ unabhängig voneinander ein Wasserstoffatom oder eine niedere Alkylgruppe;

m: eine ganze Zahl von 1 bis 3;

n: Null oder eine ganze Zahl von 1 bis 3;

Z: $—CH_2—$, —O— oder —S—;

X: $—S—R^6$ oder $—N''H—R^7$;

worin $R^6$ eine niedere Alkylgruppe und $R^7$ ein Wasserstoffatom, eine niedere Alkylgruppe oder eine Propargylgruppe bedeuten;

und worin die C-Atome in Stellung 4 und 5 des heterocyclischen Ringes der Formel (I), an die $R^4$ bzw. $R^5$ gebunden ist, miteinander über eine Einfach- oder Doppel-C-C-Bindung verbunden sind;

sowie die durch Säureaddition entstehenden Salze;

mit der Maßgabe, daß,

wenn Z die Atome —O— oder —S— bedeuten, die C-Atome in Stellung 4 und 5 durch Doppelbindung miteinander verbunden sind und n eine ganze Zahl von 1 bis 3 bedeutet;

wobei die niederen Alkyl- und die niederen Alkoxygruppen bis zu sechs Kohlenstoffatome enthalten.

2. Verbindung nach Anspruch 1, in der $R^1$, $R^2$ und $R^3$ unabhängig voneinander Chlor, Methyl oder Methoxyl bedeuten.

3. Verbindung nach Anspruch 1 oder 2, in der die Gruppe der Formel

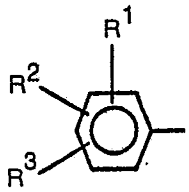

eine

Phenyl-,
4-Chlorphenyl-,
3,4-Dichlorphenyl-,
4-Methoxyphenyl-,
3,4-Dimethoxyphenyl-,
3,4,5-Trimethoxyphenyl-
4-Methylphenyl-,
5-Methylphenyl-
oder 2,4-Dimethylphenyl-Gruppe ist.

4. Verbindung nach irgendeinem der vorausgehenden Ansprüche, in der einer der Reste $R^4$ und $R^5$ eine Methylgruppe und der andere ein Wasserstoffatom ist oder beide $R^4$ und $R^5$ Wasserstoff bedeuten.

5. Verbindung nach irgendeinem der vorausgehenden Ansprüche, in der $R^6$ eine Methylgruppe ist.

6. Verbindung nach irgendeinem der Ansprüche 1 bis 4, in der $R^7$ eine Methyl-, Ethyl- oder Propylgruppe ist.

7. Verbindung nach Anspruch 1, nämlich N-cyan-N'-[4-(3-oxo-6-phenyl-2-pyridazinyl)butyl]-N''-methylguanidin.

8. Verbindung nach Anspruch 1, nämlich N-cyan-N'-[3-(3-oxo-6-phenyl-2-pyridazinyl)-propyl]-N''-ethylguanidin.

9. Verbindung nach Anspruch 1, nämlich N-cyan-N'-[4-(3-oxo-6-phenyl-2-pyridazinyl)-butyl]-N''-ethylguanidin.

10. Verbindung nach Anspruch 1, nämlich N-cyan-N'-[5-(3-oxo-6-phenyl-2-pyridazinyl)pentyl]-N''-ethylguanidin.

11. Verbindung nach Anspruch 1, nämlich N-cyan-N′-[2{(3-oxo-6-phenyl-2-pyridazinyl)-methylthio}ethyl]-N″-methylguanidin.

12. Verbindung nach Anspruch 1, nämlich N-cyan-N′-[4-(4-methyl-3-oxo-6-phenyl-2-pyridazinyl)butyl]-N″-methylguanidin.

13. Verbindung nach Anspruch 1, nämlich N-cyan-N′-[4-{6-(4-chlorphenyl)-3-oxo-2-pyridazinyl}-butyl]-N″-methylguanidin.

14. Verbindung nach Anspruch 1, nämlich N-cyan-N′-[4-{6-(4-methylphenyl)-3-oxo-2-pyridazinyl}butyl]-N″-methylguanidin.

15. Verfahren zum Herstellen einer Verbindung gemäß Formel (I) nach Anspruch 1, worin Z bedeutet —$CH_2$— und X bedeutet —$SR^6$, $R^6$ wie in Anspruch 1 definiert, bei dem eine Verbindung gemäß Formel (II)

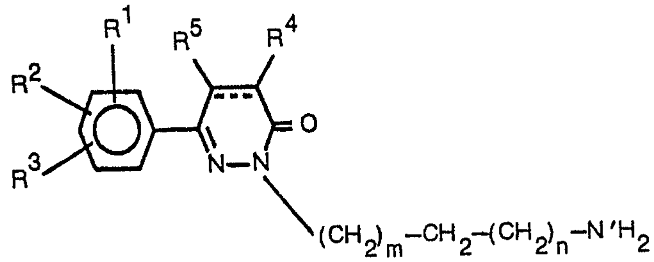

(II)

mit Dialkylcyan-dithioimido-carbonat zur Reaktion gebracht wird.

16. Verfahren zum Herstellen einer Verbindung gemäß Formel (I) nach Anspruch 1, worin Z bedeutet —$CH_2$— und X bedeutet —N″H—$R^7$, $R^7$ wie in Anspruch 1 definiert, bei dem eine Verbindung gemäß Formel (II)

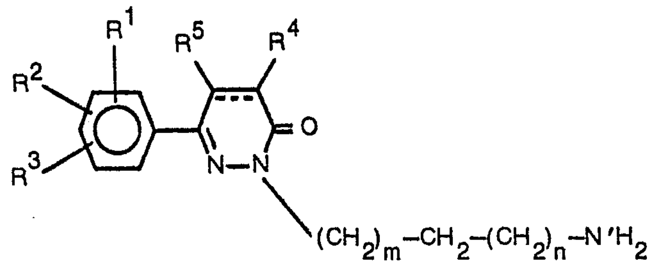

(II)

mit S-alkyl-N-cyan-N″-alkyl-isothioharnstoff zur Reaktion gebracht wird.

17. Verfahren zum Herstellen einer Verbindung gemäß Formel (I) nach Anspruch 1, worin Z bedeutet —$CH_2$— und X bedeutet —N″H—$R^7$, $R^7$ wie in Anspruch 1 definiert, bei dem eine Verbindung gemäß Formel (I) nach Anspruch 1, in der Z bedeutet —$CH_2$— und X bedeutet —S—$R^6$, $R^6$ wie in Anspruch 1 definiert, mit Ammoniak oder einem Alkylamin, welches bis zu sechs C-Atome enthält, zur Reaktion gebracht wird.

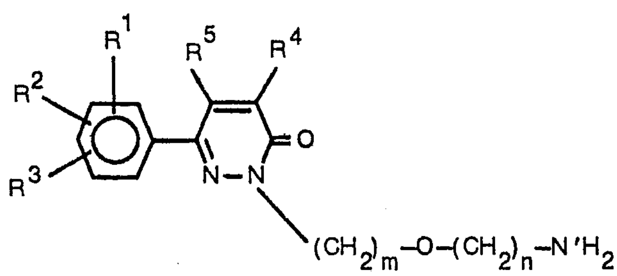

(VI)

mit Dialkylcyan-dithioimido-carbonat zur Reaktion gebracht wird.

19. Verfahren zum Herstellen einer Verbindung gemäß Formel (I) nach Anspruch 1, worin Z bedeutet —O— und X bedeutet —N″H—$R^7$, $R^7$, wie in Anspruch 1 definiert, bei dem eine Verbindung gemäß Formel (VI)

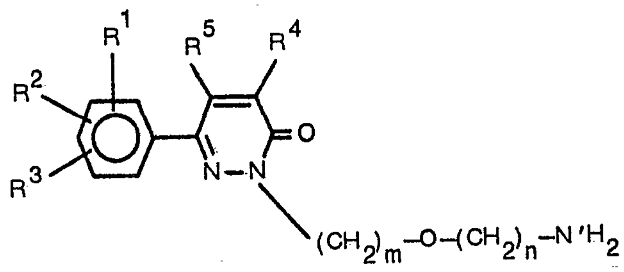

(VI)

mit S-alkyl-N-cyan-N″-alkyl-isothioharnstoff zur Reaktion gebracht wird.

20. Verfahren zum Herstellen einer Verbindung gemäß Formel (I), nach Anspruch 1, worin Z bedeutet —O— und X bedeutet —N″H—$R^7$, $R^7$ wie in Anspruch 1 definiert, bei dem eine Verbindung gemäß Formel (I) nach Anspruch 1 worin Z bedeutet —O— und X bedeutet —S—$R^6$, $R^6$ wie in Anspruch 1 definiert, mit Ammoniak oder einem Alkylamin, welches bis zu sechs C-Atome enthält, zur Reaktion gebracht wird.

21. Verfahren zum Herstellen einer Verbindung gemäß Formel (I) nach Anspruch 1, worin Z bedeutet —S— und X bedeutet —S—$R^6$, $R^6$ wie in Anspruch 1 definiert, bei dem eine Verbindung gemäß Formel (VII)

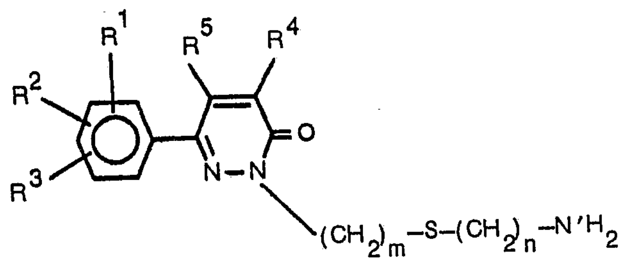

(VII)

mit Dialkylcyan-dithioimidcarbonat zur Reaktion gebracht wird.

22. Verfahren zum Herstellen einer Verbindung gemäß Formel (I) nach Anspruch 1, worin Z bedeutet —S— und X bedeutet —N″H—$R^7$, $R^7$ wie in Anspruch 1 definiert, bei dem eine Verbindung gemäß Formel (VII)

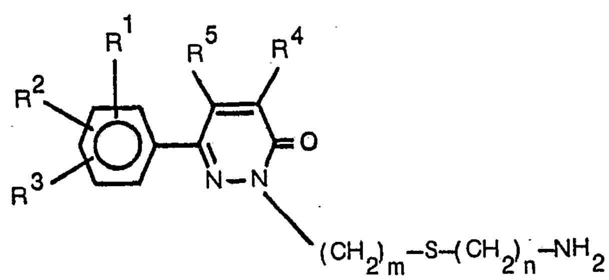

(VII)

mit S-alkyl-N-cyan-N″-alkyl-isothioharnstoff zur Reaktion gebracht wird.

23. Verfahren zum Herstellen einer Verbindung gemäß Formel (I) nach Anspruch 1, worin Z bedeutet —S— und X bedeutet —N″H—$R^7$, $R^7$ wie in Anspruch 1 definiert, bei dem eine Verbindung gemäß Formel (I) nach Anspruch 1, worin Z bedeutet —S— und X bedeutet —S—$R^6$, $R^6$ wie in Anspruch 1 definiert mit Ammoniak oder einem Alkylamin, welches bis zu sechs C-Atome enthält, zur Reaktion gebracht wird.

24. Pharmazeutische Zusammensetzung, die eine Verbindung gemäß irgendeinem der Ansprüche 1—14 oder hergestellt nach einem Verfahren gemäß irgendeinem der Ansprüche 15—23 als Wirkstoff, zusammen mit einem pharmazeutisch akzeptablen Träger- oder Hilfsstoff enthält.

**Patentansprüche für den Vertragsstaat: AT**

1. Verfahren zum Herstellen einer Verbindung der allgemeinen Formel (I-a):

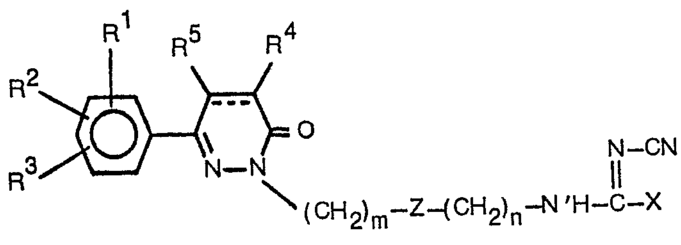

(I-a)

in der bedeuten:

$R^1$, $R^2$ und $R^3$ unabhängig voneinander ein Wasserstoffatom, ein Halogenatom, eine niedere Alkylgruppe oder eine niedere Alkoxygruppe;

$R^4$ und $R^5$ unabhängig voneinander ein Wasserstoffatom oder eine niedere Alkylgruppe;

m: eine ganze Zahl von 1 bis 3;

n: Null oder eine ganze Zahl von 1 bis 3;

Z: $-CH_2-$,

X: $-S-R^6$ worin $R^6$ eine niedere Alkylgruppe bedeutet; und worin die C-Atome in Stellung 4 und 5 des heterocyclischen Ringes der Formel (I-a), an die $R^4$ und $R^5$ gebunden ist, miteinander über eine Einfach oder Doppel-C-C-Bindung verbunden sind;

wobei die niedere Alkyl- oder niedere Alkoxygruppe bis zu sechs C-Atome enthält;

welches Verfahren die Reaktion einer Verbindung der Formel (II):

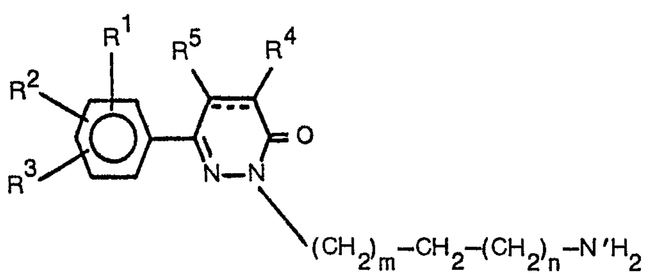

(II)

mit Dialkylcyan-dithioimidocarbonat umfaßt.

2. Verfahren zum Herstellen einer Verbindung der Formel (I-a) nach Anspruch 1, mit der Abweichung, daß X bedeutet $-N''-H-R^7$, wobei $R^7$ ein Wasserstoffatom, eine Alkylgruppe mit bis zu sechs Kohlenstoffatomen oder eine Propargylgruppe bedeutet, welches Verfahren die Reaktion einer Verbindung gemäß der Formel (II):

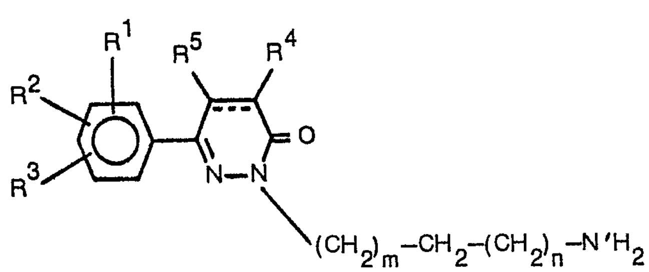

(II)

mit S-alkyl-N-cyan-N''-alkyl-isothioharnstoff umfaßt.

3. Verfahren zum Herstellen einer Verbindung der Formel (I-a) nach Anspruch 1 mit der Abweichung, daß X bedeutet $-N''-H-R^7$ ($R^7$ wie definiert in Anspruch 2), welches Verfahren die Reaktion einer Verbindung der Formel (I-a) nach Anspruch 1 mit Ammoniak oder einem Alkylamin mit bis zu sechs Kohlenstoffatomen umfaßt.

4. Verfahren zum Herstellen einer Verbindung gemäß der allgemeinen Formel (I-c):

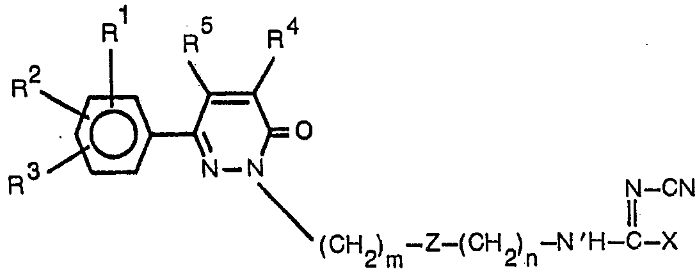

(I-c)

worin bedeuten:

$R^1$, $R^2$ und $R^3$ unabhängig voneinander ein Wasserstoffatom, ein Halogenatom, eine niedere Alkylgruppe oder eine niedere Alkoxygruppe;

$R^4$ und $R^5$ unabhängig voneinander ein Wasserstoffatom oder eine niedere Alkylgruppe;

m: eine ganze Zahl von 1 bis 3;

n: eine ganze Zahl von 1 bis 3;

z: —O—;

x: —S—$R^6$, wobei $R^6$ eine niedere Alkylgruppe bedeutet und wobei die niedere Alkyl- und niedere Alkoxygruppe bis zu sechs Kohlenstoffatome enthalten; welches Verfahren die Reaktion einer Verbindung gemäß der Formel (VI):

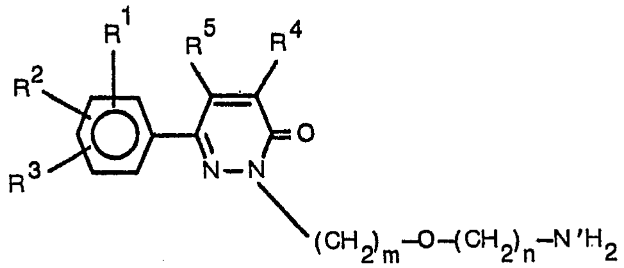

(VI)

mit Dialkylcyan-dithioimidocarbonat umfaßt.

5. Verfahren zum Herstellen einer Verbindung der Formel (I-c) nach Anspruch 4 mit der Abweichung, daß X —N″—H—$R^7$ bedeutet, $R^7$ wie definiert in Anspruch 2, welches Verfahren die Reaktion einer Verbindung nach der Formel (VI):

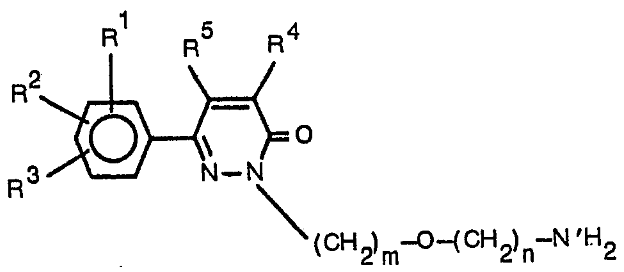

(VI)

mit S-alkyl-N-cyan-N″-alkyl-isothioharnstoff umfaßt.

6. Verfahren zum Herstellen einer Verbindung gemäß Formel (I-c) nach Anspruch 4 mit der Abweichung, daß X bedeutet —N″—H—$R^7$, $R^7$ wie definiert in Anspruch 2, welches Verfahren die Reaktion einer Verbindung der Formel (I-c) nach Anspruch 4 mit Ammoniak oder Alkylamin mit bis zu sechs Kohlenstoffatomen umfaßt.

7. Verfahren zum Herstellen einer Verbindung gemäß der allgemeinen Formel (I-e):

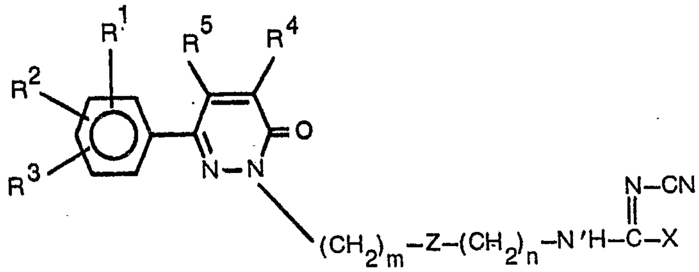

(I-e)

worin bedeuten:

$R^1$, $R^2$ und $R^3$ unabhängig voneinander ein Wasserstoffatom, ein Halogenatom, eine niedere Alkylgruppe oder eine niedere Alkoxygruppe;

$R^4$ und $R^5$ unabhängig voneinander ein Wasserstoffatom oder eine niedere Alkylgruppe;

m: eine ganze Zahl von 1 bis 3;

n: eine ganze Zahl von 1 bis 3;

z: —S—;

x: —S—$R^6$, wobei $R^6$ eine niedere Alkylgruppe bedeutet und die niedere Alkyl- und niedere Alkoxygruppe bis zu sechs Kohlenstoffatome aufweisen;

welches Verfahren die Reaktion einer Verbindung gemäß der Formel (VII):

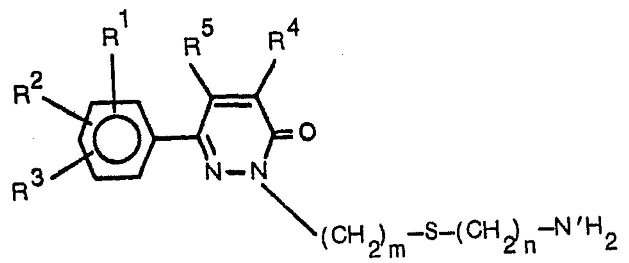

(VII)

mit Dialkylcyan-dithioimidocarbonat umfaßt.

8. Verfahren zum Herstellen einer Verbindung der Formel (I-e) nach Anspruch 7 mit der Abweichung, daß x —NB''—H—$R^7$ bedeutet, $R^7$ wie definiert in Anspruch 2, welches Verfahren die Reaktion einer Verbindung gemäß der Formel (VII):

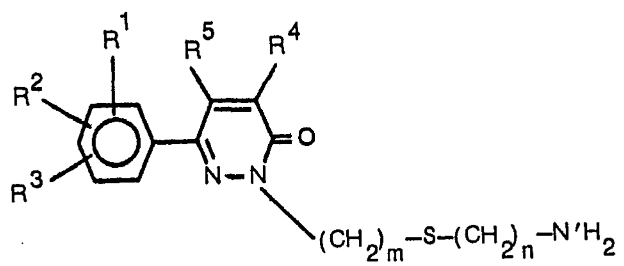

(VII)

mit S-alkyl-N-cyan-N''-alkyl-isothioharnstoff umfaßt.

9. Verfahren zum Herstellen F einer Verbindung der Formel (I-e) gemäß Anspruch 7 mit der Abweichung, daß x —N''—H—$R^7$ bedeutet, $R^7$ wie definiert in Anspruch 2, welches Verfahren die Reaktion einer Verbindung der Formel (I-e) gemäß Anspruch 7 mit Ammoniak oder einem Alkylamin mit bis zu sechs Kohlenstoffatomen umfaßt.

**Revendications pour les Etats contractants: BE CH DE FR GB IT LI NL**

1. Un composé représenté par la formule générale (I):

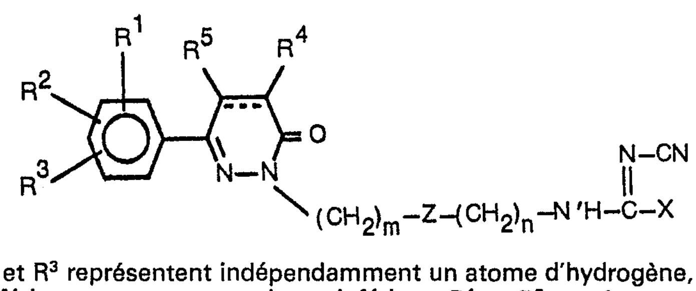

(I)

dans laquelle $R^1$, $R^2$ et $R^3$ représentent indépendamment un atome d'hydrogène, un atome d'halogène, un groupe alkyle inférieur ou un groupe alcoxy inférieur; $R^4$ et $R^5$ représentent indépendamment un atome d'hydrogène ou un groupe alkyle inférieur; m représente un nombre entier de 1 à 3; n représente zéro ou un nombre entier de 1 à 3; Z représente —$CH_2$—, —O— ou —S—; X représente —S—$R^6$ ou —N''H—$R^7$, où $R^6$ représente un groupe alkyle inférieur et $R^7$ représente un atome d'hydrogène, un groupe alkyle inférieur ou un groupe propargyle; et les atomes de carbone aux positions 4 et 5 du noyau hétérocyclique de formule (I) auxquels $R^4$ et $R^5$ sont respectivement fixés, sont liés soit par une simple liaison soit par une double liaison carbone-carbone; et leurs sels d'addition avec des acides; sous réserve que, lorsque Z représente —O— ou —S—, lesdits atomes de carbone aux positions 4 et 5 sont liés par une double liaison et n représente un nombre entier de 1 à 3; les groupes alkyle inférieurs

et alcoxy inférieurs contenant jusqu'à 6 atomes de carbone.

2. Un composé selon la revendication 1, dans lequel $R^1$, $R^2$ et $R^3$ représentent indépendamment un chlore, un méthyle ou un méthoxy.

3. Un composé selon la revendication 1 ou 2, dans lequel le groupe de formule:

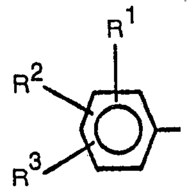

est un groupe phényle, 4-chlorophényle, 3,4-dichlorophényle, 4-méthoxyphényle, 3,4-diméthoxyphényle, 3,4,5-triméthoxyphényle, 4-méthylphényle, 5-méthylphényle ou 2,4-diméthylphényle.

4. Un composé selon l'une quelconque des revendications précédentes, où un de $R^4$ et $R^5$ est un groupe méthyle et l'autre est un hydrogène ou $R^4$ et $R^5$ représentent tous deux un hydrogène.

5. Un composé selon l'une quelconque des revendications précédentes, où $R^6$ est un groupe méthyle.

6. Un composé selon l'une quelconque des revendications 1 à 4, où $R^7$ est un groupe méthyle, éthyle ou propyle.

7. Un composé selon la revendication 1, qui est la N-cyano-N'-[4-(3-oxo-6-phényl-2-pyridazinyl)butyl]-N''-méthylguanidine.

8. Un composé selon la revendication 1, qui est la N-cyano-N'-[3-(3-oxo-6-phényl-2-pyridazinyl)propyl]-N''-éthylguanidine.

9. Un composé selon la revendication 1, qui est la N-cyano-N'-[4-(3-oxo-6-phényl-2-pyridazinyl)butyl]-N''-éthylguanidine.

10. Un composé selon la revendication 1, qui est la N-cyano-N'-[5-(3-oxo-6-phényl-2-pyridazinyl)pentyl]-N''-éthylguanidine.

11. Un composé selon la revendication 1, qui est la N-cyano-N'-[2-{(3-oxo-6-phényl-2-pyridazinyl)méthylthio}éthyl]-N''-méthylguanidine.

12. Un composé selon la revendication 1, qui est la N-cyano-N'[4-(4-méthyl-3-oxo-6-phényl-2-pyridazinyl)butyl]-N''-méthylguanidine.

13. Un composé selon la revendication 1, qui est la N-cyano-N'-[4-{6-(4-chlorophényl)-3-oxo-2-pyridazinyl}butyl]-N''-méthylguanidine.

14. Un composé selon la revendication 1, qui est la N-cyano-N'-[4-{6-(4-méthylphényl)-3-oxo-2-pyridazinyl}butyl]-N''-méthylguanidine.

15. Un procédé de préparation d'un composé de formule (I) tel que défini à la revendication 1, dans lequel Z représente —$CH_2$— et X représente —S—$R^6$, $R^6$ étant tel que défini à la revendication 1, ce procédé comprenant la réaction d'un composé représenté par la formule (II):

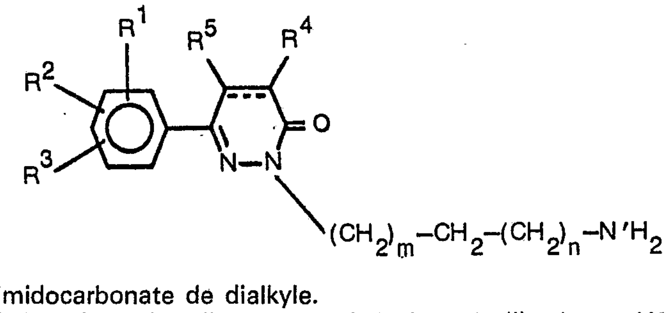

(II)

avec un cyanodithioimidocarbonate de dialkyle.

16. Un procédé de préparation d'un composé de formule (I) tel que défini à la revendication 1, dans lequel Z représente —$CH_2$— et X représente —N''H—$R^7$, $R^7$ étant tel que défini à la revendication 1, ce procédé comprenant la réaction d'un composé représenté par la formule (II):

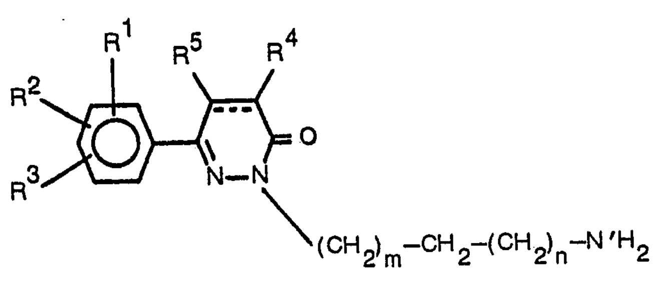

(II)

avec une S-alkyl-N-cyano-N″-alkyl-isothiourée.

17. Un procédé de préparation d'un composé de formule (I) tel que défini à la revendication 1, dans lequel Z représente —$CH_2$— et X représente —N″H—$R^7$, $R^7$ étant tel que défini à la revendication 1, ce procédé comprenant la réaction d'un composé de formule (I) tel que défini à la revendication 1, dans lequel Z représente —$CH_2$— et X représente —S—$R^6$, $R^6$ étant tel que défini à la revendication 1, avec l'ammoniac ou une alkylamine contenant jusqu'à 6 atomes de carbone.

18. Un procédé de préparation d'un composé de formule (I) tel que défini à la revendication 1, dans lequel Z représente —O— et X représente —S—$R^6$, $R^6$ étant tel que défini à la revendication 1, ce procédé comprenant la réaction d'un composé représenté par la formule (VI):

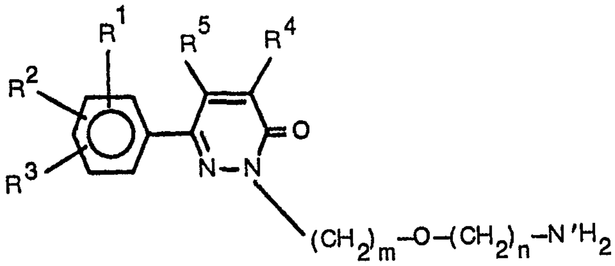

(VI)

avec un cyanodithioimidocarbonate de dialkyle.

19. Un procédé de préparation d'un composé de formule (I) tel que défini à la revendication 1, dans lequel Z représente —O— et X représente —N″H—$R^7$, $R^7$ étant tel que défini à la revendication 1, ce procédé comprenant la réaction d'un composé représenté par la formule (VI):

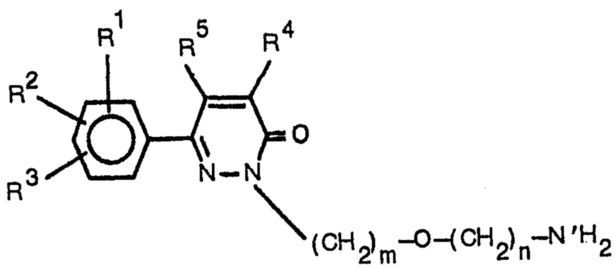

(VI)

avec une S-alkyl-N-cyano-N″-alkyl-isothiourée.

20. Un procédé de préparation d'un composé de formule (I) tel que défini à la revendication 1, dans lequel Z représente —O— et X représente —N″H—$R^7$, $R^7$ étant tel que défini à la revendication 1, ce procédé comprenant la réaction d'un composé de formule (I) tel que défini à la revendication 1, dans lequel Z représente —O— et X représente —S—$R^6$, $R^6$ étant tel que défini à la revendication 1, avec l'ammoniac ou une alkylamine contenant jusqu'à 6 atomes de carbone.

21. Un procédé de préparation d'un composé de formule (I) tel que défini à la revendication 1, dans lequel z représente —S— et X représente —S—$R^6$, $R^6$ étant tel que défini à la revendication 1, ce procédé comprenant la réaction d'un composé représenté par la formule (VII):

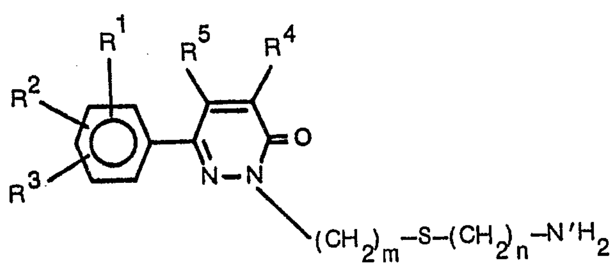

(VII)

avec un cyanodithioimidocarbonate de dialkyle.

22. Un procédé de préparation d'un composé de formule (I) tel que défini à la revendication 1, dans lequel Z représente —S— et X représente —N″H—$R^7$, $R^7$ étant tel que défini à la revendication 1, ce procédé comprenant la réaction d'un composé représenté par la formule (VII):

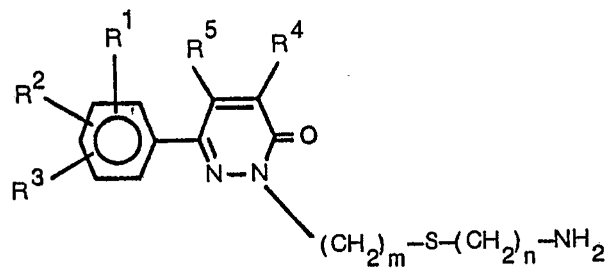

(VII)

avec une S-alkyl-N-cyano-N″-alkyl-isothiourée.

23. Un procédé de préparation d'un composé de formule (I) tel que défini à la revendication 1, dans lequel Z représente —S— et X représente —N″H—$R^7$, $R^7$ étant tel que défini à la revendication 1, ce procédé comprenant la réaction d'un composé de formule (I) tel que défini à la revendication 1, dans lequel Z représente —S— et X représente —S—$R^6$, $R^6$ étant tel que défini à la revendication 1, avec l'ammoniac ou une alkylamine contenant jusqu'à 6 atomes de carbone.

24. Une composition pharmaceutique comprenant comme ingrédient actif un composé tel que revendiqué à l'une quelconque des revendications 1 à 14 ou qui a été préparé par un procédé tel que revendiqué à l'une quelconque des revendications 15 à 23, avec un véhicule ou adjuvant pharmaceutiquement acceptable.

**Revendications pour l'Etat contractant: AT**

1. Un procédé de préparation d'un composé représenté par la formule générale (I-a):

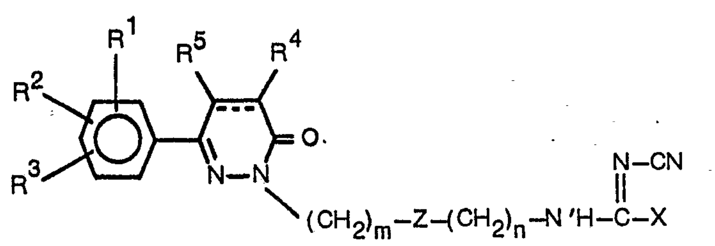

(I-a)

dans laquelle $R^1$, $R^2$ et $R^3$ représentent indépendamment un atome d'hydrogène, un atome d'halogène, un groupe alkyle inférieur ou un groupe alcoxy inférieur; $R^4$ et $R^5$ représentent indépendamment un atome d'hydrogène ou un groupe alkyle inférieur; m représente un nombre entier de 1 à 3; n représente zéro ou un nombre entier de 1 à 3; Z représente —$CH_2$—; X représente —S—$R^6$ où $R^6$ représente un groupe alkyle inférieur; et les atomes de carbone aux positions 4 et 5 du noyau hétérocyclique de la formule (I-a) auxquels $R^4$ et $R^5$ sont respectivement fixés, sont liés soit par une simple liaison soit par une double liaison carbone-carbone; les groupes alkyle inférieurs et alcoxy inférieurs contenant jusqu'à 6 atomes de carbone; ce procédé comprenant la réaction d'un composé représenté par la formule (II):

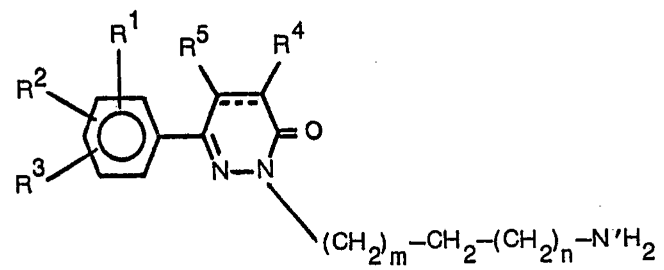

(II)

avec un cyanodithioimidocarbonate de dialkyle.

2. Un procédé de préparation d'un composé de formule (I-a) tel que défini à la revendication 1, si ce n'est que X représente —N″H—$R^7$ où $R^7$ représente un atome d'hydrogène, un groupe alkyle ayant jusqu'à 6 atomes de carbone ou un groupe propargyle, ce procédé comprenant la rèaction d'un composé représenté par la formule (II):

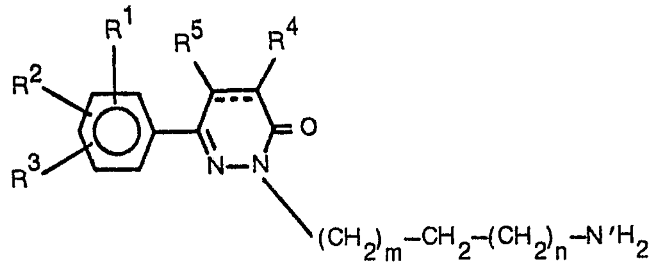

(II)

avec une S-alkyl-N-cyano-N″-alkyl-isothiourée.

3. Un procédé de préparation d'un composé de formule (I-a) tel que défini à la revendication 1, si ce n'est que X représente —NH″—$R^7$, $R^7$ étant tel que défini à la revendication 2, ce procédé comprenant la réaction d'un composé de formule (I-a) tel que défini à la revendication 1 avec l'ammoniac ou une alkylamine contenant jusqu'à 6 atomes de carbone.

4. Un procédé de préparation d'un composé représenté par la formule générale (I-c):

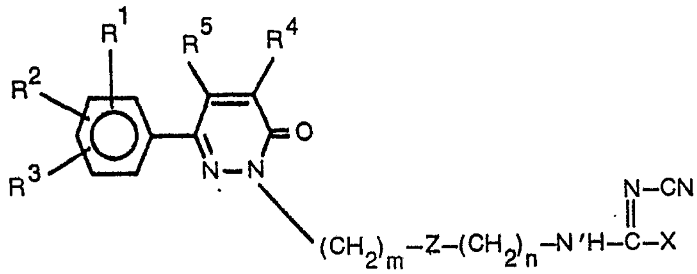

(I-c)

dans laquelle $R^1$, $R^2$ et $R^3$ représentent indépendamment un atome d'hydrogène, un atome d'halogène, un groupe alkyle inférieur ou un groupe alcoxy inférieur; $R^4$ et $R^5$ représentent indépendamment un atome d'hydrogène ou un groupe alkyle inférieur; m représente un nombre entier de 1 à 3; n représente un nombre entier de 1 à 3; Z représente —O—; X représente —S—$R^6$ où $R^6$ représente un groupe alkyle inférieur, les groupes alkyle inférieurs et alcoxy inférieurs contenant jusqu'à 6 atomes de carbone; ce procédé comprenant la réaction d'un composé représenté par la formule (VI):

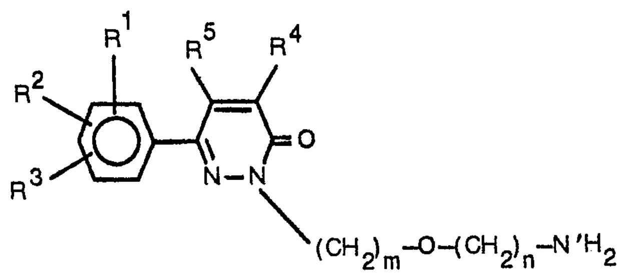

(VI)

avec un cyano-dithioimidocarbonate de dialkyle.

5. Un procédé de préparation d'un composé de formule (I-c) tel que défini à la revendication 4, si ce n'est que X représente —N″H—$R^7$, $R^7$ étant tel que défini à la revendication 2, ce procédé comprenant la réaction d'un composé représenté par la formule (VI):

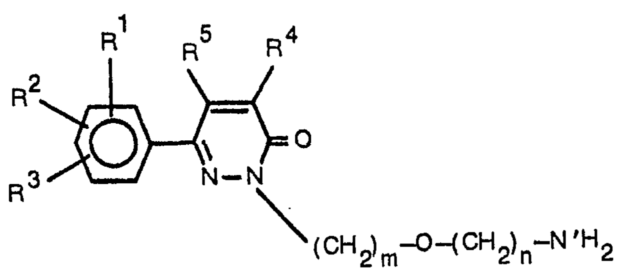

(VI)

avec une S-alkyl-N-cyano-N″-alkyl-isothiourée.

6. Un procédé de préparation d'un composé de formule (I-c) tel que défini à la revendication 4, si ce n'est que X représente —N″H—$R^7$, $R^7$ étant tel que défini à la revendication 2, ce procédé comprenant la réaction d'un composé de formule (I-c) tel que défini à la revendication 4, avec

l'ammoniac ou une alkylamine contenant jusqu'à 6 atomes de carbone.

7. Un procédé de préparation d'un composé représenté par la formule générale (I-e):

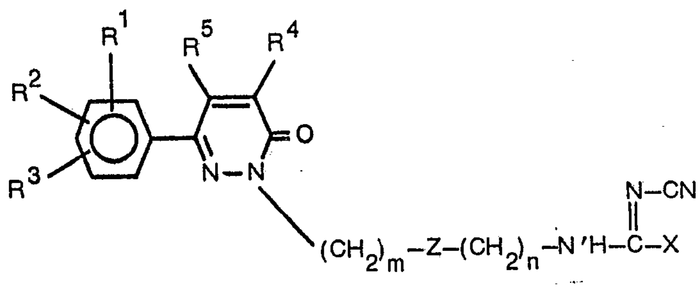

(I-e)

dans laquelle $R^1$, $R^2$ et $R^3$ représentent indépendamment un atome d'hydrogène, un atome d'halogène, un groupe alkyle inférieur ou un groupe alcoxy inférieur; $R^4$ et $R^5$ représentent indépendamment un atome d'hydrogène ou un groupe alkyle inférieur; m représente un nombre entier de 1 à 3; n représente un nombre entier de 1 à 3; Z représente —S—; X représente —S—$R^6$ où $R^6$ représente un groupe alkyle inférieur, les groupes alkyle inférieurs et alcoxy inférieurs contenant jusqu'à 6 atomes de carbone; ce procédé comprenant la réaction d'un composé représenté par la formule (VII):

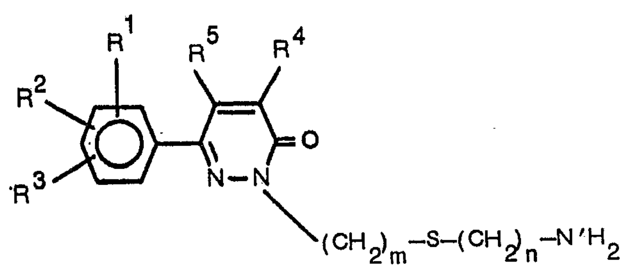

(VII)

avec un cyanodithioimidocarbonate de dialkyle.

8. Un procédé de préparation d'un composé de formule (I-e) tel que défini à la revendication 7, si ce n'est que X représente —N"H—$R^7$, $R^7$ étant tel que défini à la revendication 2, ce procédé comprenant la réaction d'un composé représenté par la formule (VII):

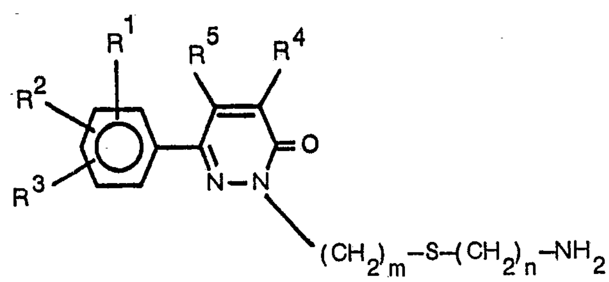

(VII)

avec une S-alkyl-N-cyano-N"-alkyl-isothiourée.

9. Un procédé de préparation d'un composé de formule (I-e) tel que défini à la revendication 7, si ce n'est que X représente —N"H—$R^7$, $R^7$ étant tel que défini à la revendication 2, ce procédé comprenant la réaction d'un composé de formule (I-e) tel que défini à la revendication 7, avec l'ammoniac ou une alkylamine contenant jusqu'à 6 atomes de carbone.

FIG. 1

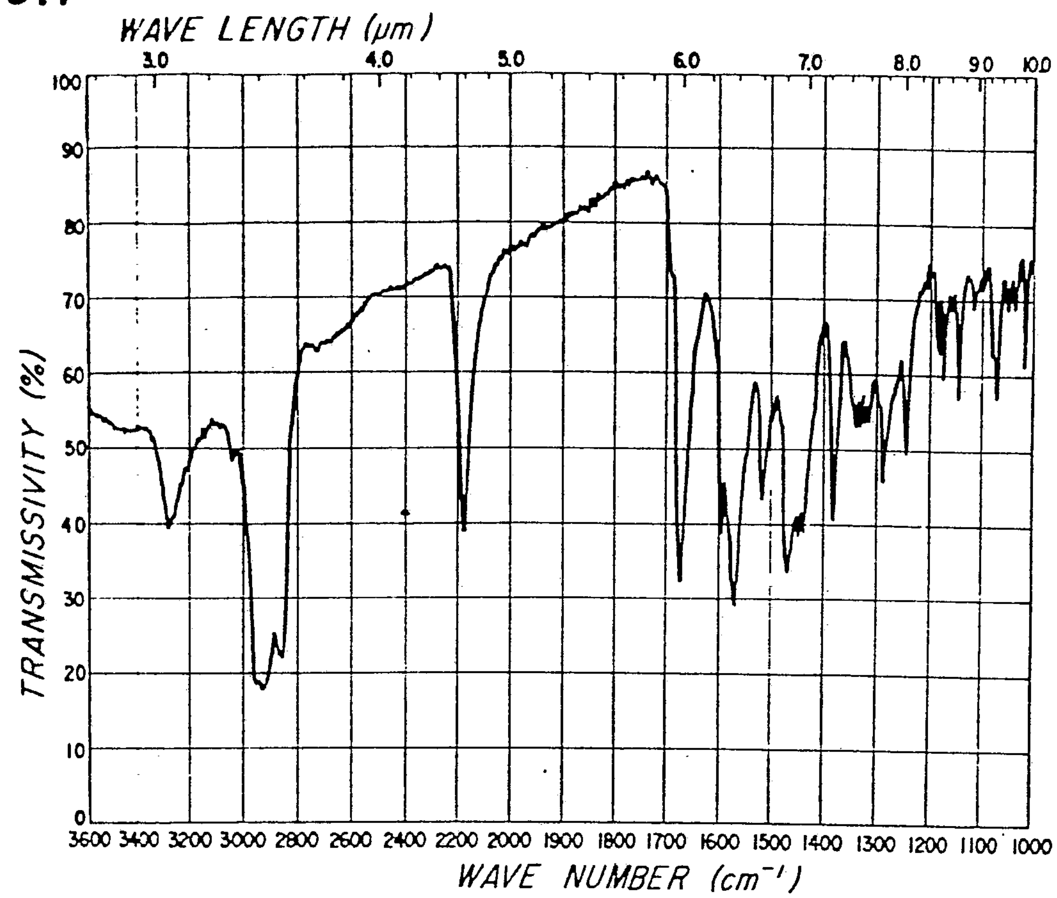
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG. 2

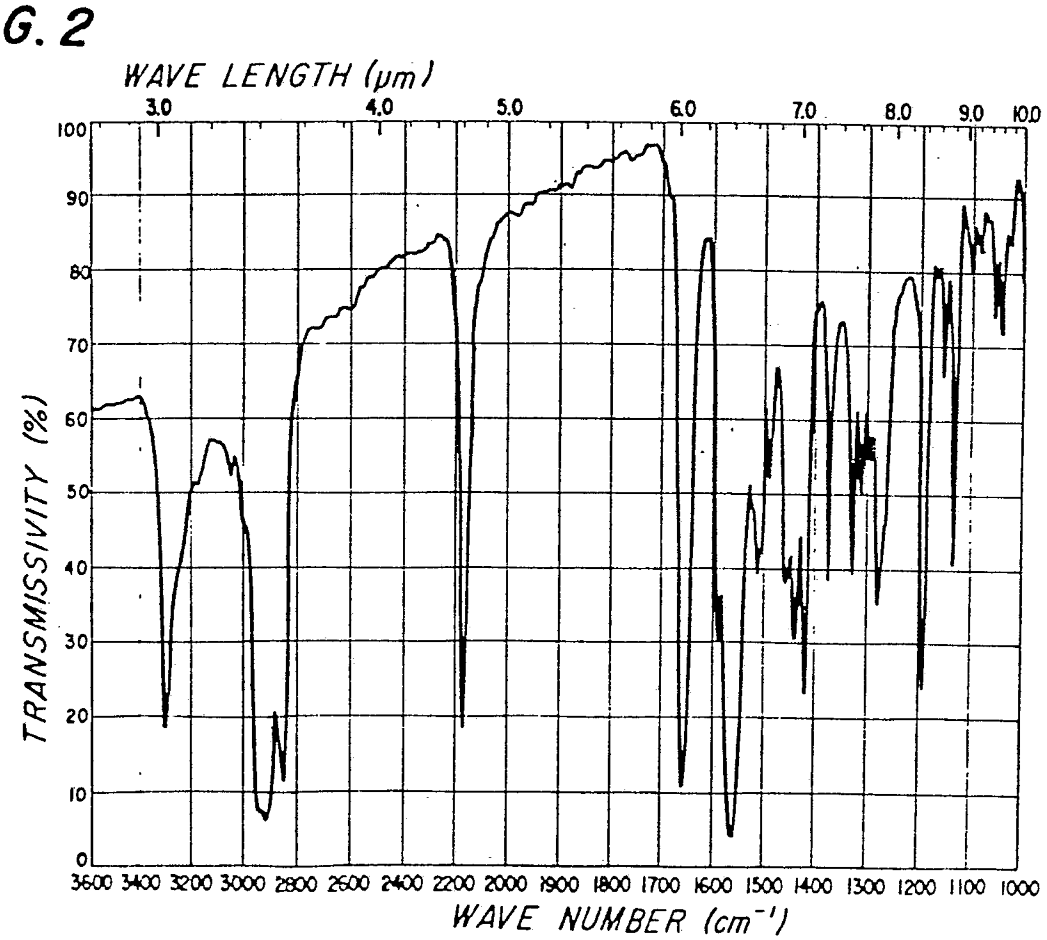
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG. 3

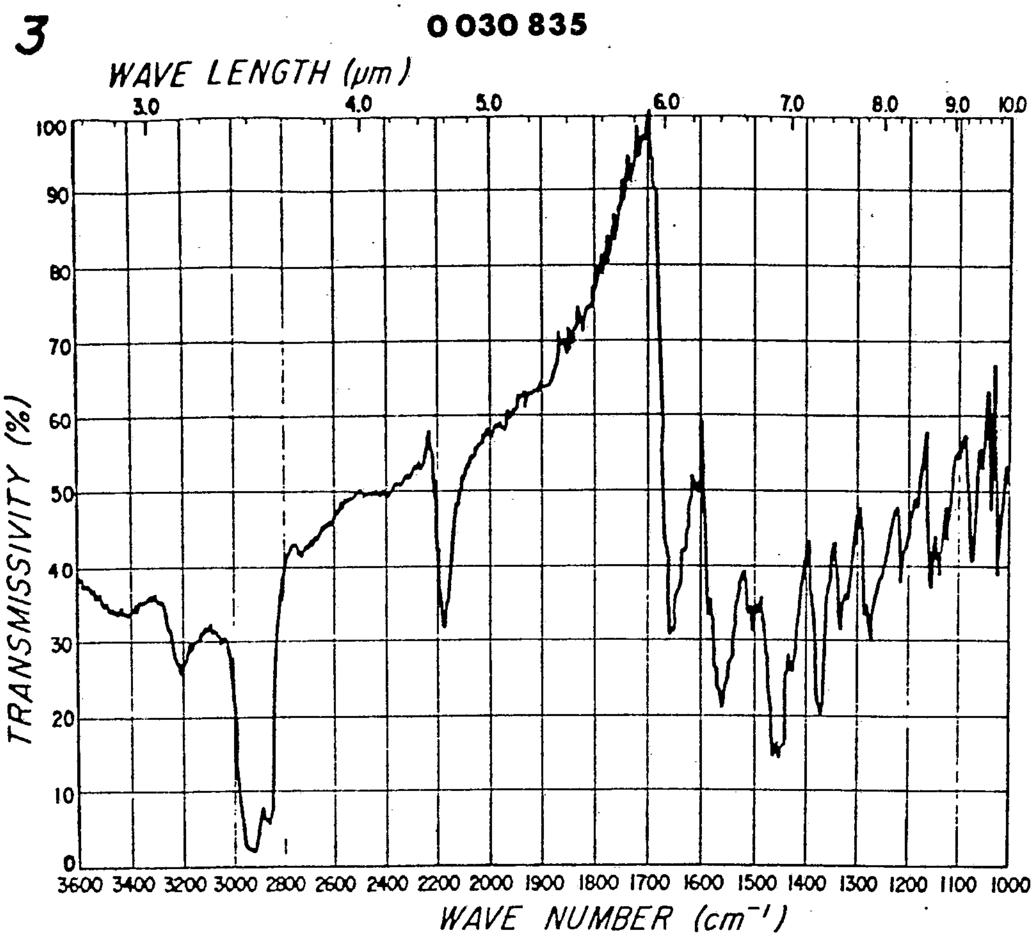
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 4

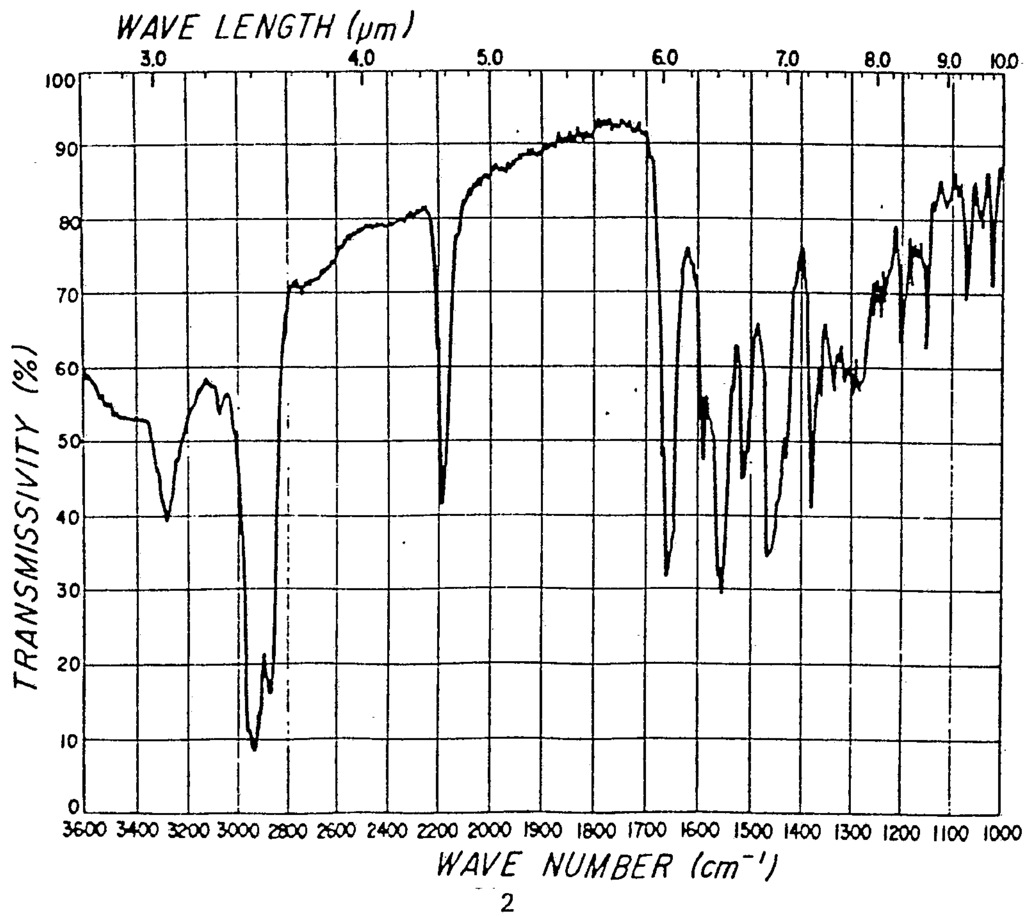
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 5

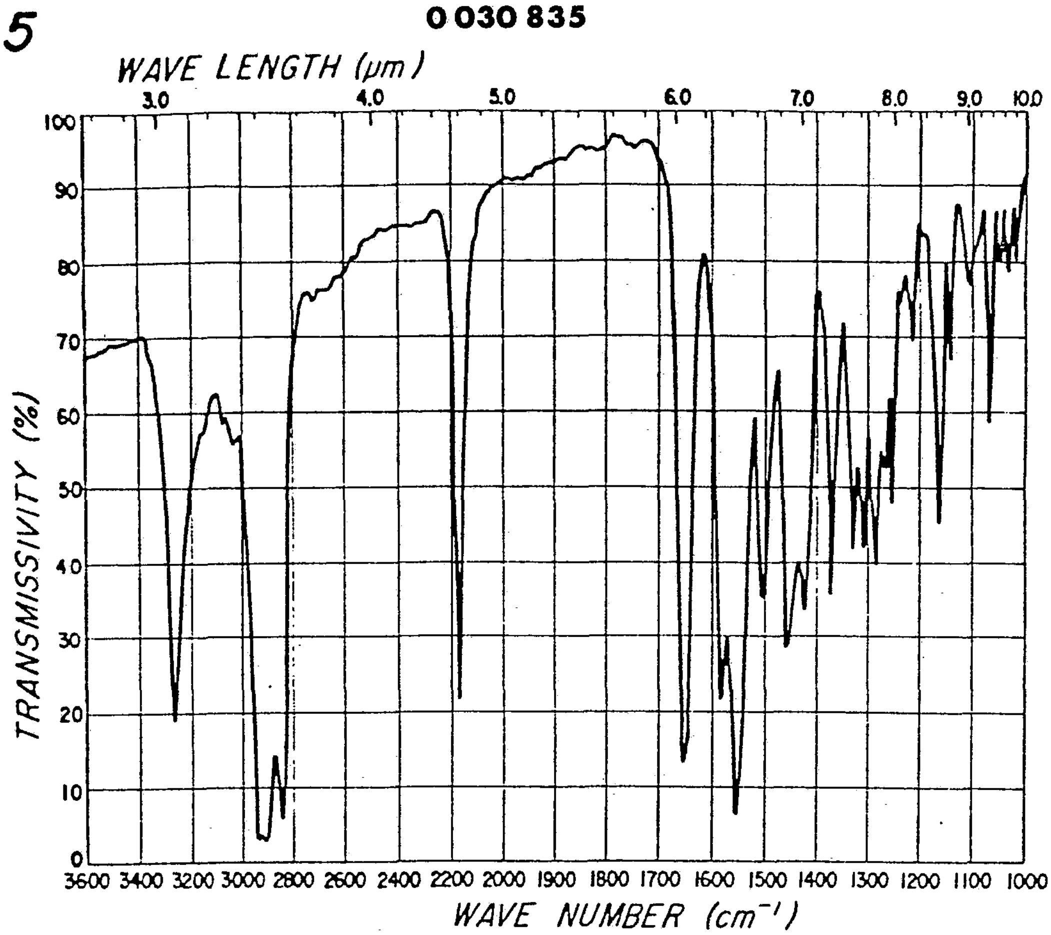
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 6

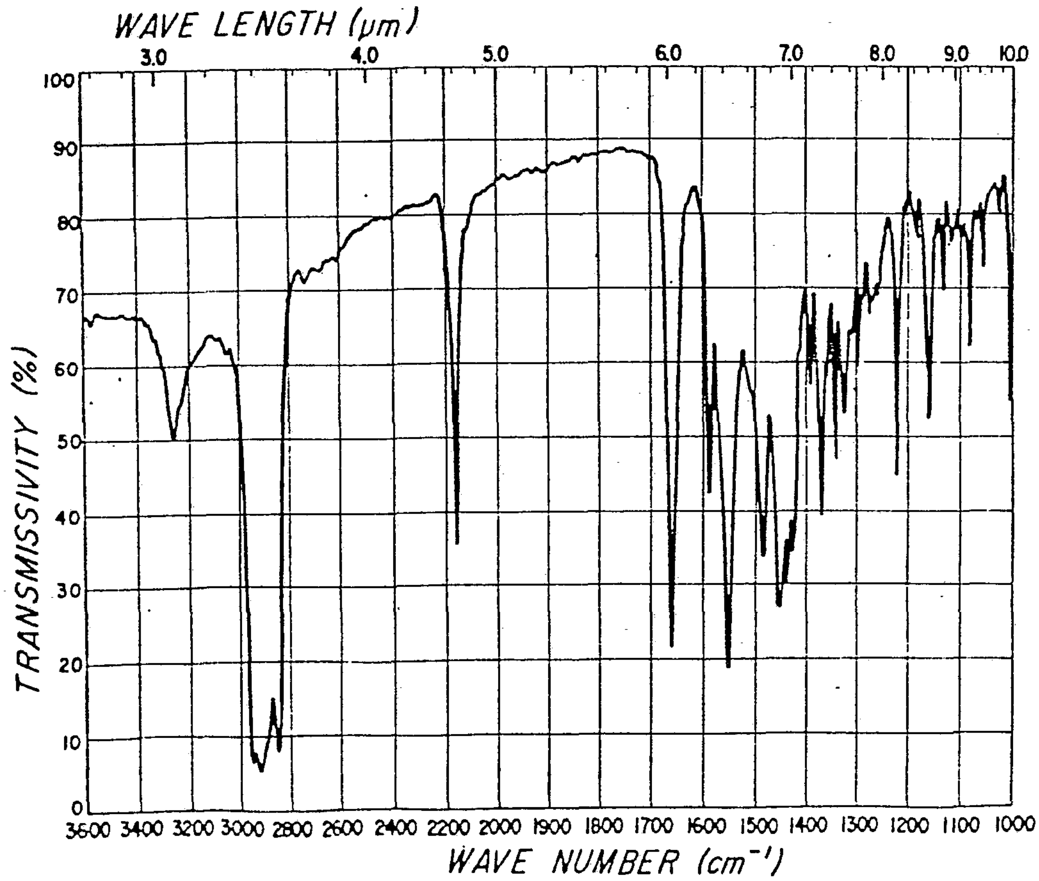
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 7

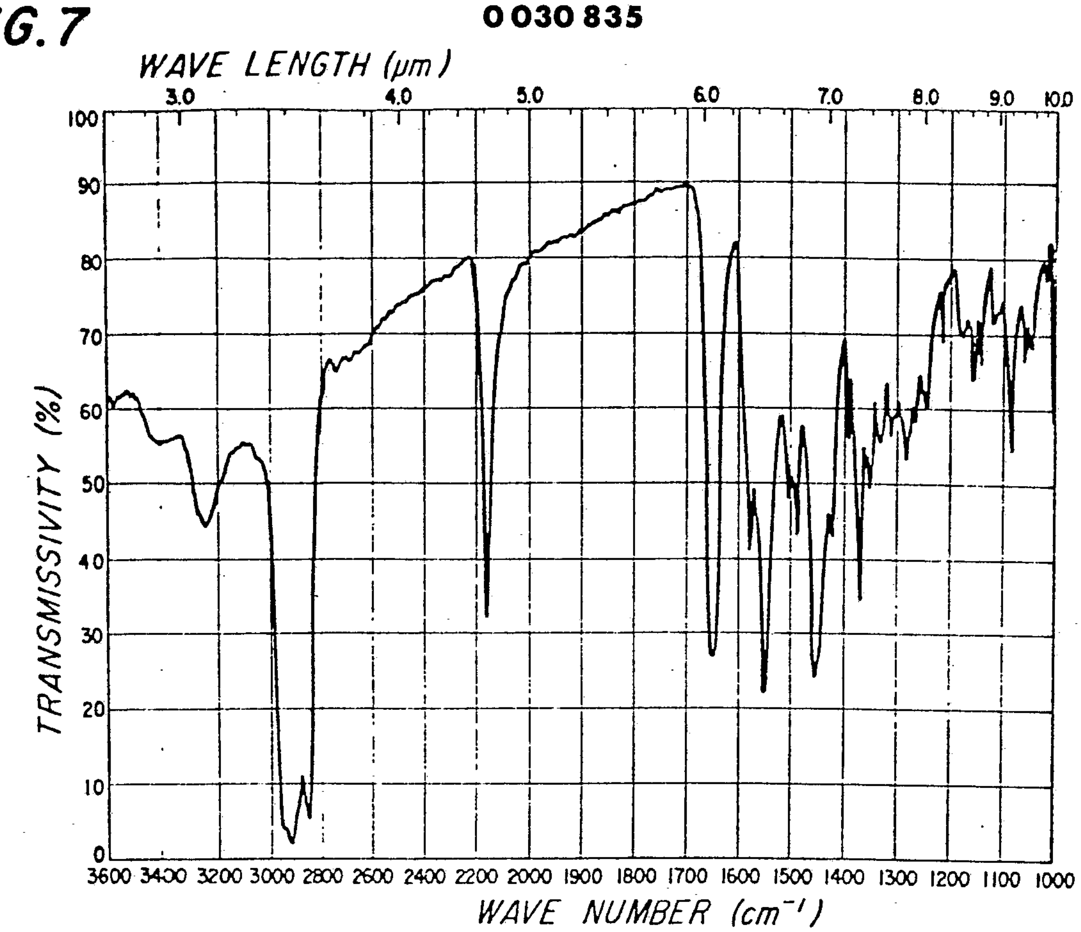
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 8

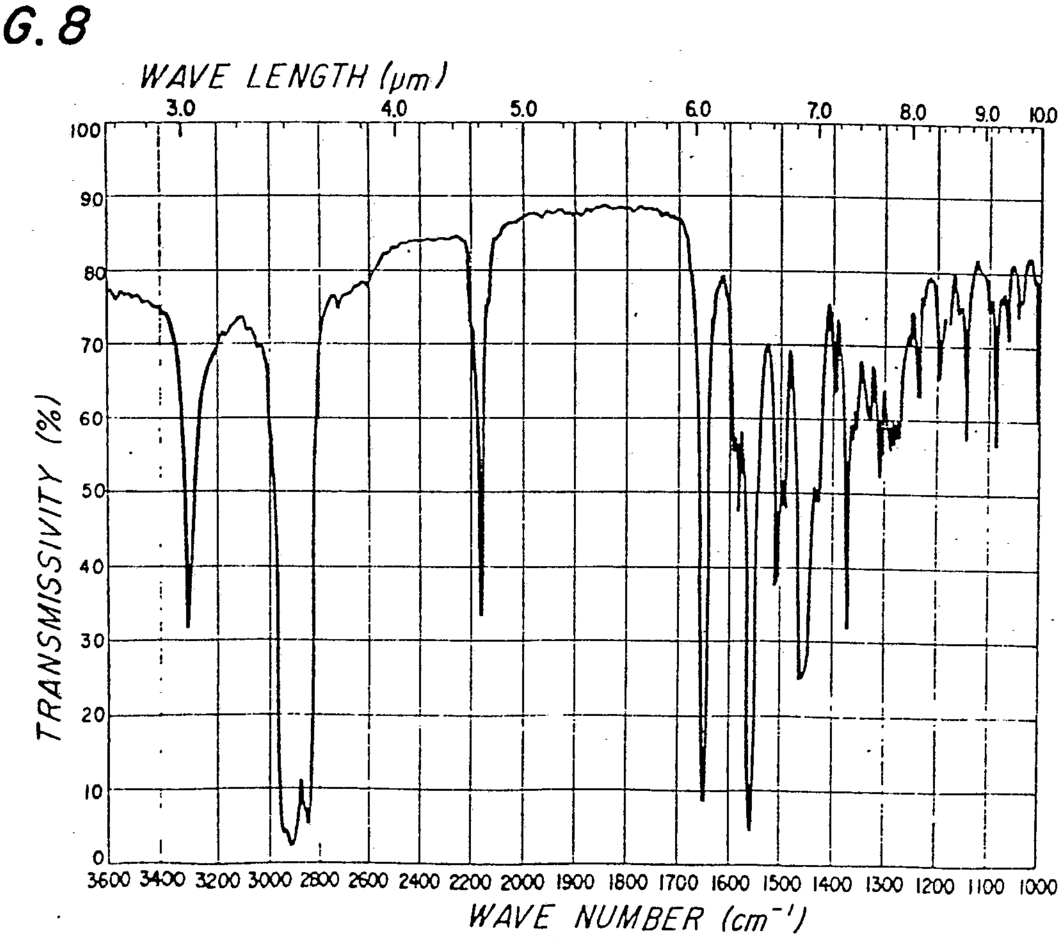
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 9

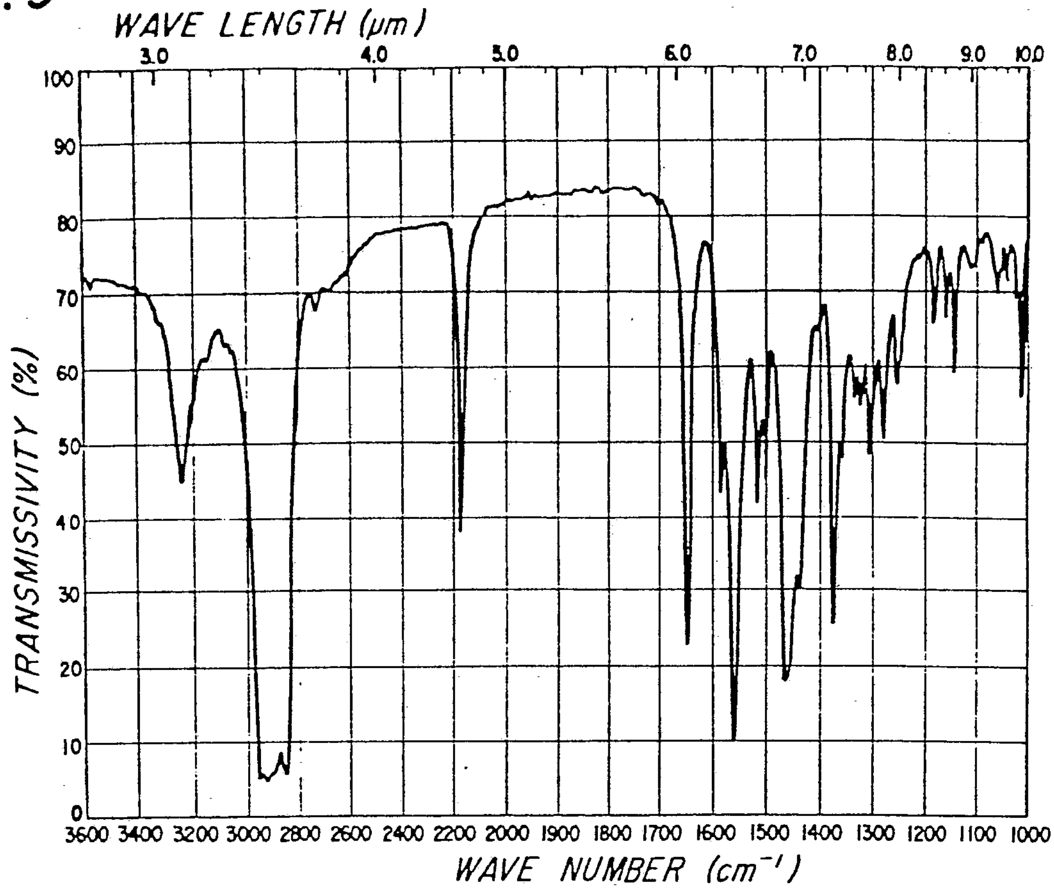
WAVE LENGTH (μm)
3.0 4.0 5.0 6.0 7.0 8.0 9.0 10.0
TRANSMISSIVITY (%)
100 90 80 70 60 50 40 30 20 10 0
3600 3400 3200 3000 2800 2600 2400 2200 2000 1900 1800 1700 1600 1500 1400 1300 1200 1100 1000
WAVE NUMBER (cm⁻¹)

FIG. 10

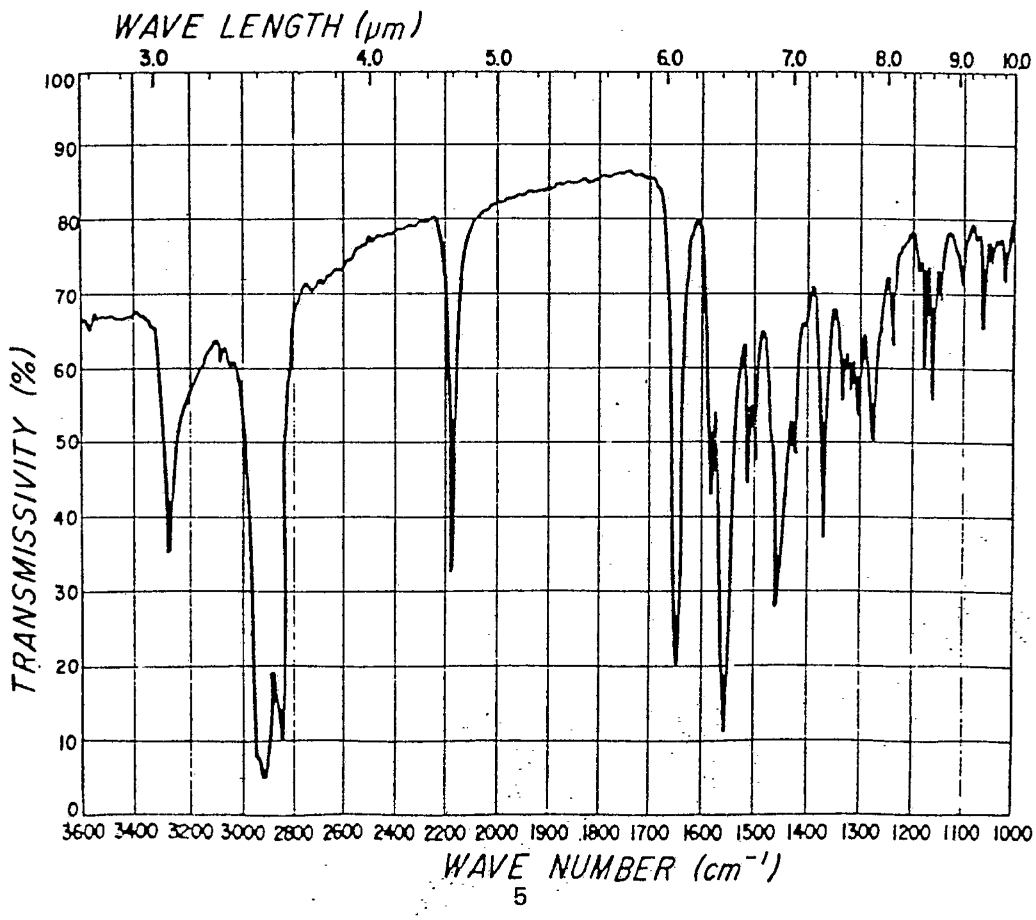
WAVE LENGTH (μm)
3.0 4.0 5.0 6.0 7.0 8.0 9.0 10.0
TRANSMISSIVITY (%)
100 90 80 70 60 50 40 30 20 10 0
3600 3400 3200 3000 2800 2600 2400 2200 2000 1900 1800 1700 1600 1500 1400 1300 1200 1100 1000
WAVE NUMBER (cm⁻¹)

FIG. 11

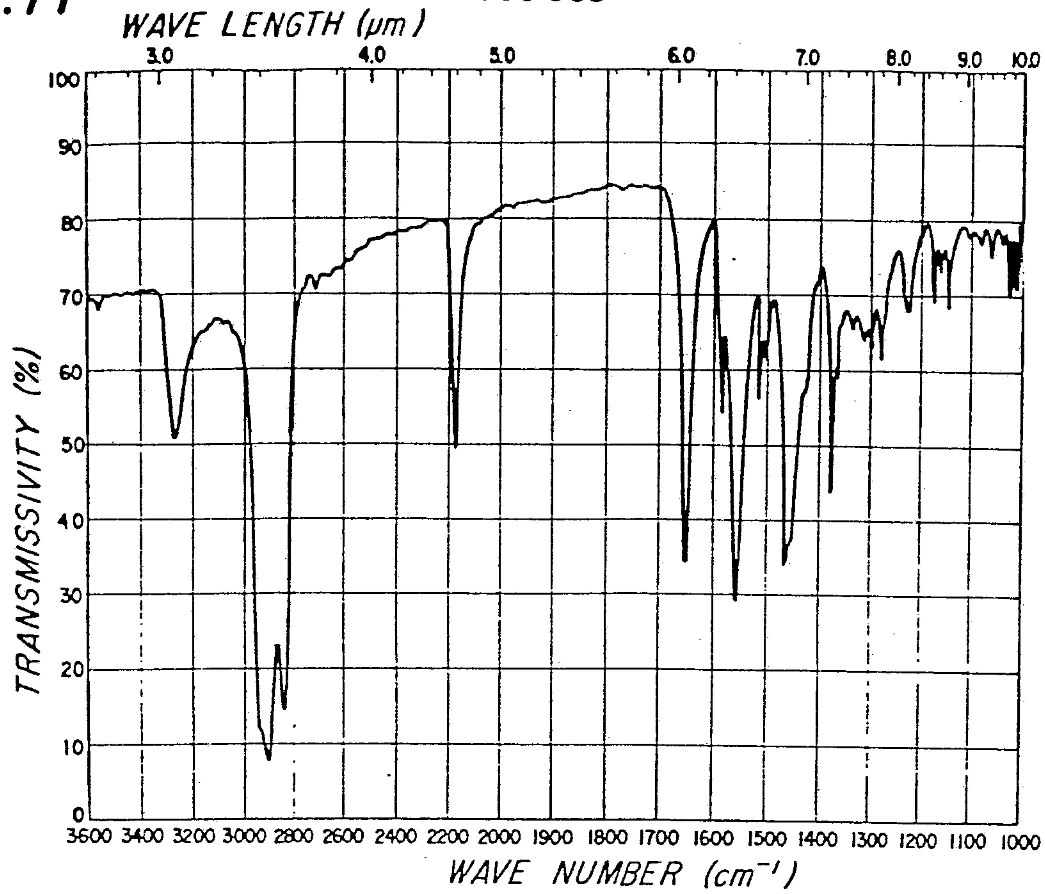
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 12

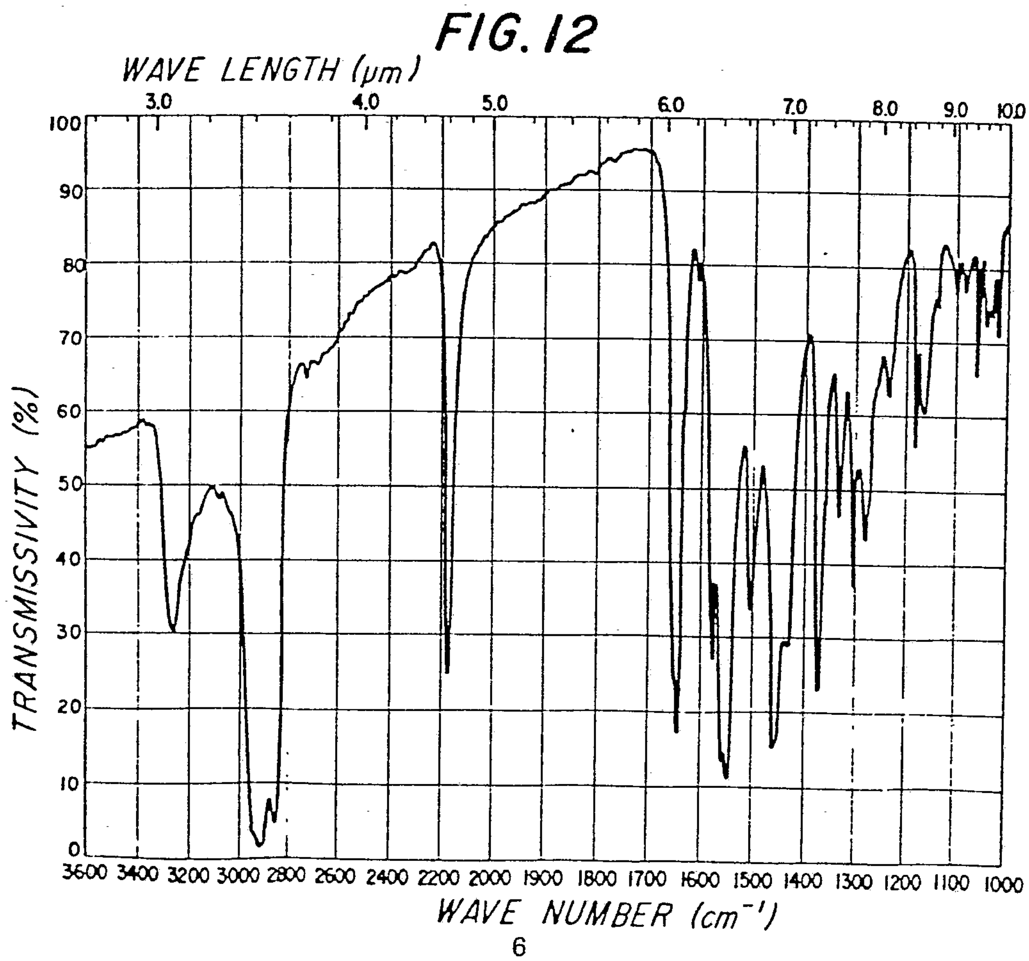
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 13

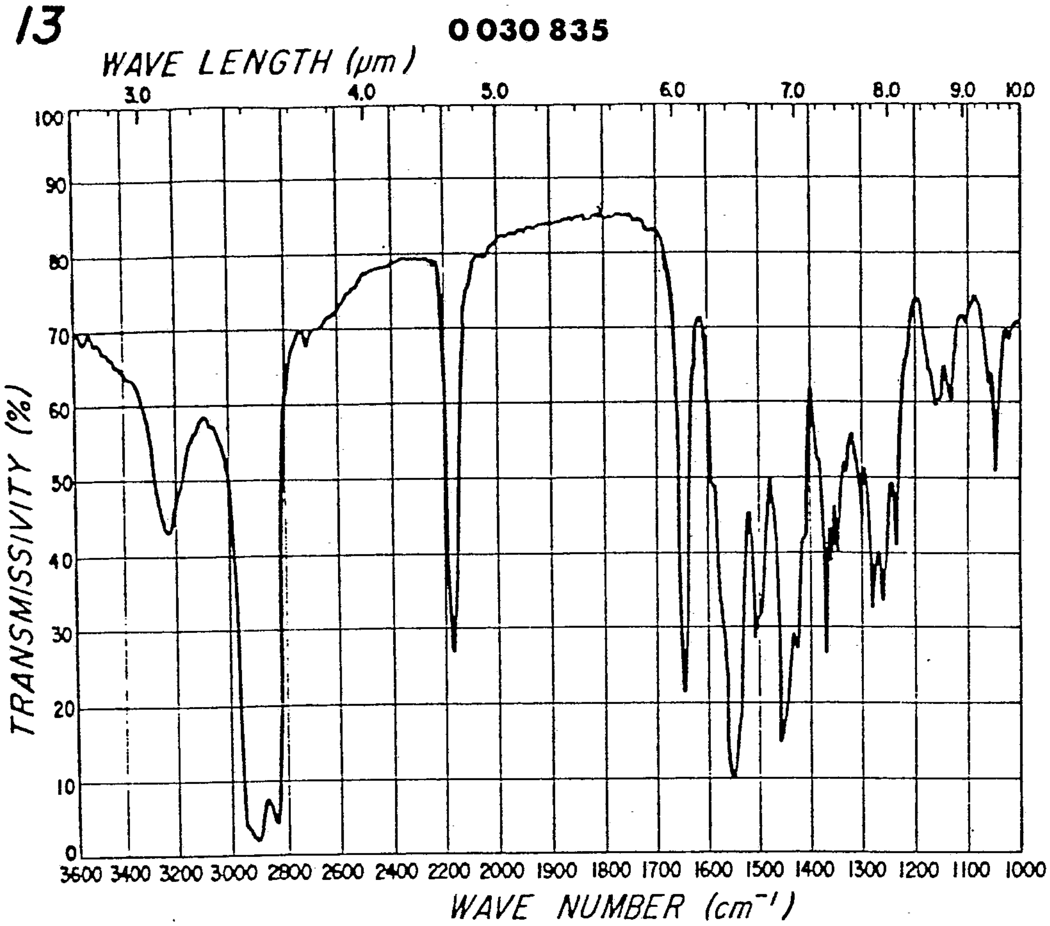
WAVE LENGTH (μm)
3.0 4.0 5.0 6.0 7.0 8.0 9.0 10.0
TRANSMISSIVITY (%)
100 90 80 70 60 50 40 30 20 10 0
3600 3400 3200 3000 2800 2600 2400 2200 2000 1900 1800 1700 1600 1500 1400 1300 1200 1100 1000
WAVE NUMBER (cm⁻¹)

FIG. 14

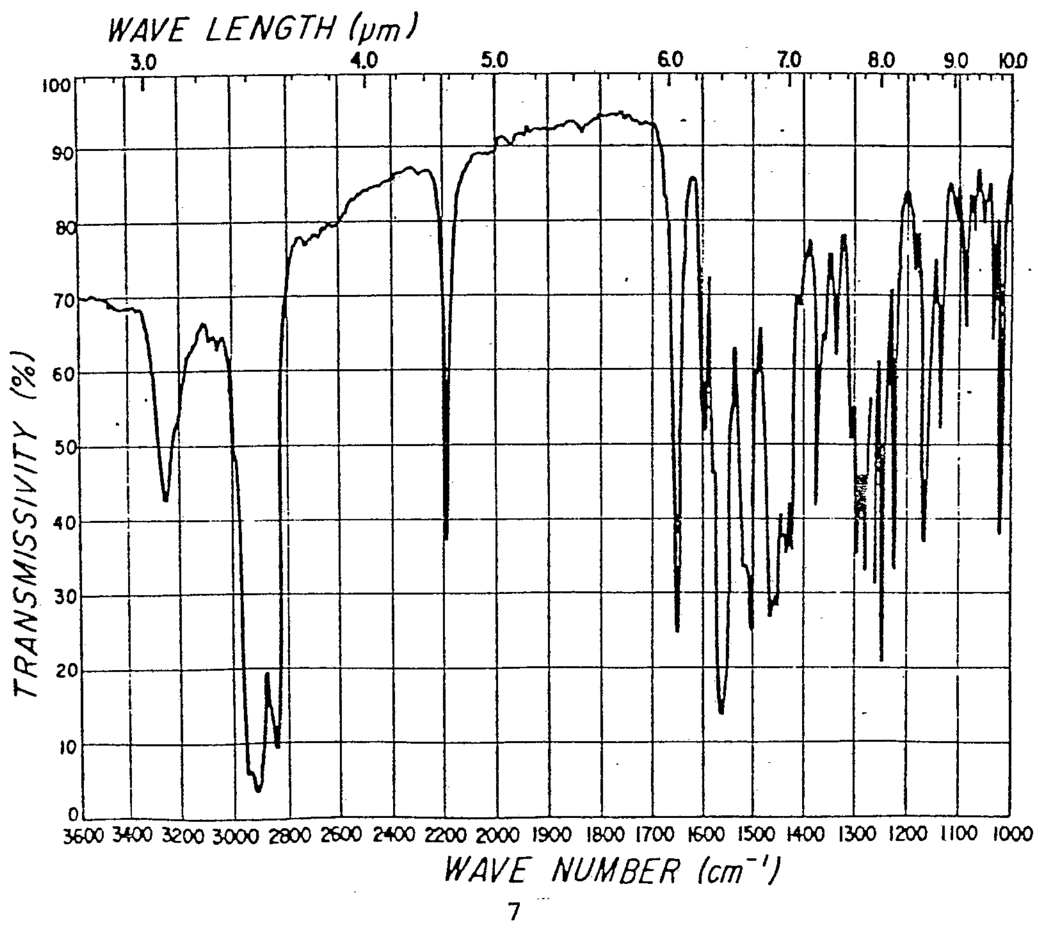
WAVE LENGTH (μm)
3.0 4.0 5.0 6.0 7.0 8.0 9.0 10.0
TRANSMISSIVITY (%)
100 90 80 70 60 50 40 30 20 10 0
3600 3400 3200 3000 2800 2600 2400 2200 2000 1900 1800 1700 1600 1500 1400 1300 1200 1100 1000
WAVE NUMBER (cm⁻¹)

FIG. 15

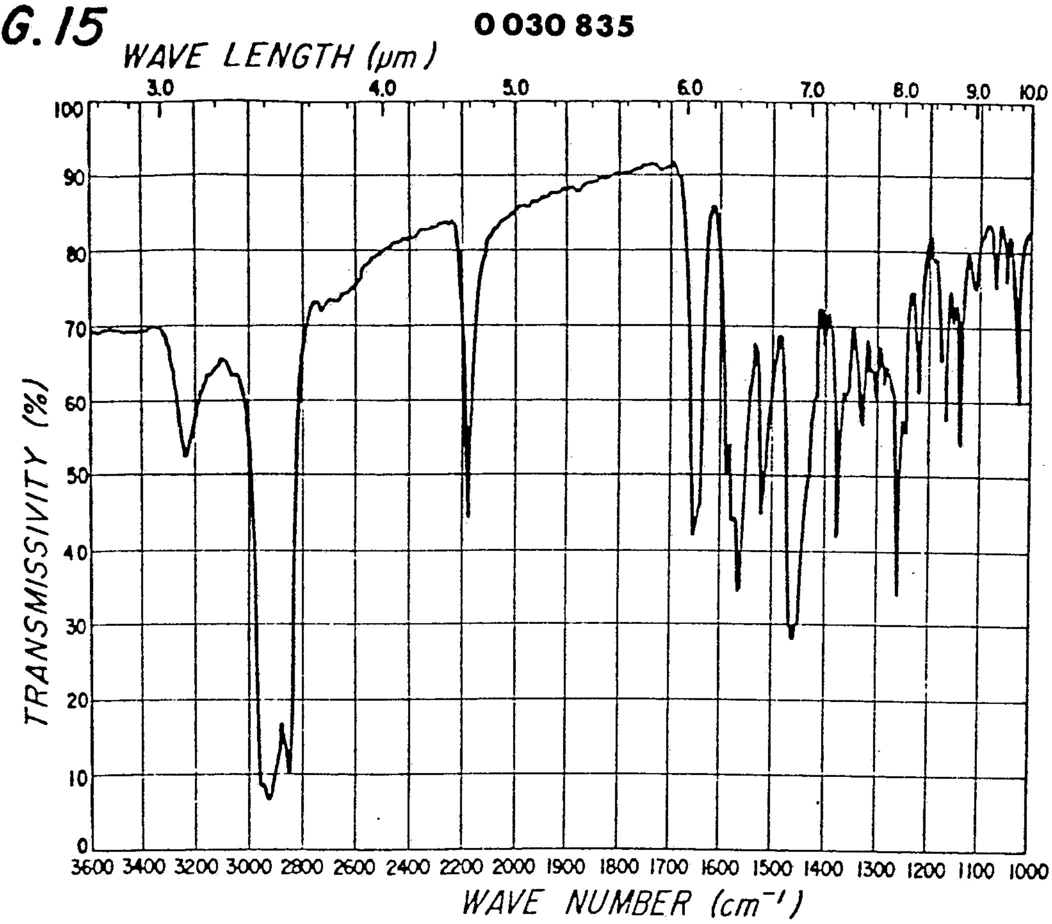
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 16

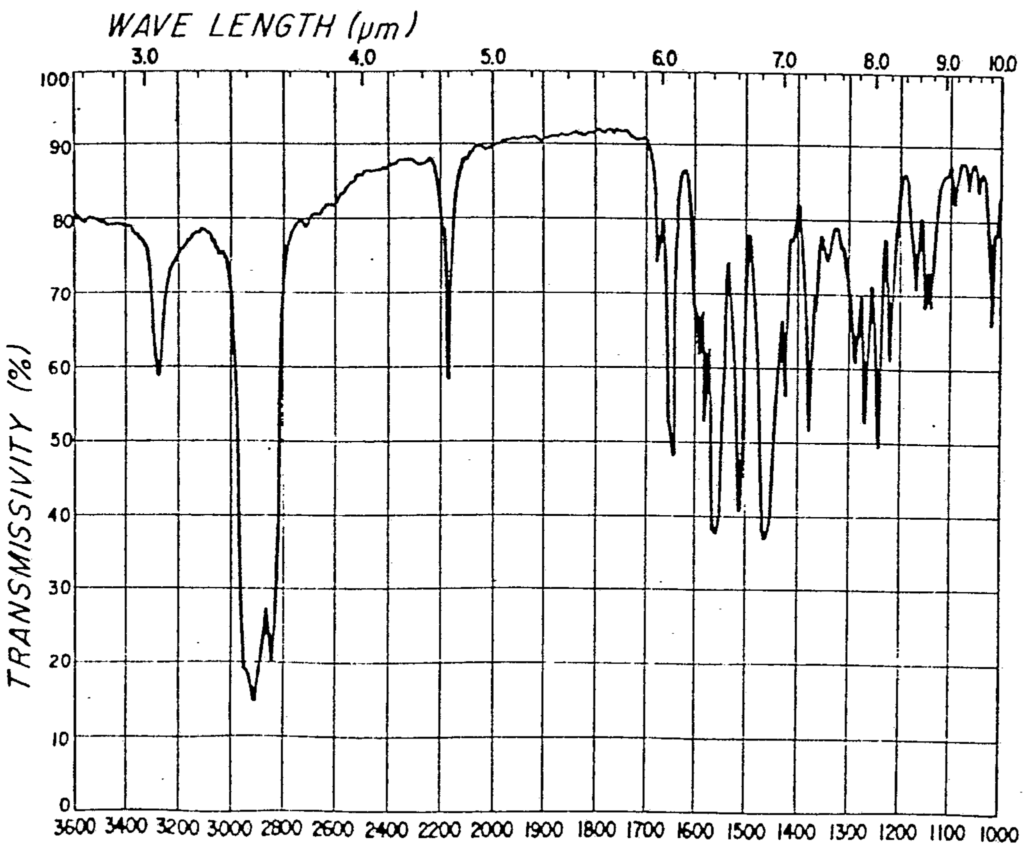
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 17

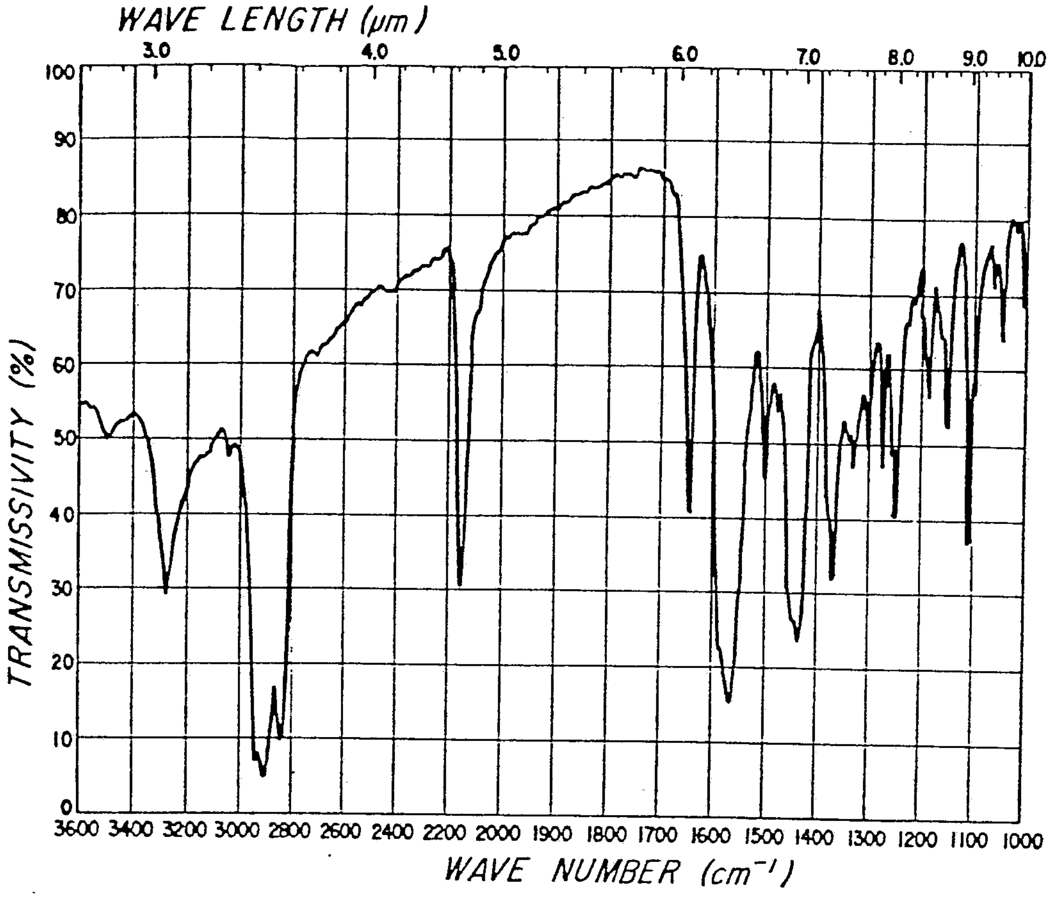
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER ($cm^{-1}$)

FIG. 18

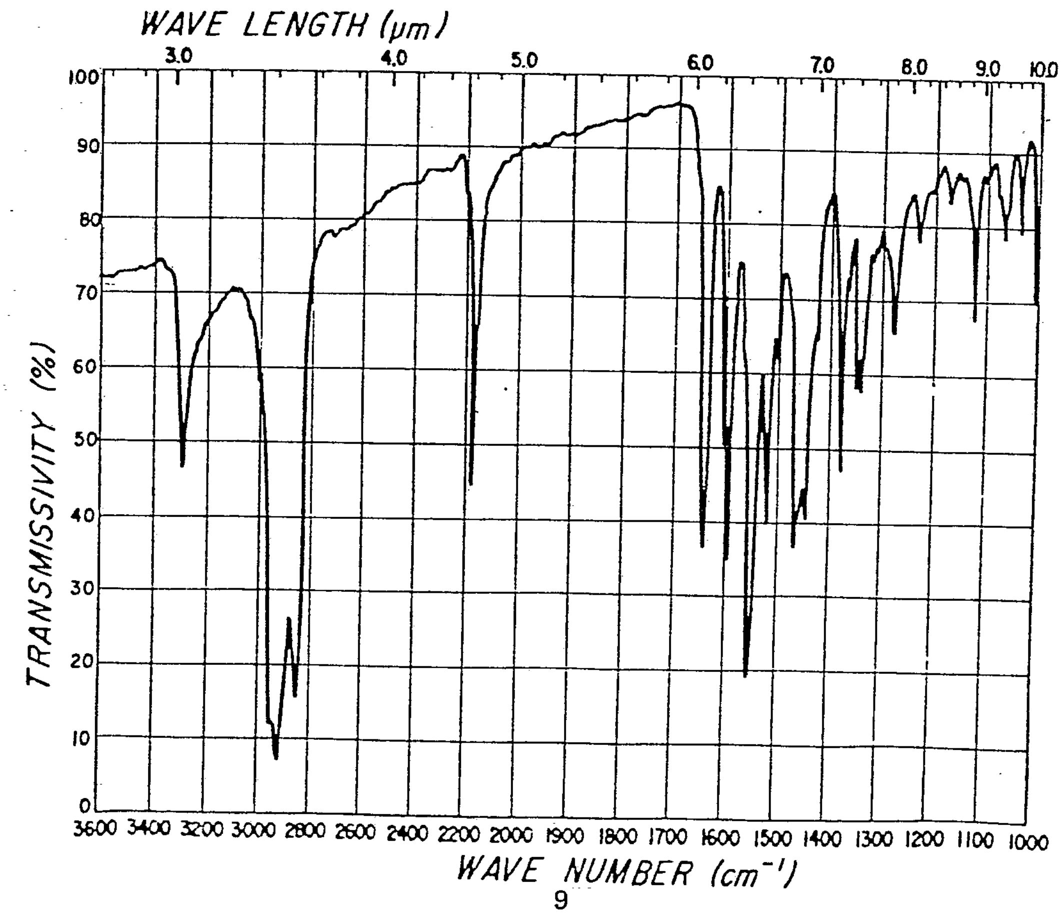
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER ($cm^{-1}$)

FIG. 19

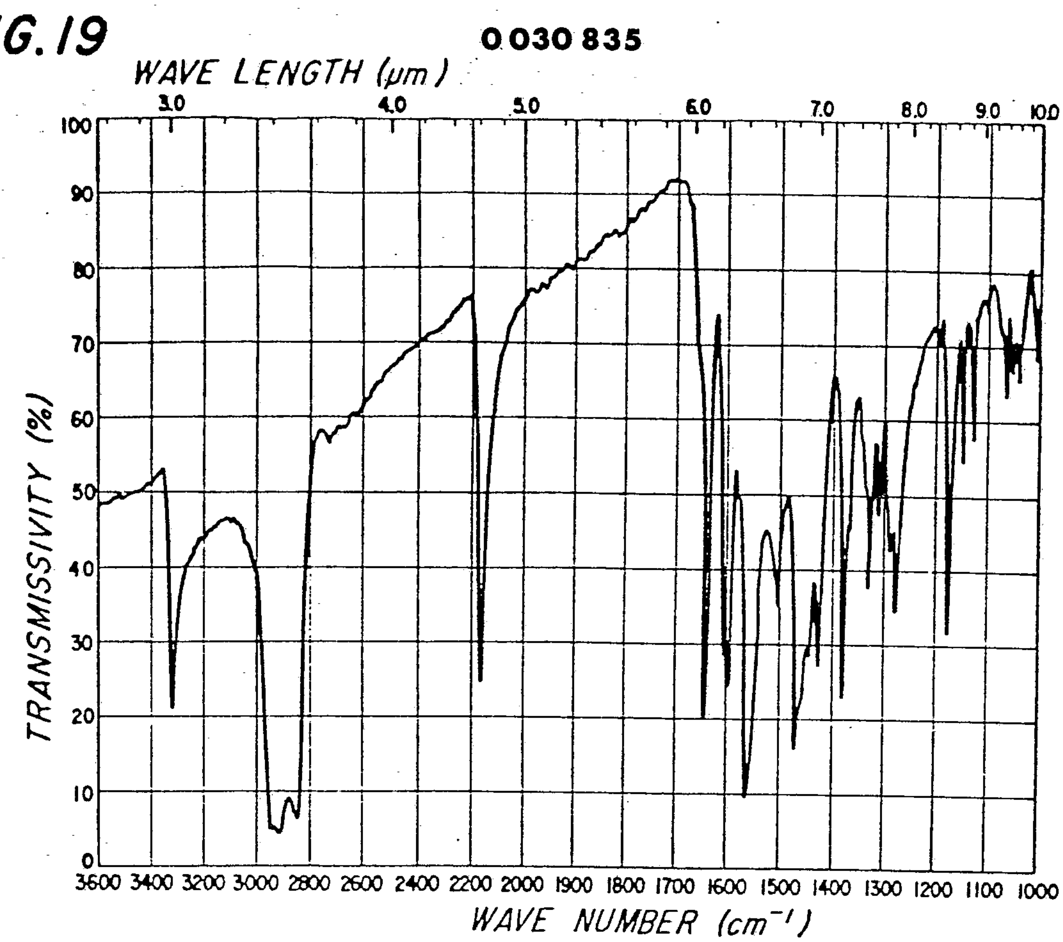
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 20

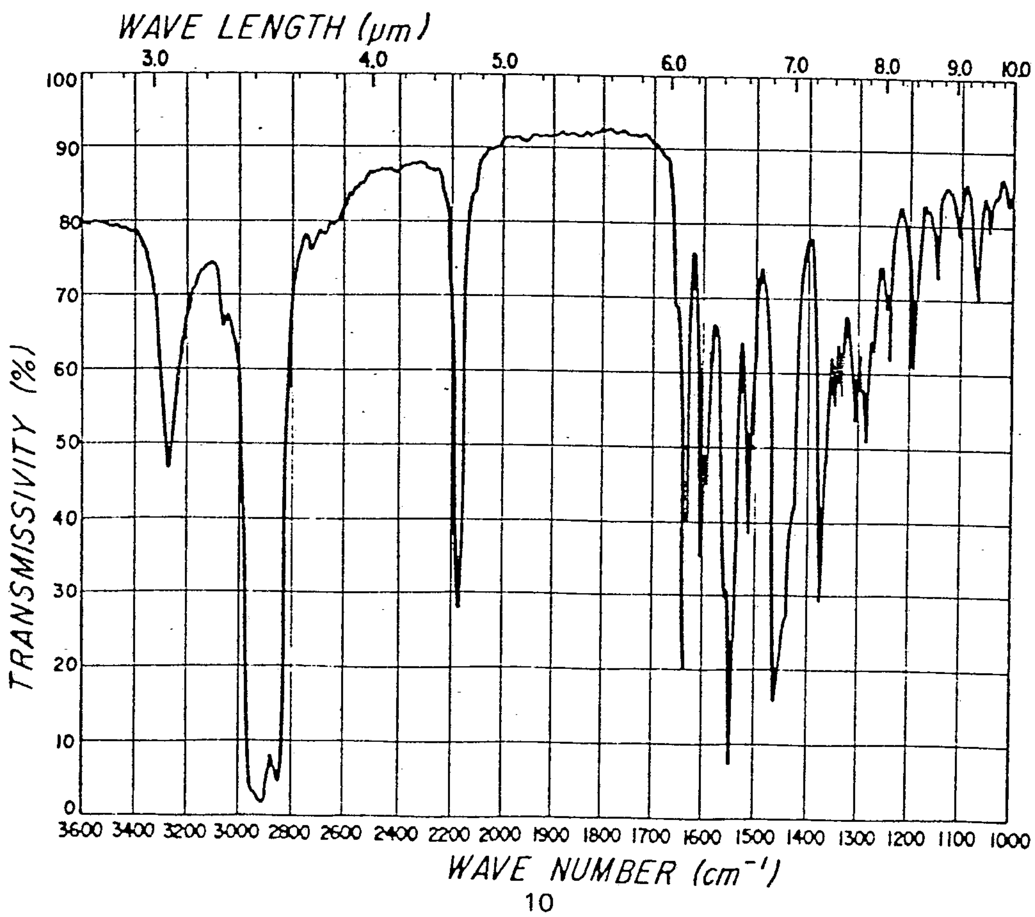
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 21

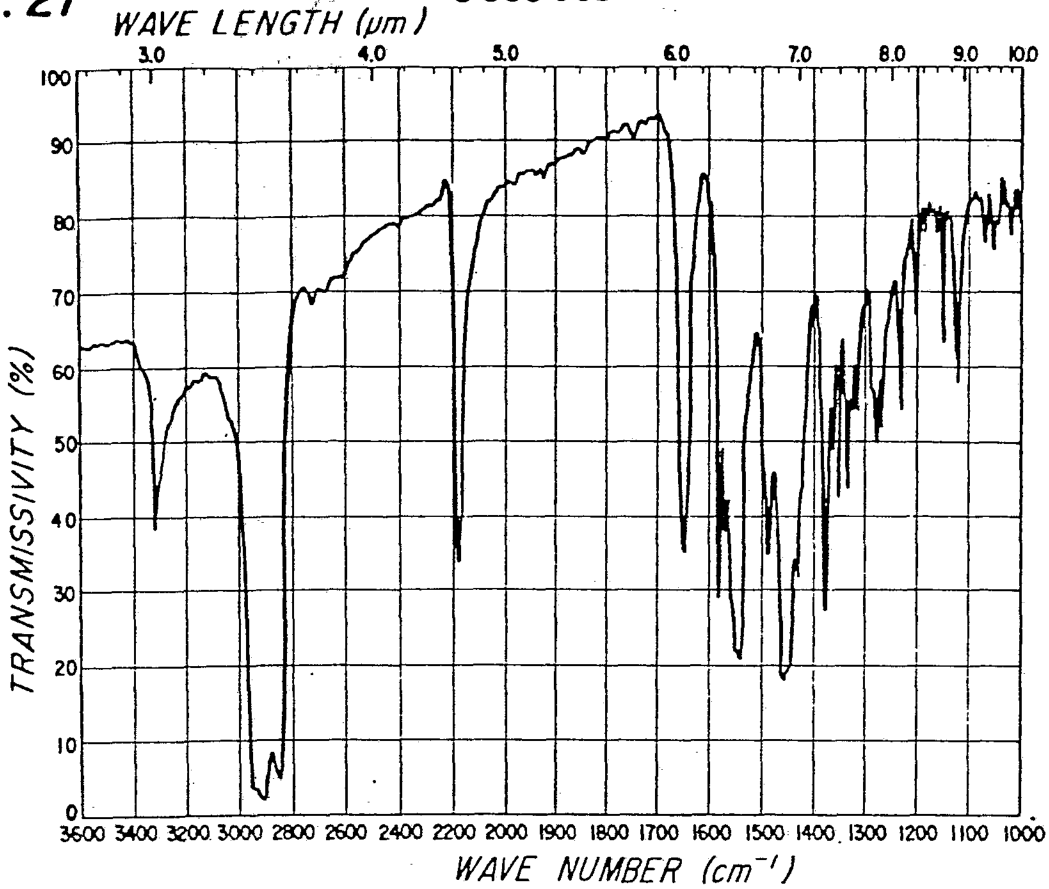
WAVE LENGTH (µm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 22

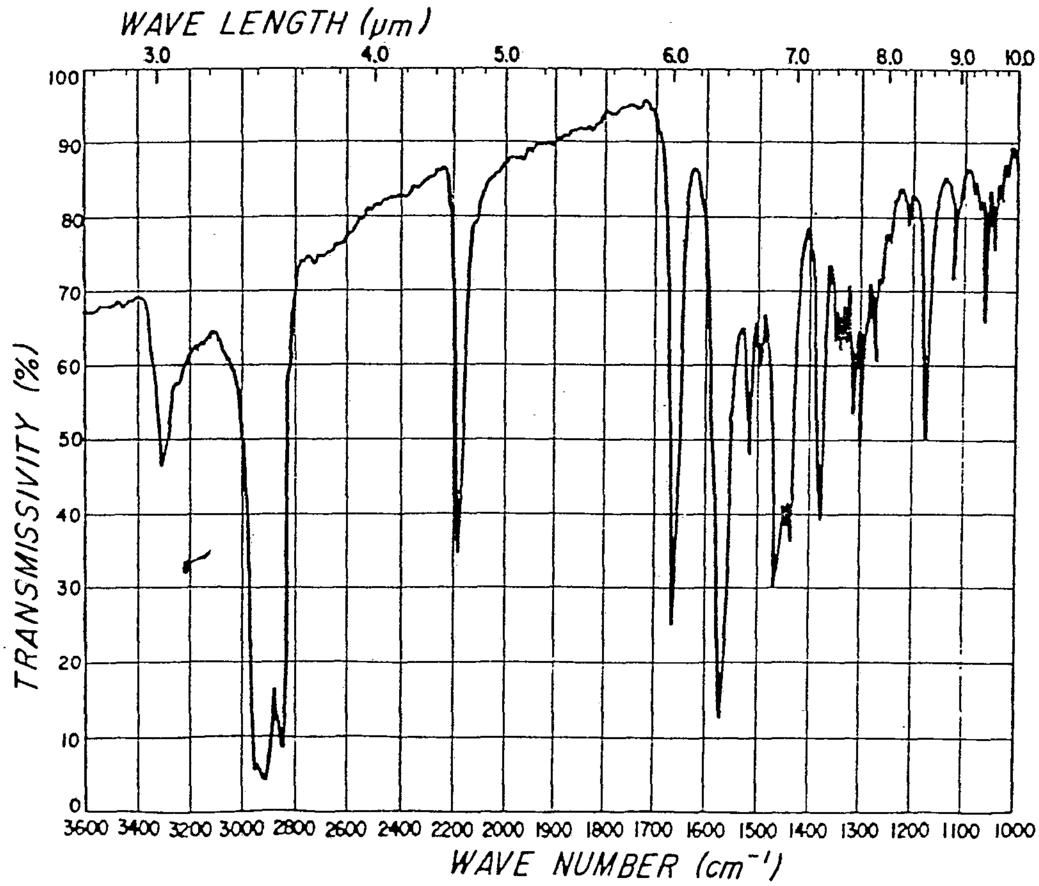
WAVE LENGTH (µm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 23

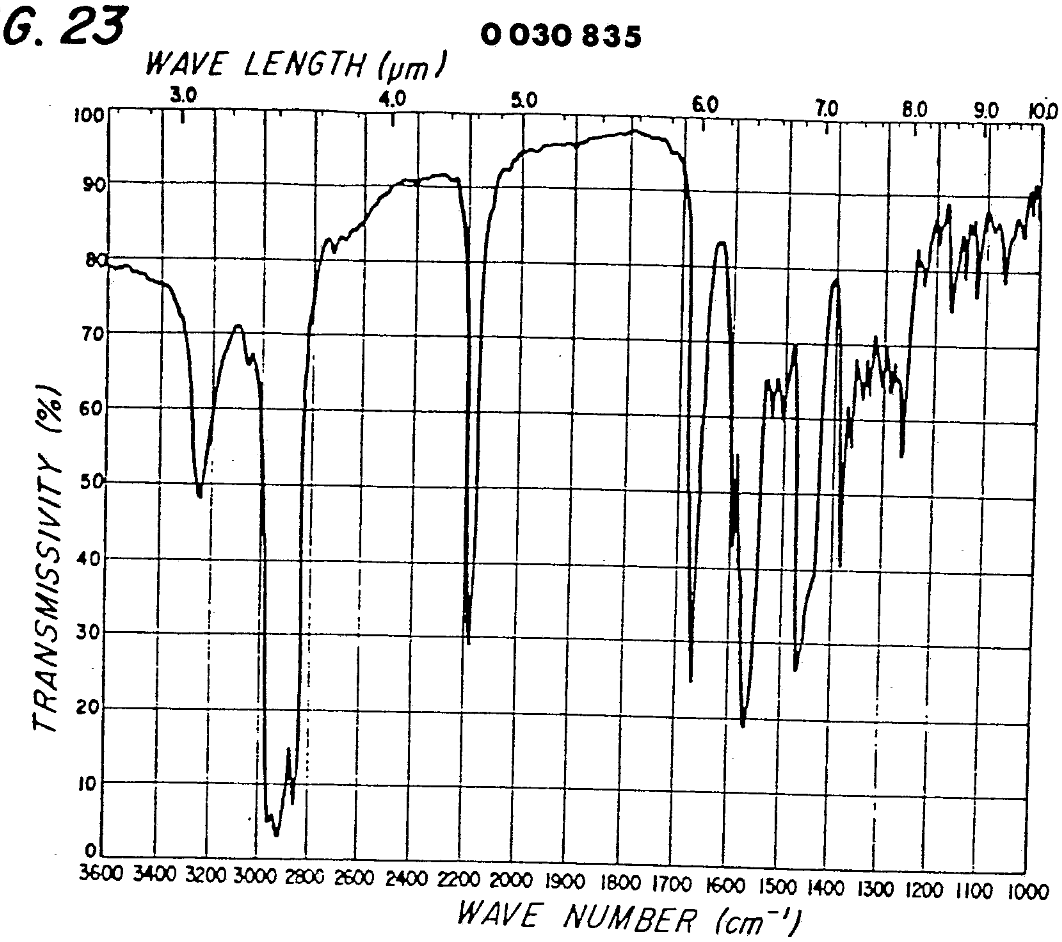
WAVE LENGTH (μm)
3.0 4.0 5.0 6.0 7.0 8.0 9.0 10.0
TRANSMISSIVITY (%)
100 90 80 70 60 50 40 30 20 10 0
3600 3400 3200 3000 2800 2600 2400 2200 2000 1900 1800 1700 1600 1500 1400 1300 1200 1100 1000
WAVE NUMBER (cm⁻¹)

FIG. 24

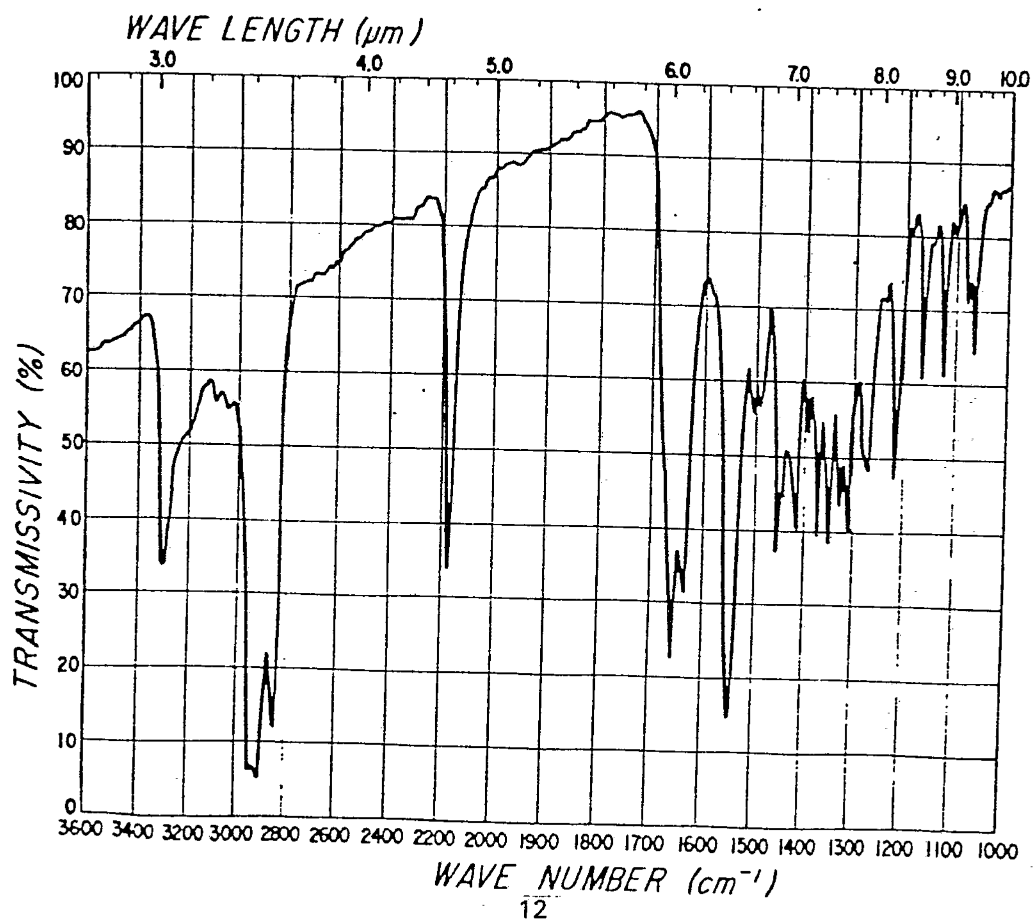
WAVE LENGTH (μm)
3.0 4.0 5.0 6.0 7.0 8.0 9.0 10.0
TRANSMISSIVITY (%)
100 90 80 70 60 50 40 30 20 10 0
3600 3400 3200 3000 2800 2600 2400 2200 2000 1900 1800 1700 1600 1500 1400 1300 1200 1100 1000
WAVE NUMBER (cm⁻¹)

FIG. 25

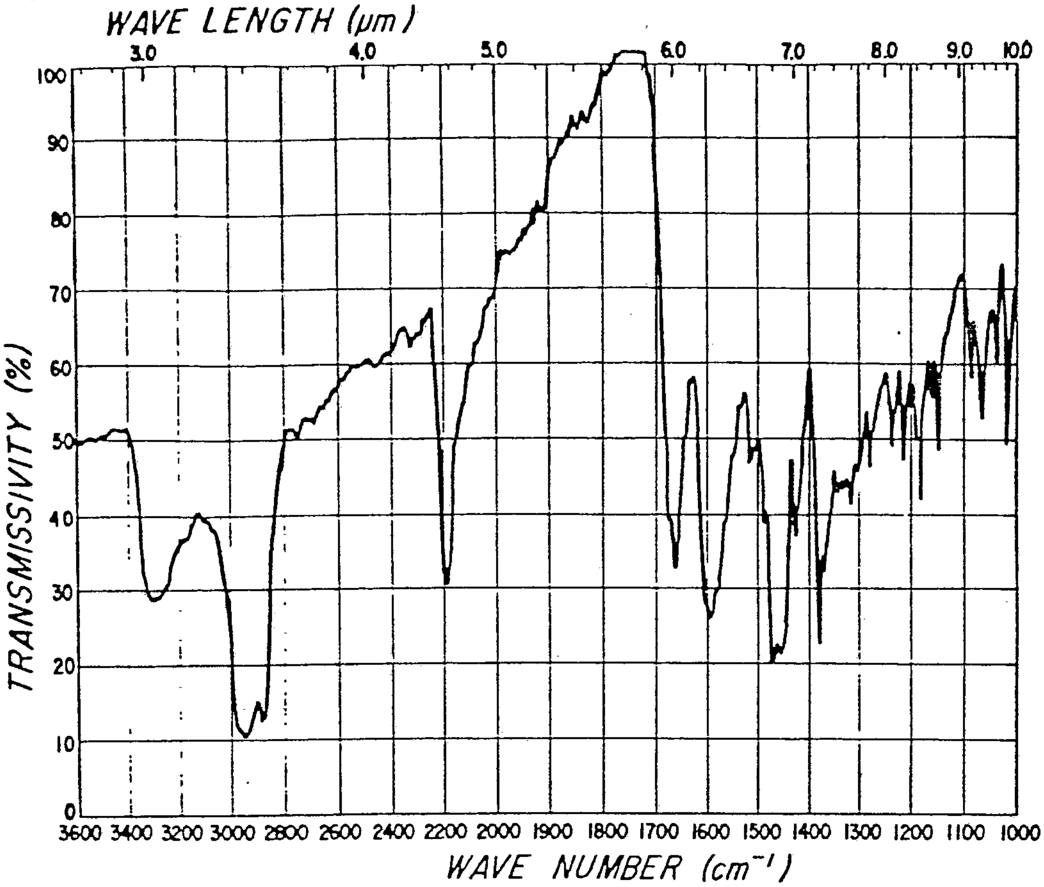
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 26

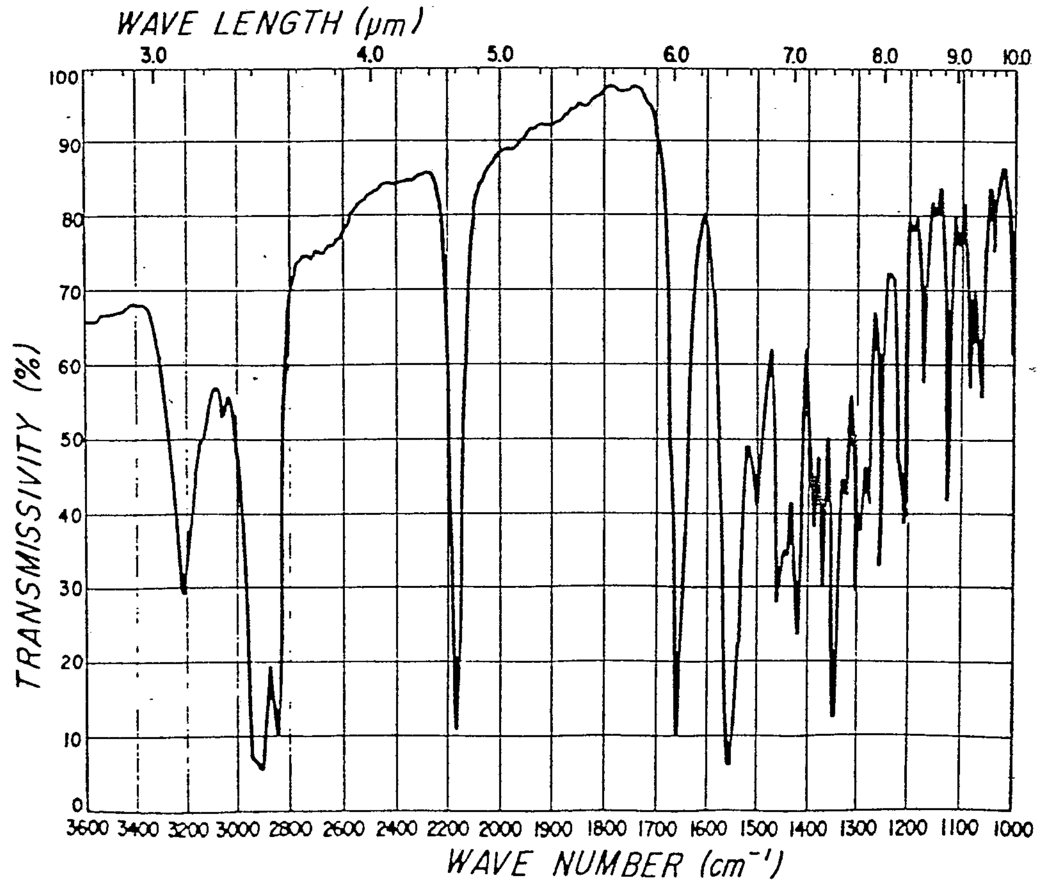
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 27

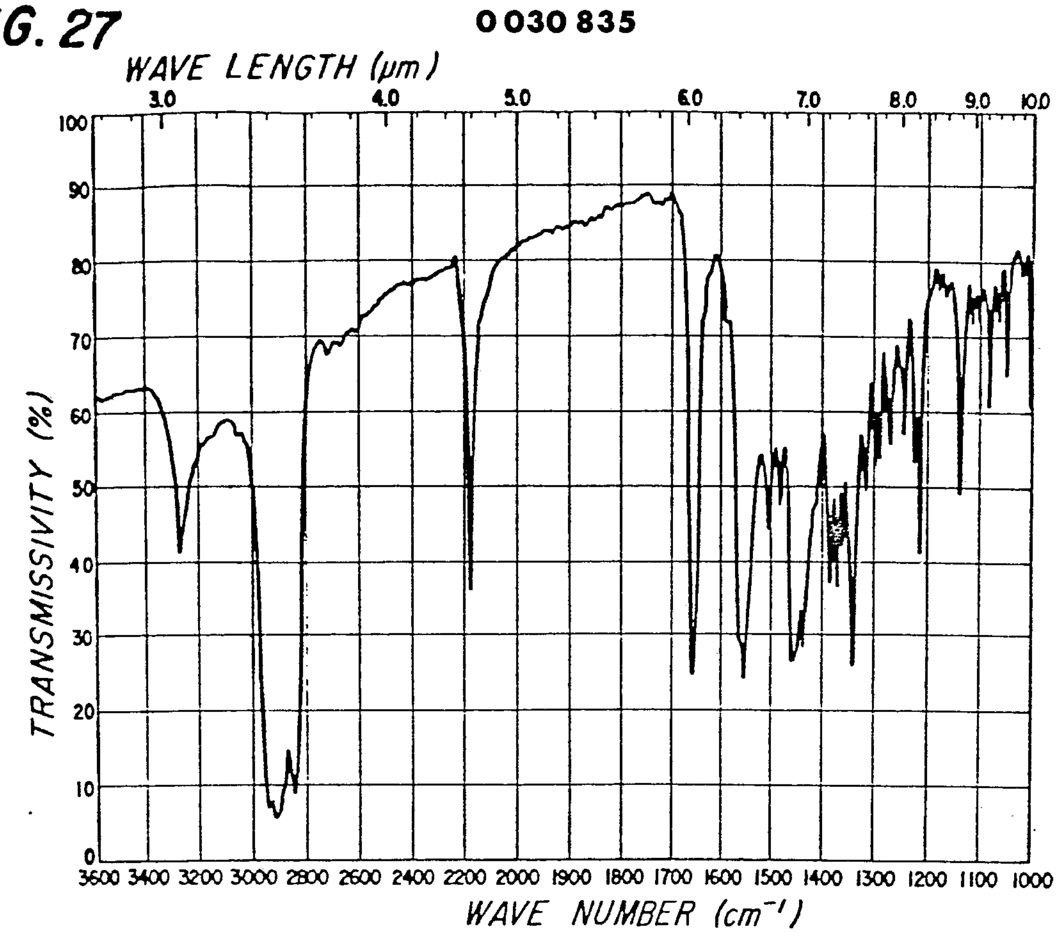
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 28

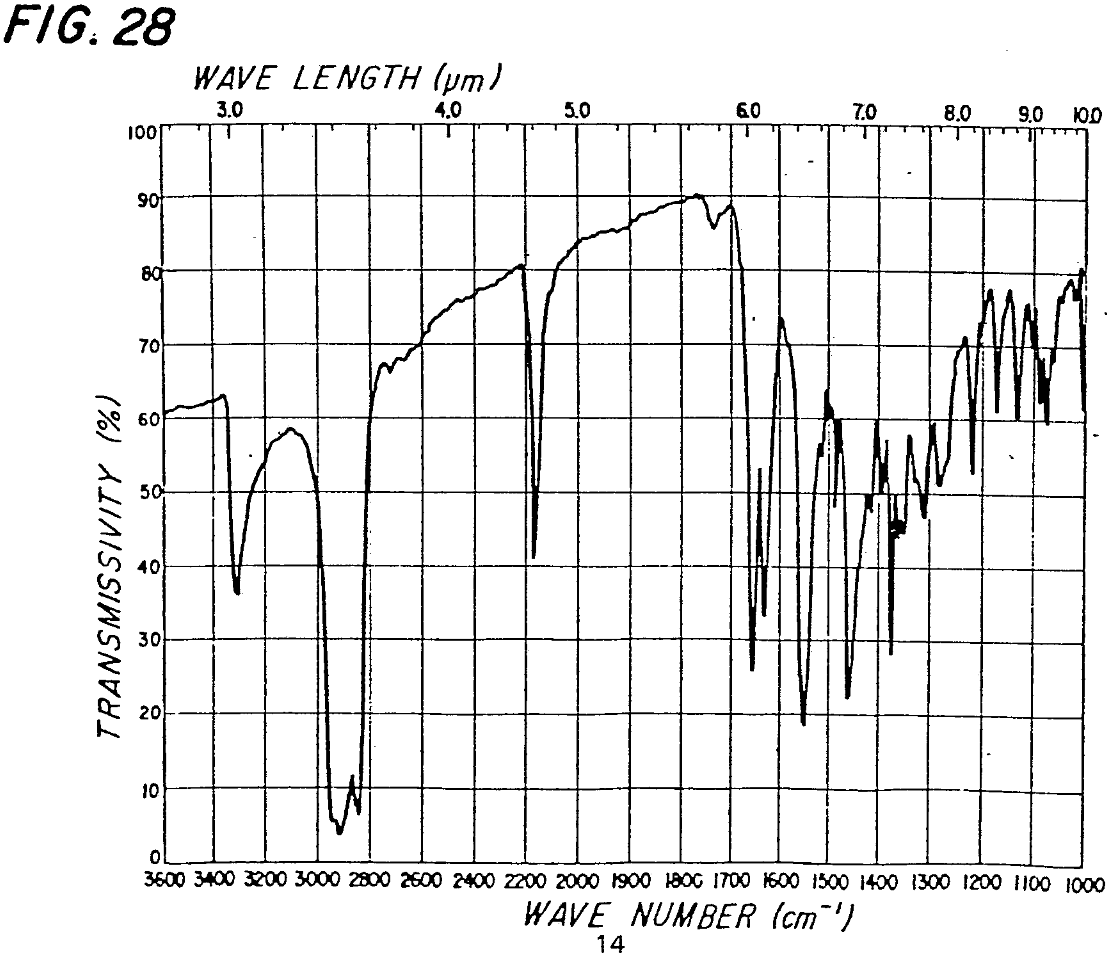
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 29

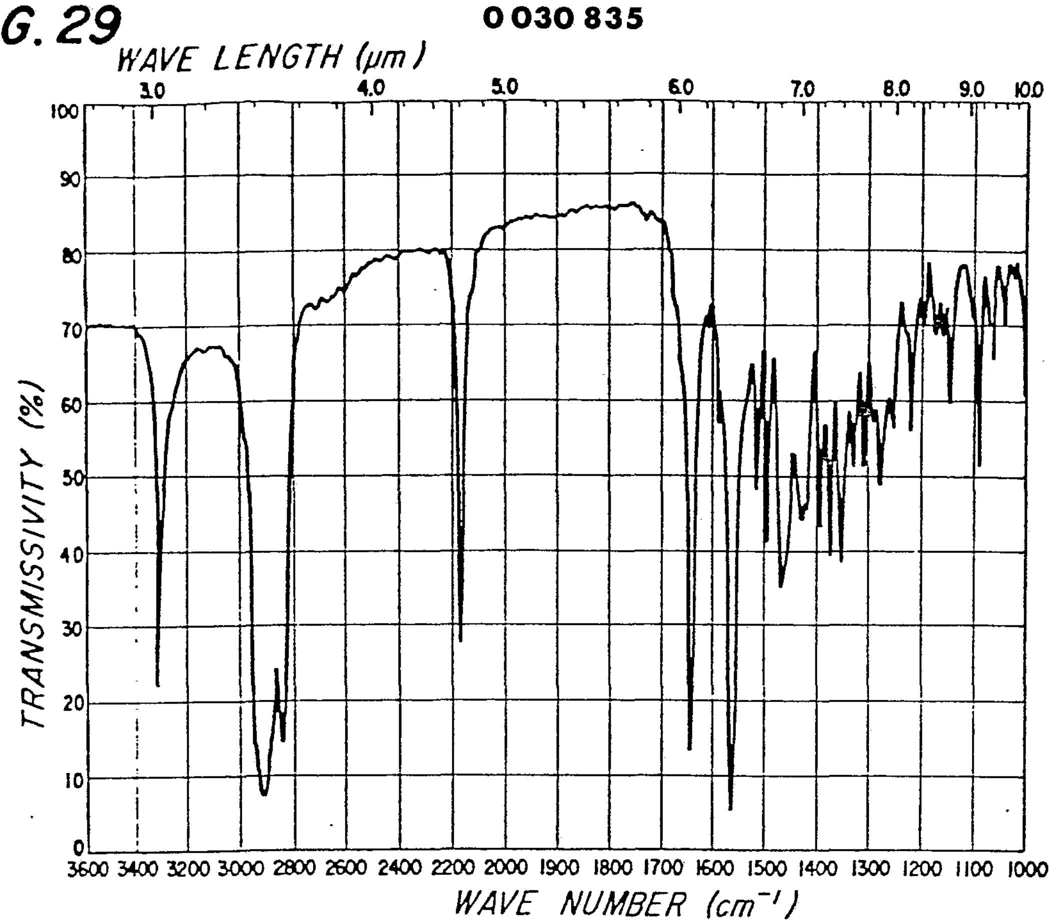
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER ($cm^{-1}$)

FIG. 30

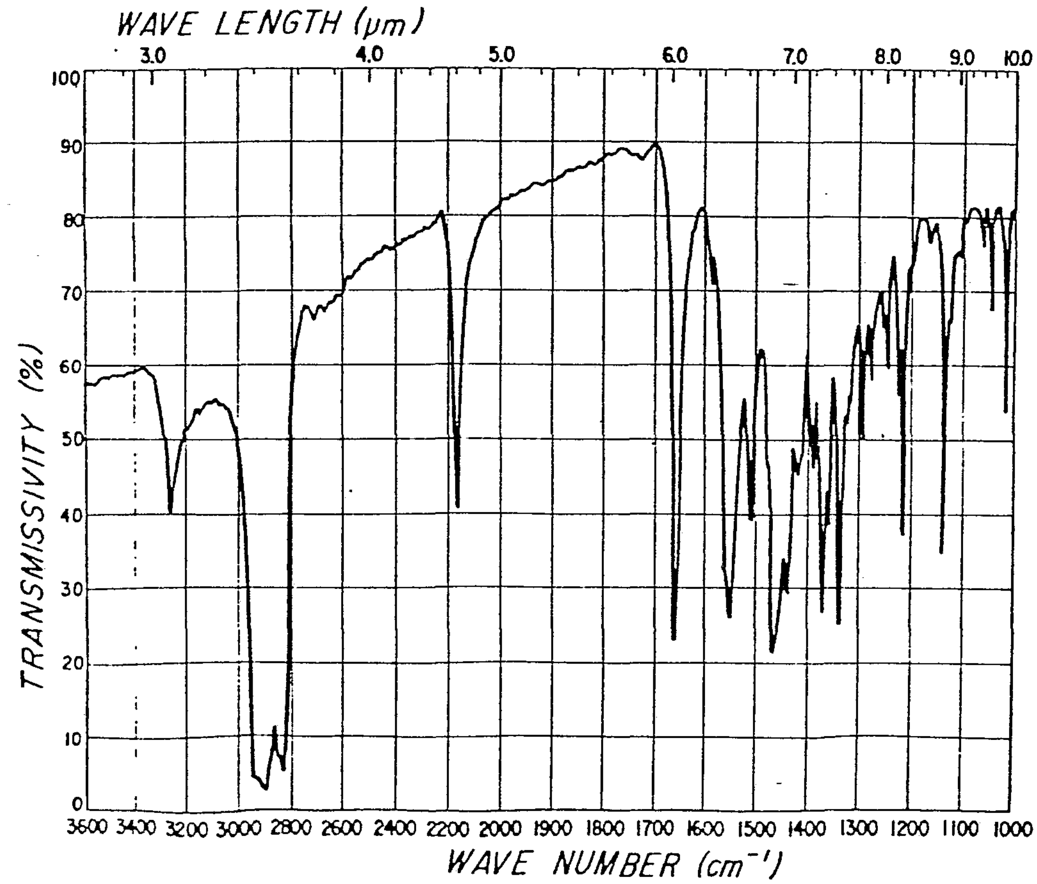
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER ($cm^{-1}$)

FIG. 31

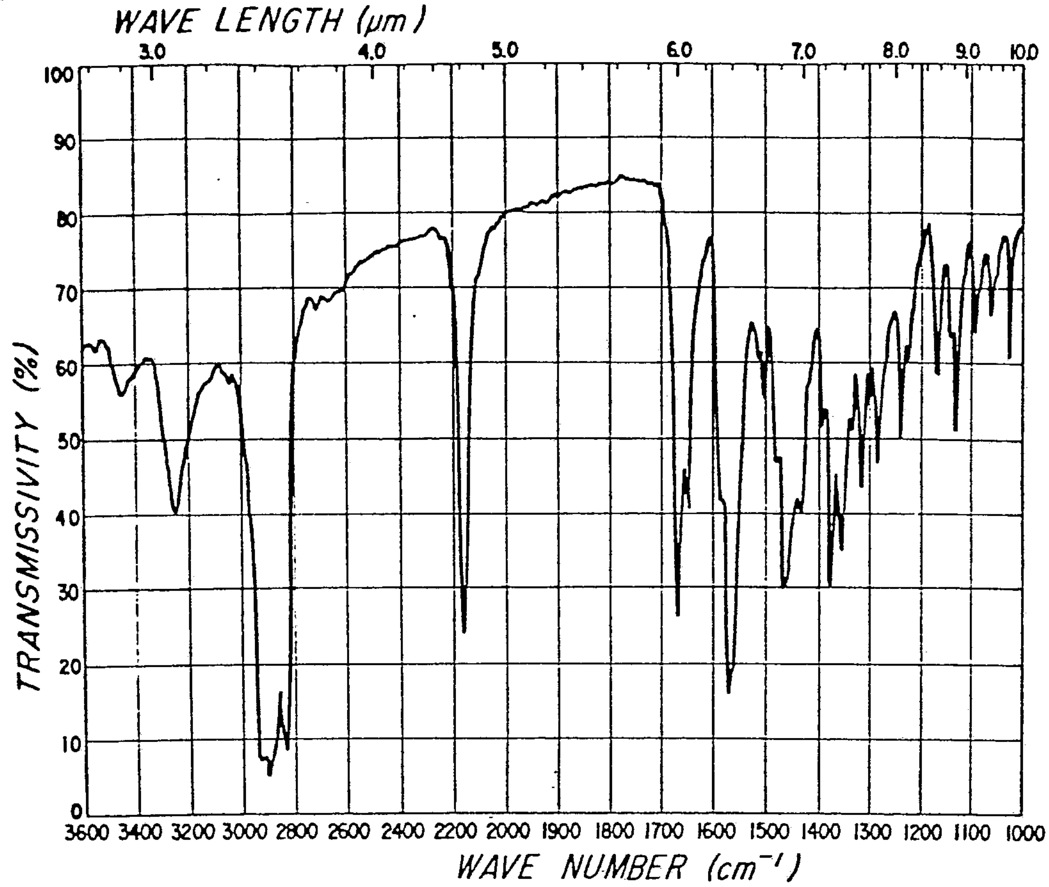
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG. 32

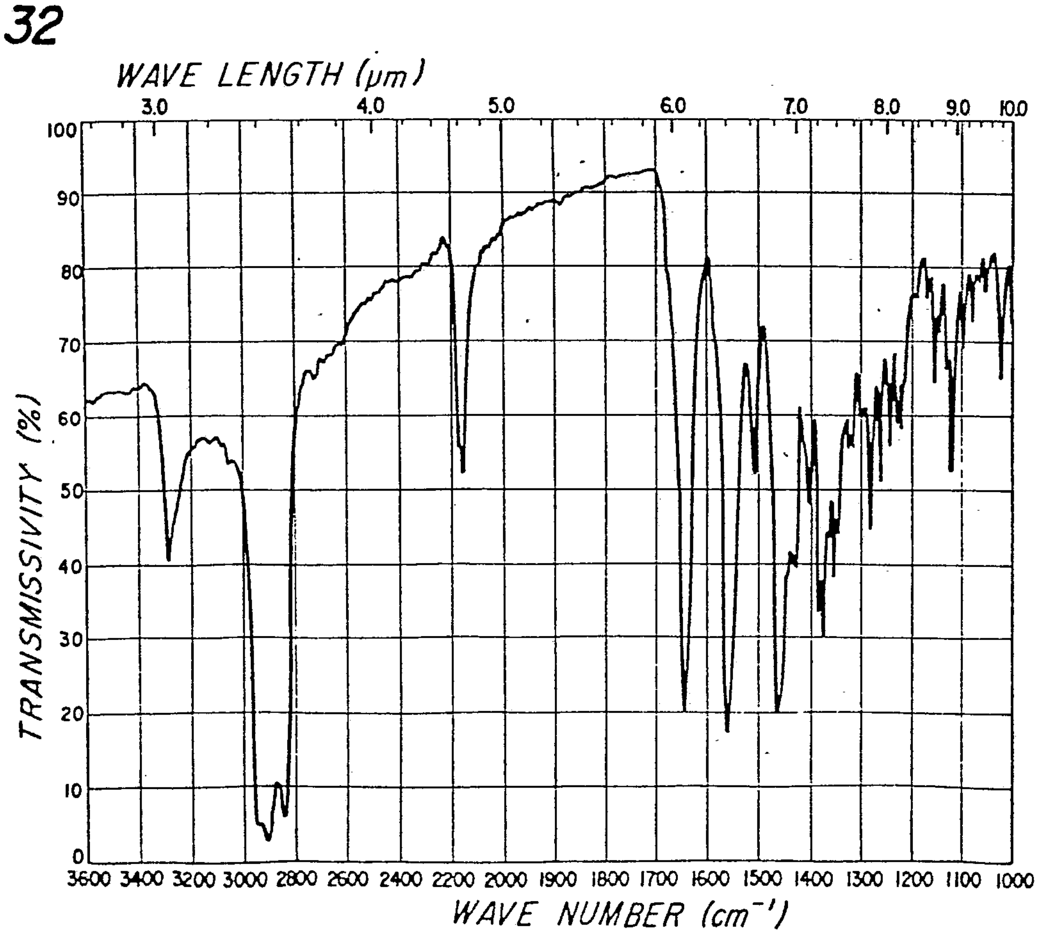
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG. 33

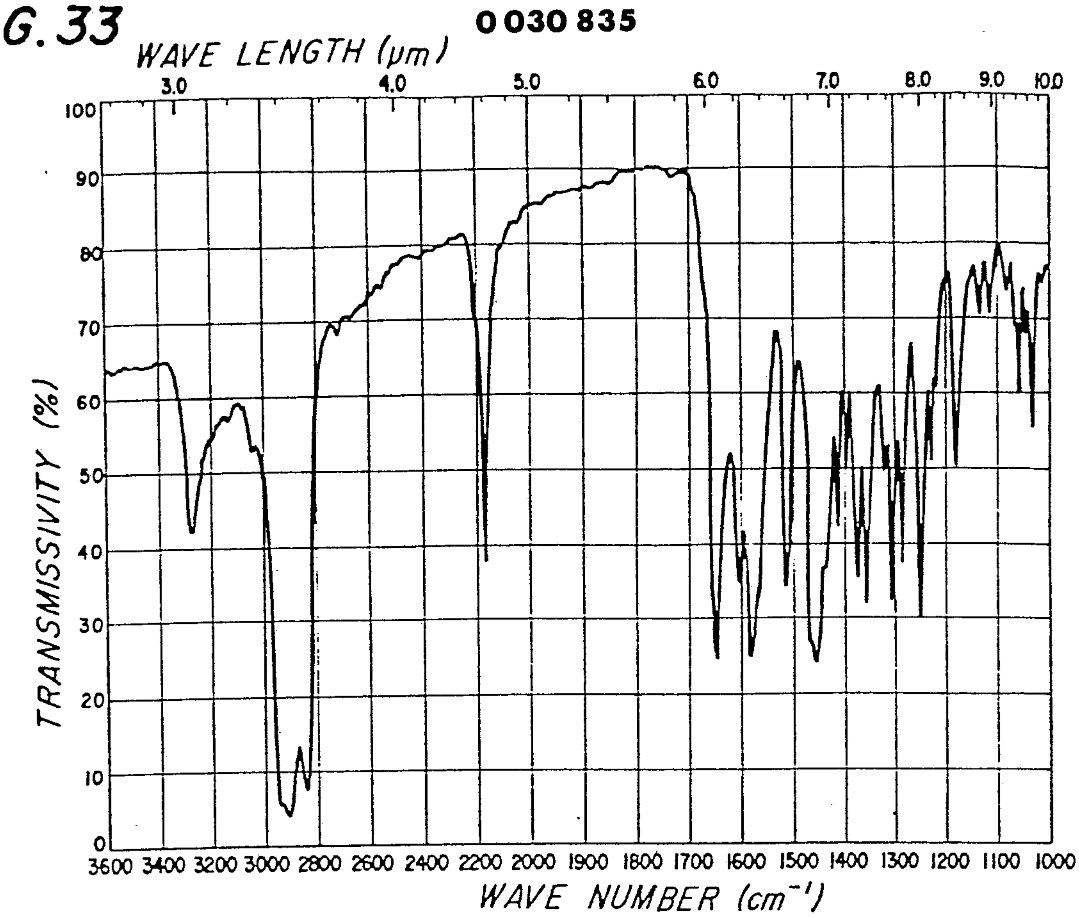
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 34

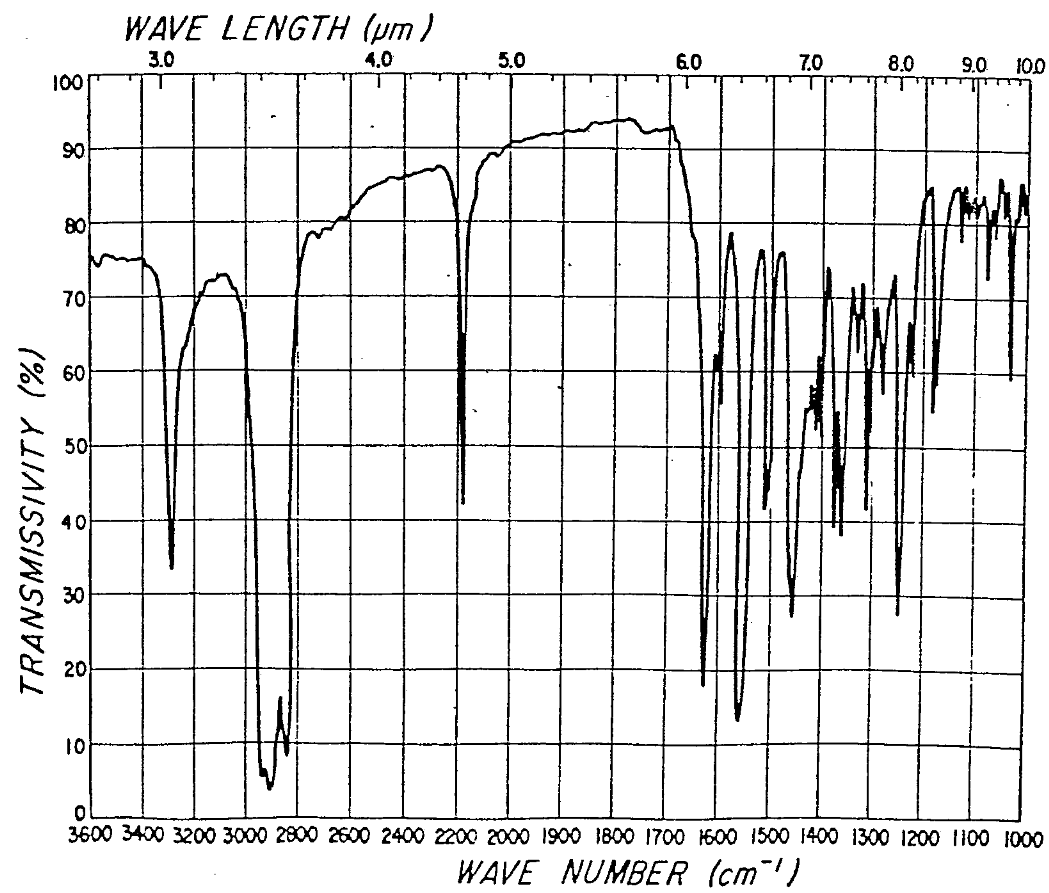
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 35

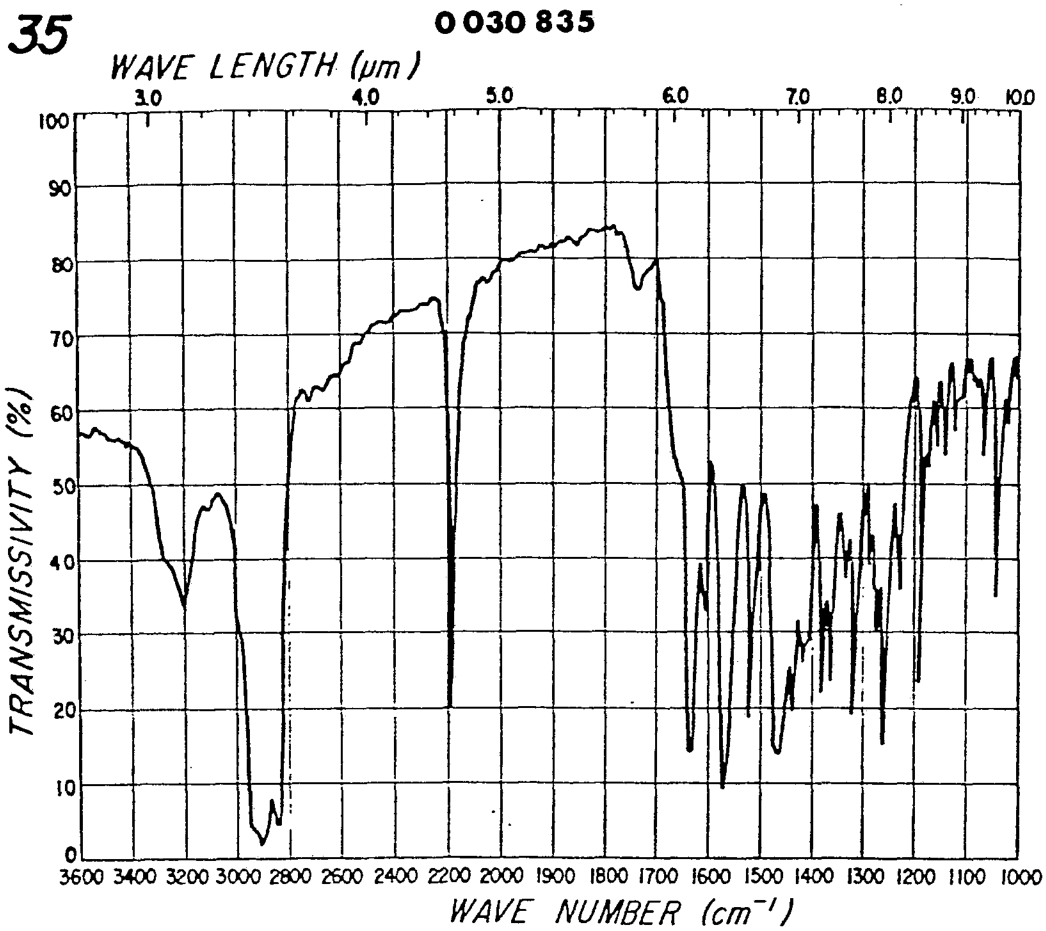
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 36

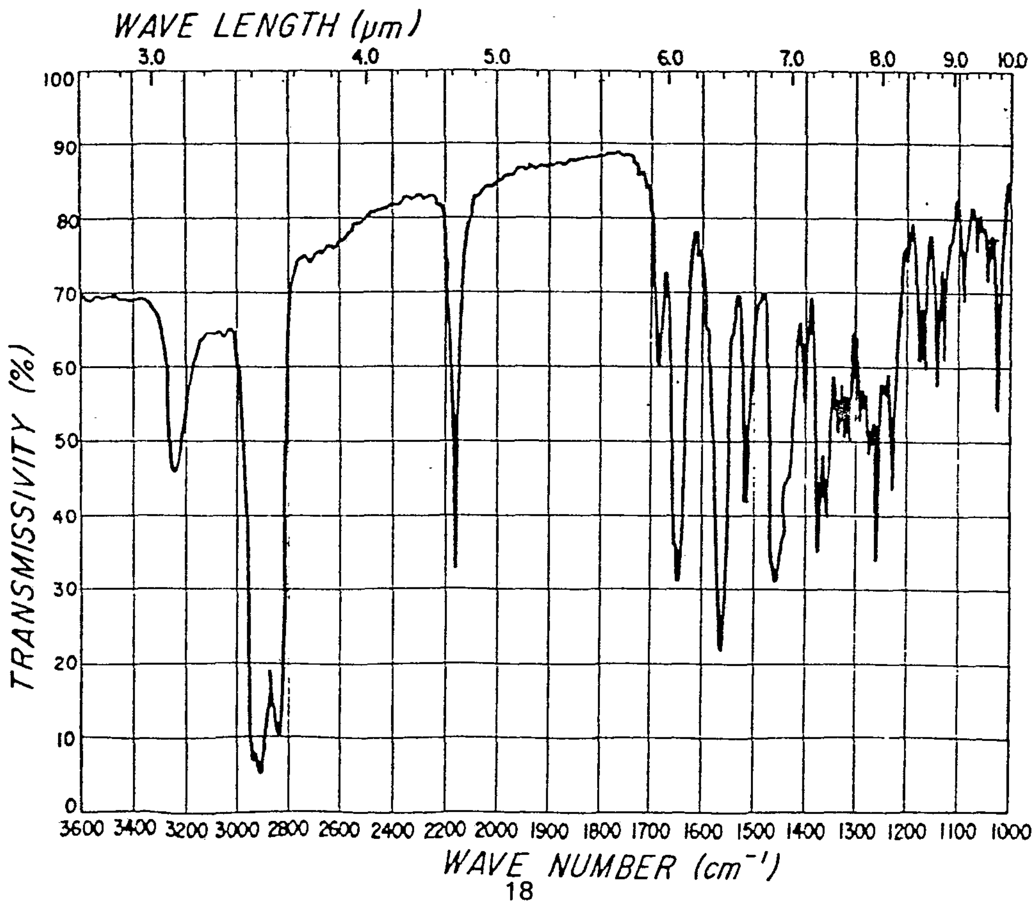
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 37

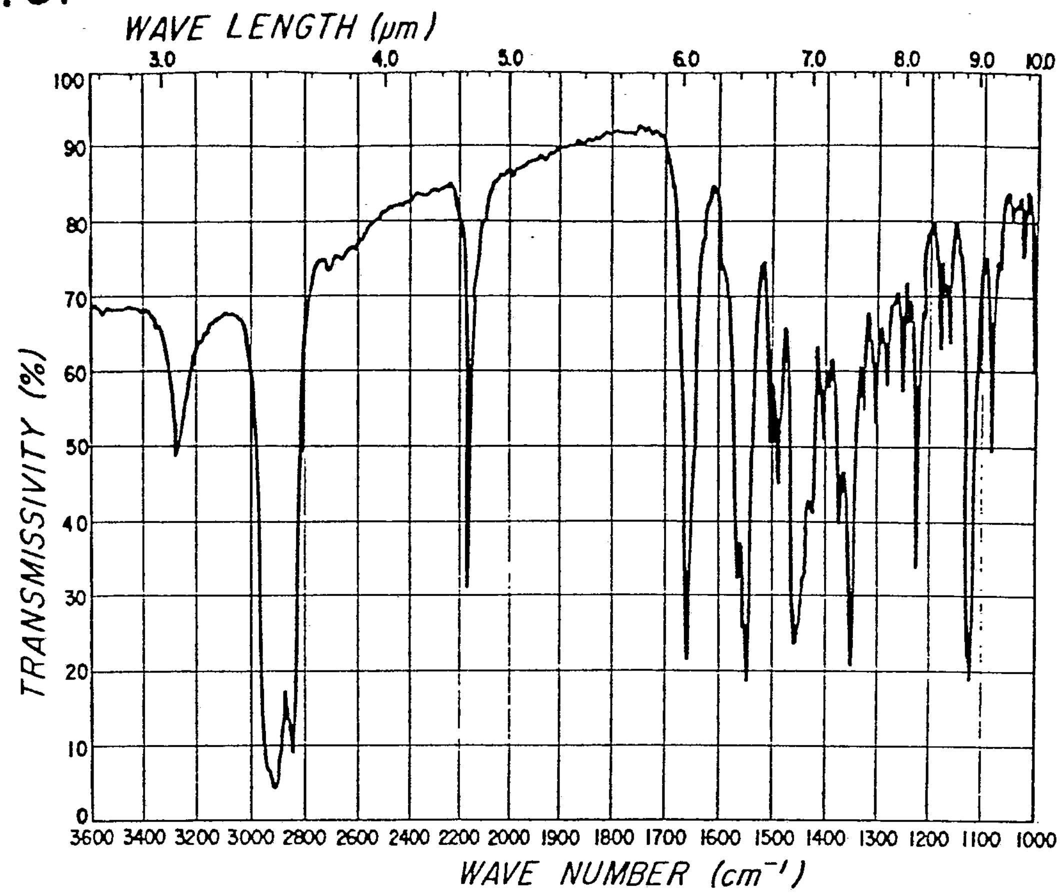
WAVE LENGTH (µm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG. 38

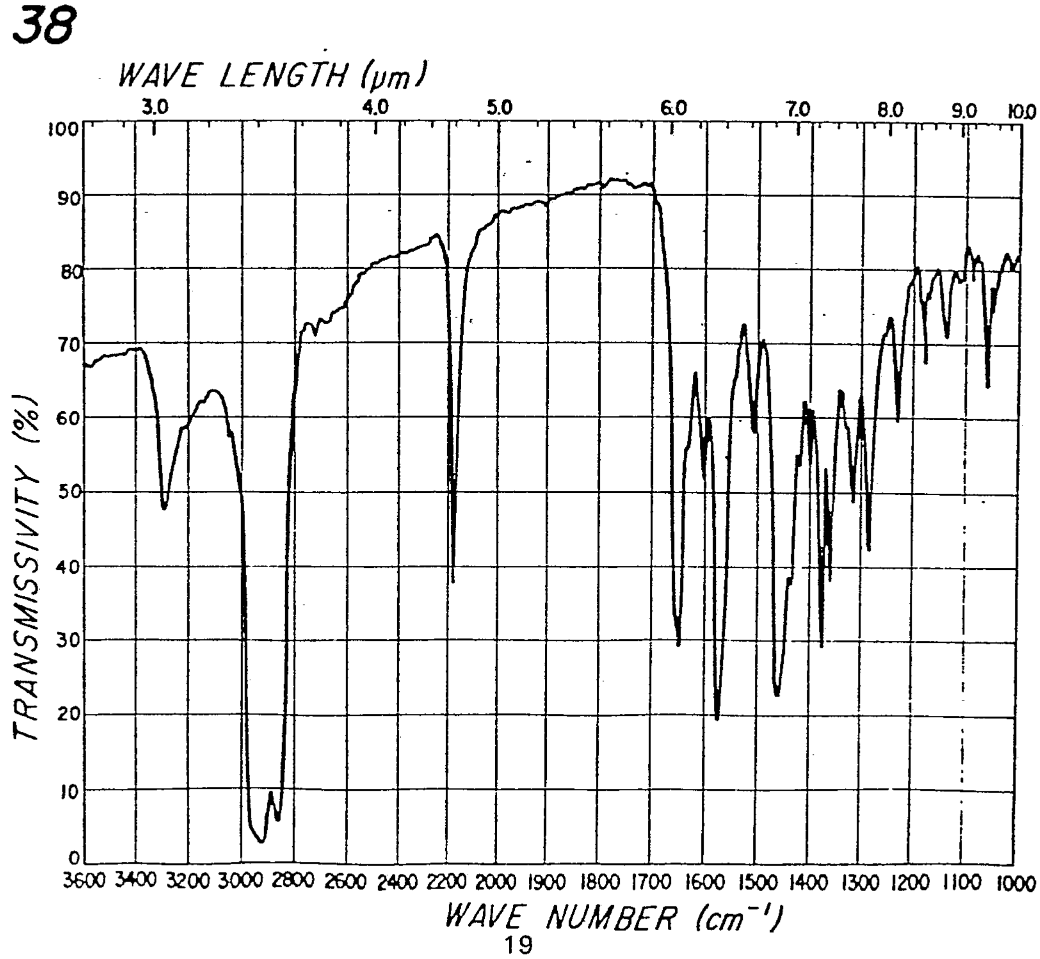
WAVE LENGTH (µm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG. 39

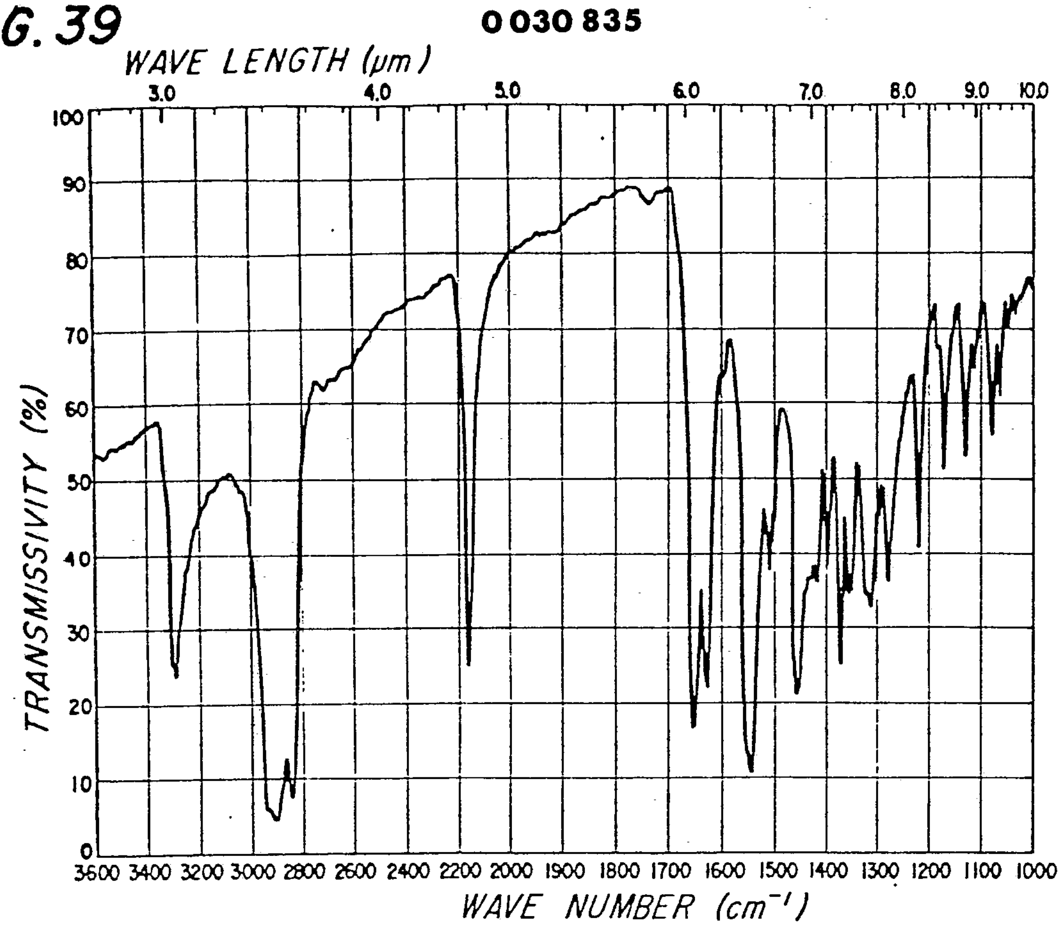
WAVE LENGTH (μm)
3.0
4.0
5.0
6.0
7.0
8.0
9.0
10.0
TRANSMISSIVITY (%)
0
10
20
30
40
50
60
70
80
90
100
3600 3400 3200 3000 2800 2600 2400 2200 2000 1900 1800 1700 1600 1500 1400 1300 1200 1100 1000
WAVE NUMBER (cm⁻¹)

FIG. 40

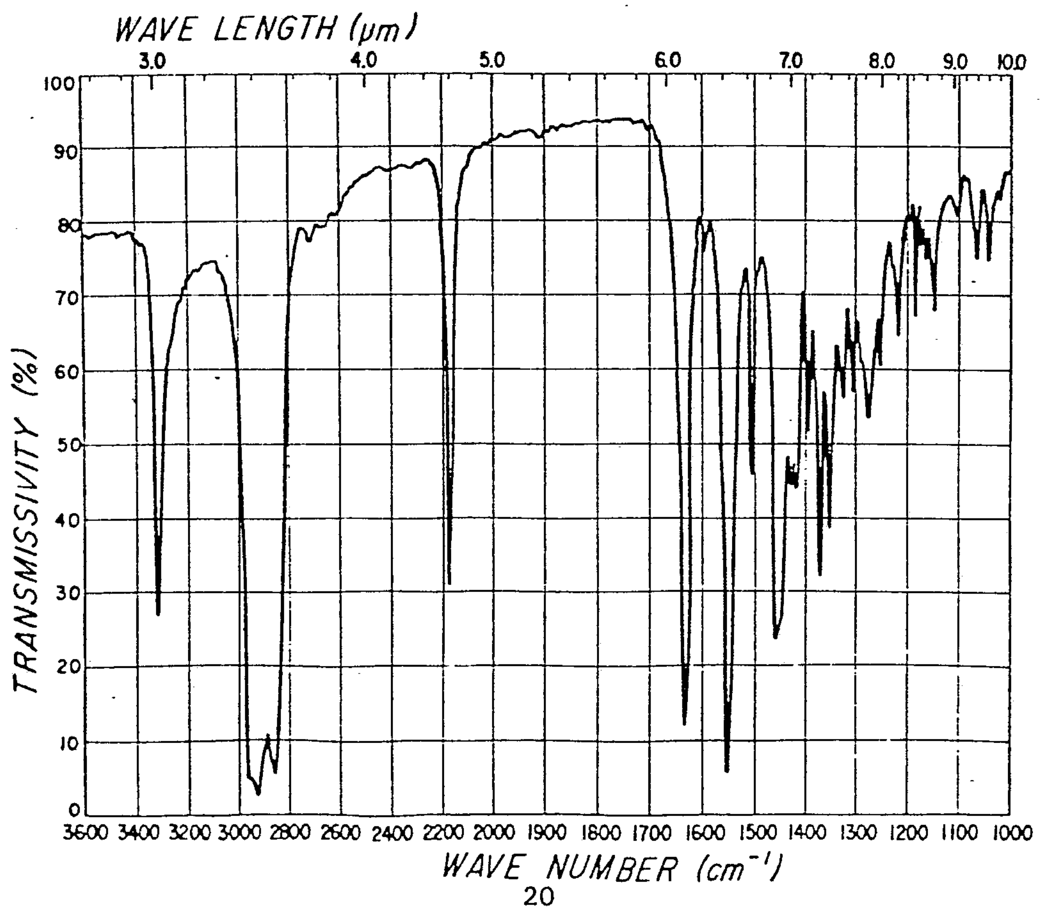
WAVE LENGTH (μm)
3.0
4.0
5.0
6.0
7.0
8.0
9.0
10.0
TRANSMISSIVITY (%)
0
10
20
30
40
50
60
70
80
90
100
3600 3400 3200 3000 2800 2600 2400 2200 2000 1900 1800 1700 1600 1500 1400 1300 1200 1100 1000
WAVE NUMBER (cm⁻¹)

FIG. 41

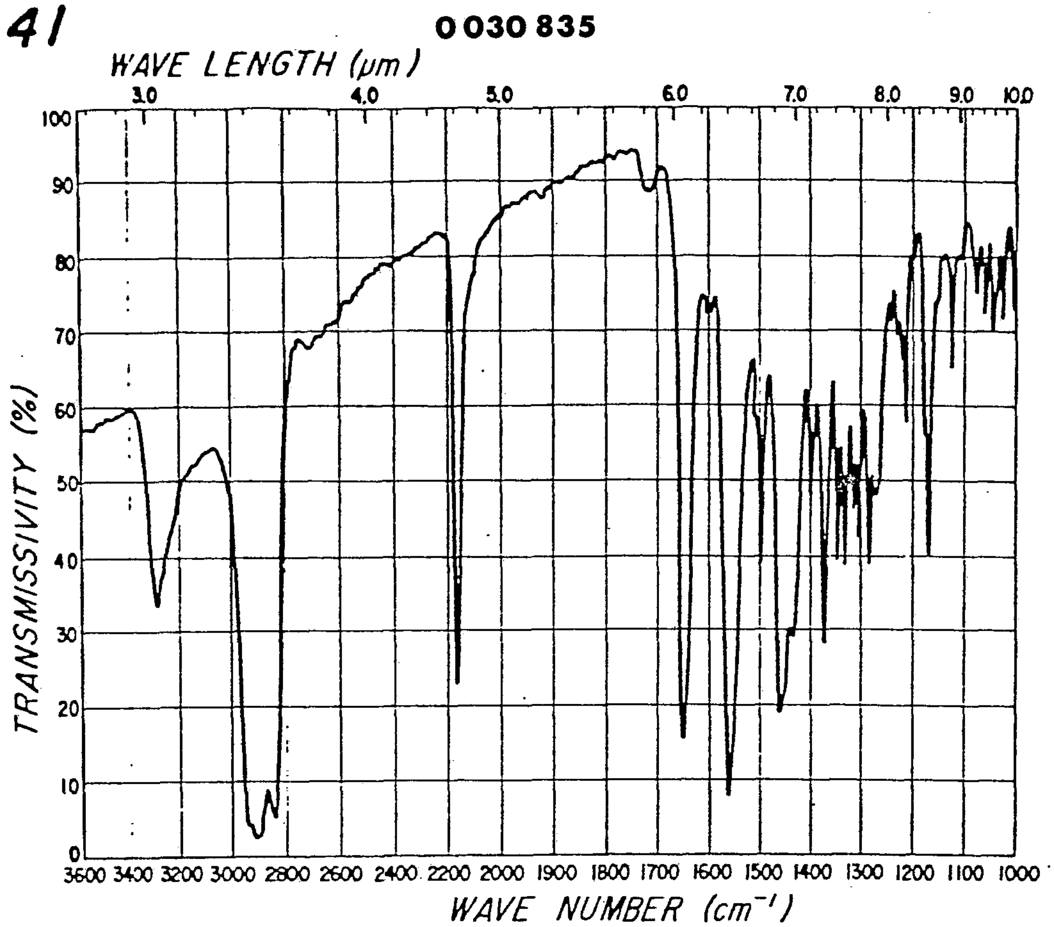
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 42

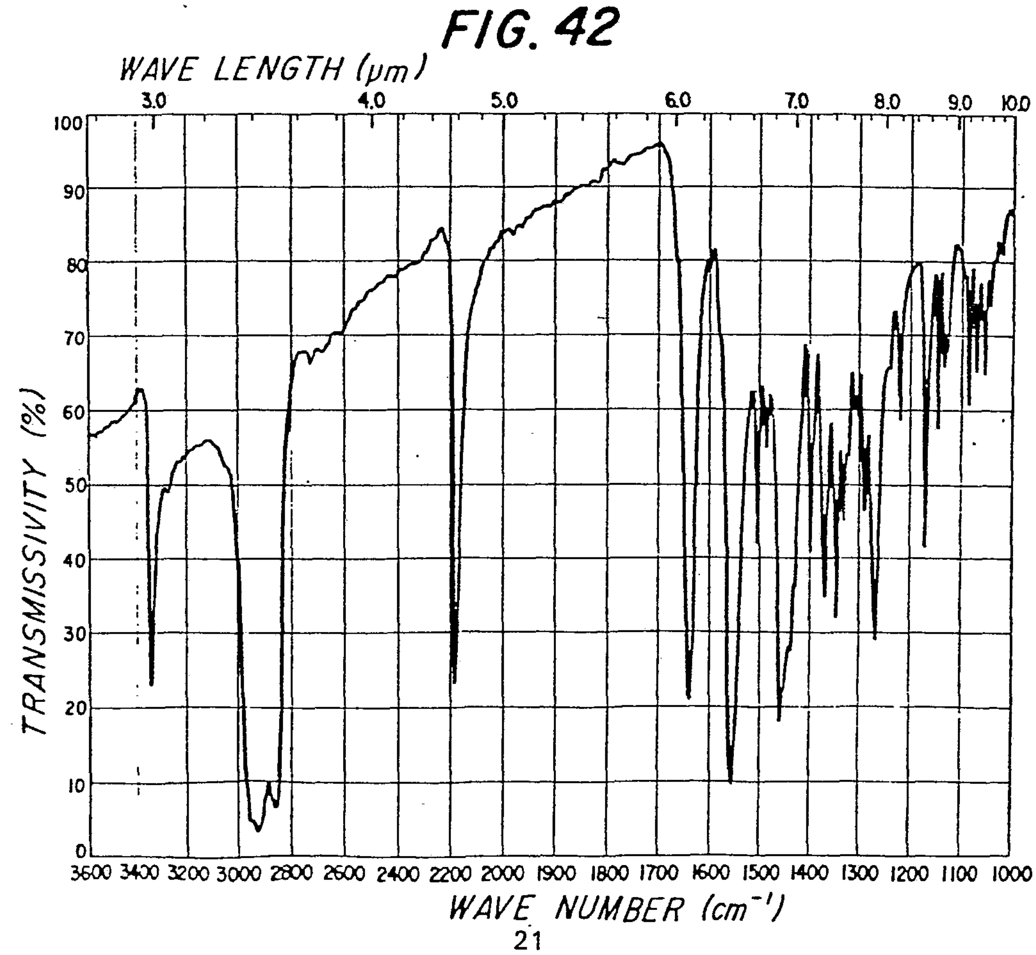
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 43

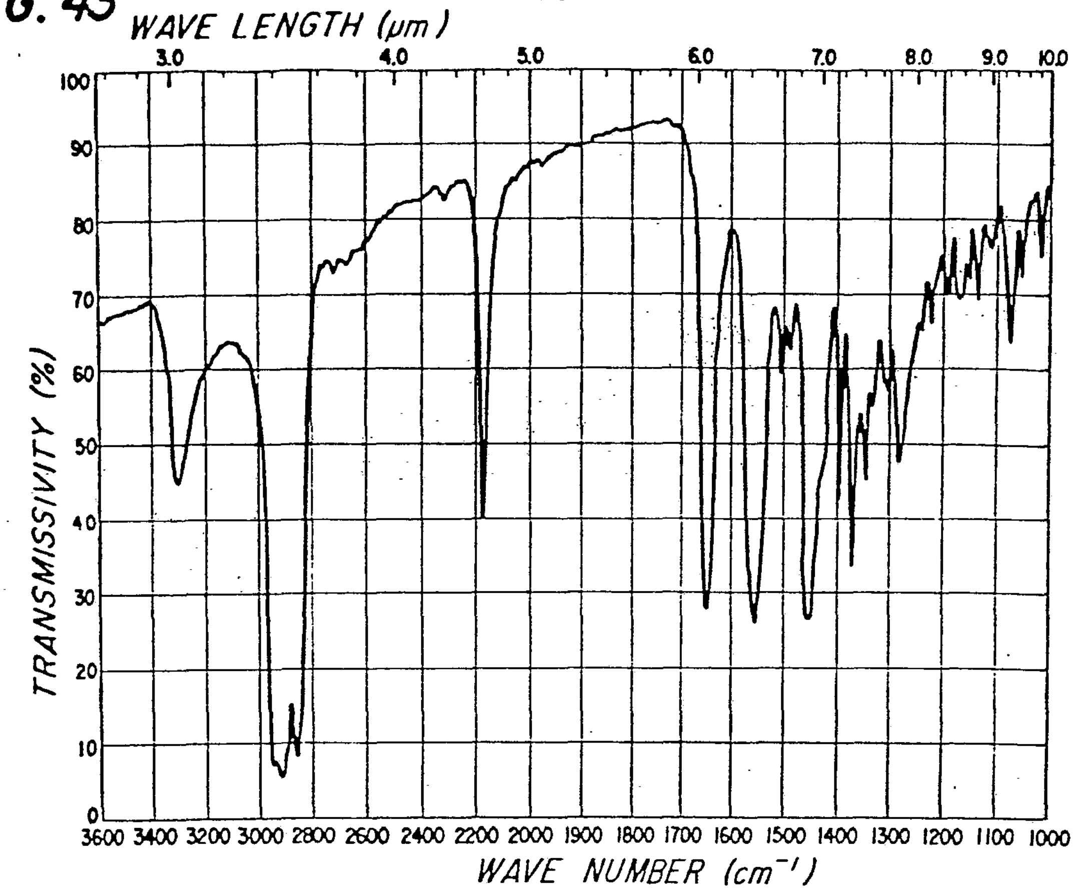
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 44

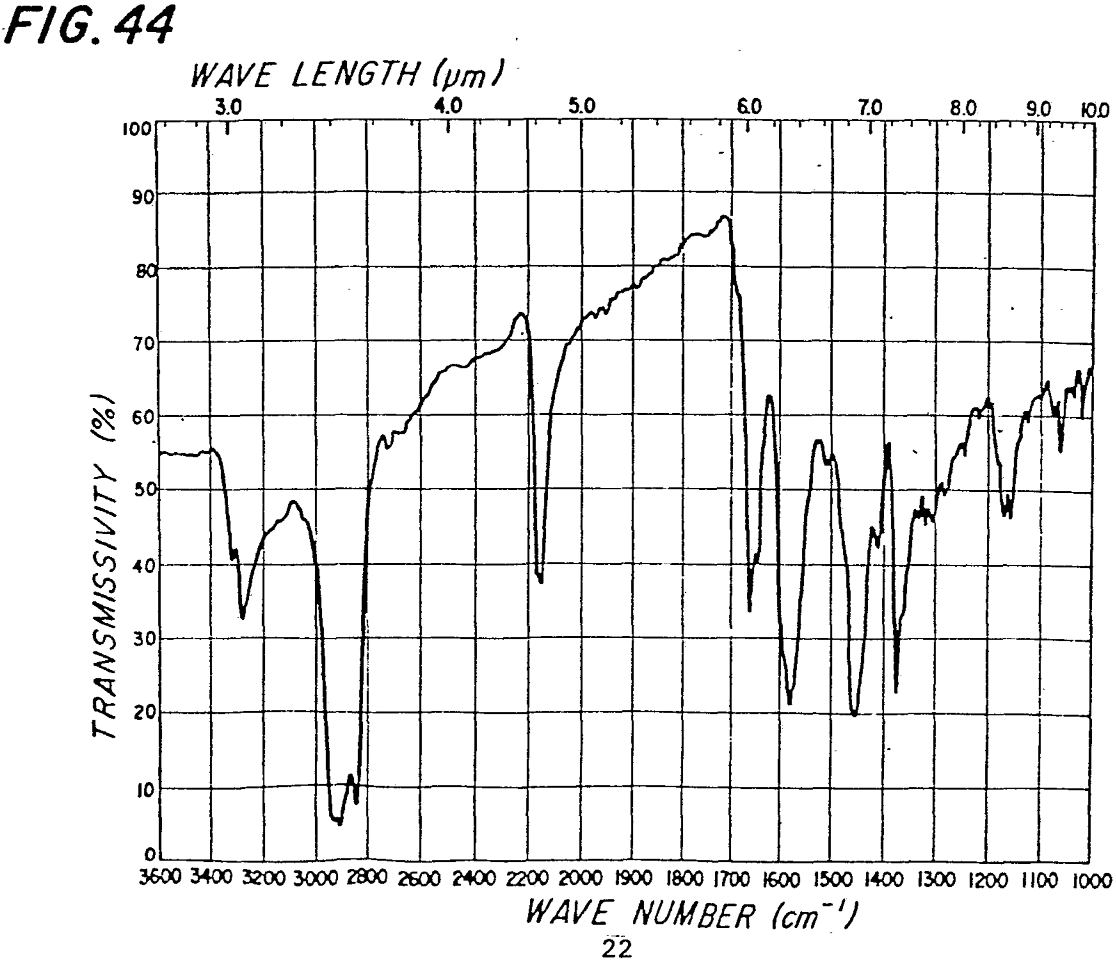
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 45

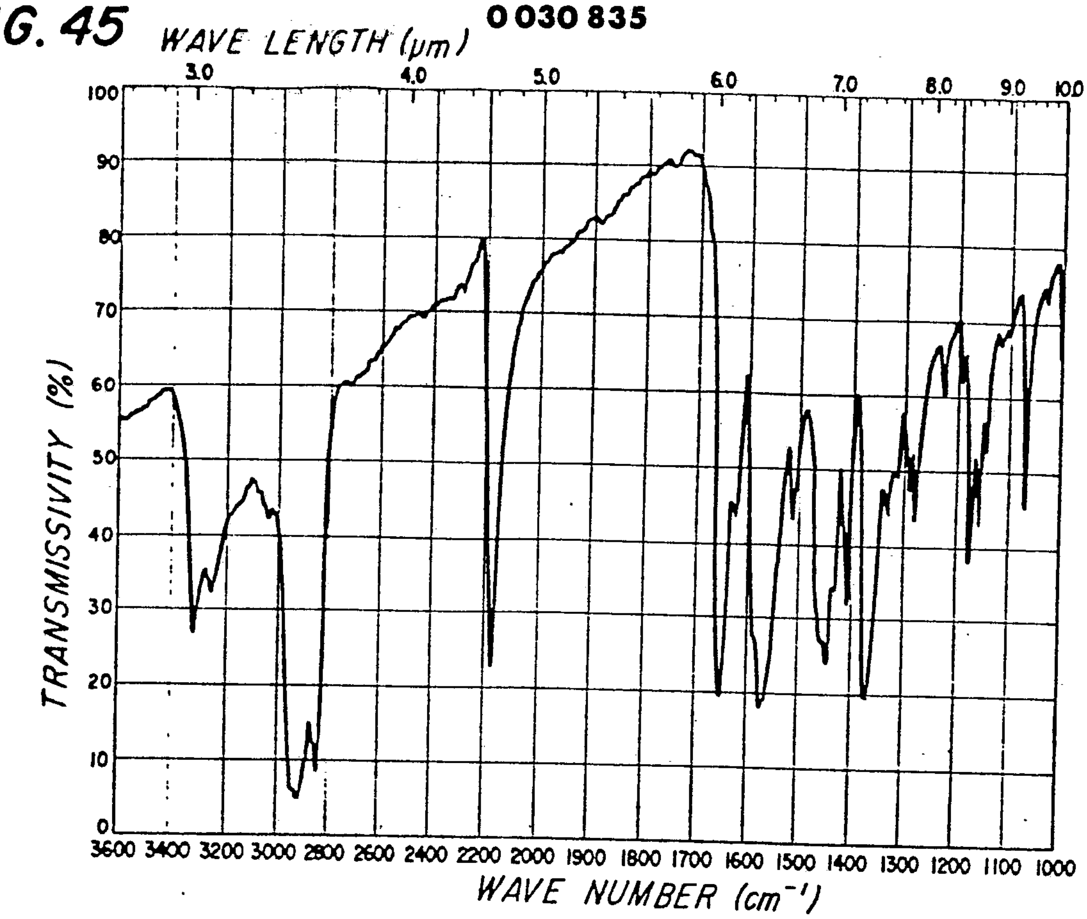
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 46

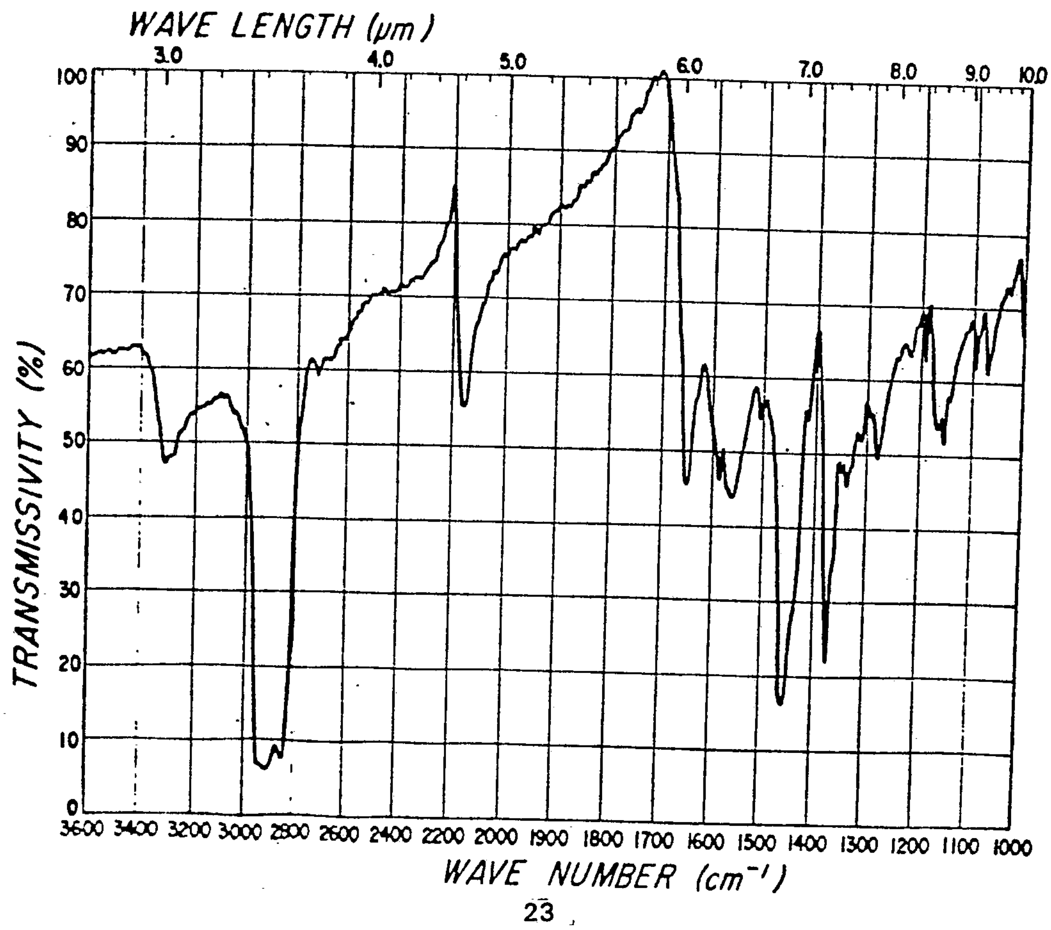
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 47

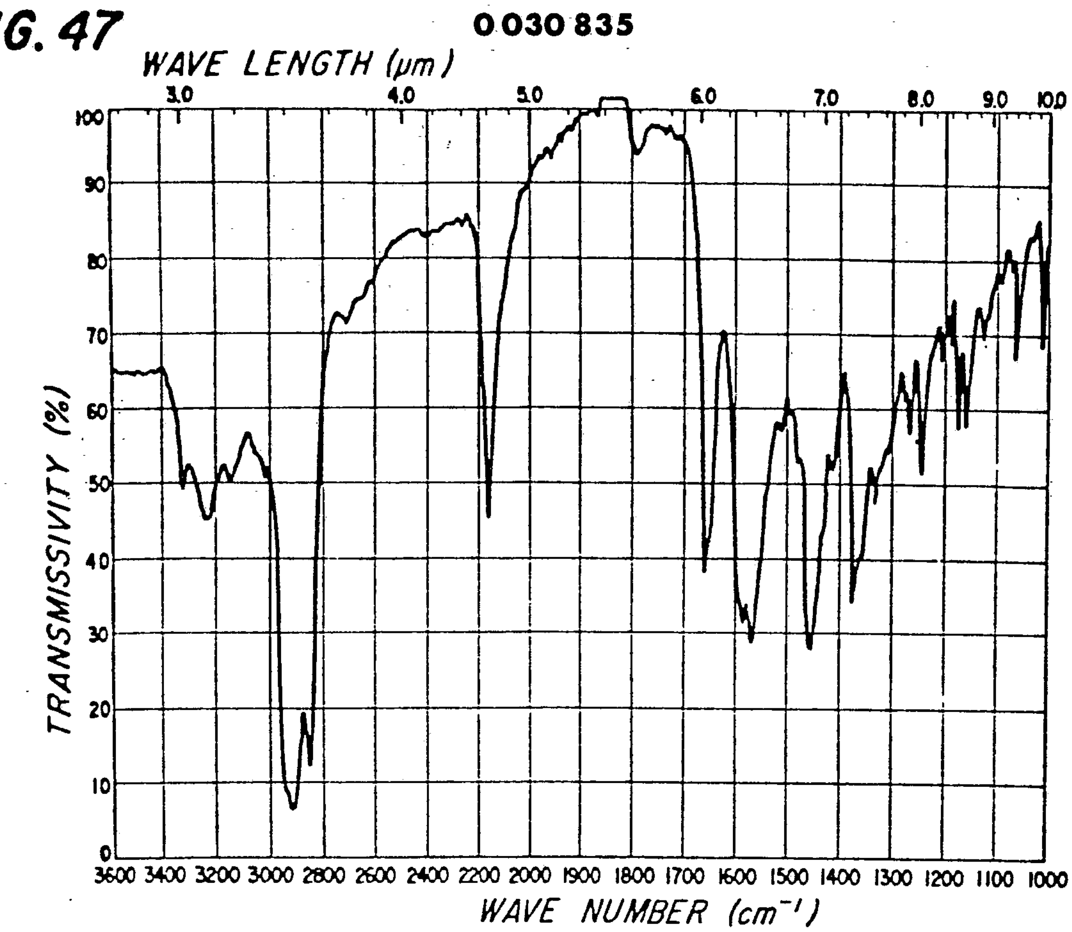
WAVE LENGTH (μm)
3.0 4.0 5.0 6.0 7.0 8.0 9.0 10.0
100 90 80 70 60 50 40 30 20 10 0
TRANSMISSIVITY (%)
3600 3400 3200 3000 2800 2600 2400 2200 2000 1900 1800 1700 1600 1500 1400 1300 1200 1100 1000
WAVE NUMBER ($cm^{-1}$)

FIG. 48

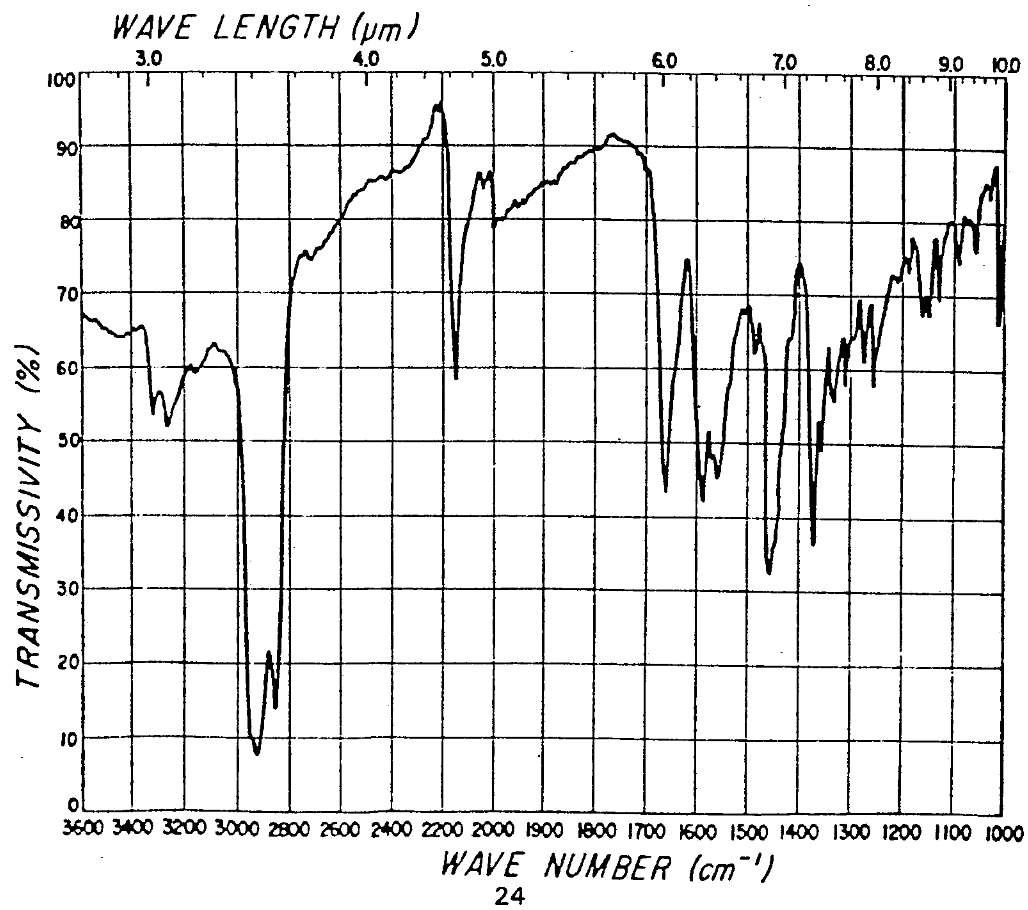
WAVE LENGTH (μm)
3.0 4.0 5.0 6.0 7.0 8.0 9.0 10.0
100 90 80 70 60 50 40 30 20 10 0
TRANSMISSIVITY (%)
3600 3400 3200 3000 2800 2600 2400 2200 2000 1900 1800 1700 1600 1500 1400 1300 1200 1100 1000
WAVE NUMBER ($cm^{-1}$)

FIG. 49

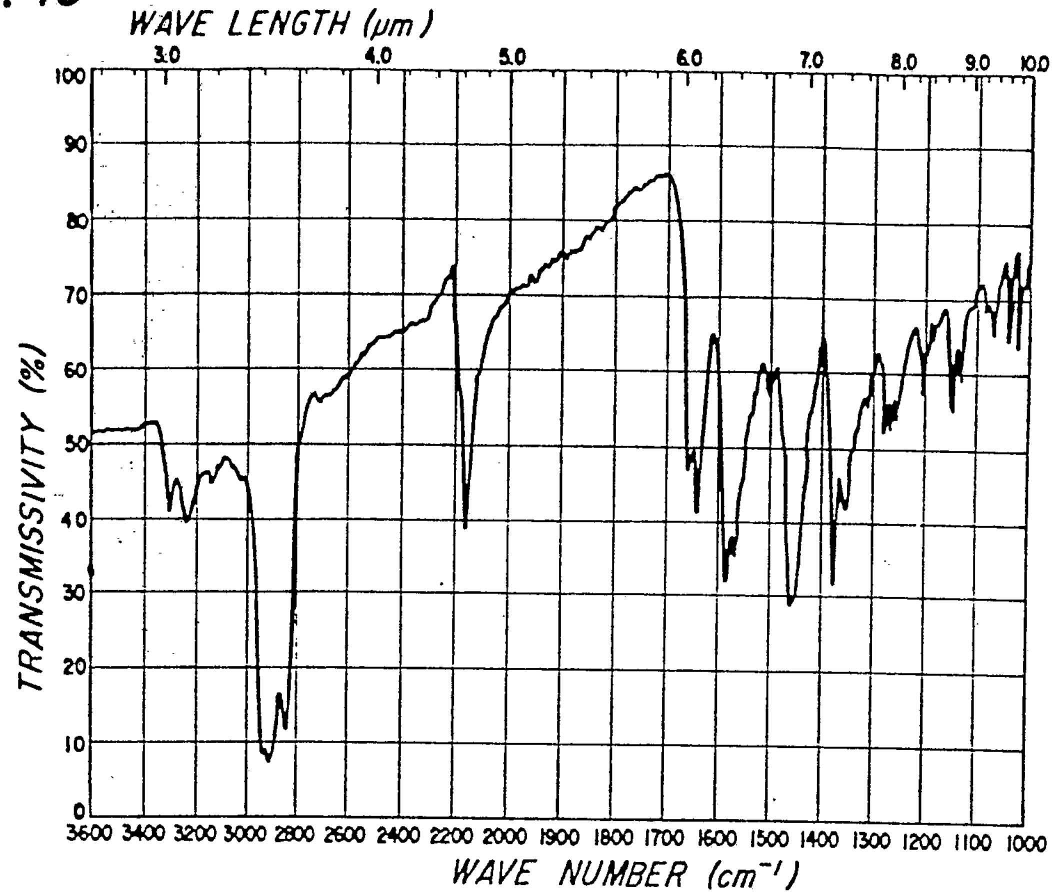
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER ($cm^{-1}$)

FIG. 50

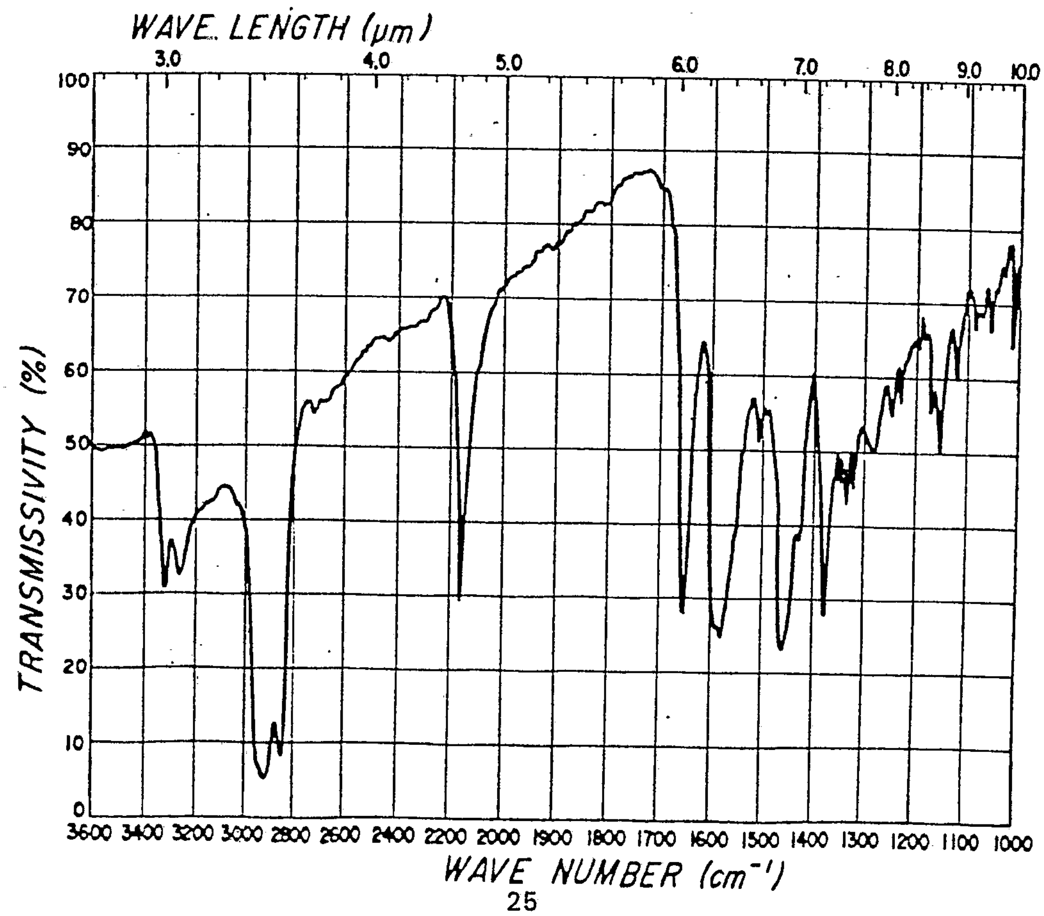
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER ($cm^{-1}$)

FIG. 51

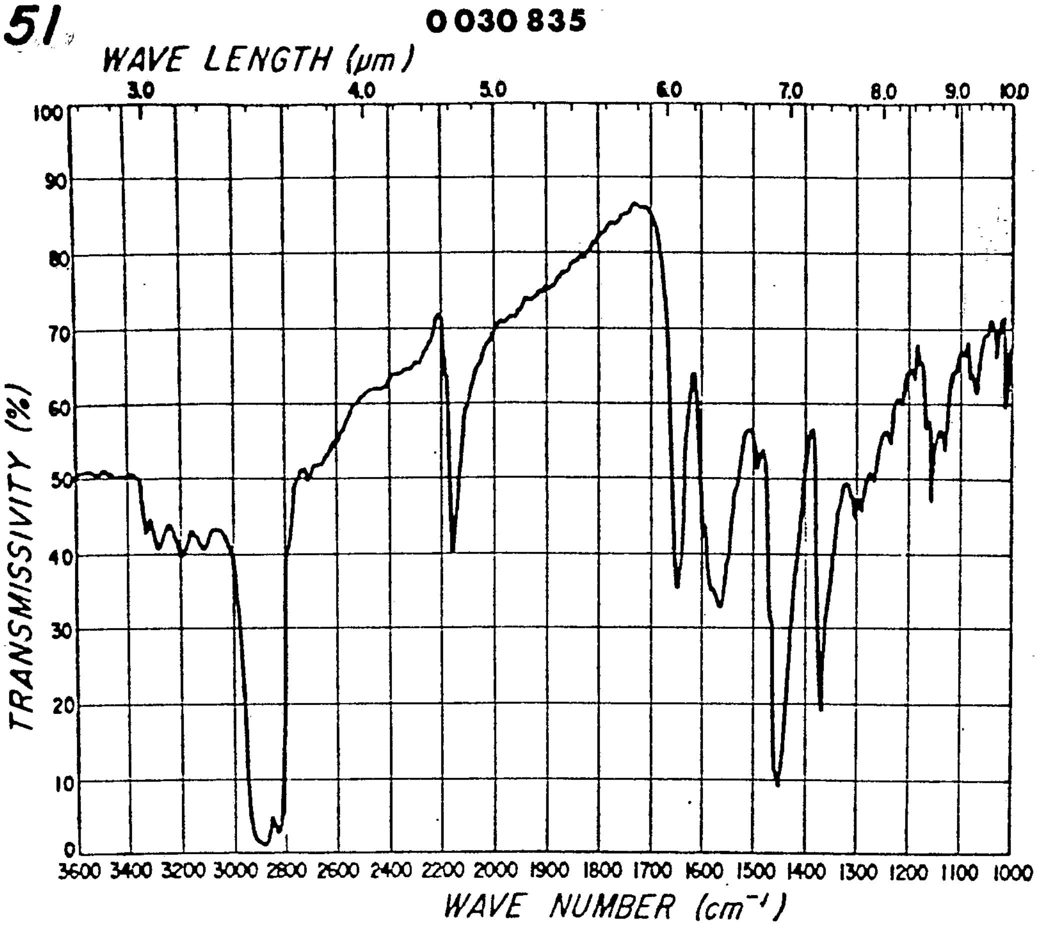
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER ($cm^{-1}$)

FIG. 52

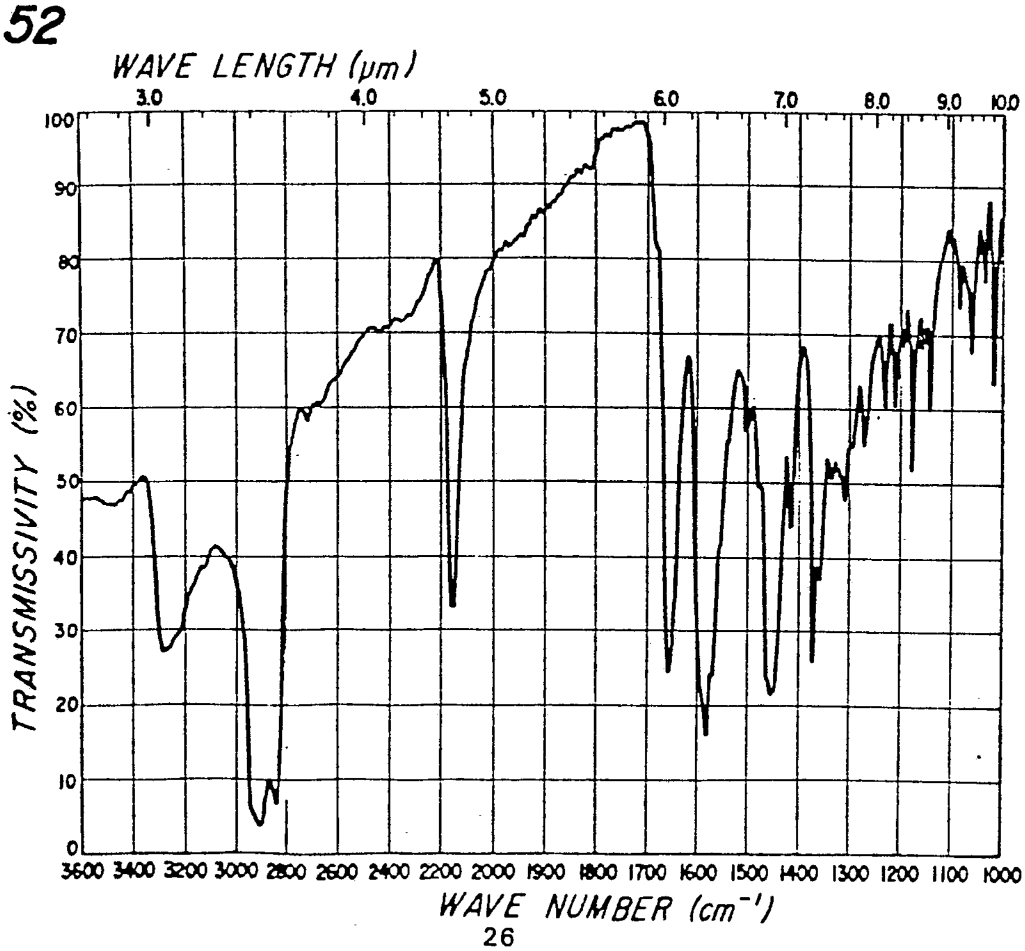
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER ($cm^{-1}$)

FIG. 53

0 030 835

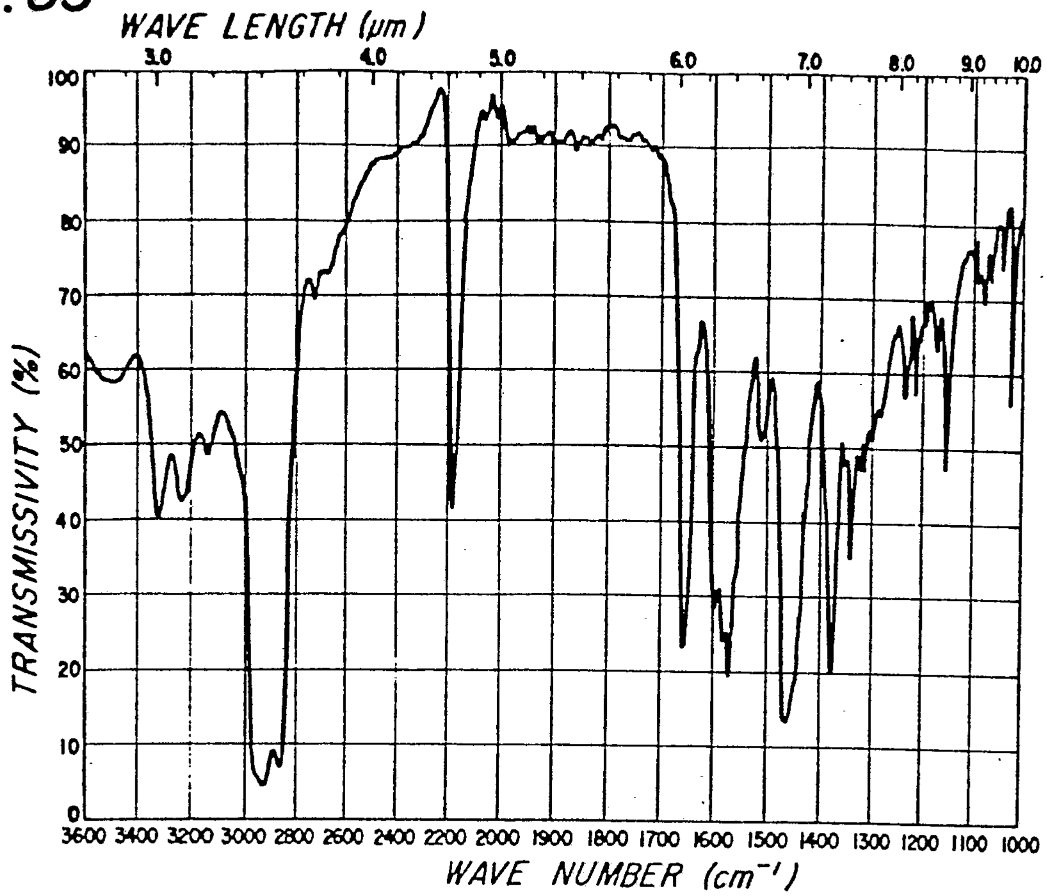


FIG. 54

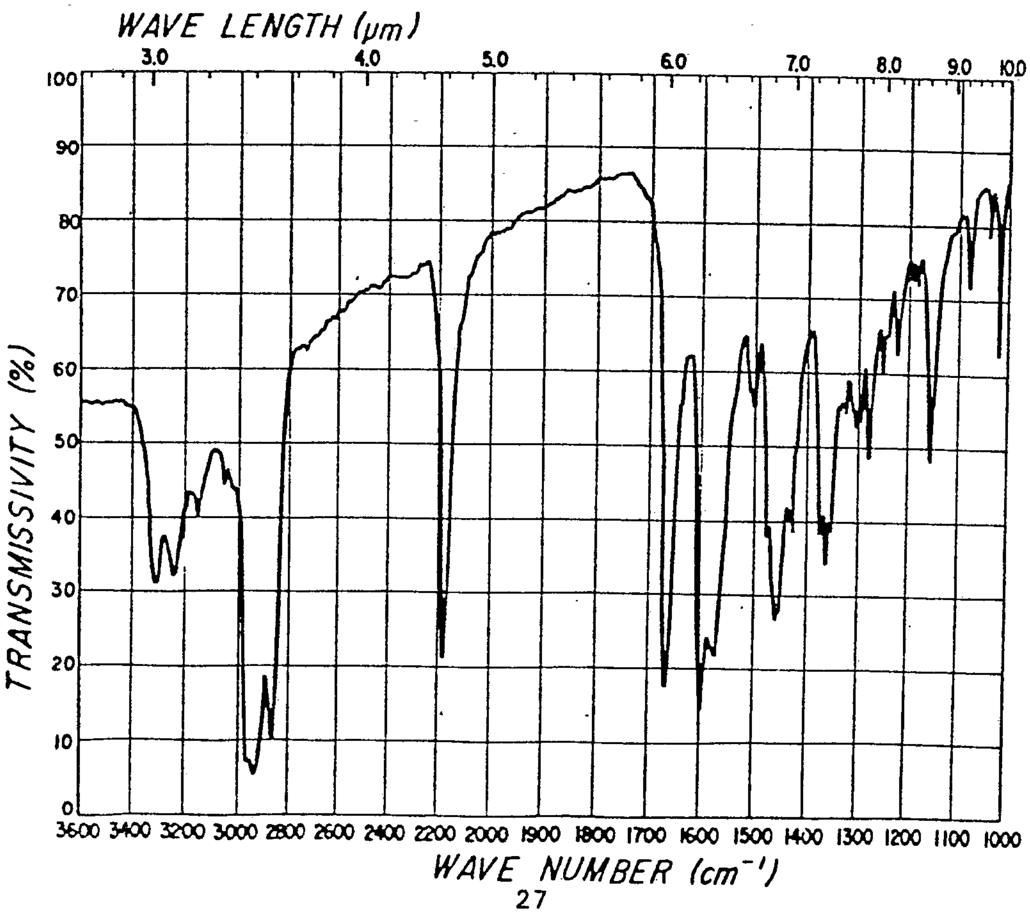

FIG. 55

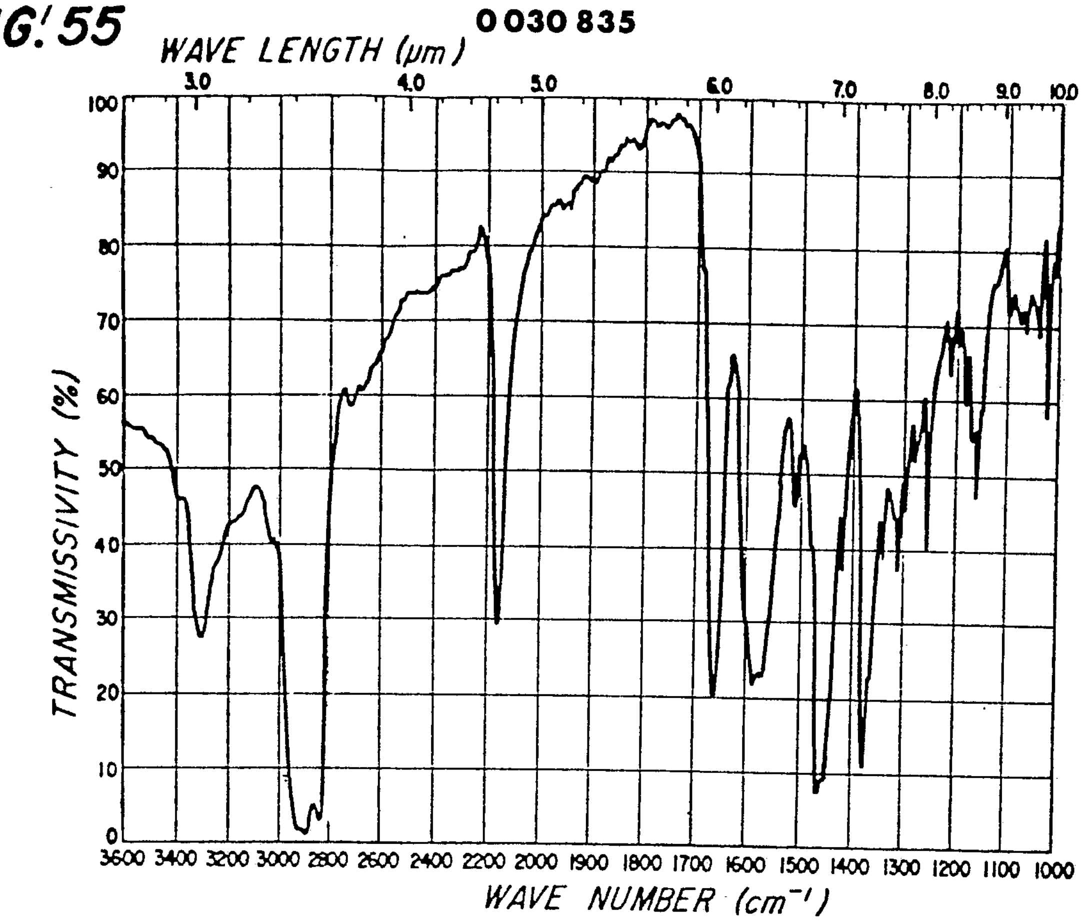
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 56

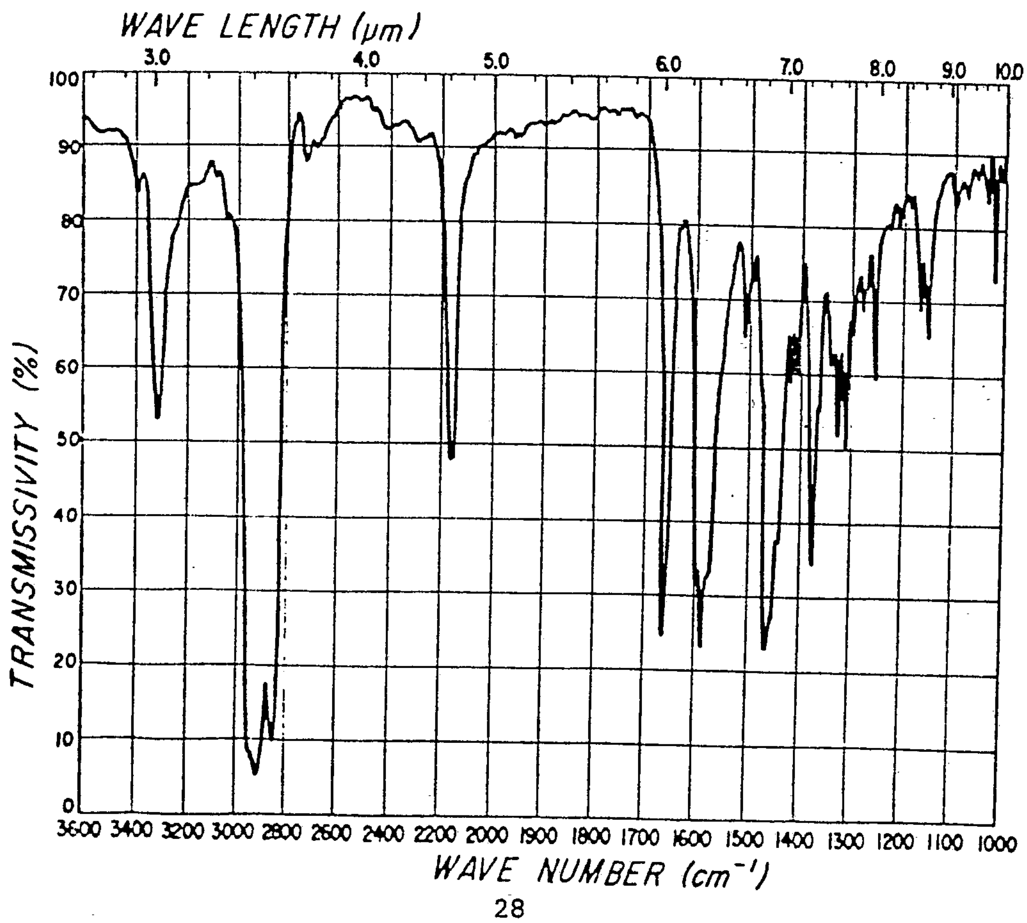
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 57

0030835

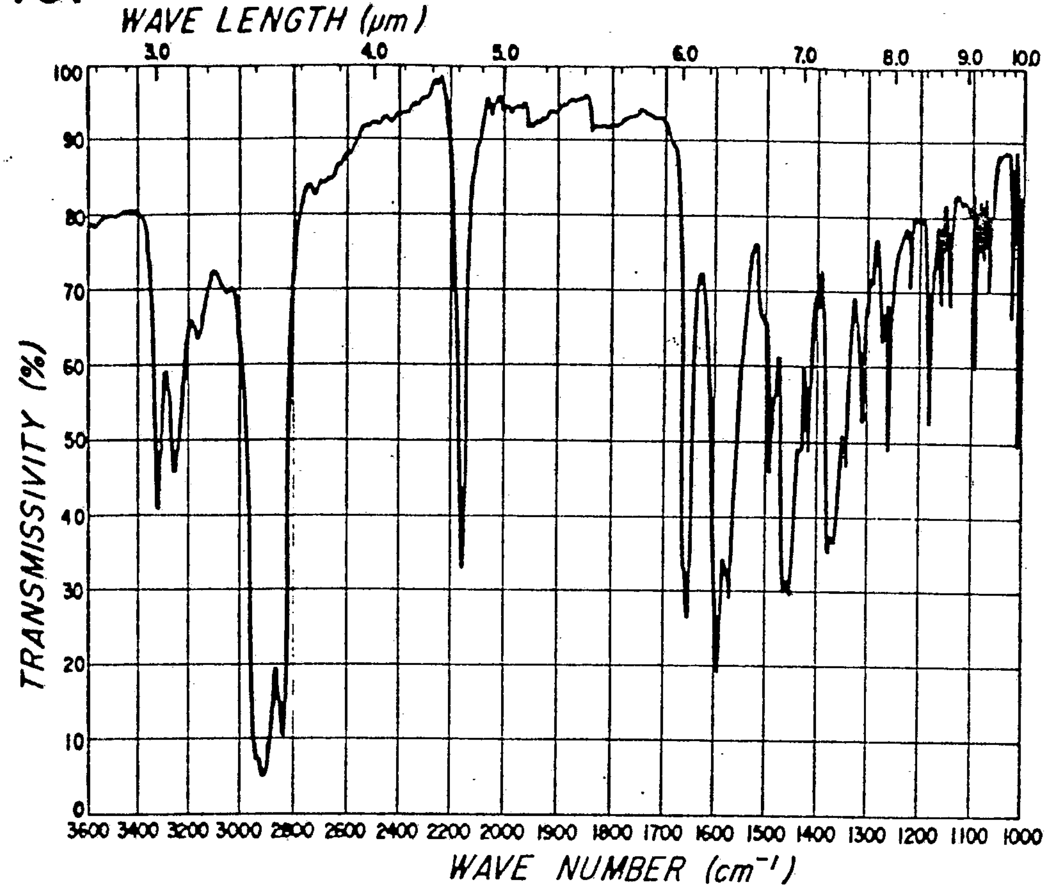


FIG. 58

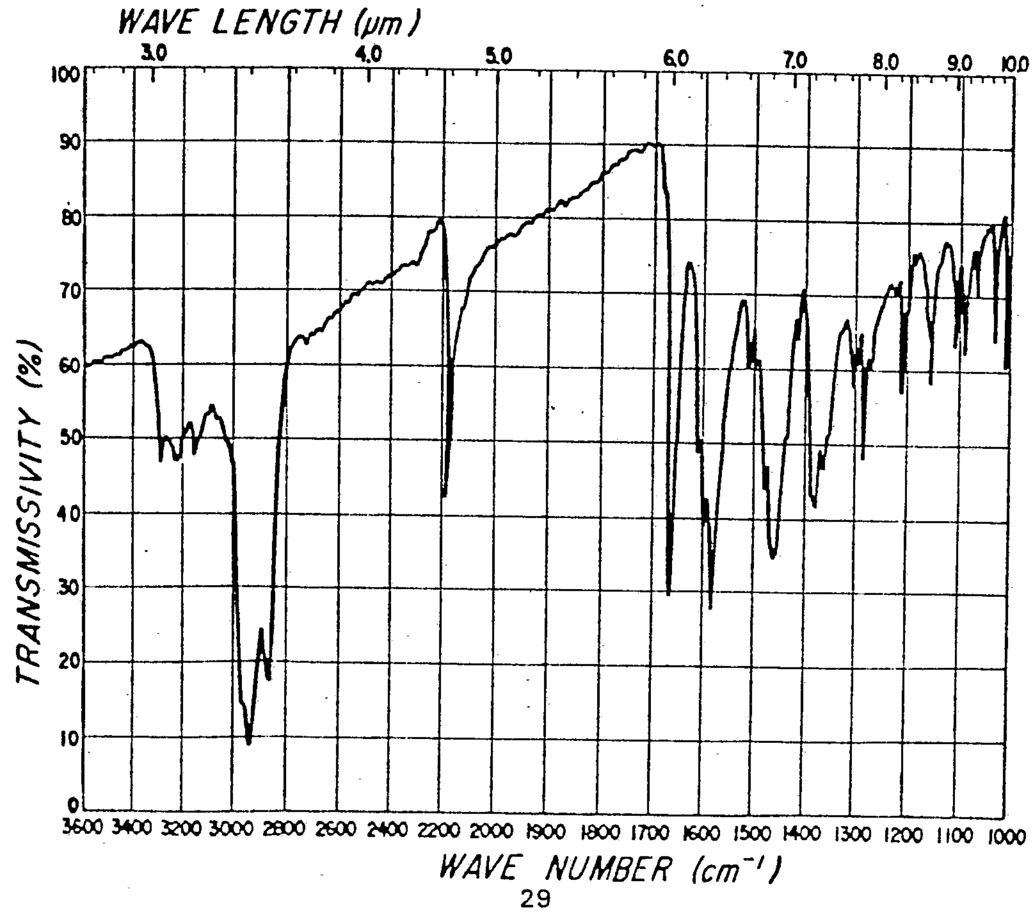

FIG. 59

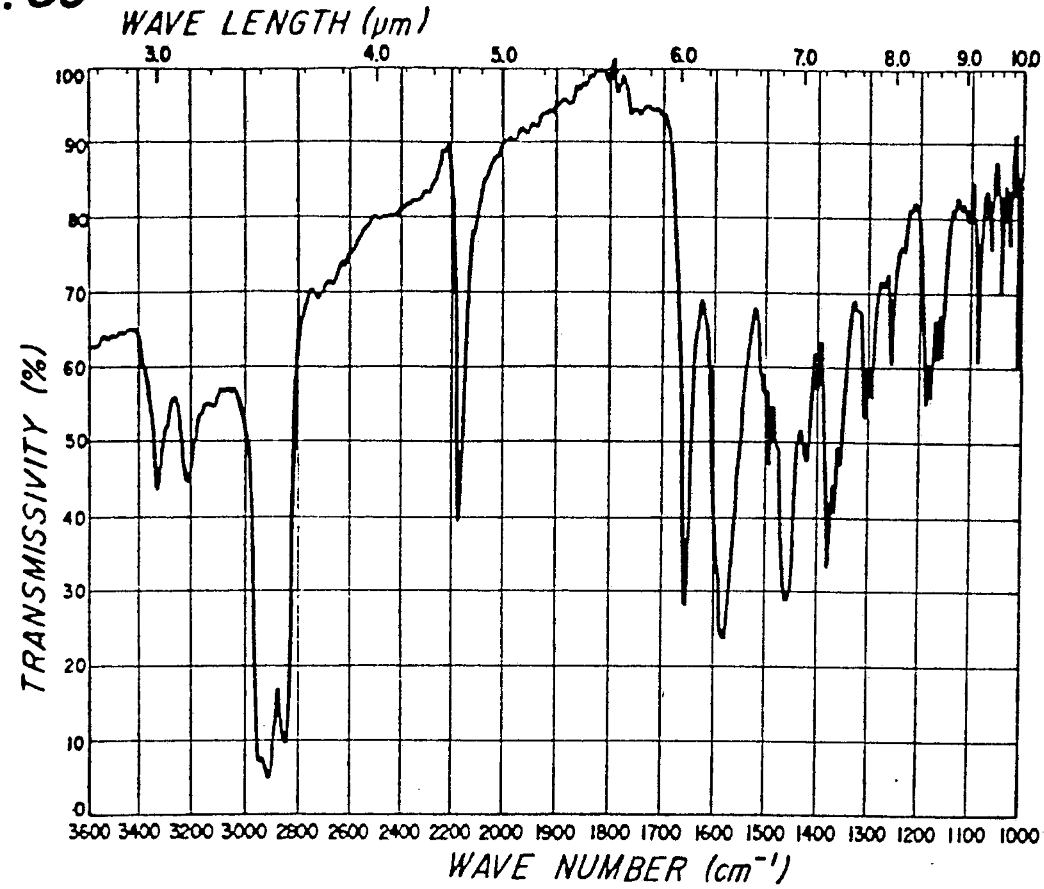
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 60

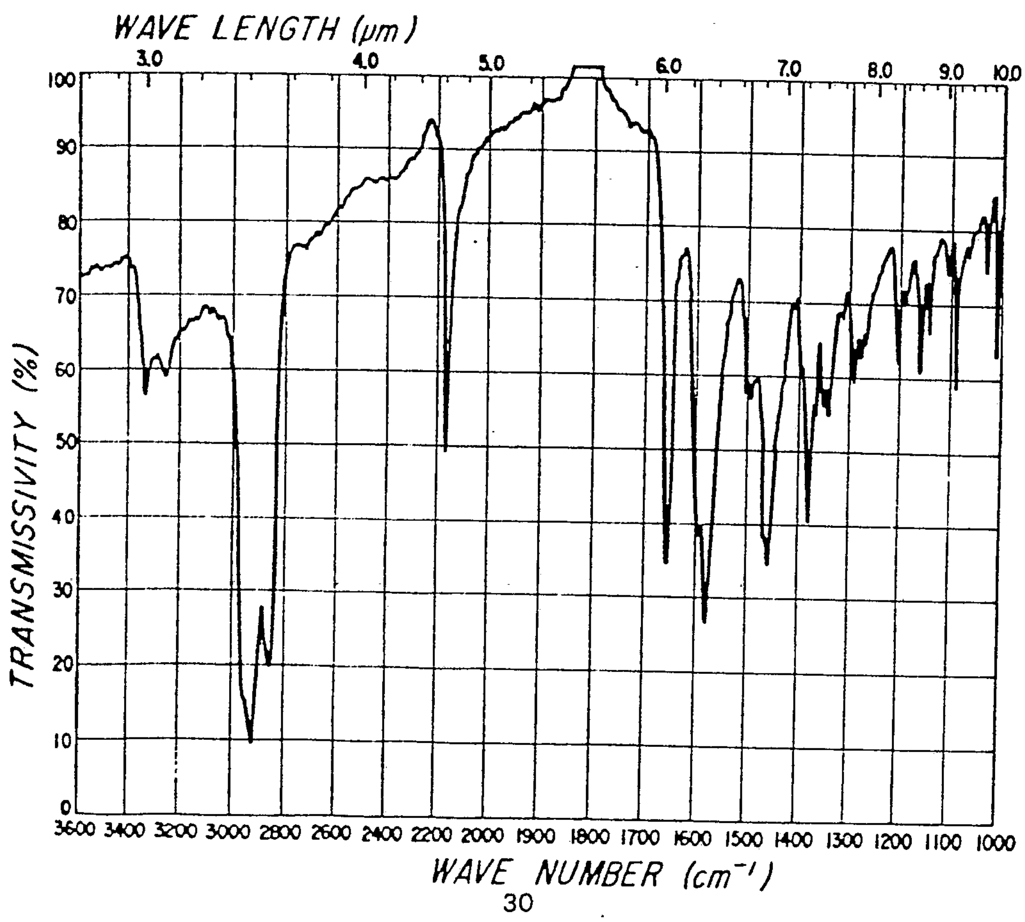
WAVE LENGTH (μm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 61

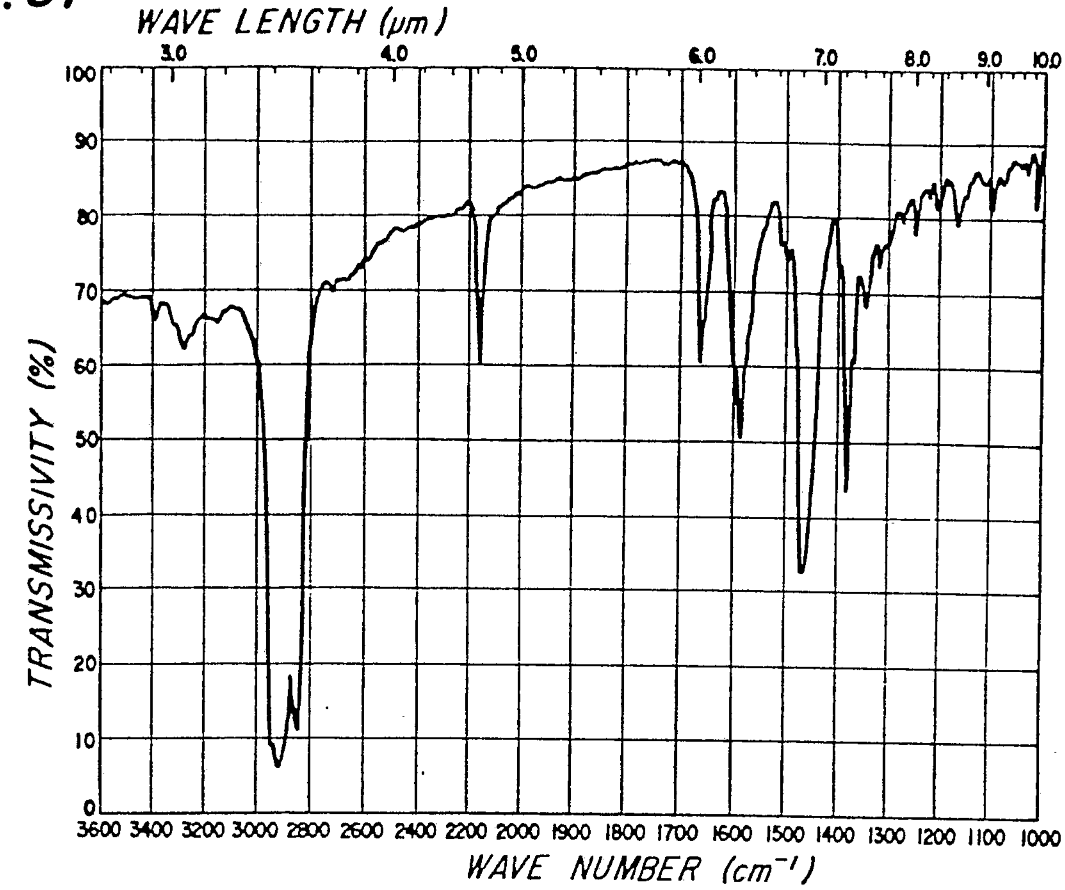
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 62

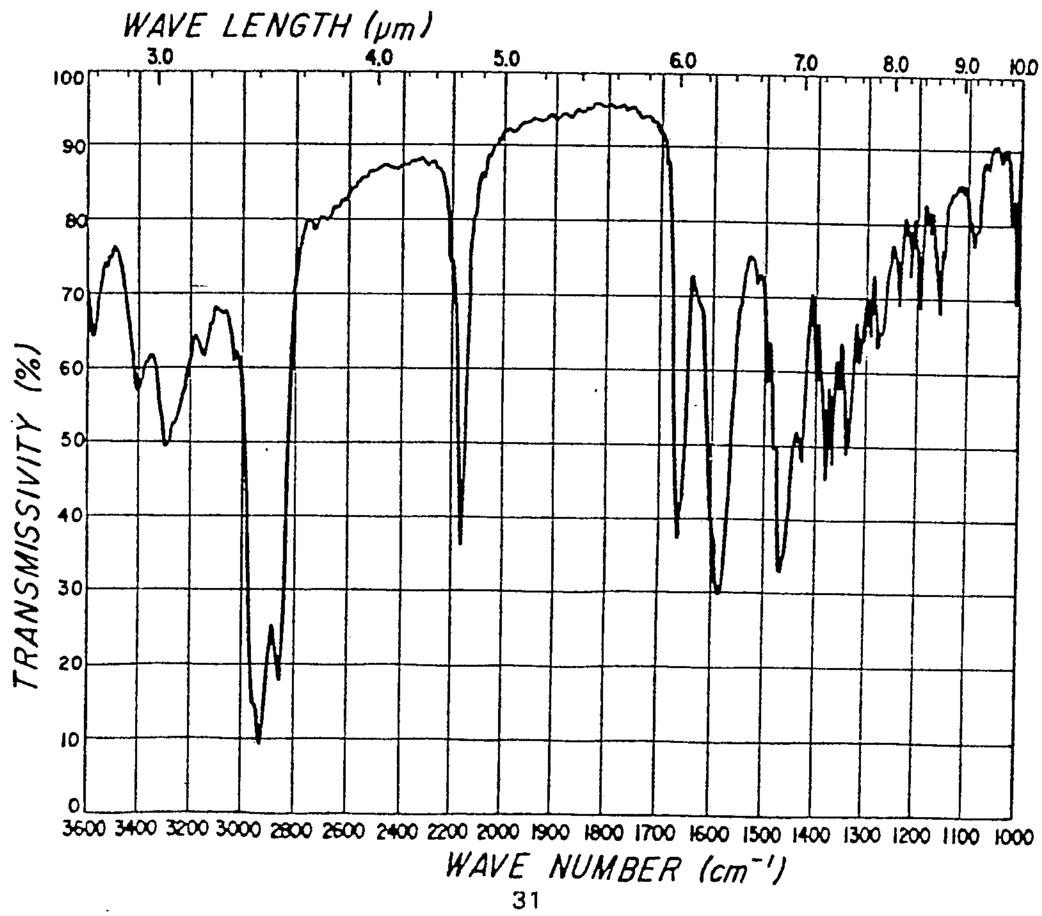
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 63

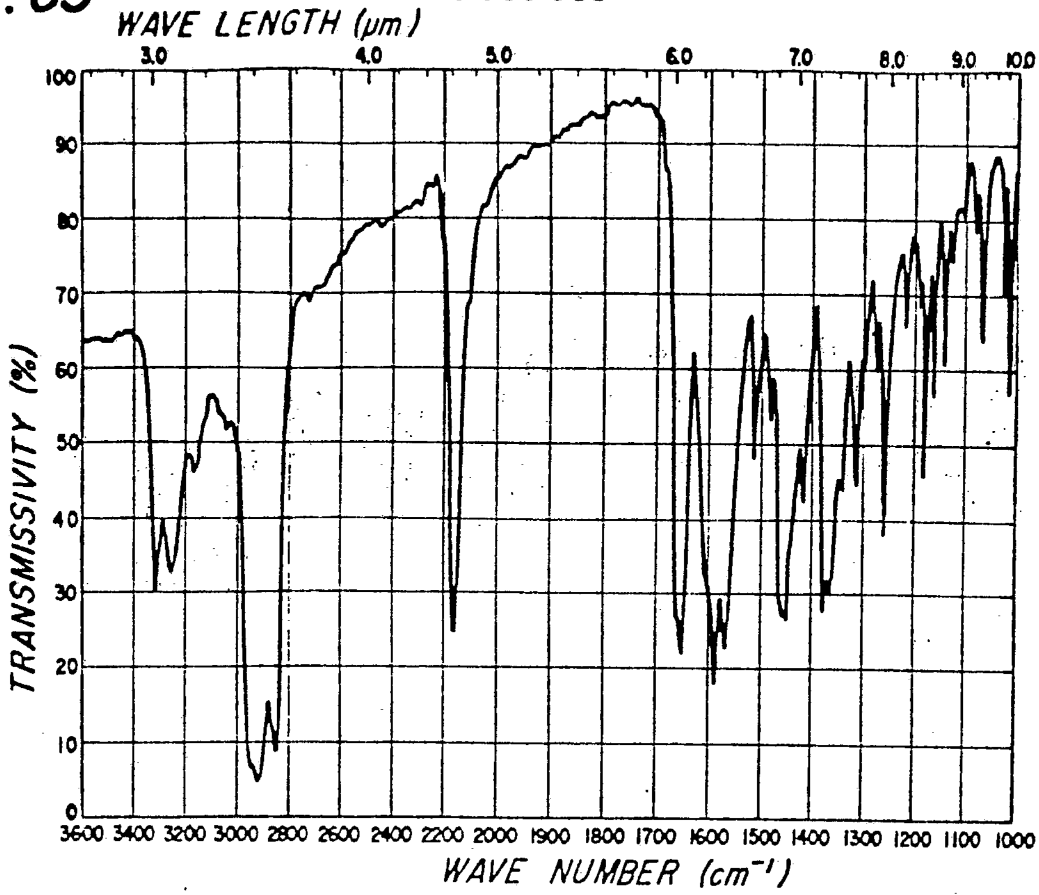
WAVE LENGTH (µm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 64

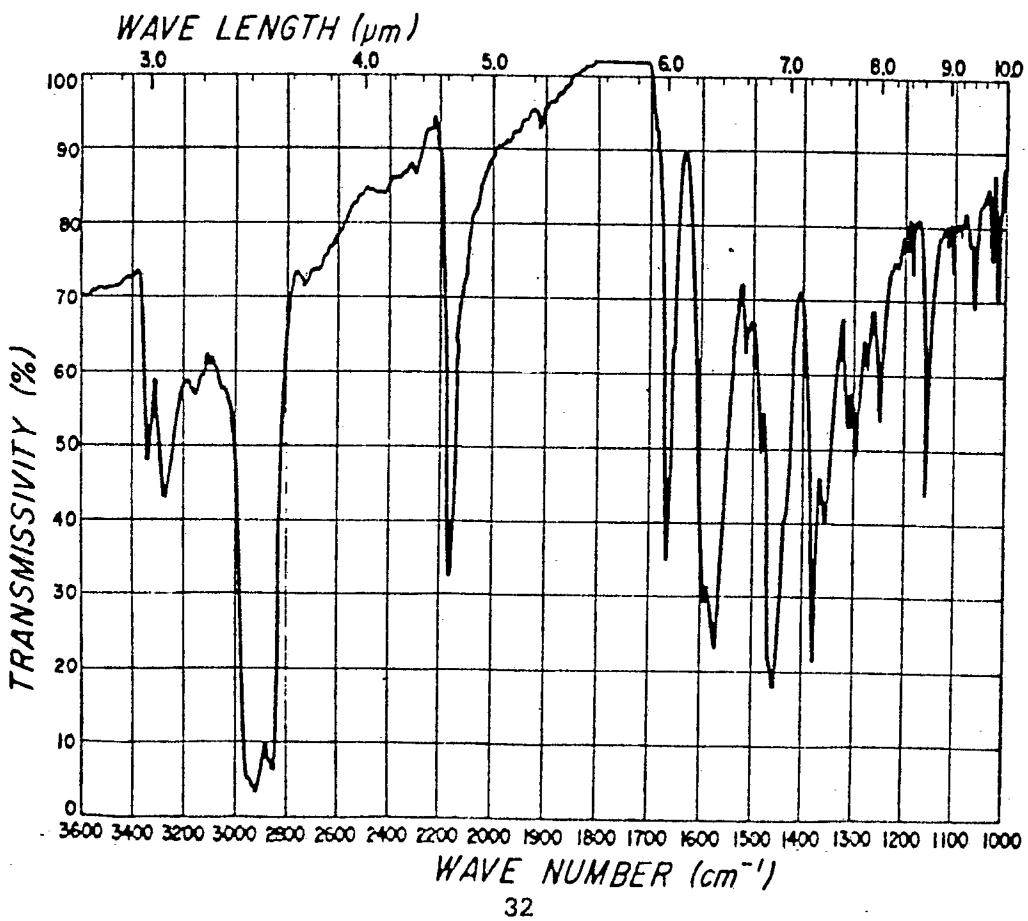
WAVE LENGTH (µm)
3.0 4.0 5.0 6.0 7.0 8.0 9.0 10.0
TRANSMISSIVITY (%)
0 10 20 30 40 50 60 70 80 90 100
3600 3400 3200 3000 2800 2600 2400 2200 2000 1900 1800 1700 1600 1500 1400 1300 1200 1100 1000
WAVE NUMBER (cm⁻¹)

FIG. 65

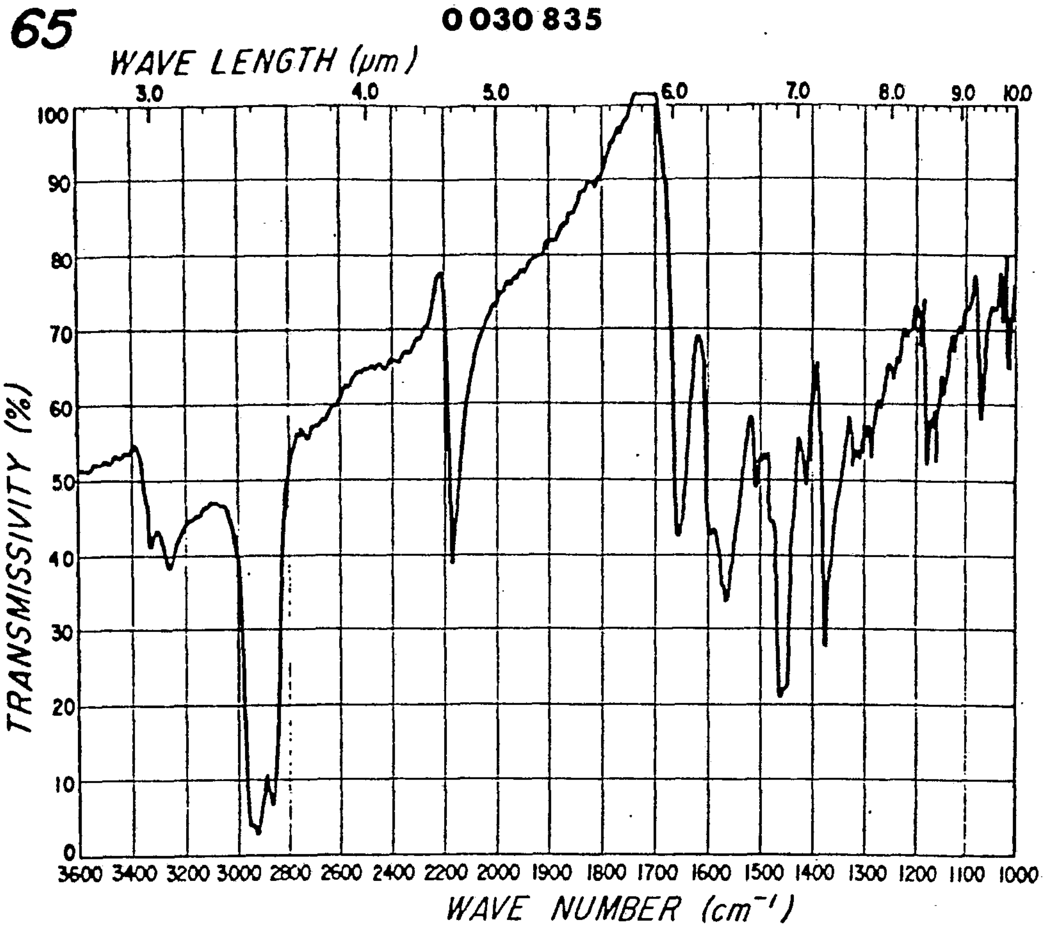
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG. 66

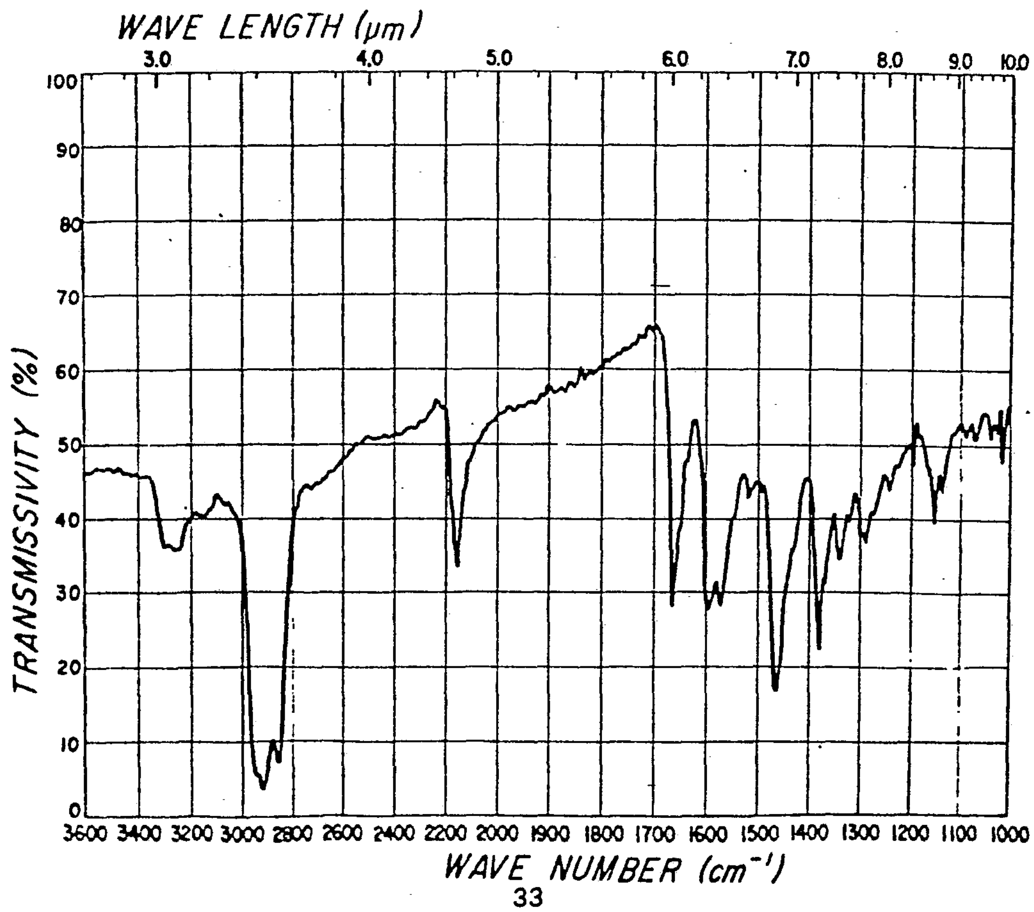
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG. 67

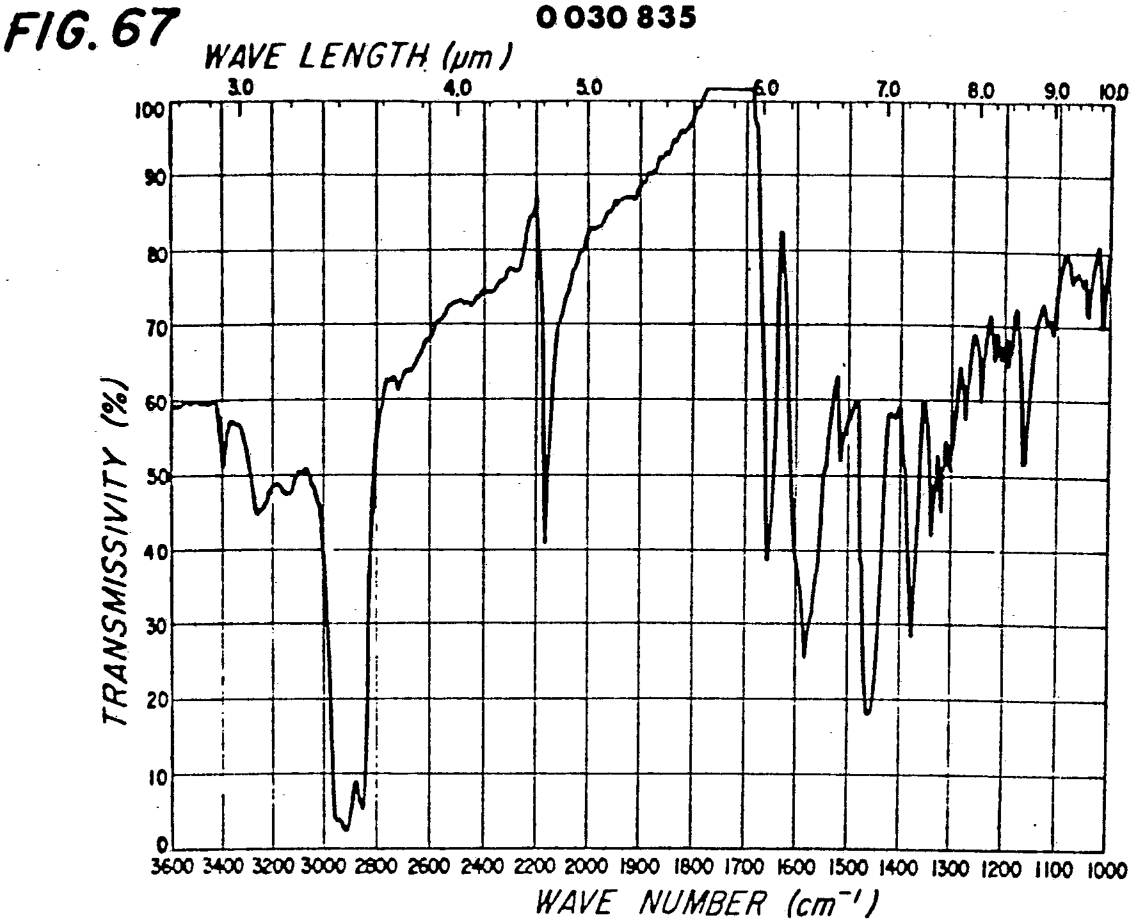
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 68

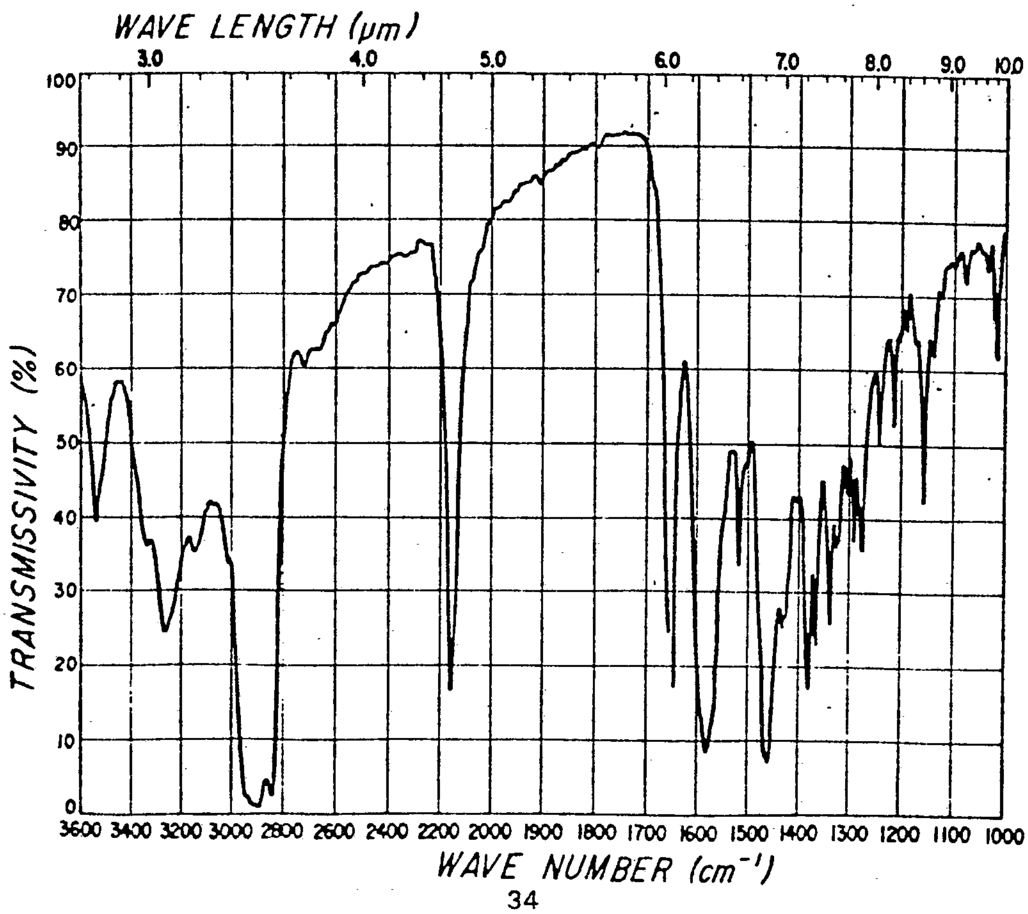
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 69

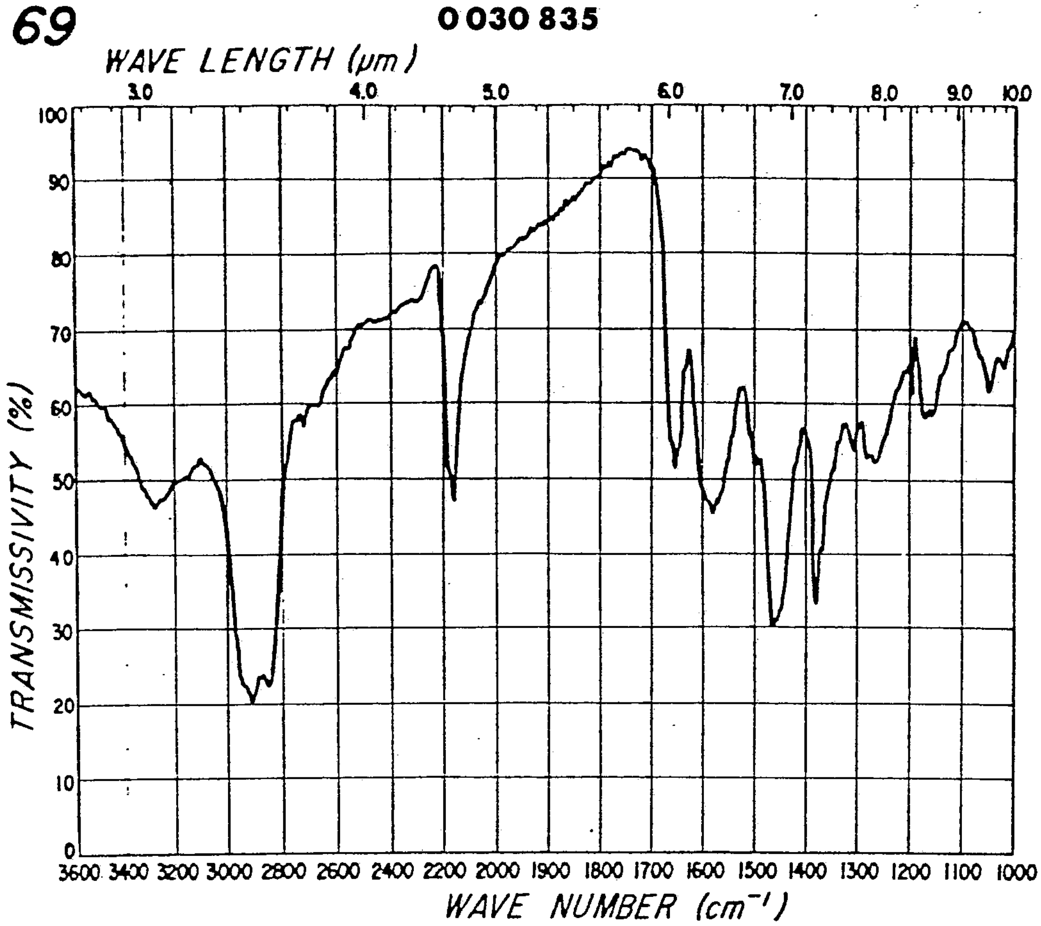
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 70

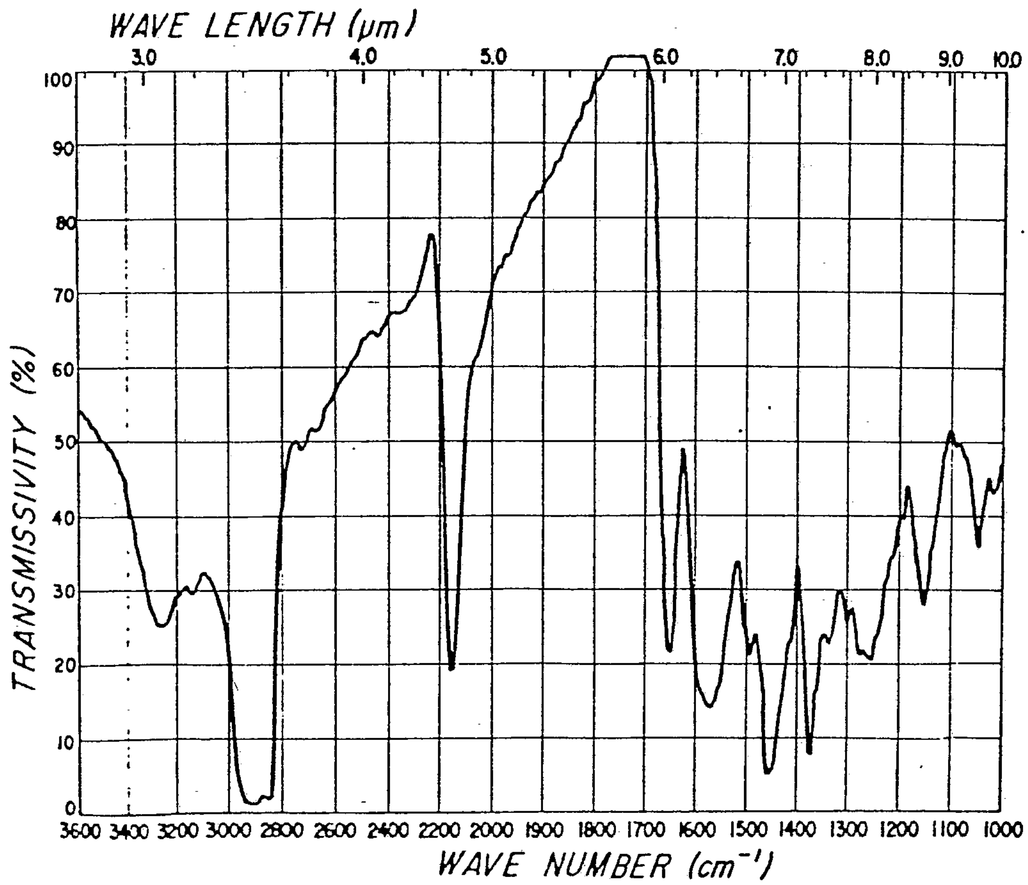
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG.71

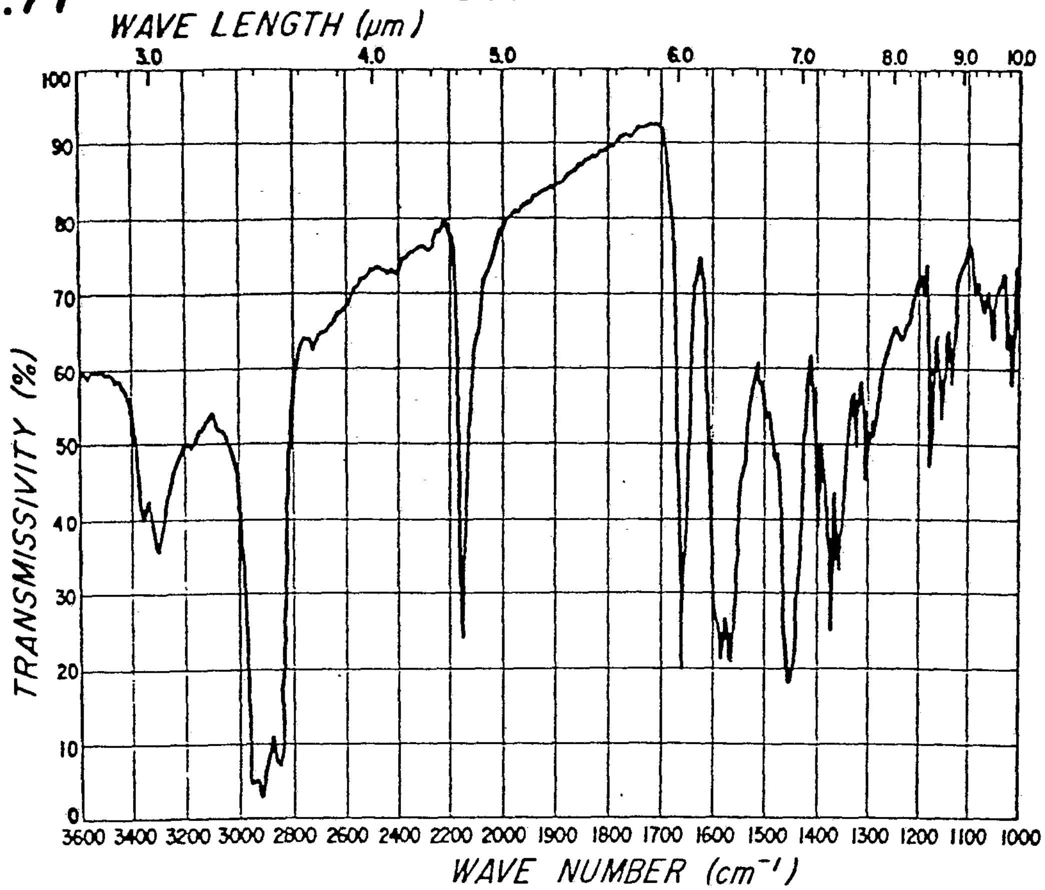
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG.72

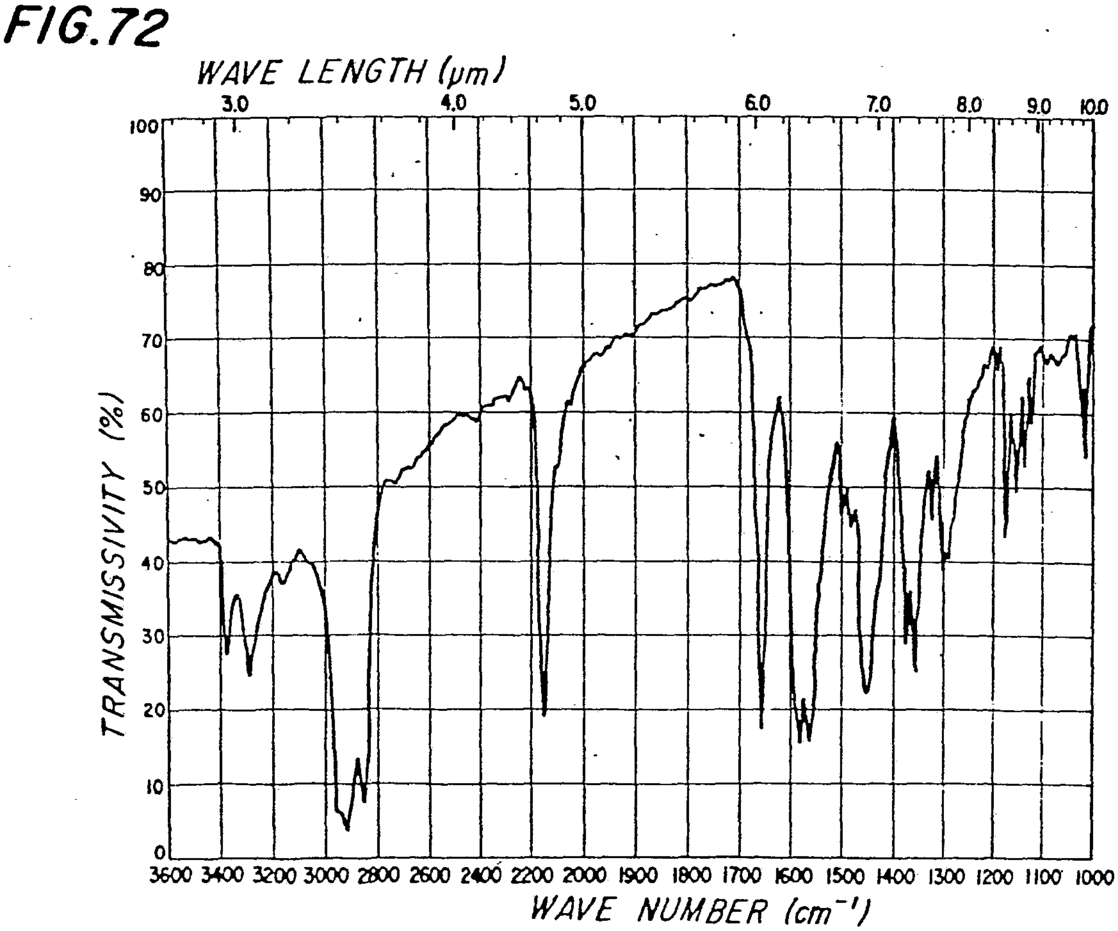
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG. 73

0030835

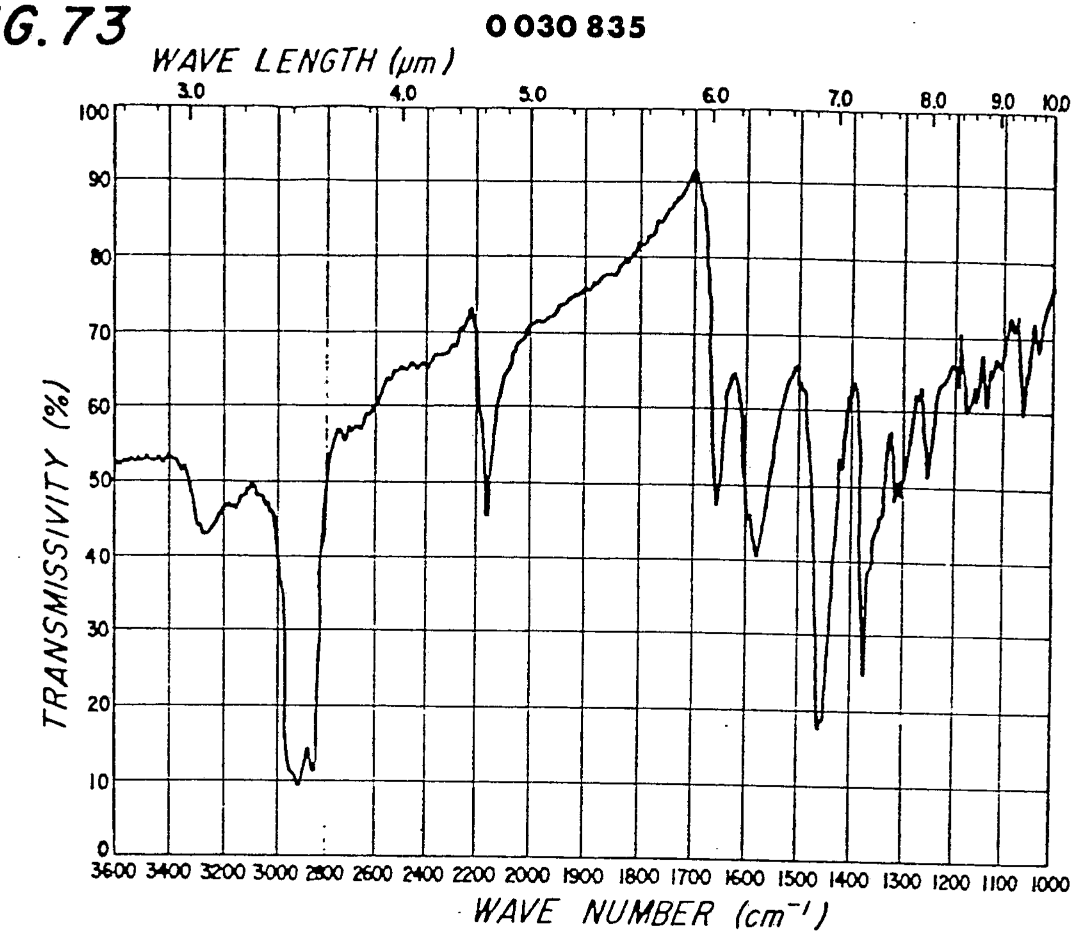


FIG. 74

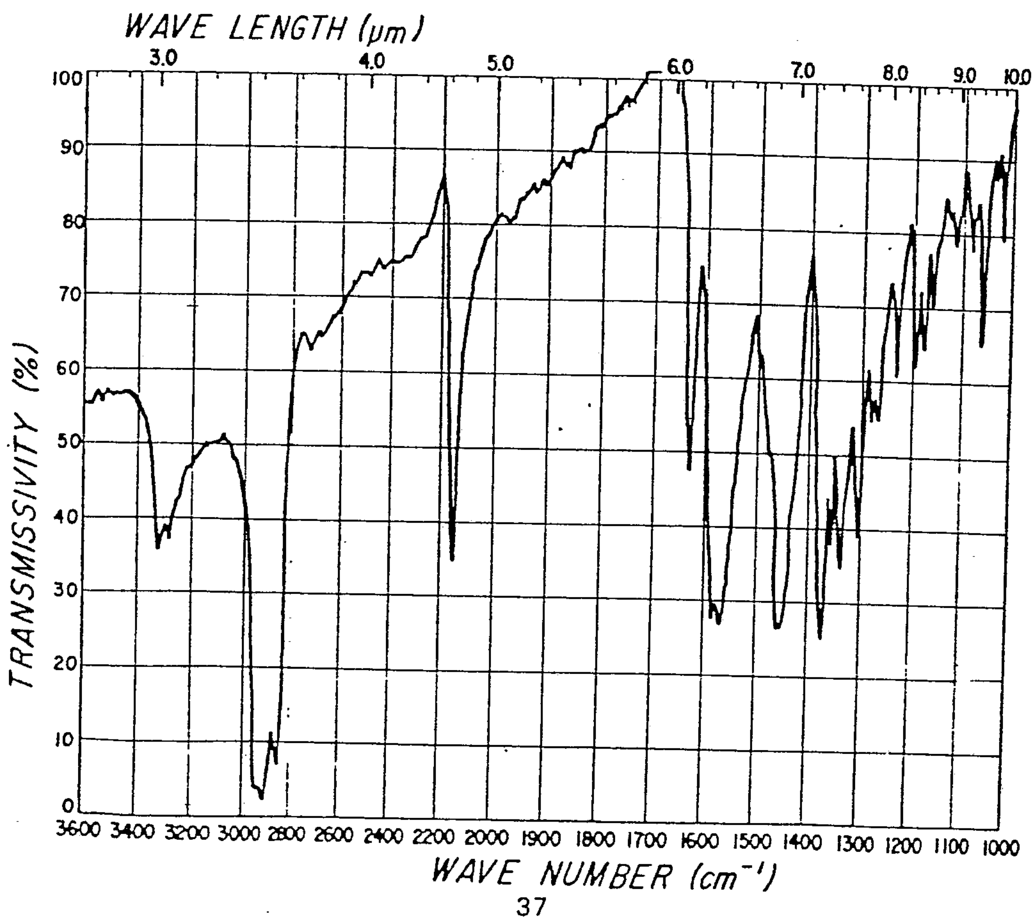

FIG.75

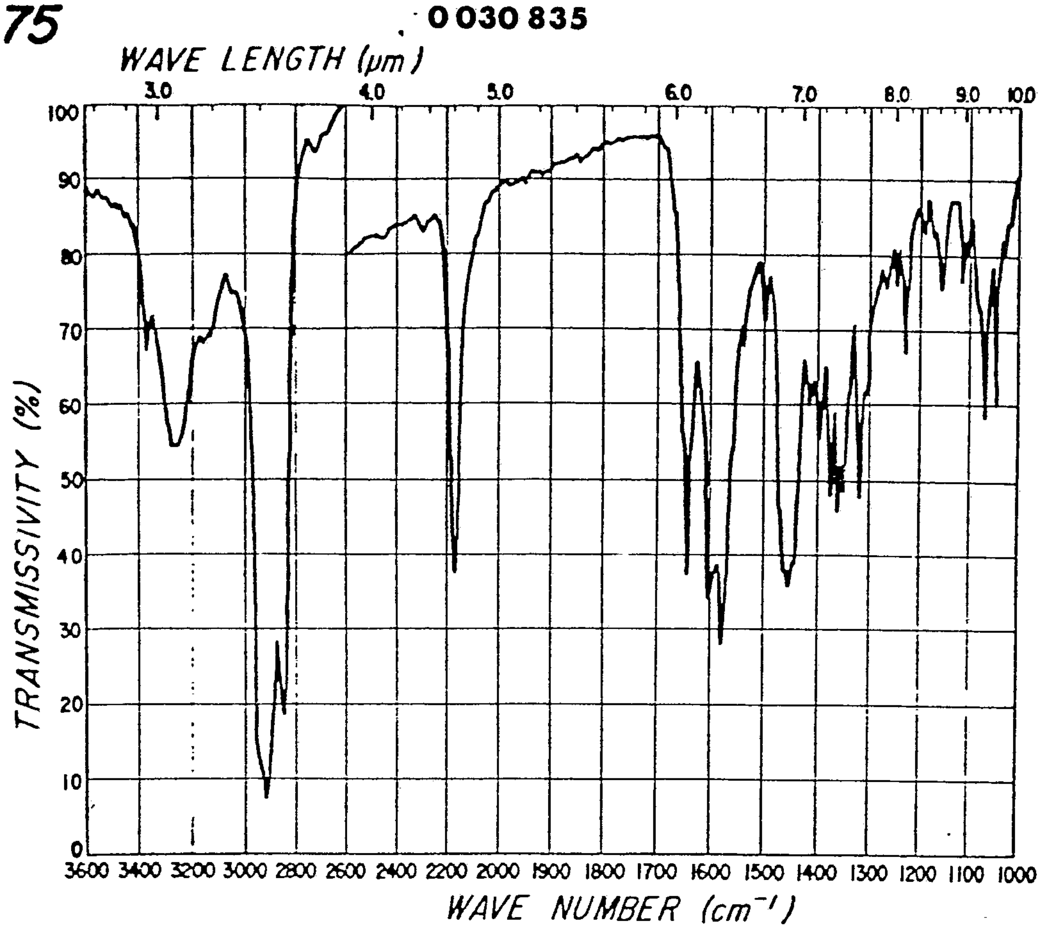
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG.76

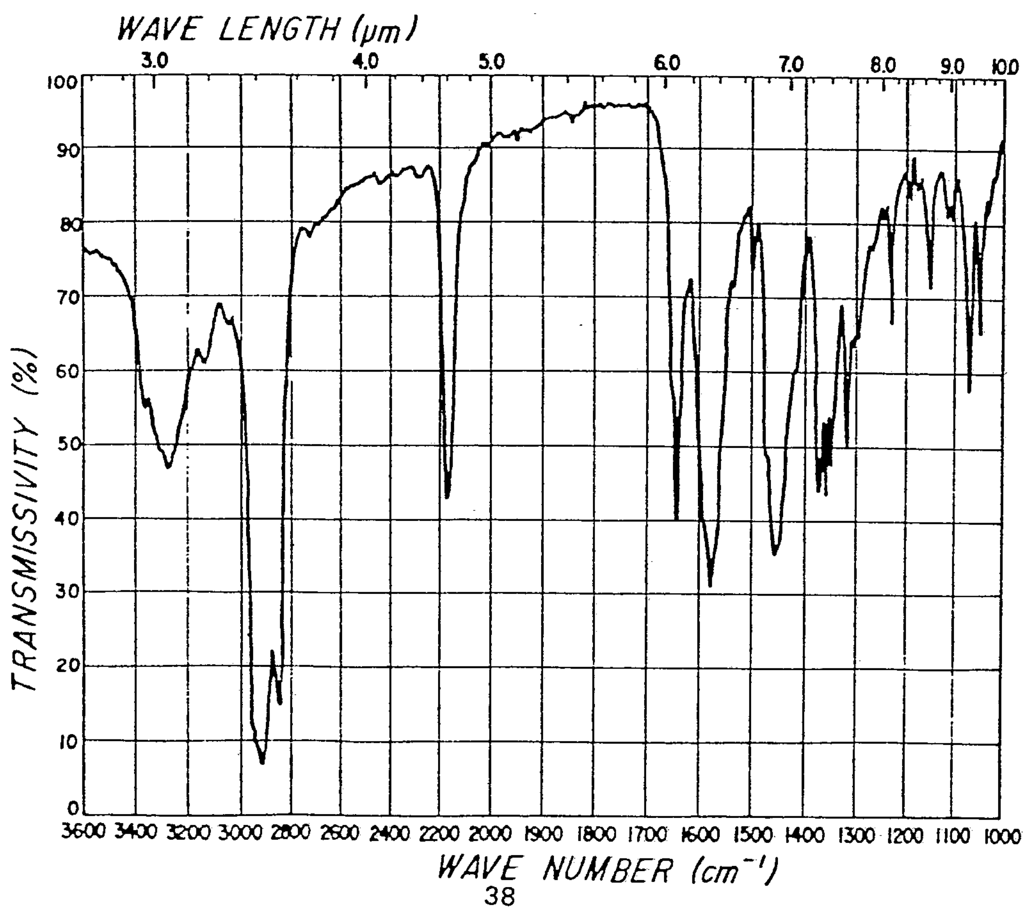
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)

FIG.77

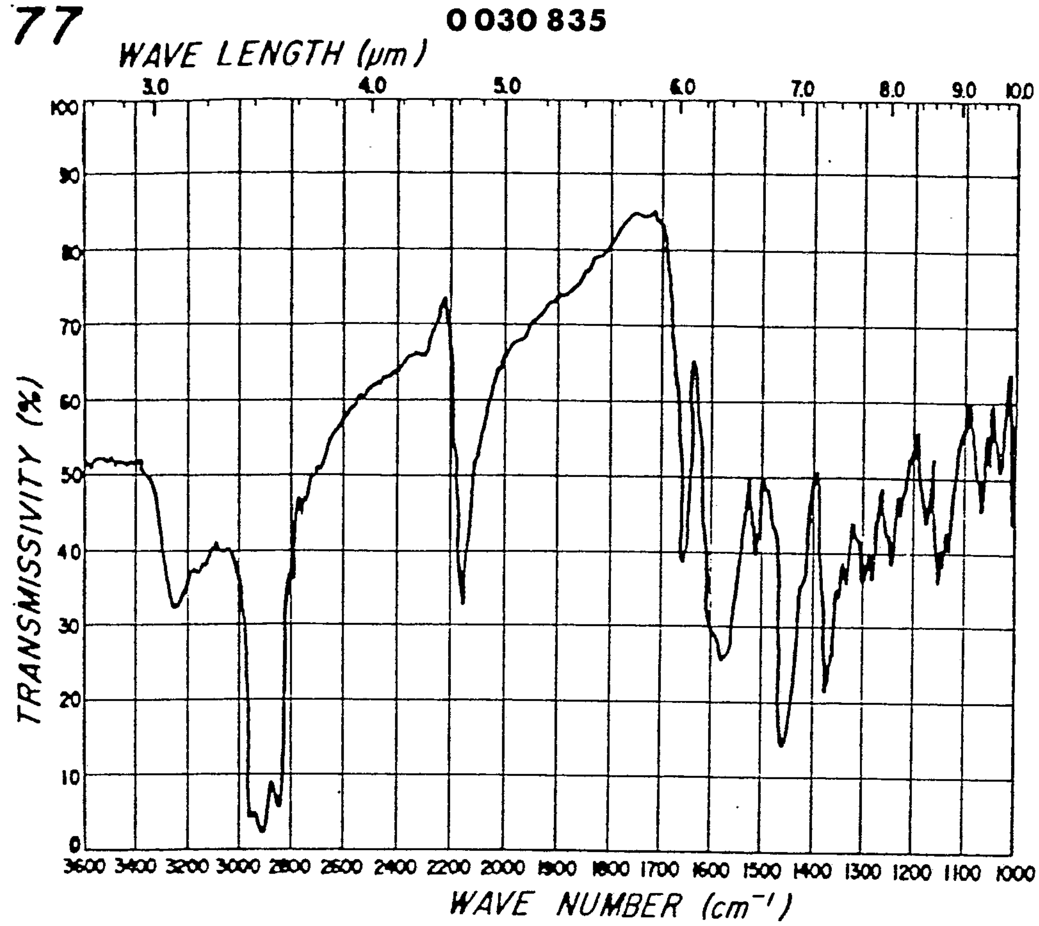
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG.78

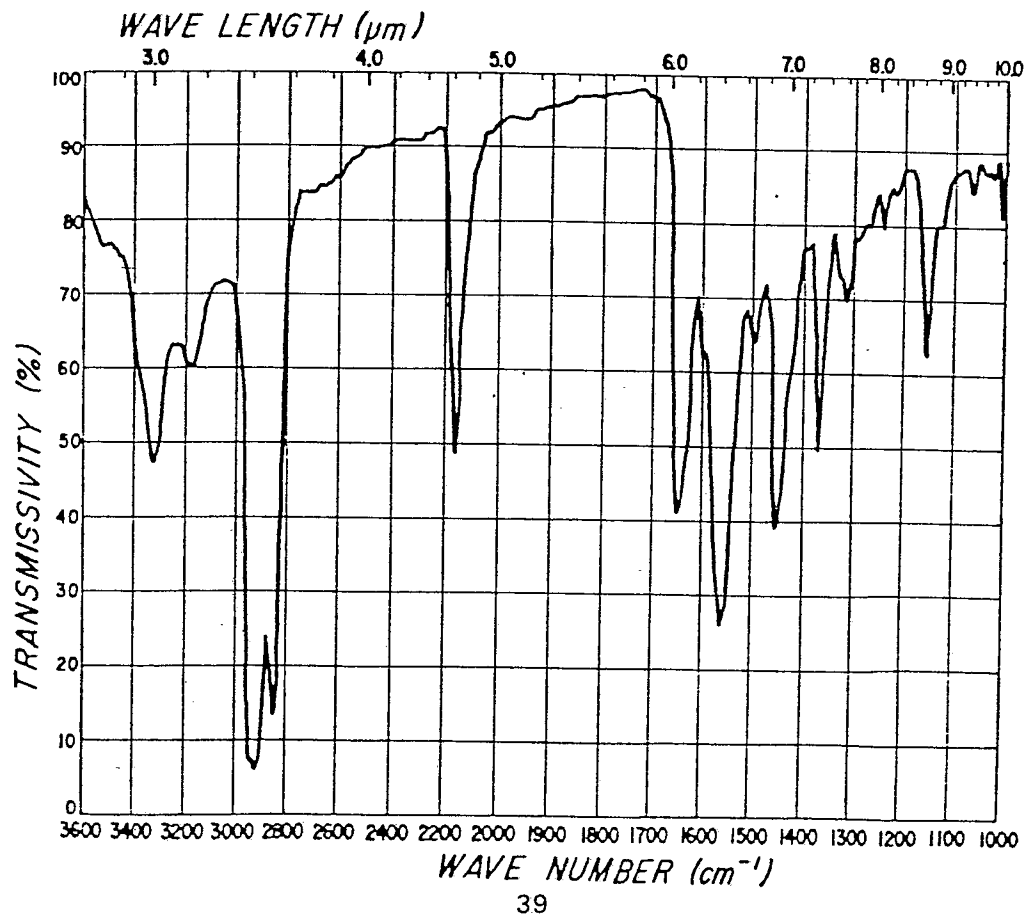
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 79

0 030 835

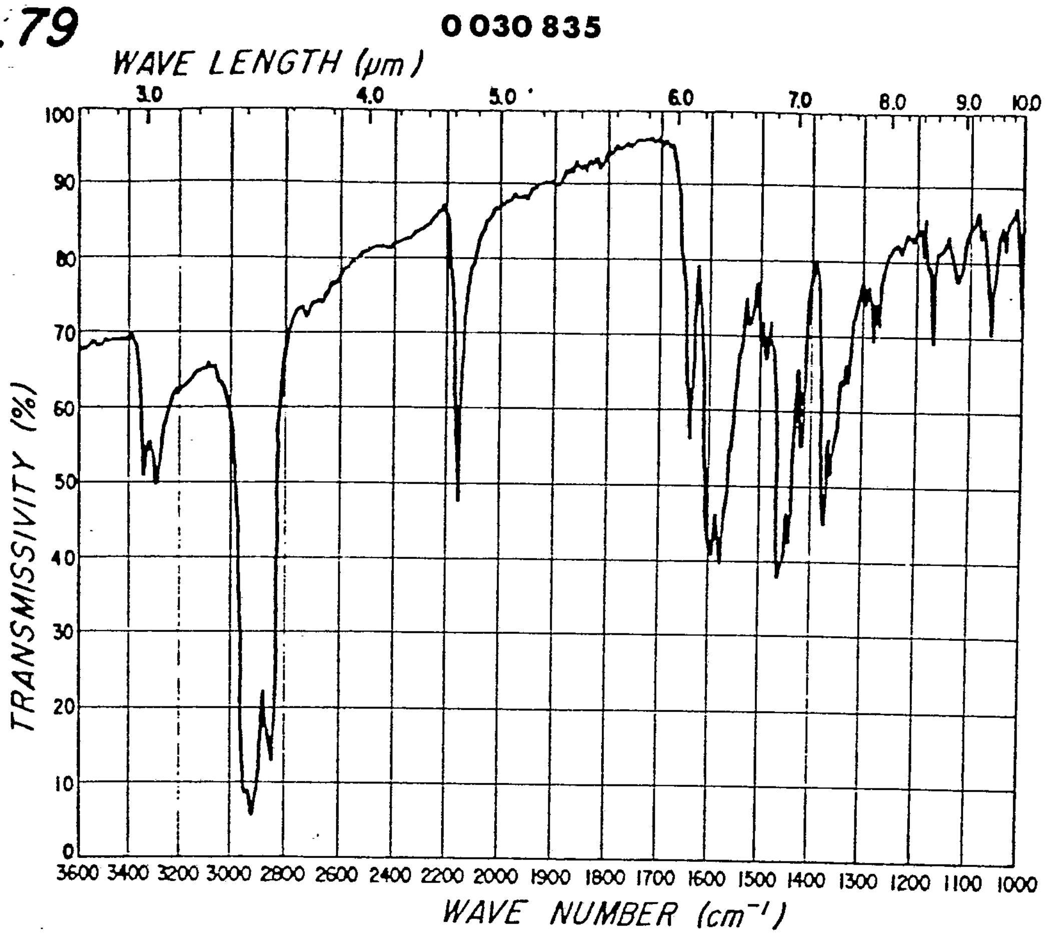


FIG. 80

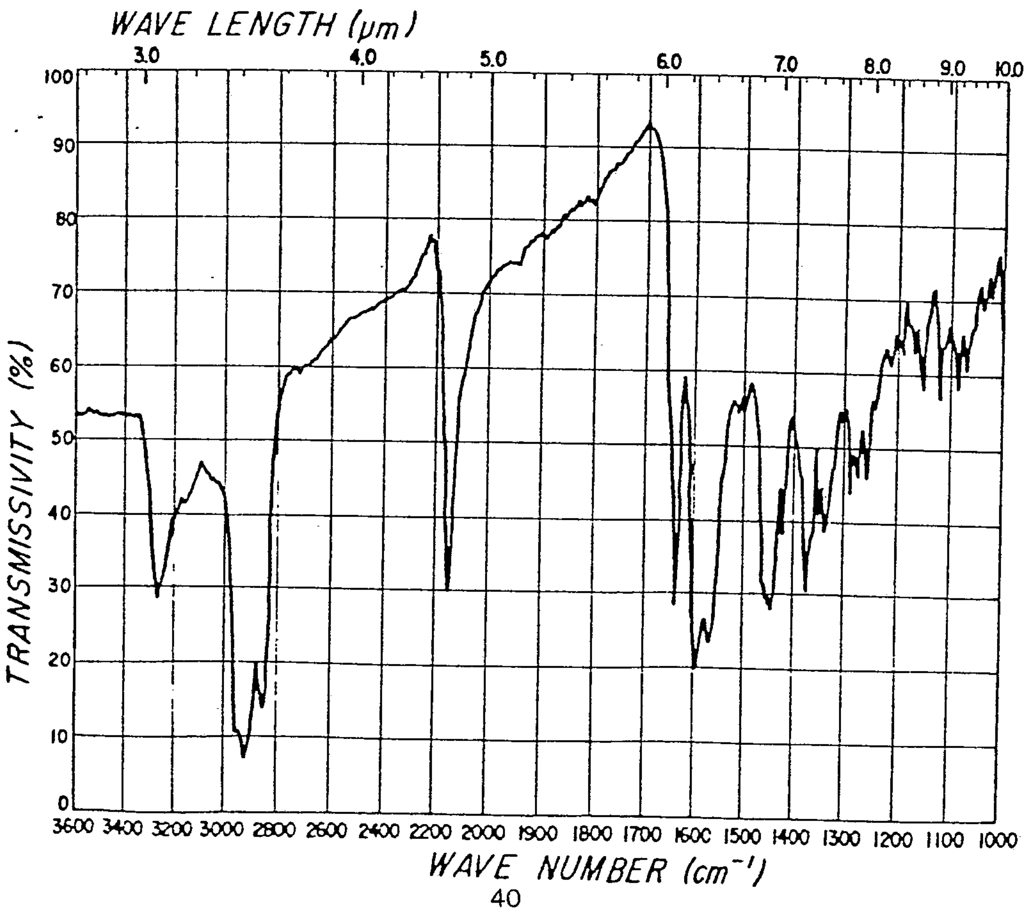

FIG. 81

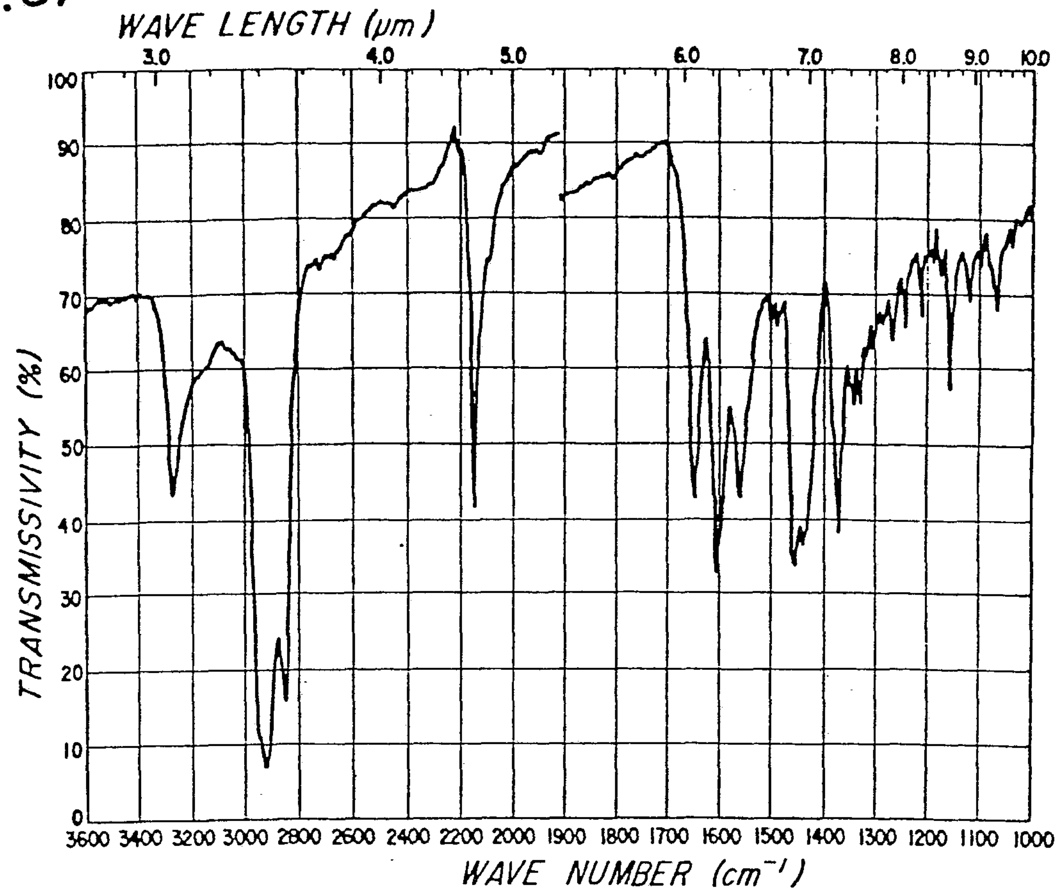
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 82

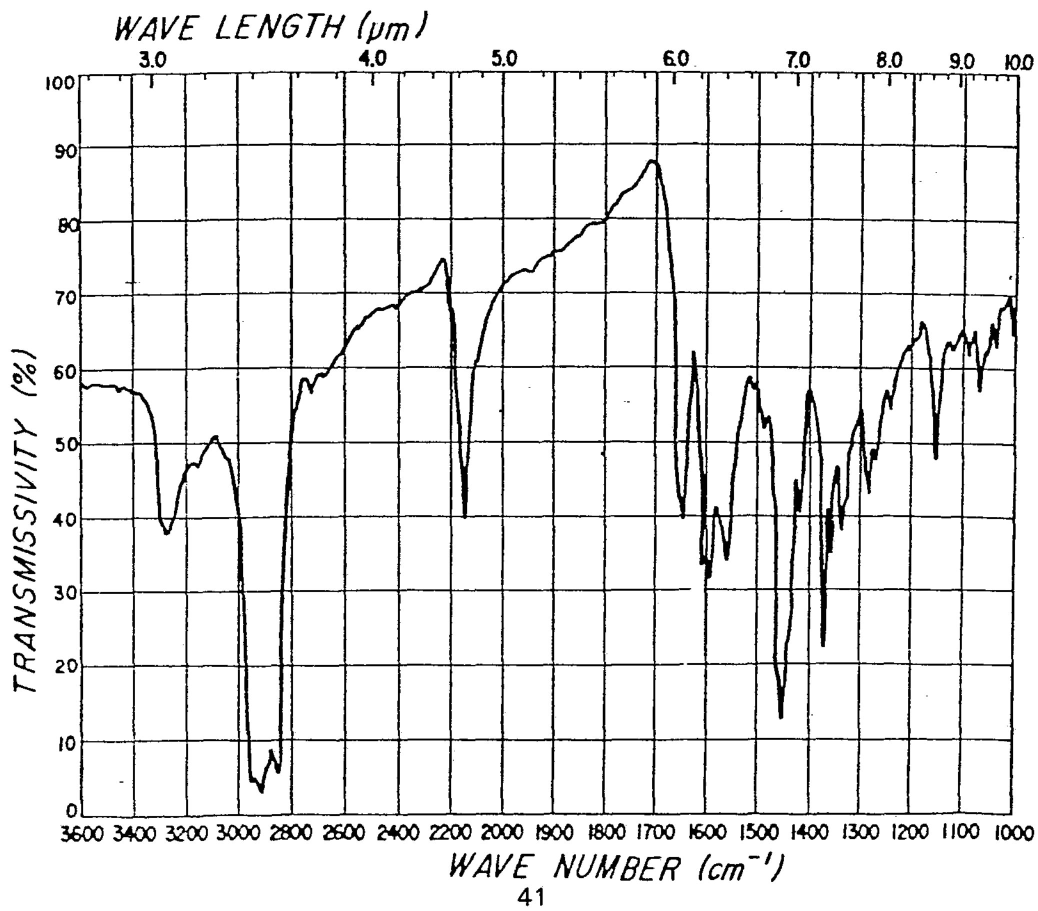
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 83



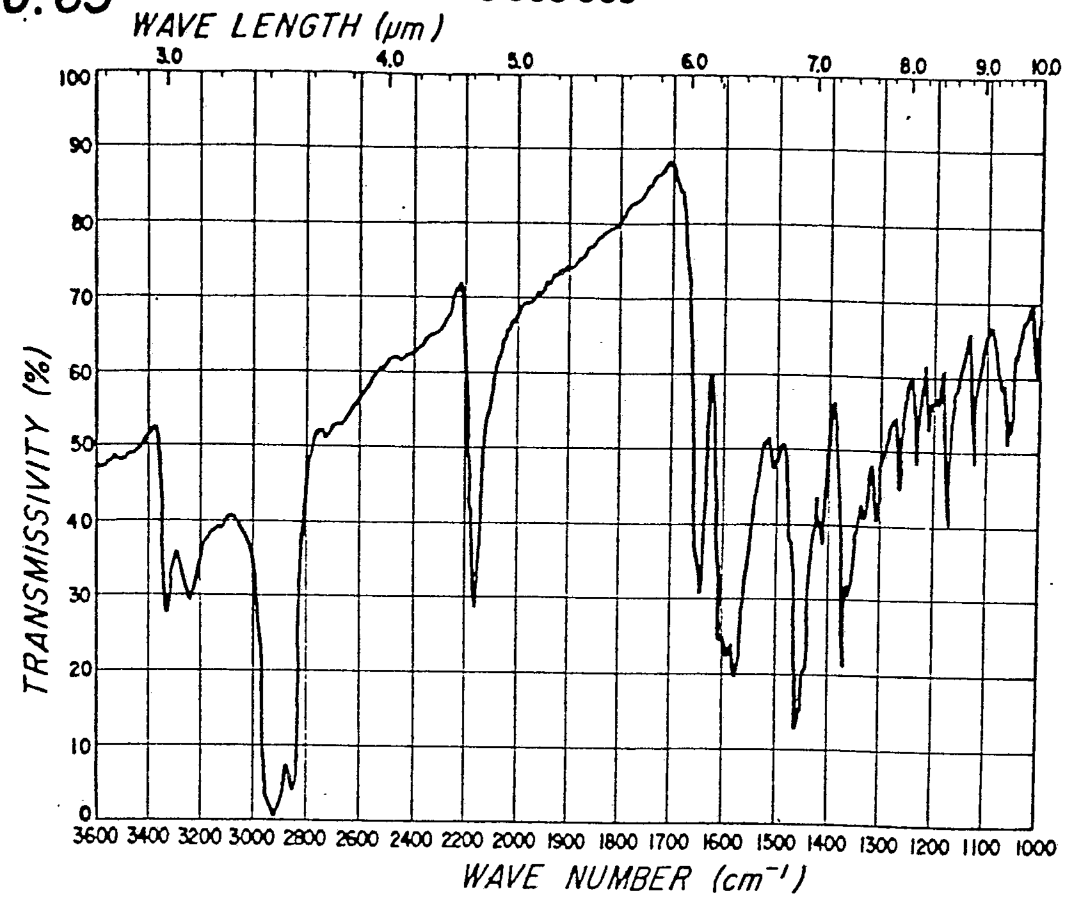
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER ($cm^{-1}$)

FIG. 84

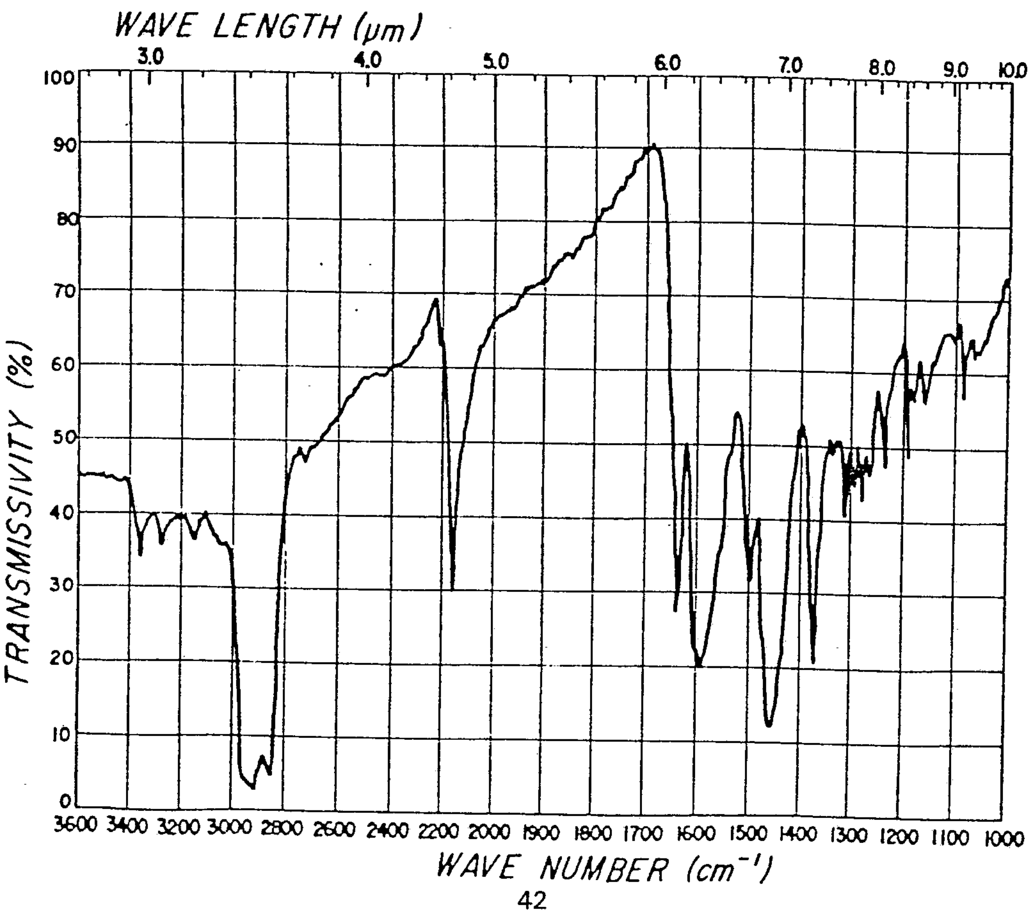
WAVE LENGTH (μm)
3.0
4.0
5.0
6.0
7.0
8.0
9.0
10.0
100
90
80
70
60
50
40
30
20
10
0
TRANSMISSIVITY (%)
3600 3400 3200 3000 2800 2600 2400 2200 2000 1900 1800 1700 1600 1500 1400 1300 1200 1100 1000
WAVE NUMBER ($cm^{-1}$)

FIG. 85

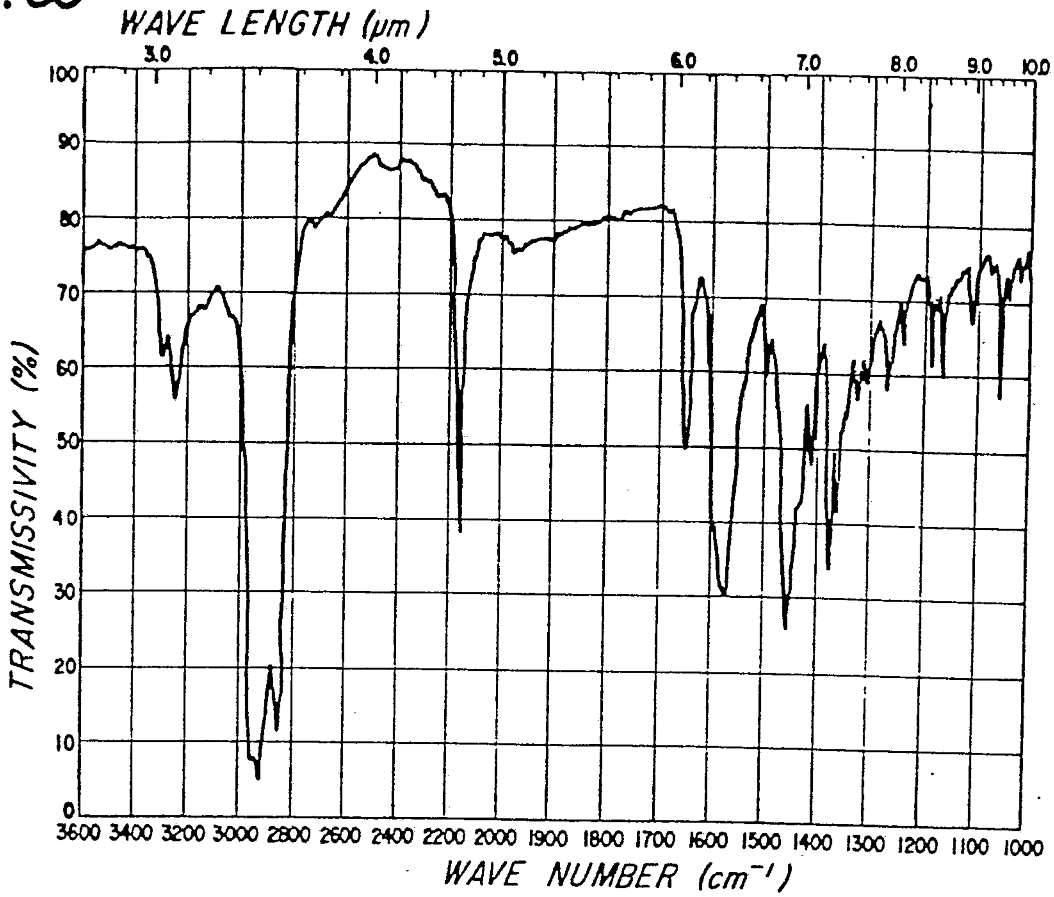
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER ($cm^{-1}$)

FIG. 86

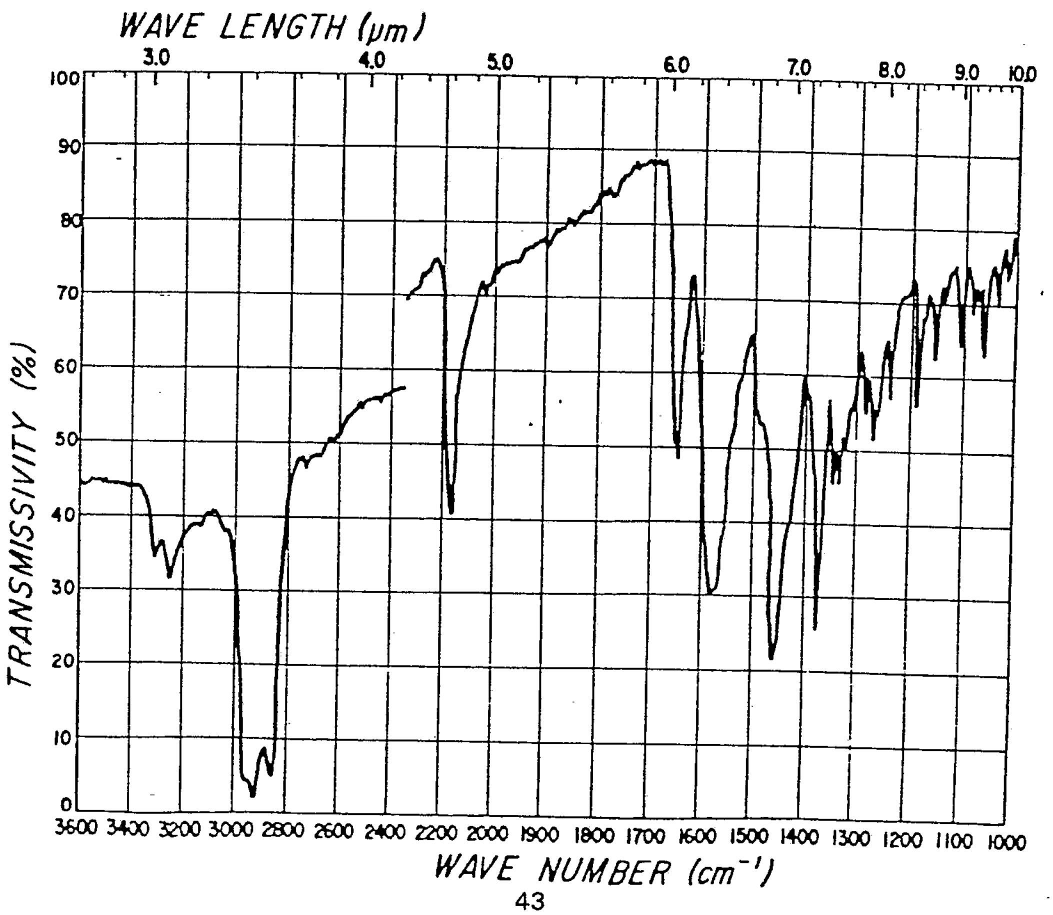
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER ($cm^{-1}$)

FIG. 87

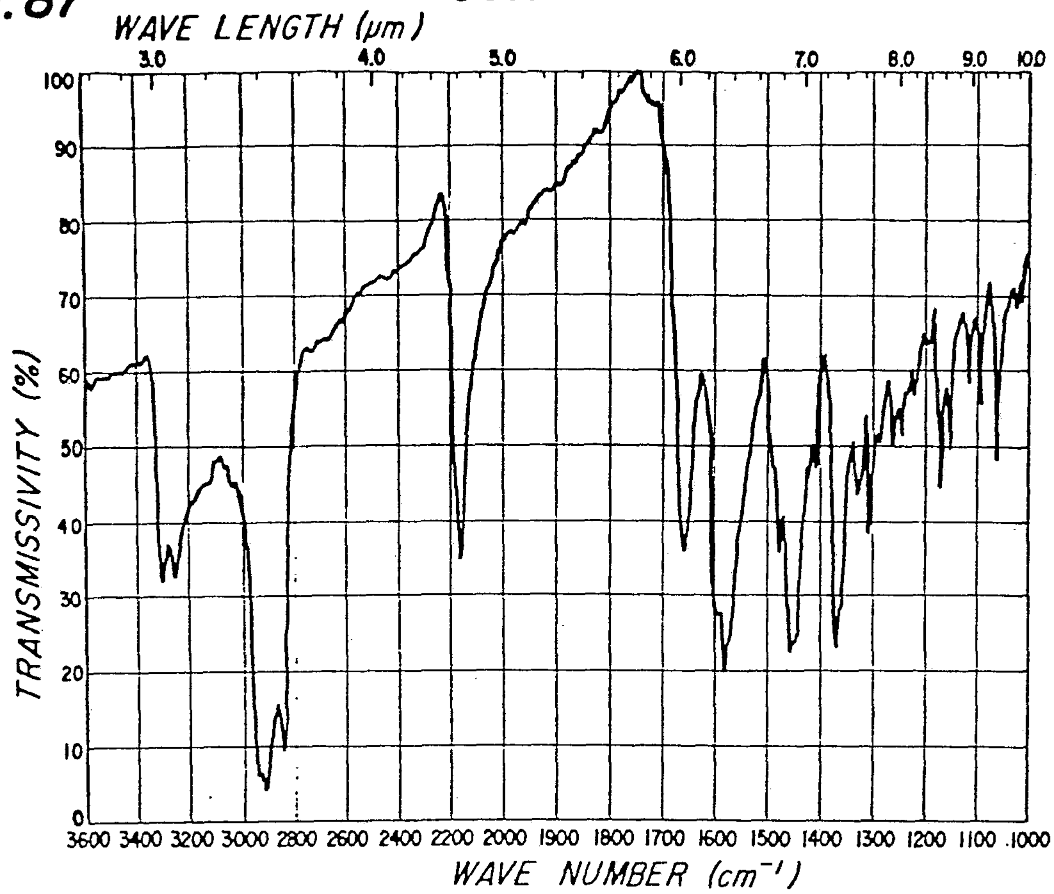
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 88

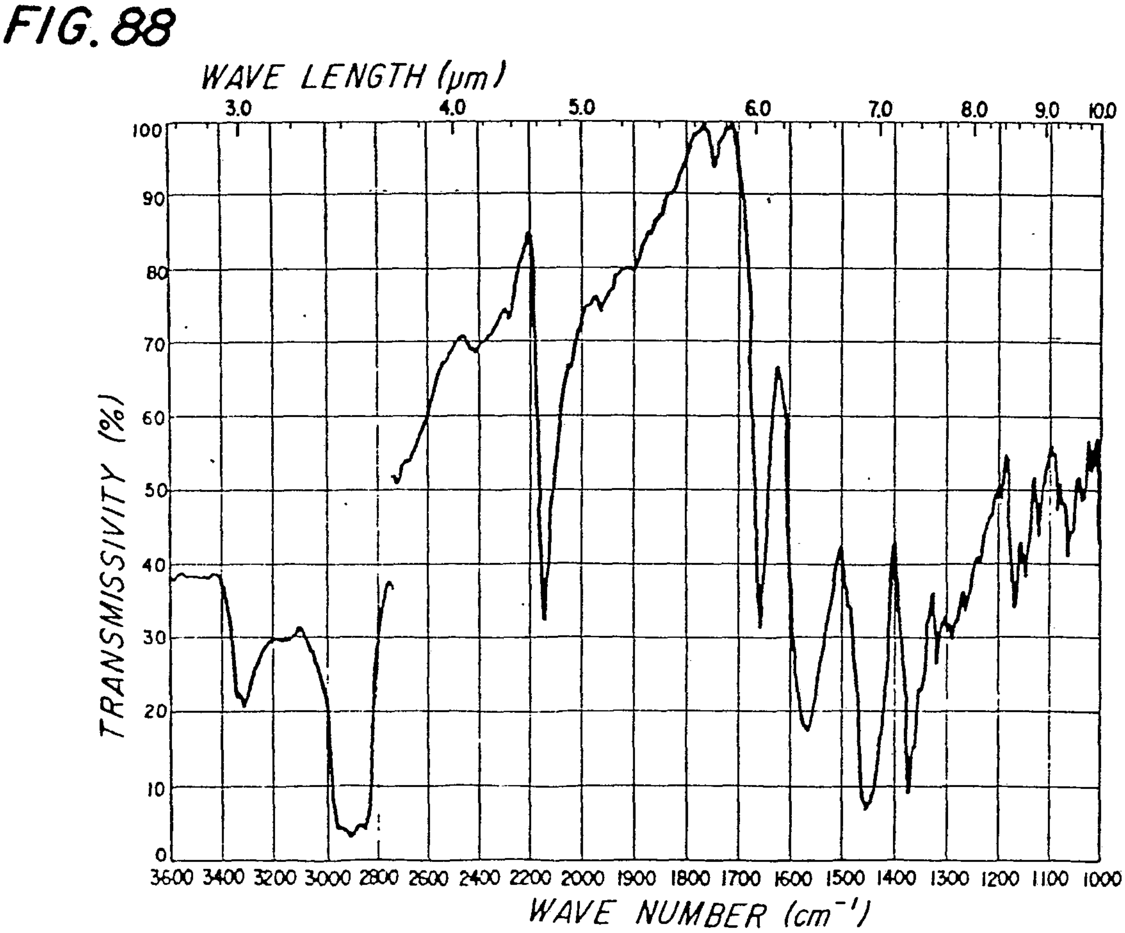
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 89

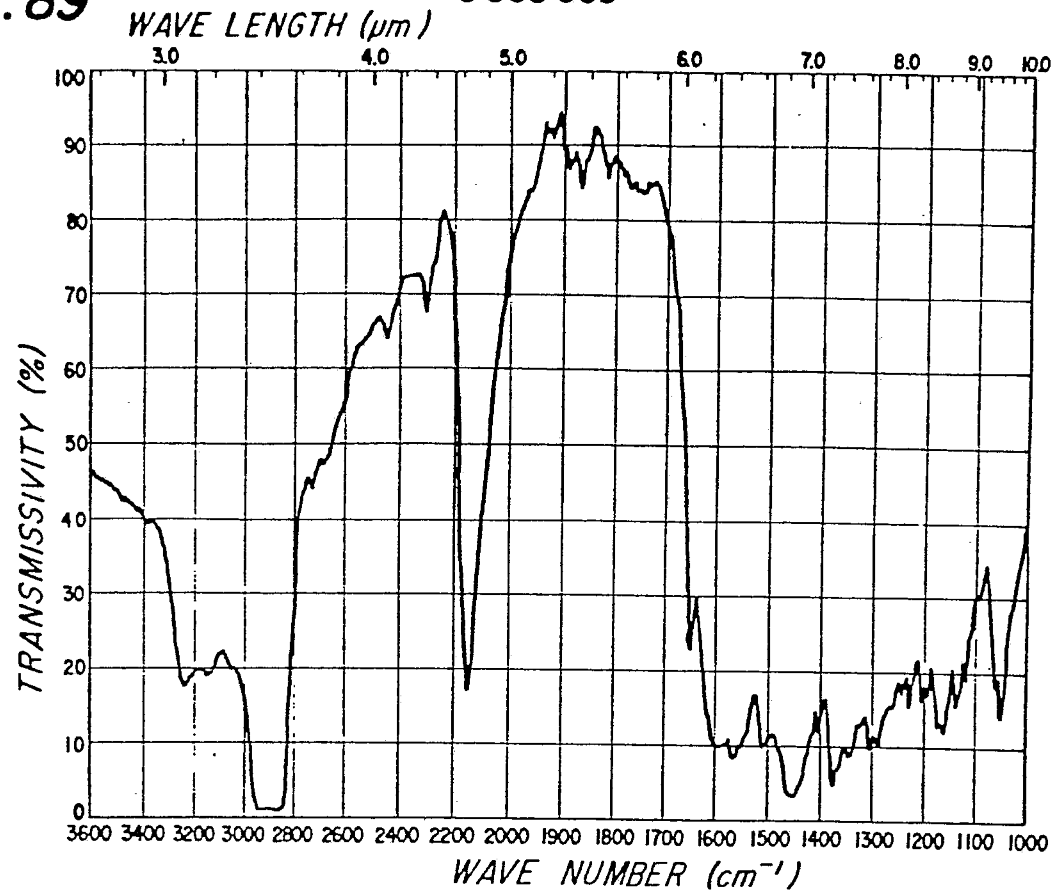
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 90

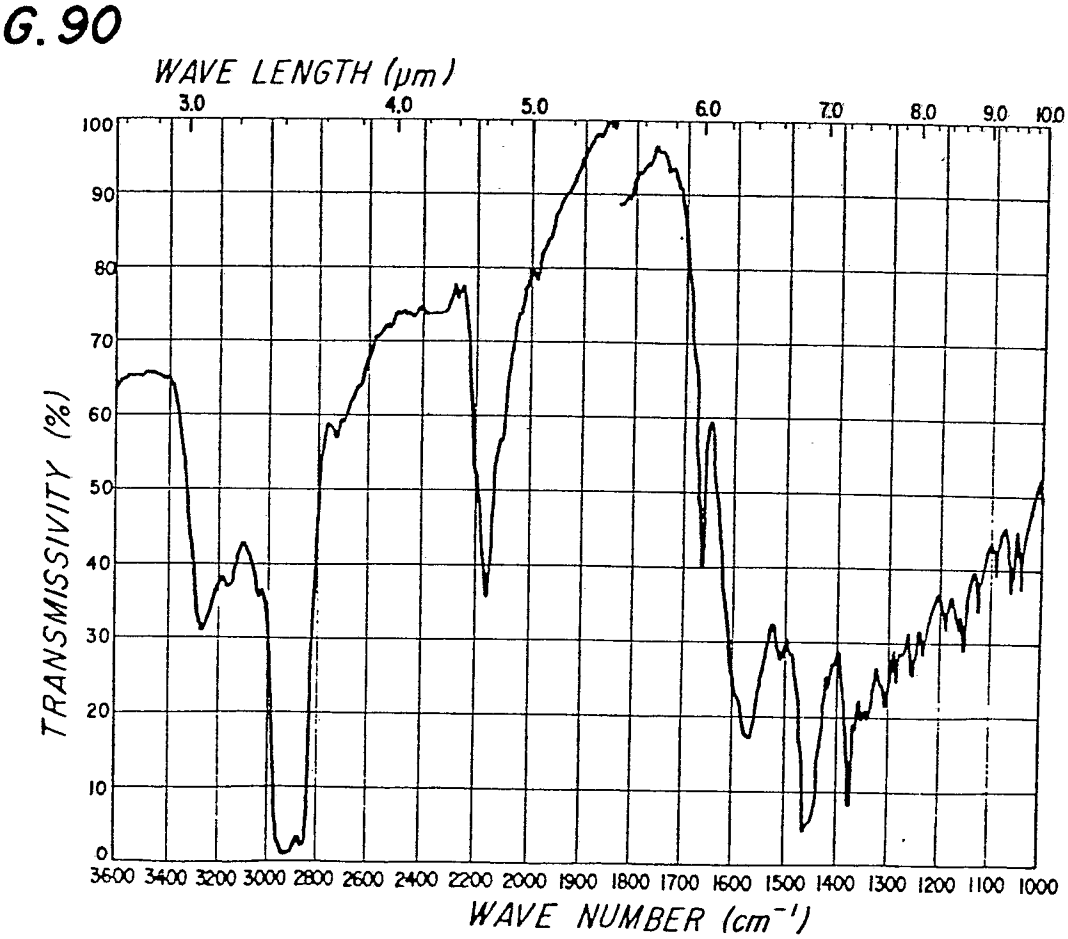
WAVE LENGTH (μm)
3.0
4.0
5.0
6.0
7.0
8.0
9.0
10.0
TRANSMISSIVITY (%)
100
90
80
70
60
50
40
30
20
10
0
3600 3400 3200 3000 2800 2600 2400 2200 2000 1900 1800 1700 1600 1500 1400 1300 1200 1100 1000
WAVE NUMBER (cm⁻¹)

FIG. 91

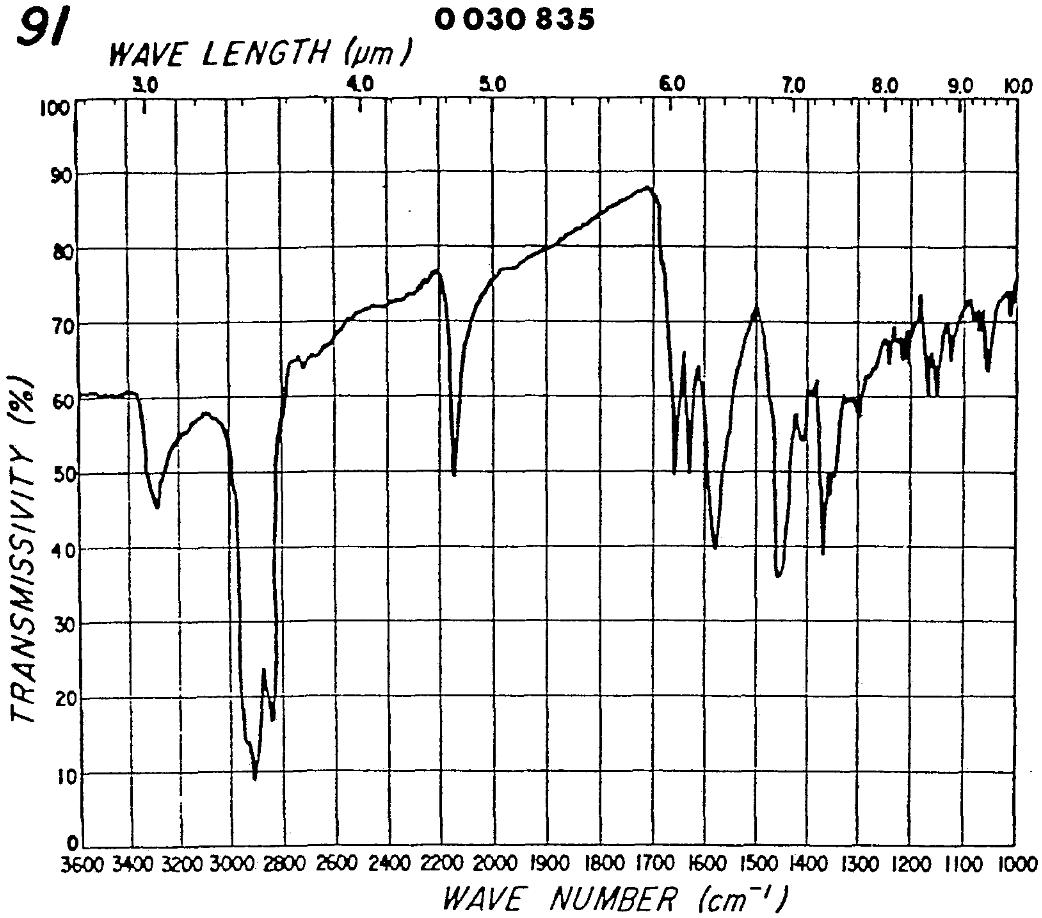
WAVE LENGTH (μm)
3.0 4.0 5.0 6.0 7.0 8.0 9.0 10.0
TRANSMISSIVITY (%)
100 90 80 70 60 50 40 30 20 10 0
3600 3400 3200 3000 2800 2600 2400 2200 2000 1900 1800 1700 1600 1500 1400 1300 1200 1100 1000
WAVE NUMBER (cm⁻¹)

FIG. 92

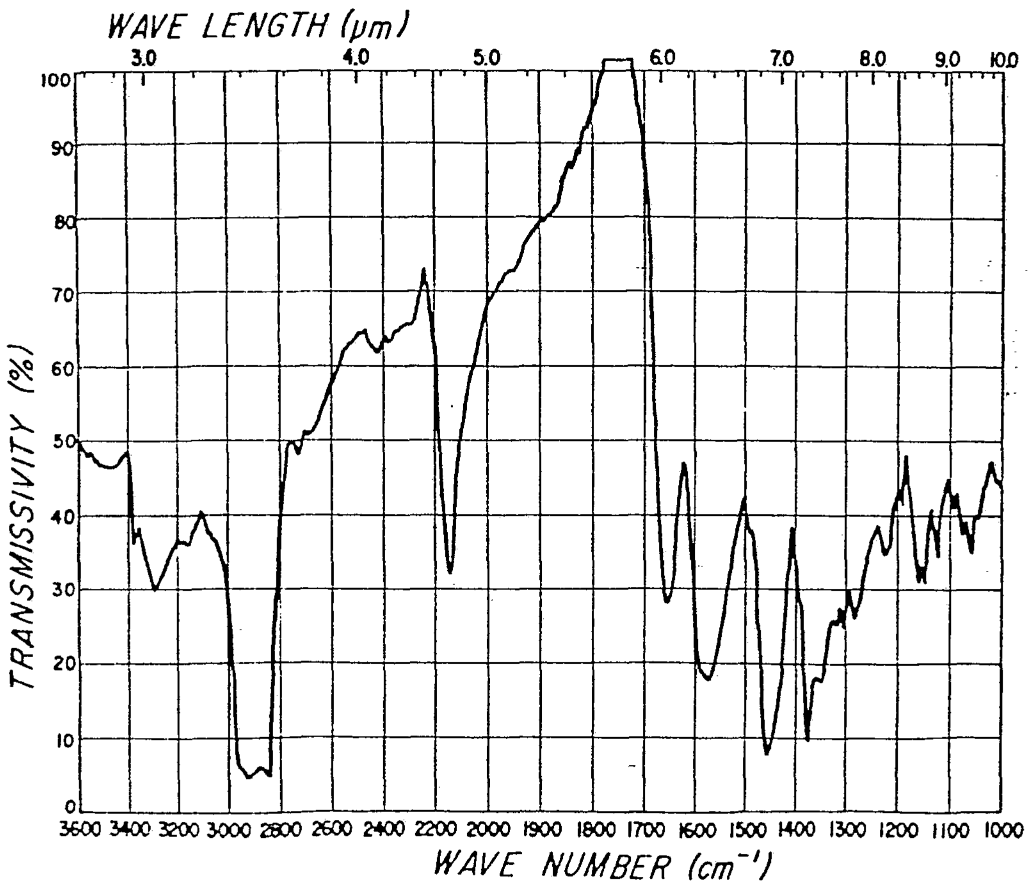
WAVE LENGTH (μm)
3.0 4.0 5.0 6.0 7.0 8.0 9.0 10.0
TRANSMISSIVITY (%)
100 90 80 70 60 50 40 30 20 10 0
3600 3400 3200 3000 2800 2600 2400 2200 2000 1900 1800 1700 1600 1500 1400 1300 1200 1100 1000
WAVE NUMBER (cm⁻¹)

FIG. 93

0 030 835

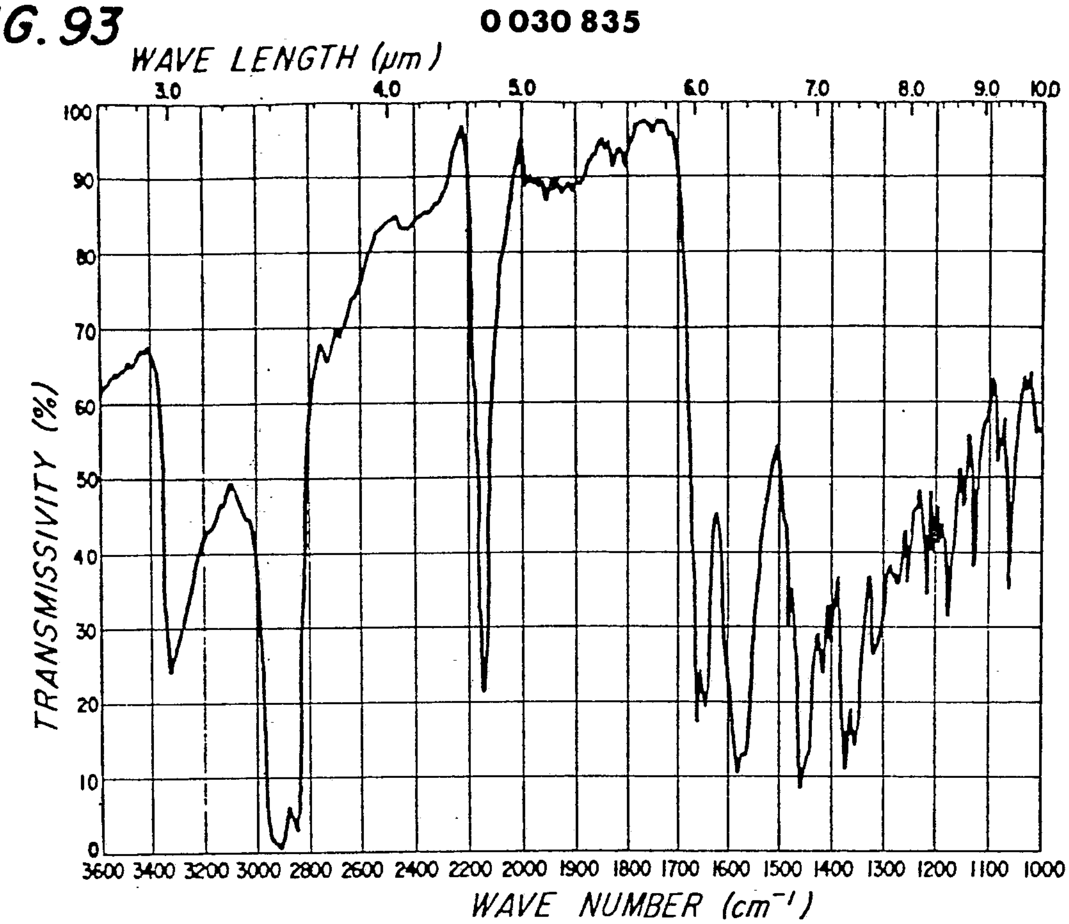


FIG. 94

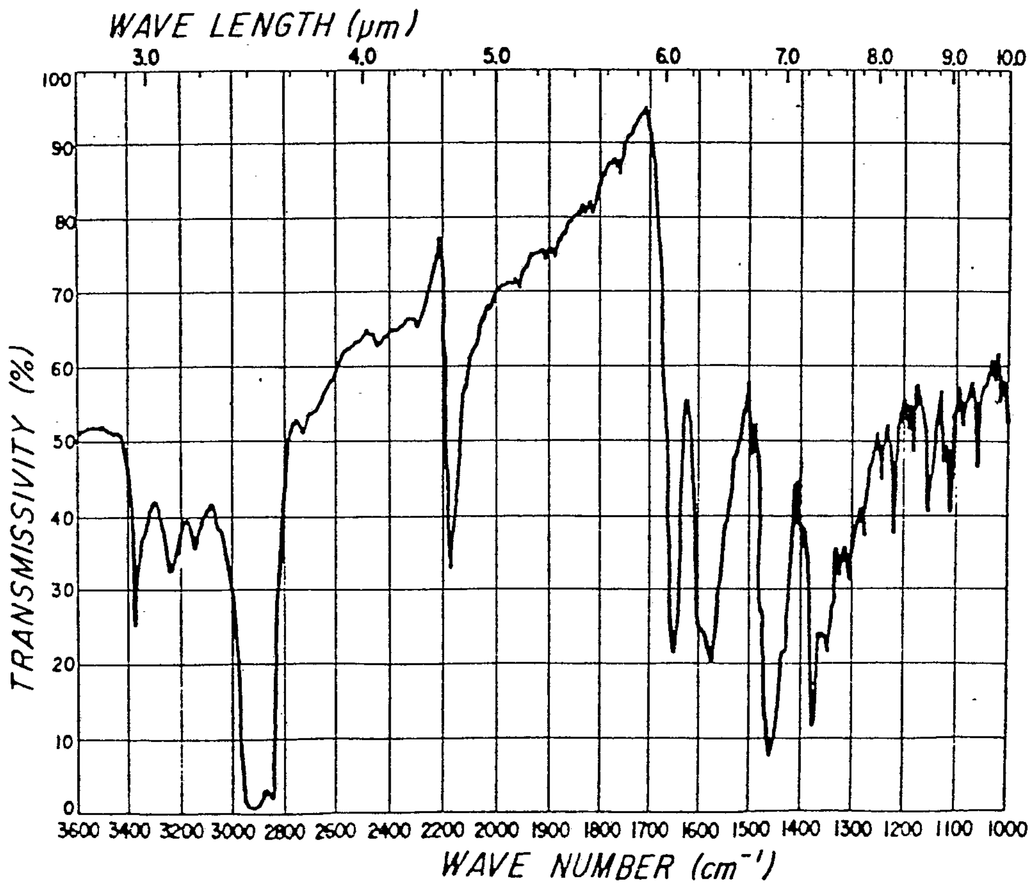

FIG. 95



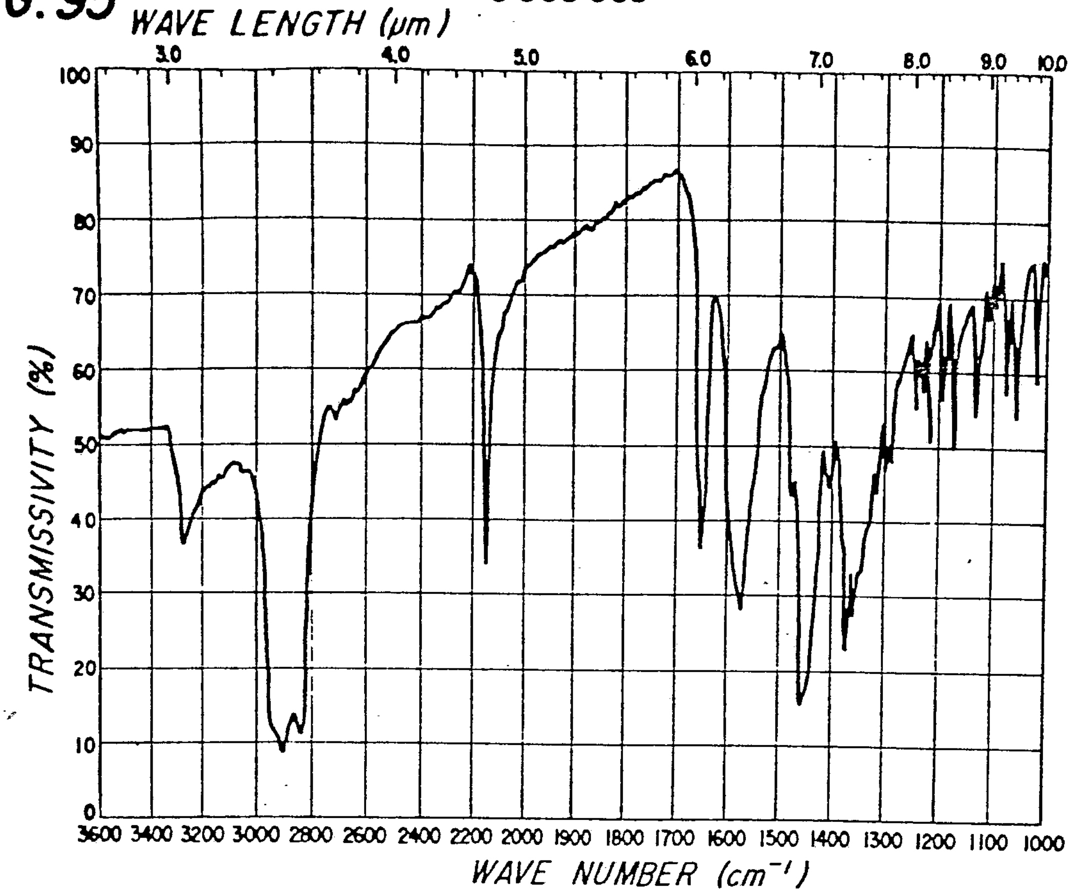
WAVE LENGTH (μm)
3.0
4.0
5.0
6.0
7.0
8.0
9.0
10.0
TRANSMISSIVITY (%)
0
10
20
30
40
50
60
70
80
90
100
3600 3400 3200 3000 2800 2600 2400 2200 2000 1900 1800 1700 1600 1500 1400 1300 1200 1100 1000
WAVE NUMBER (cm⁻¹)

FIG. 96

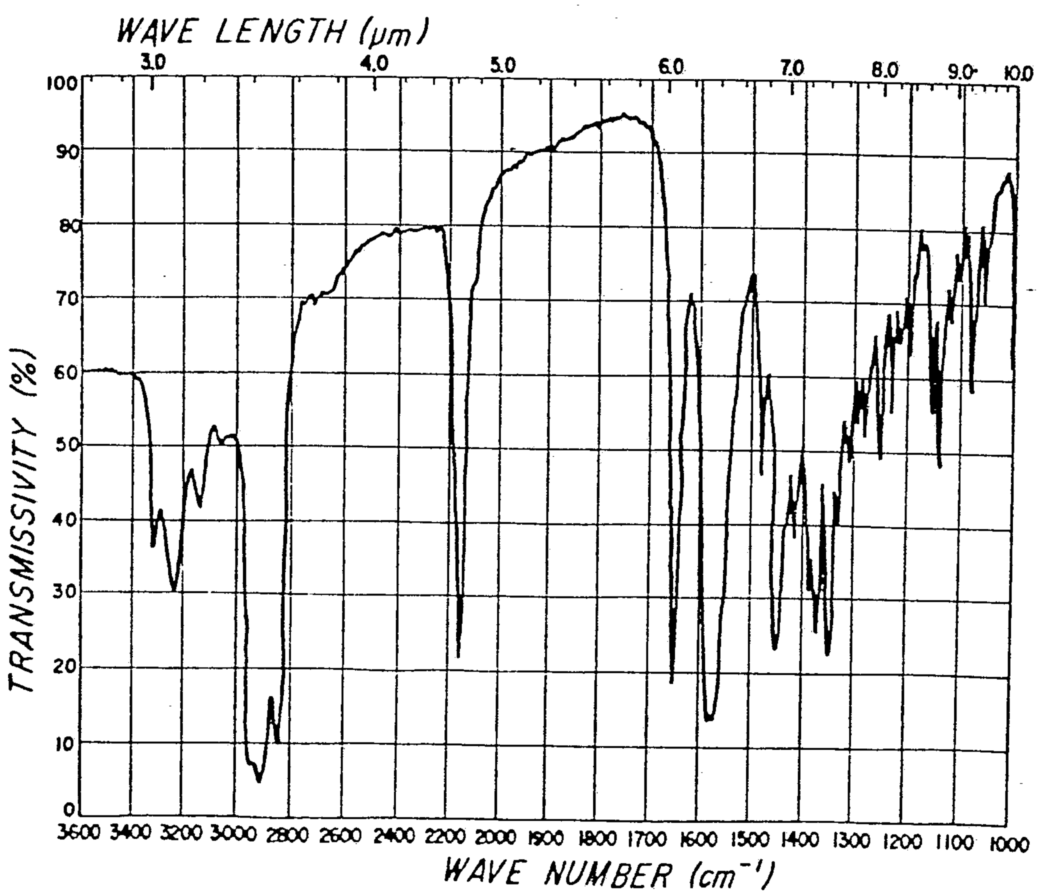
WAVE LENGTH (μm)
3.0
4.0
5.0
6.0
7.0
8.0
9.0
10.0
TRANSMISSIVITY (%)
0
10
20
30
40
50
60
70
80
90
100
3600 3400 3200 3000 2800 2600 2400 2200 2000 1900 1800 1700 1600 1500 1400 1300 1200 1100 1000
WAVE NUMBER (cm⁻¹)

FIG. 97

0 030 835

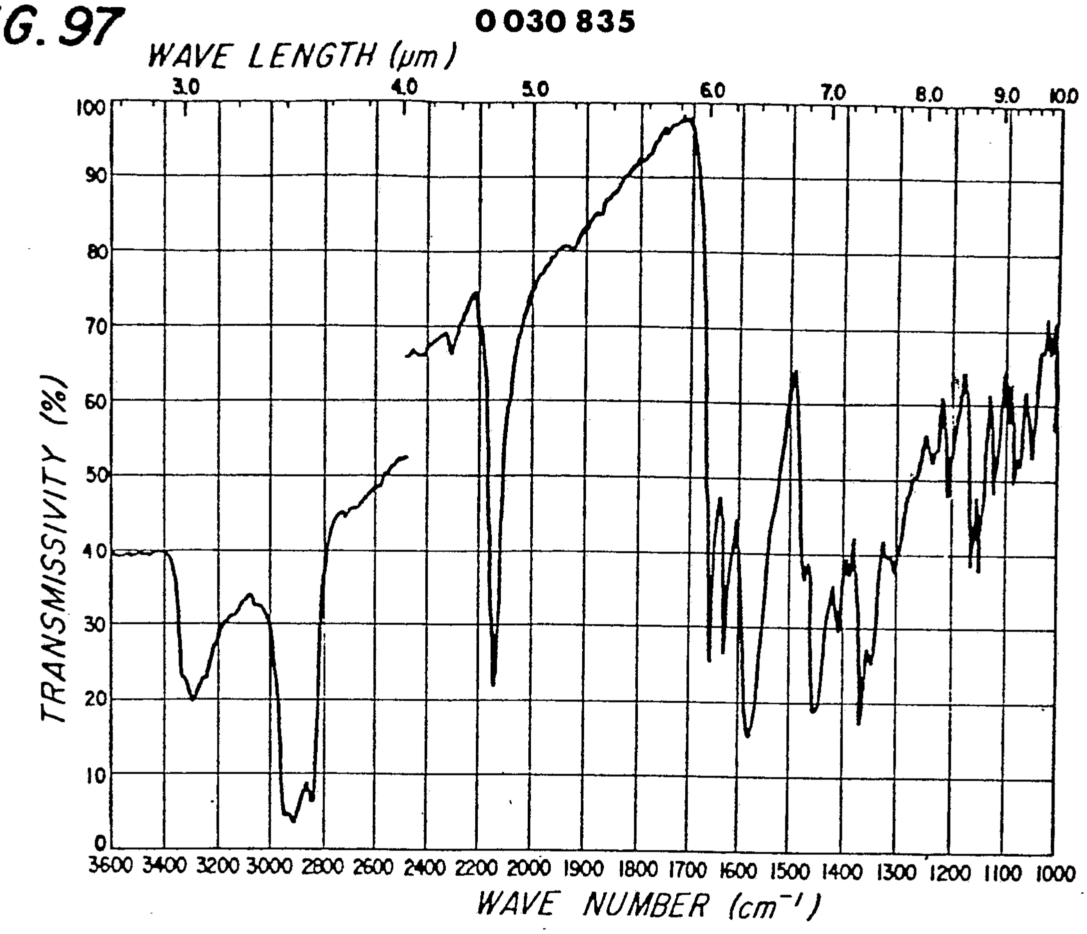


FIG. 98

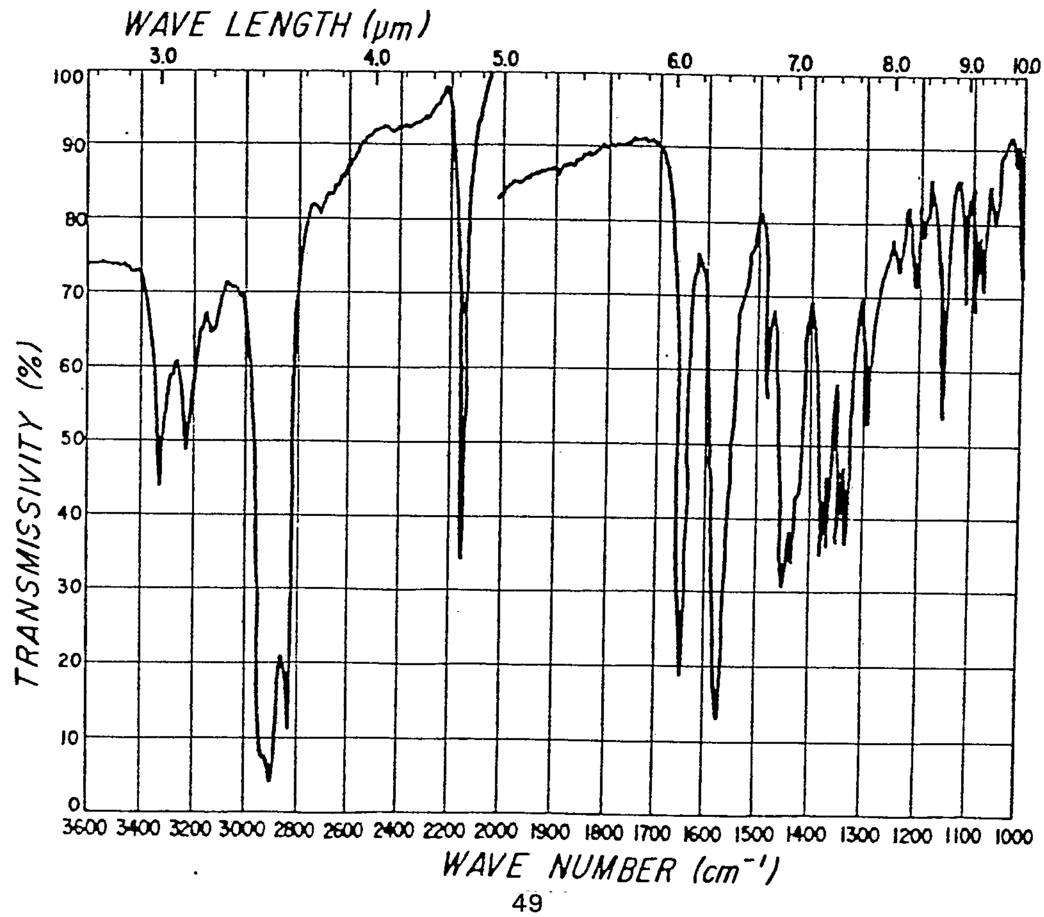

FIG. 99

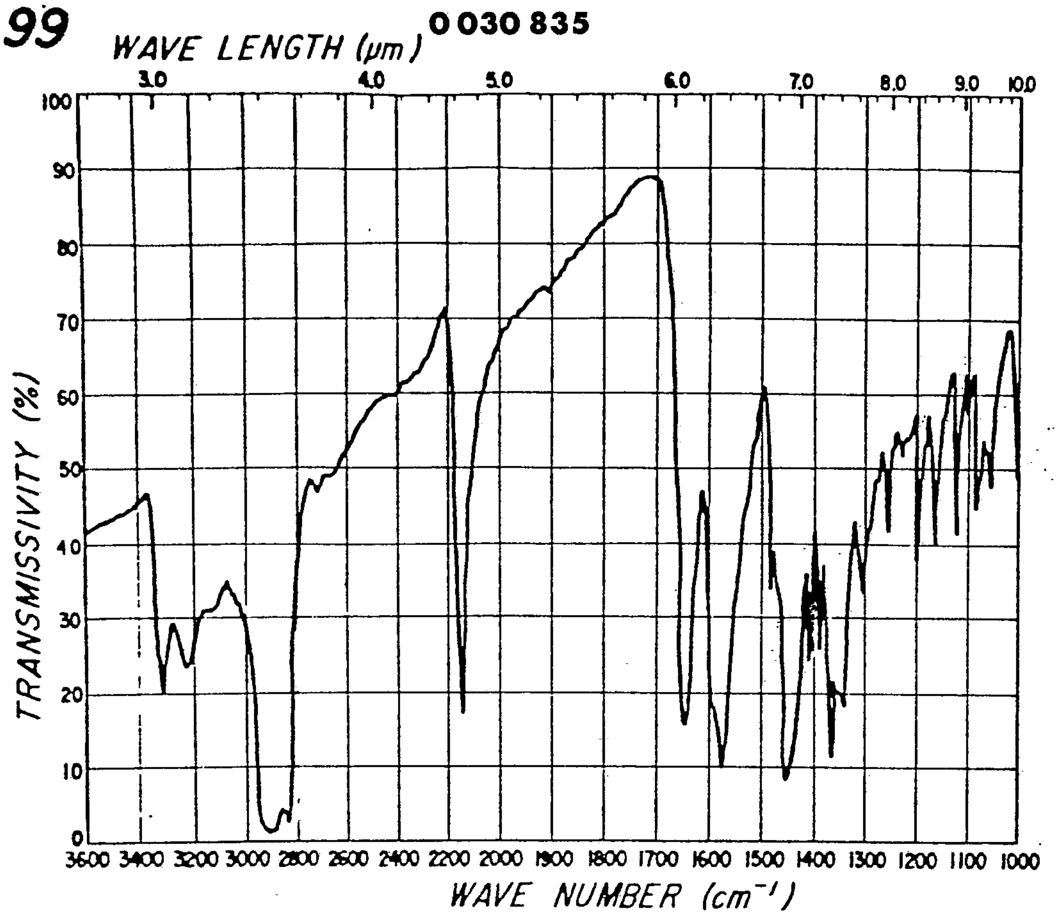


FIG. 100

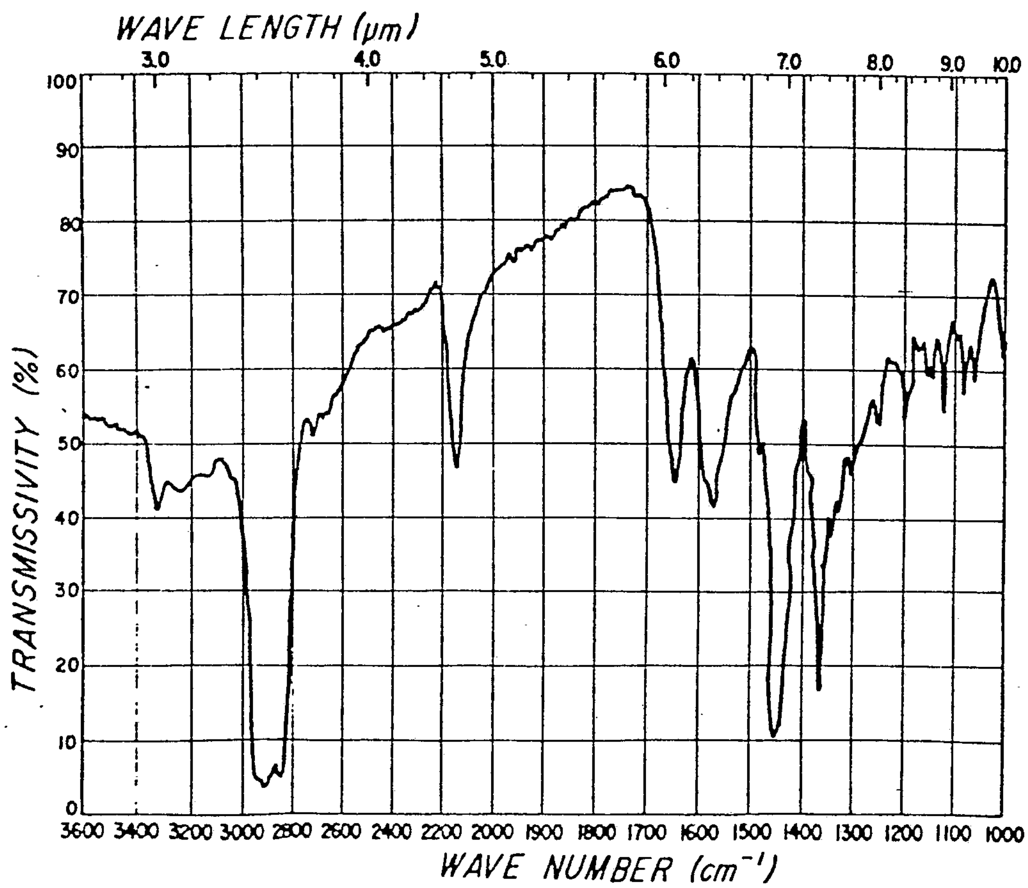

FIG. 101

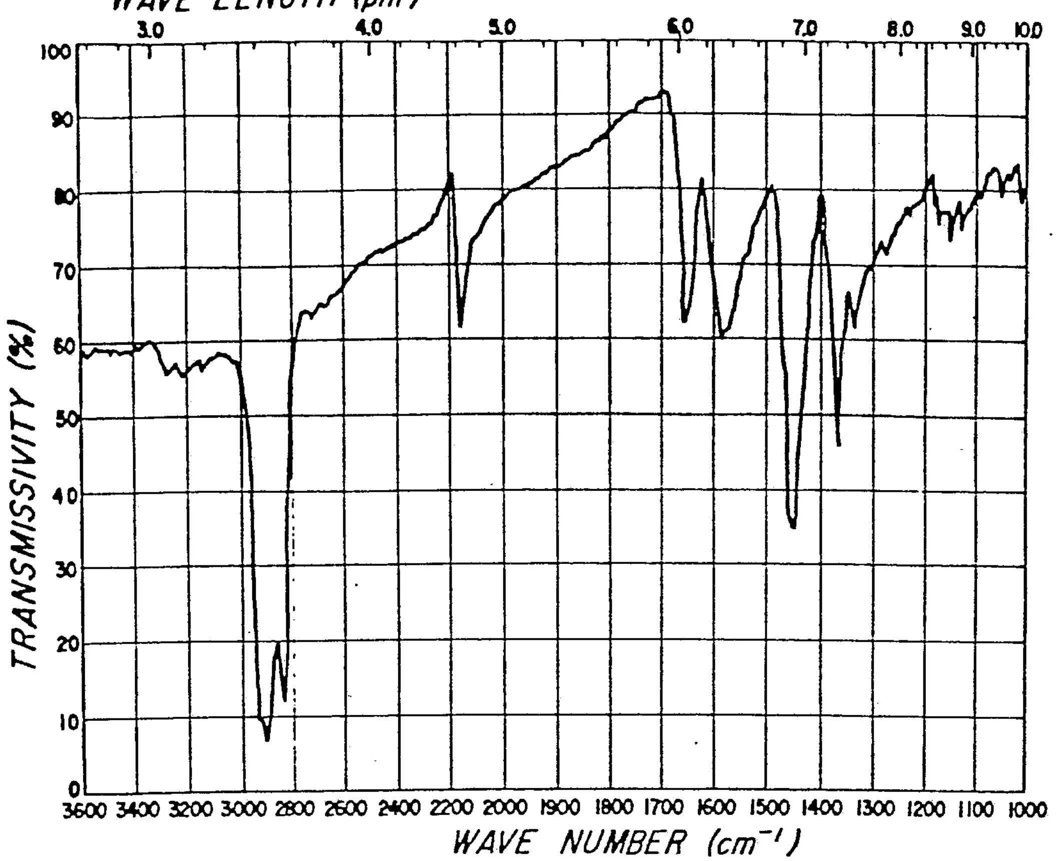


FIG. 102

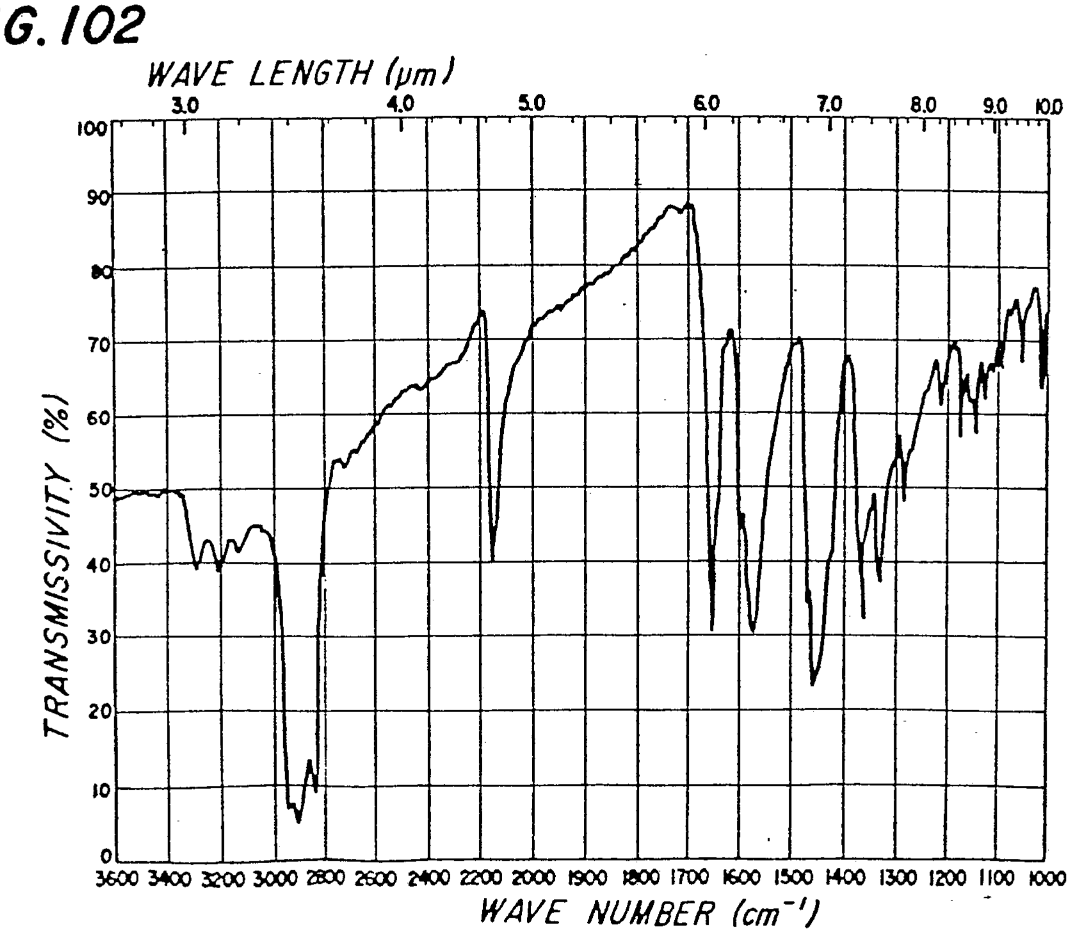

FIG. 103

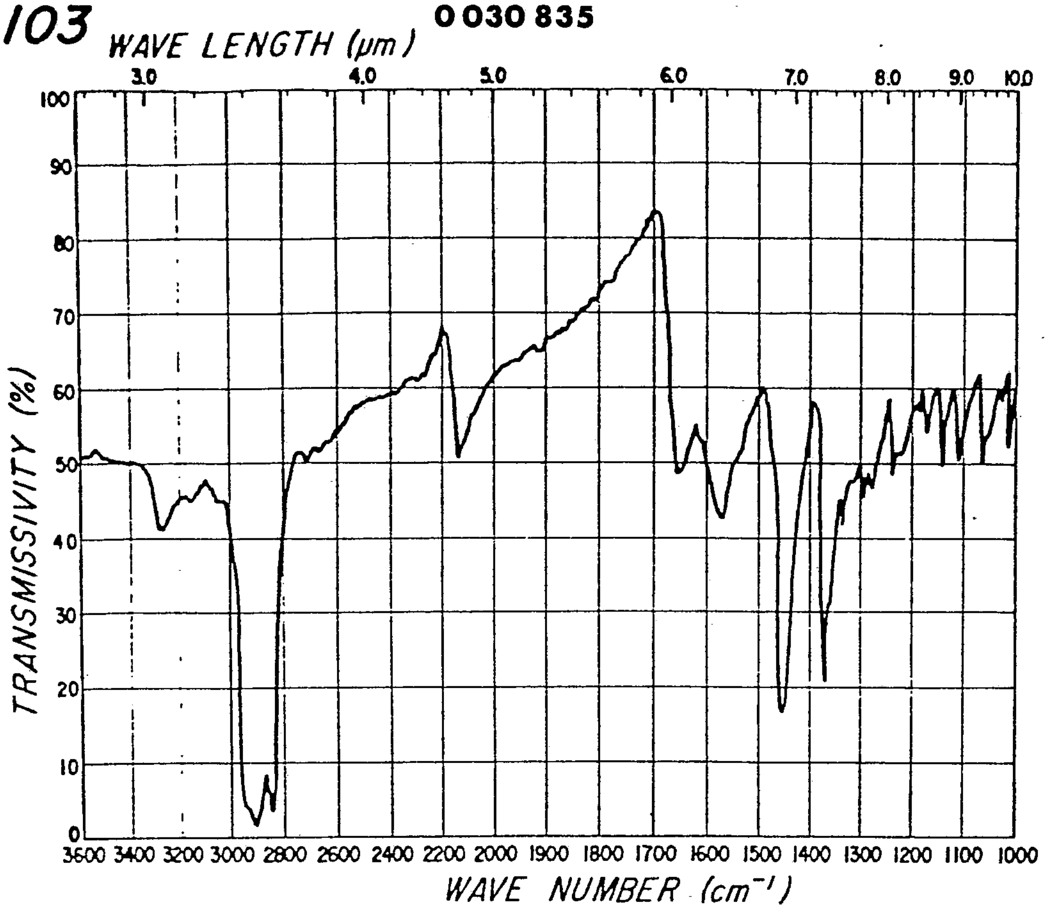


FIG. 104

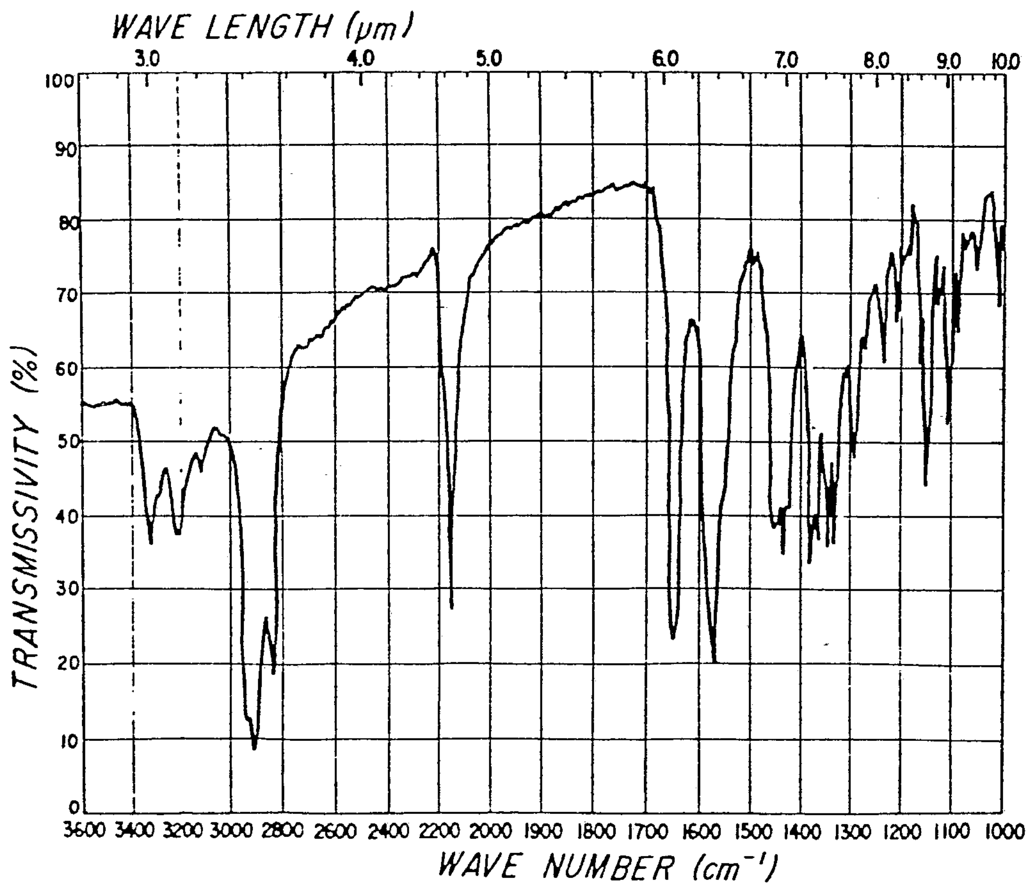

FIG. 105

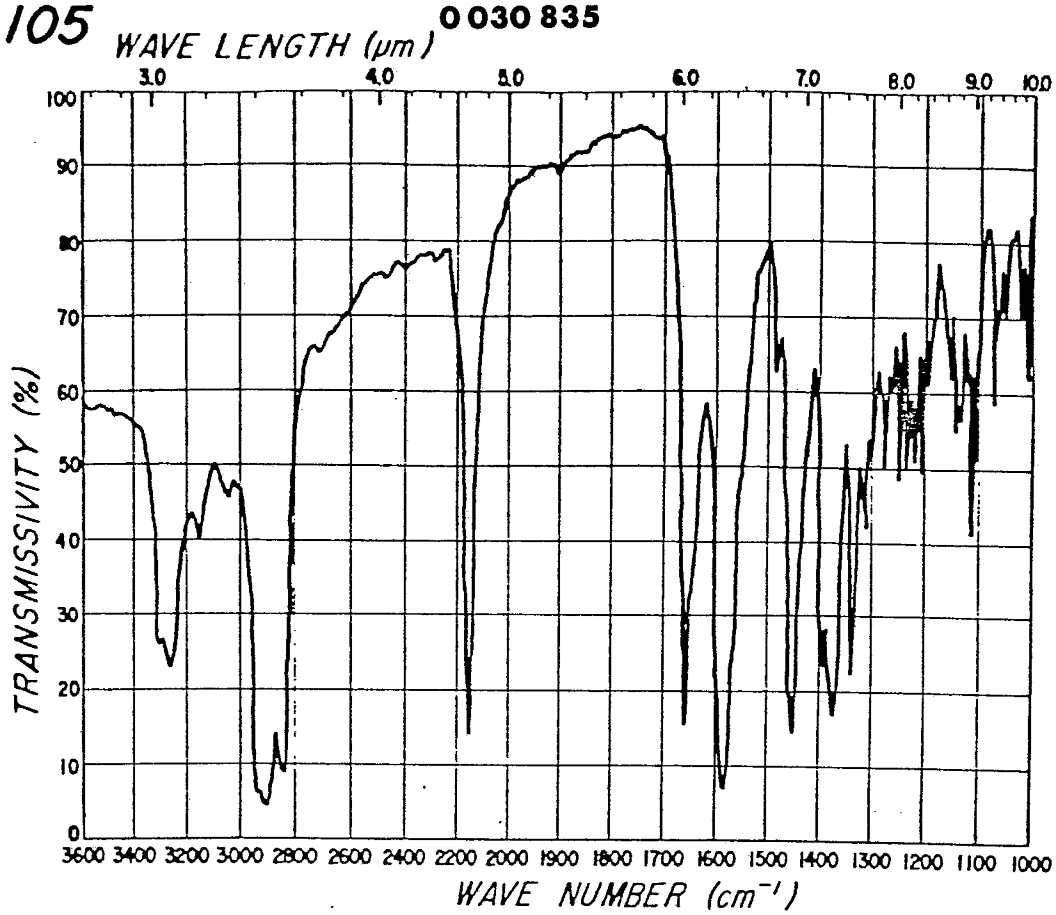


FIG. 106

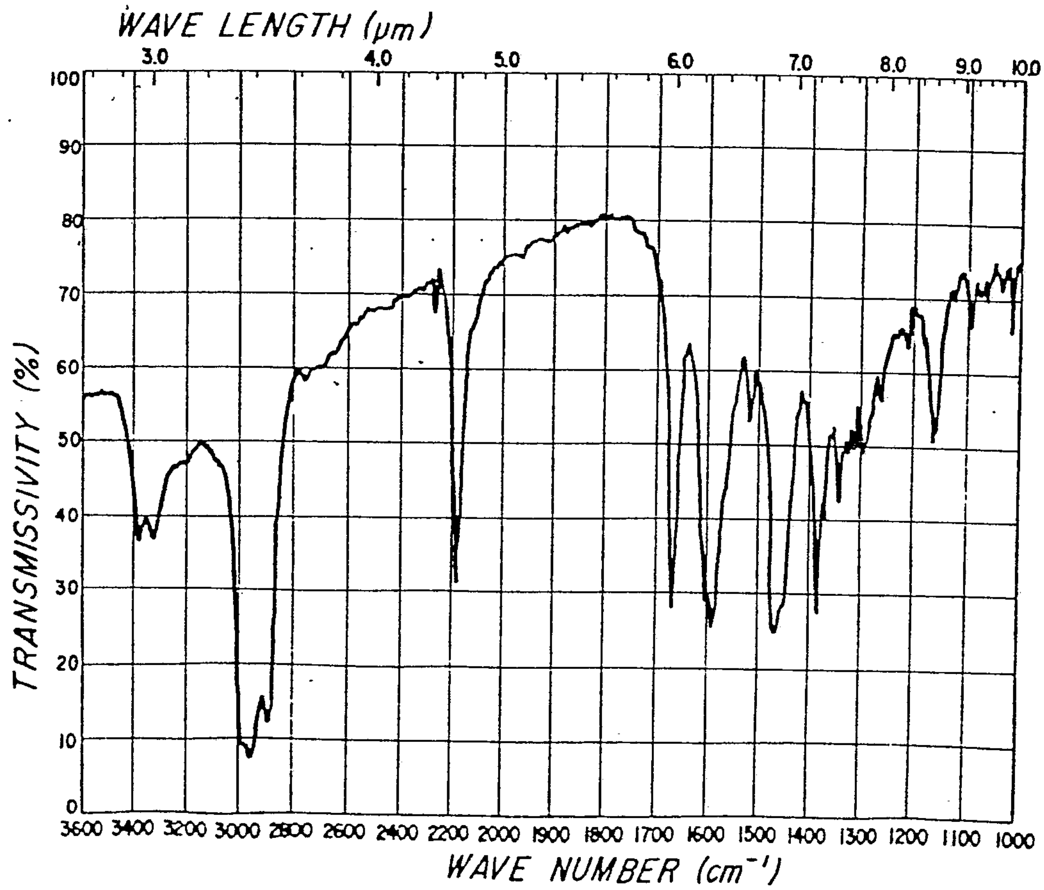

FIG. 107

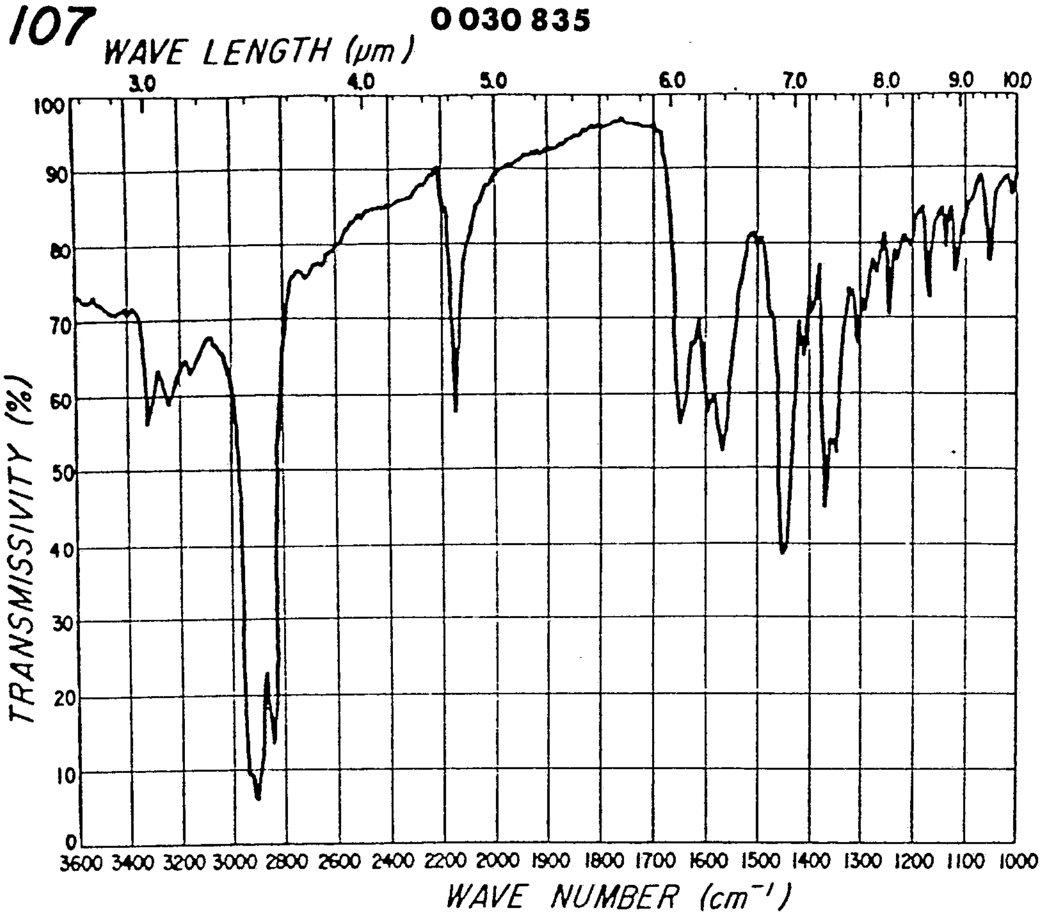


FIG. 108

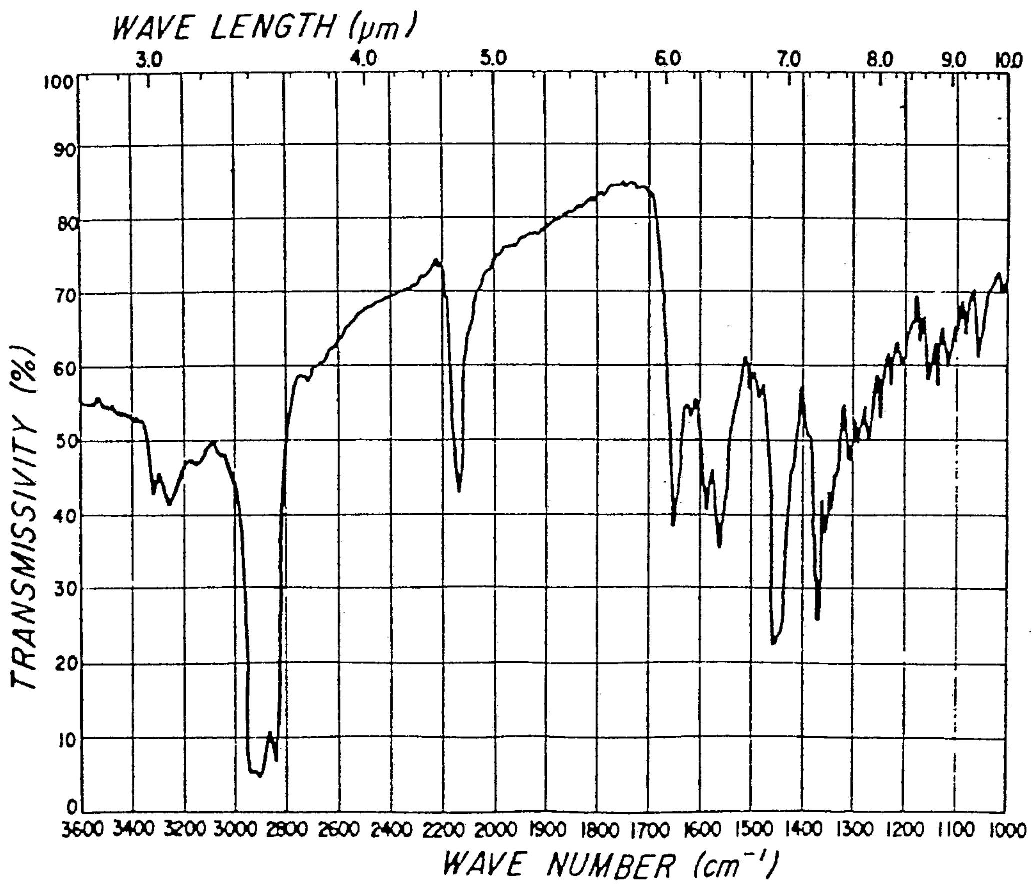

FIG. 109



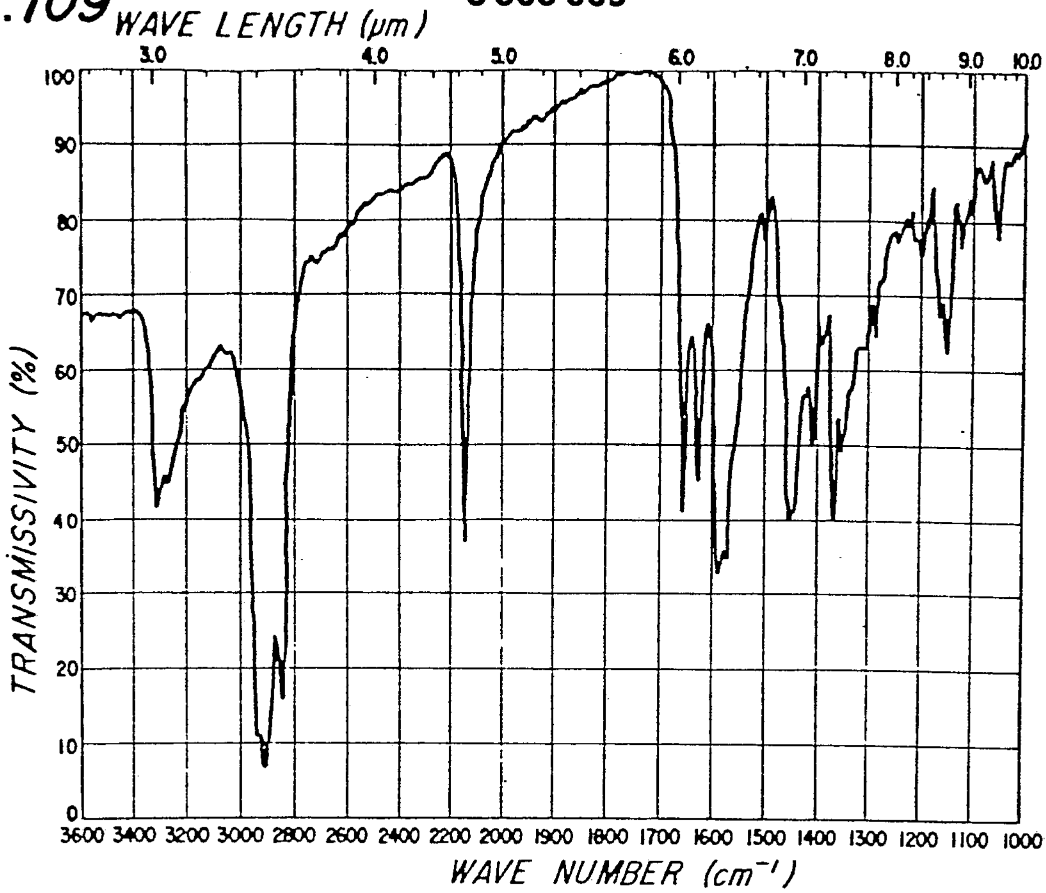


FIG. 110

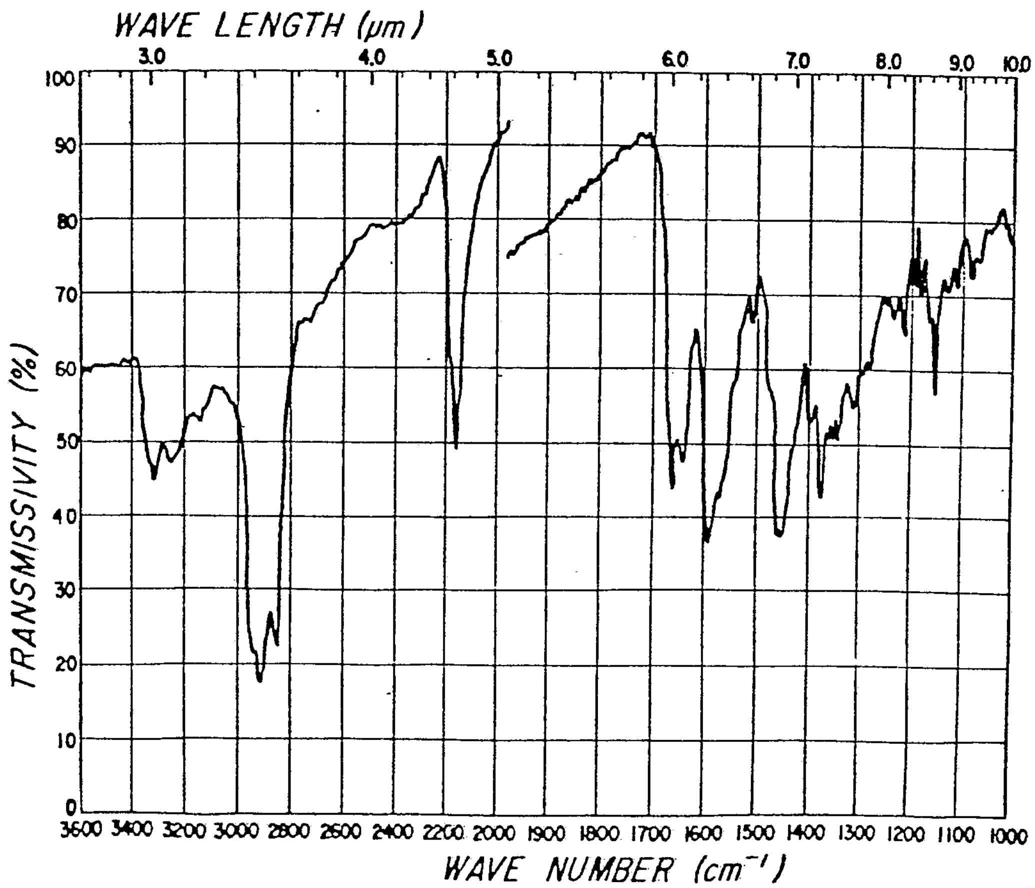

FIG. 111

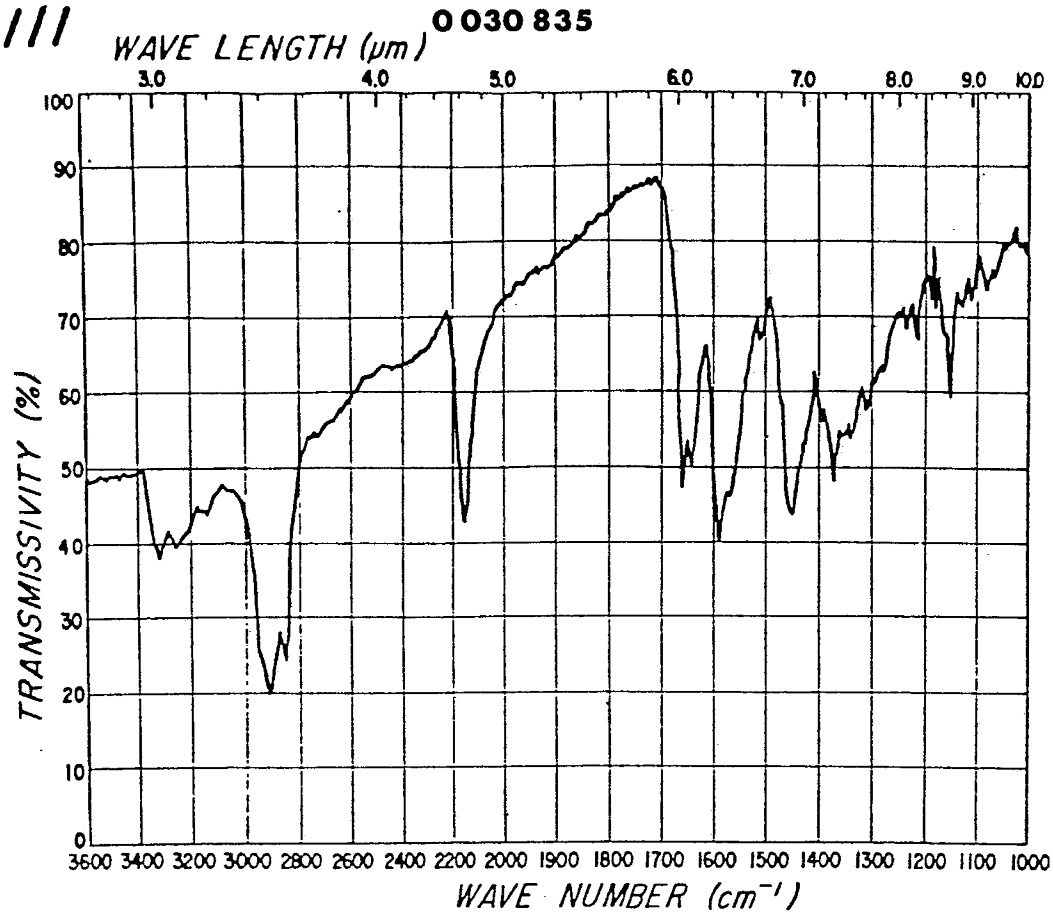


FIG. 112

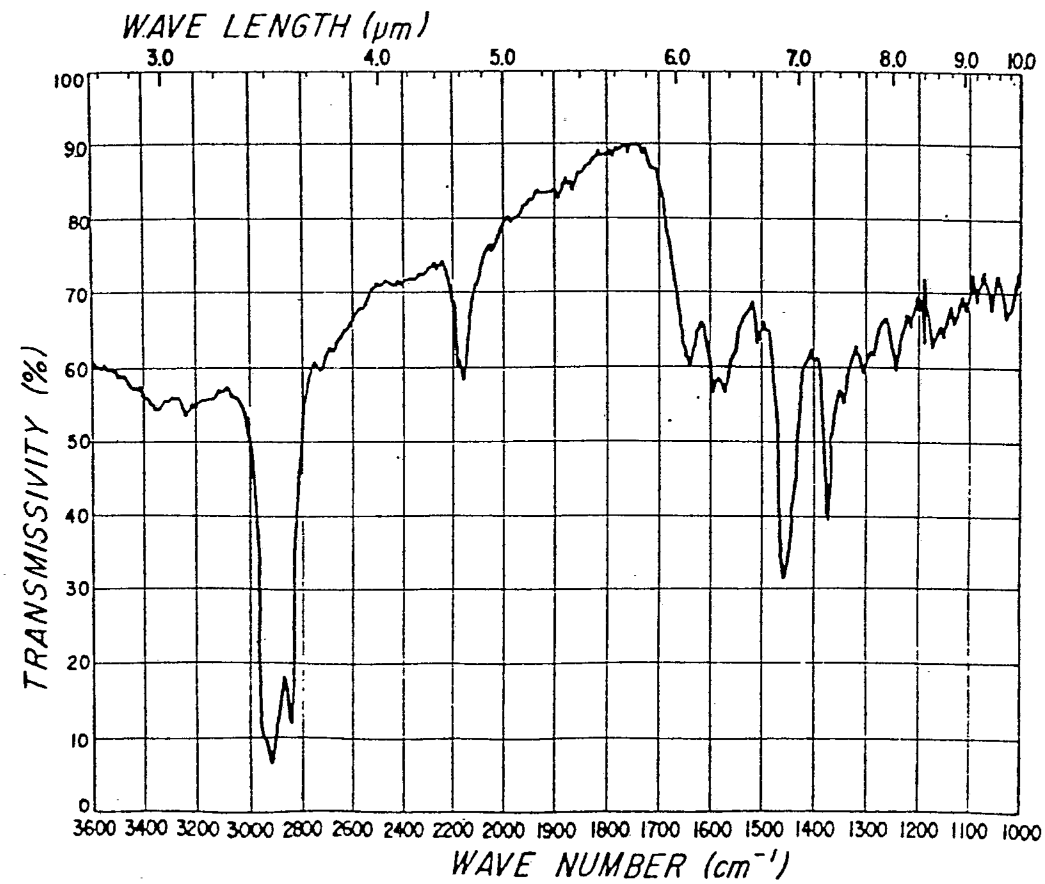

FIG. 113

0 030 835

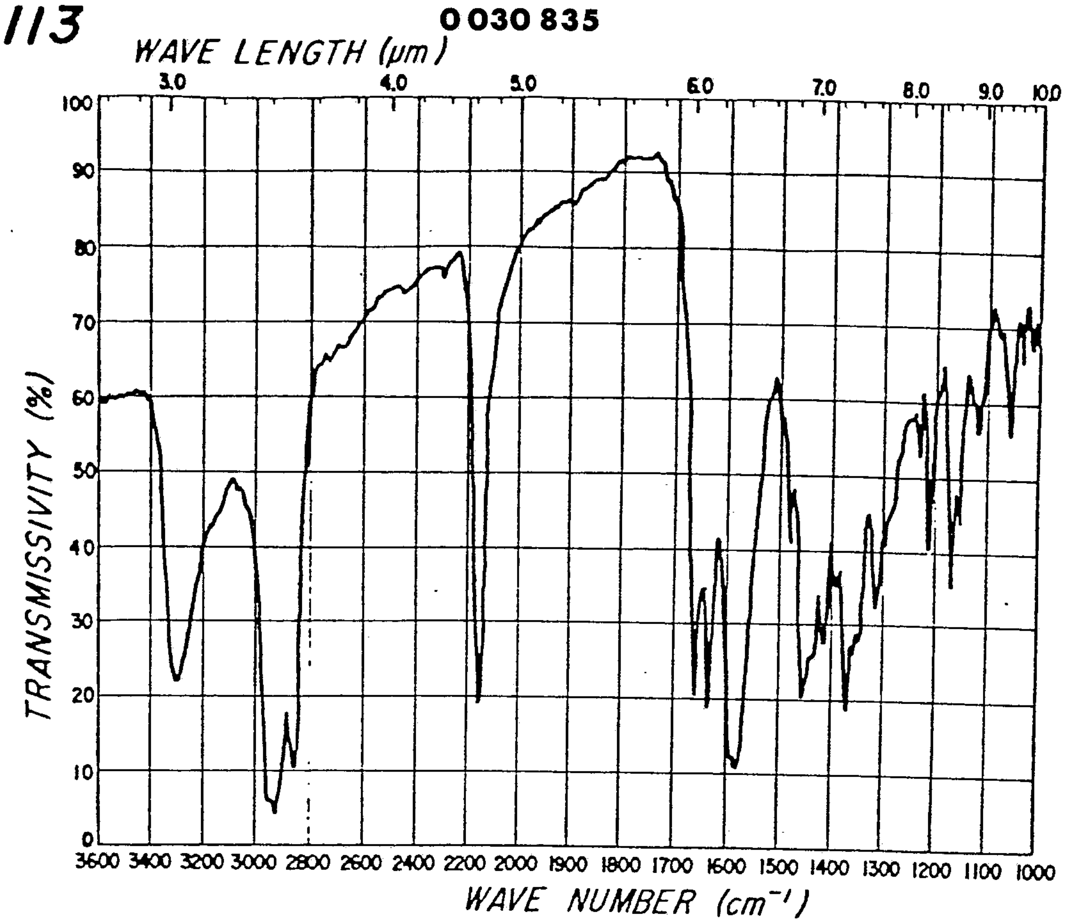


FIG. 114

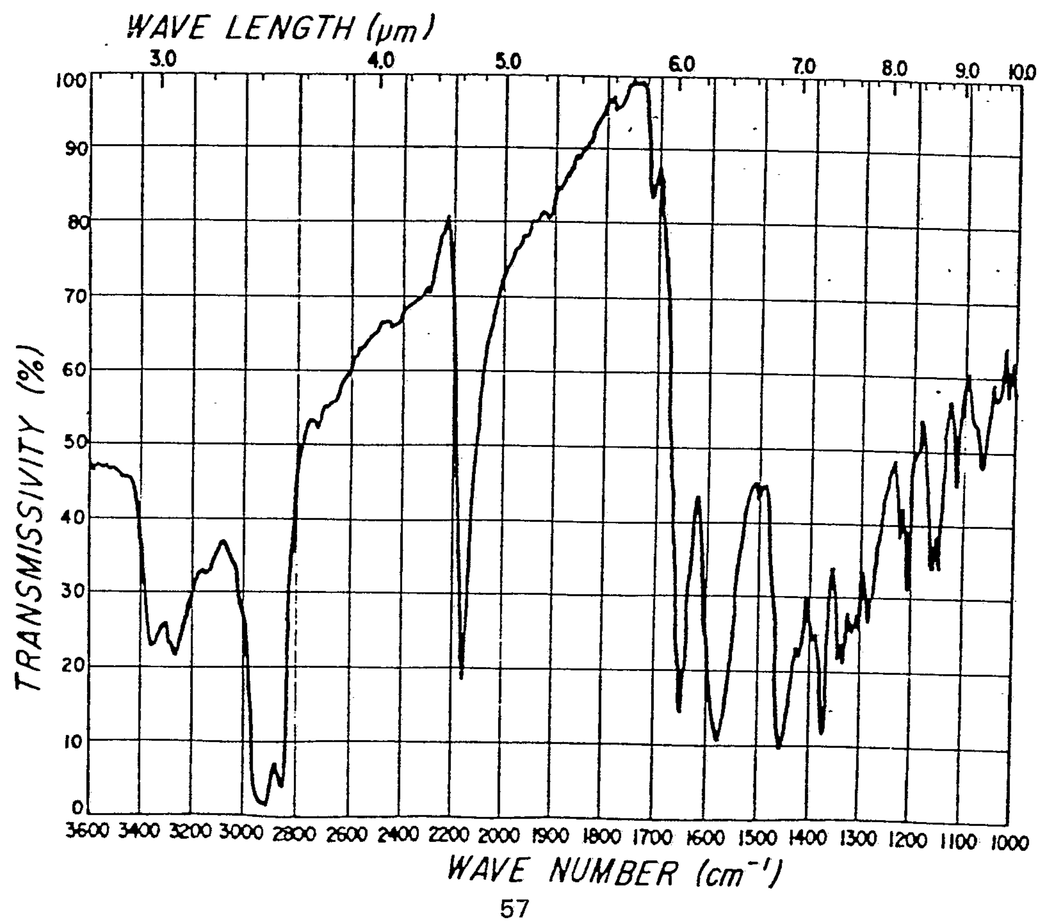

0 030 835

FIG. 115

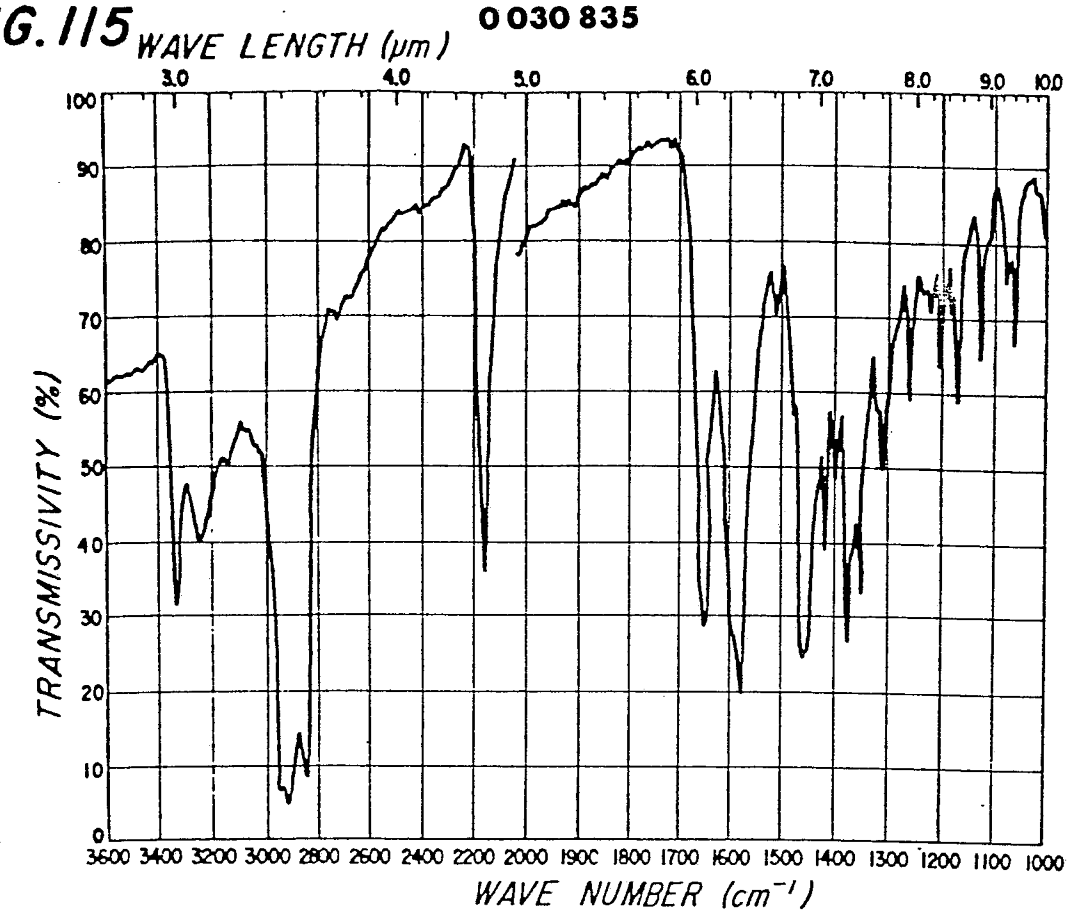


FIG. 116

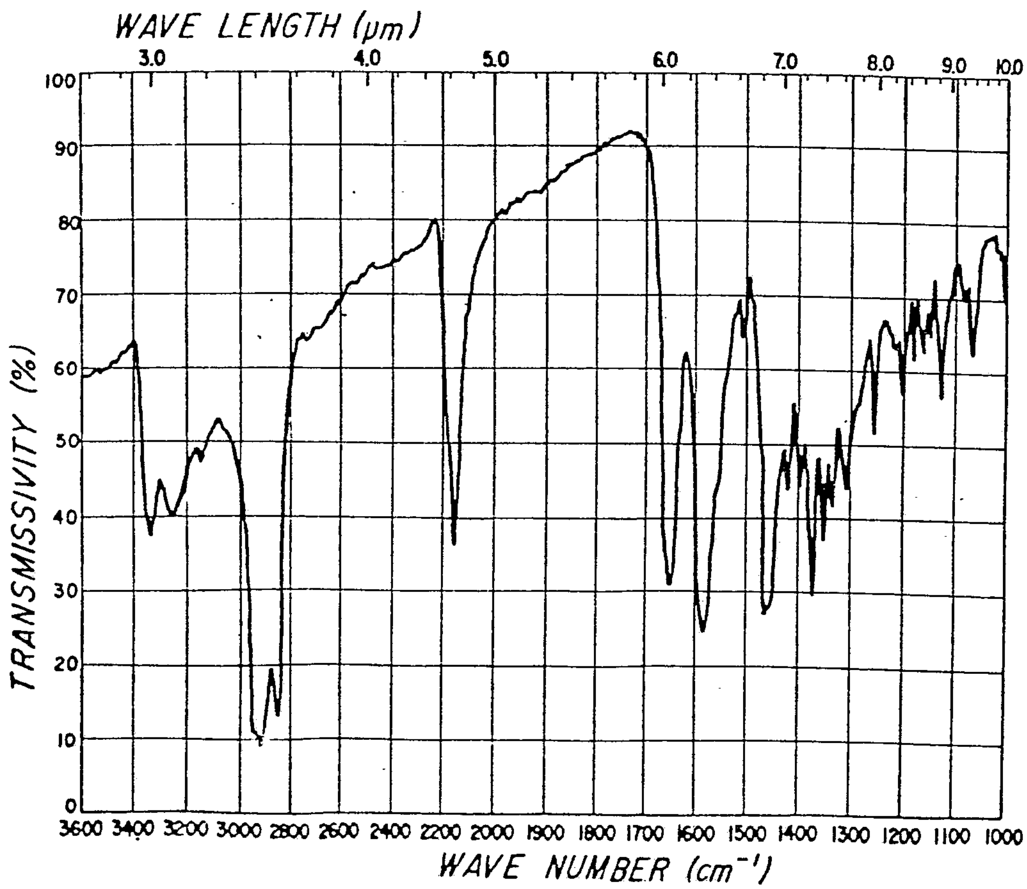

0030 835

FIG. 117

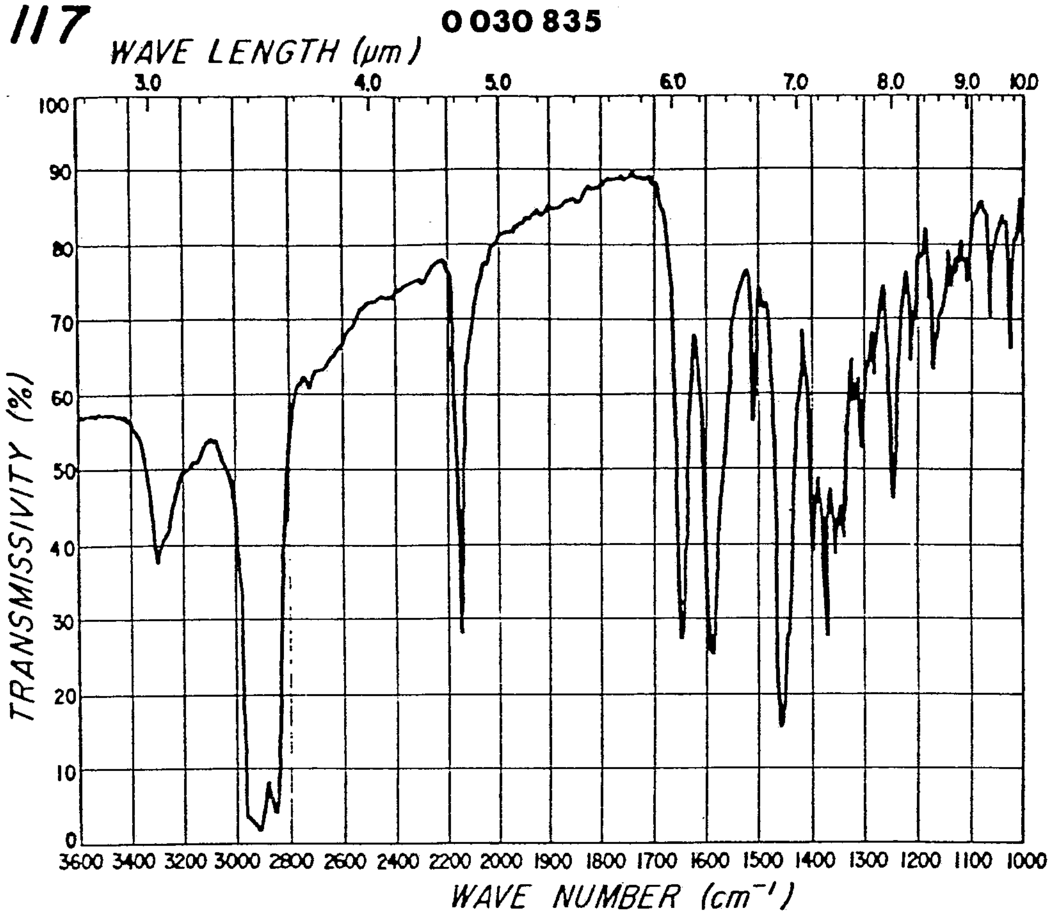


FIG. 118

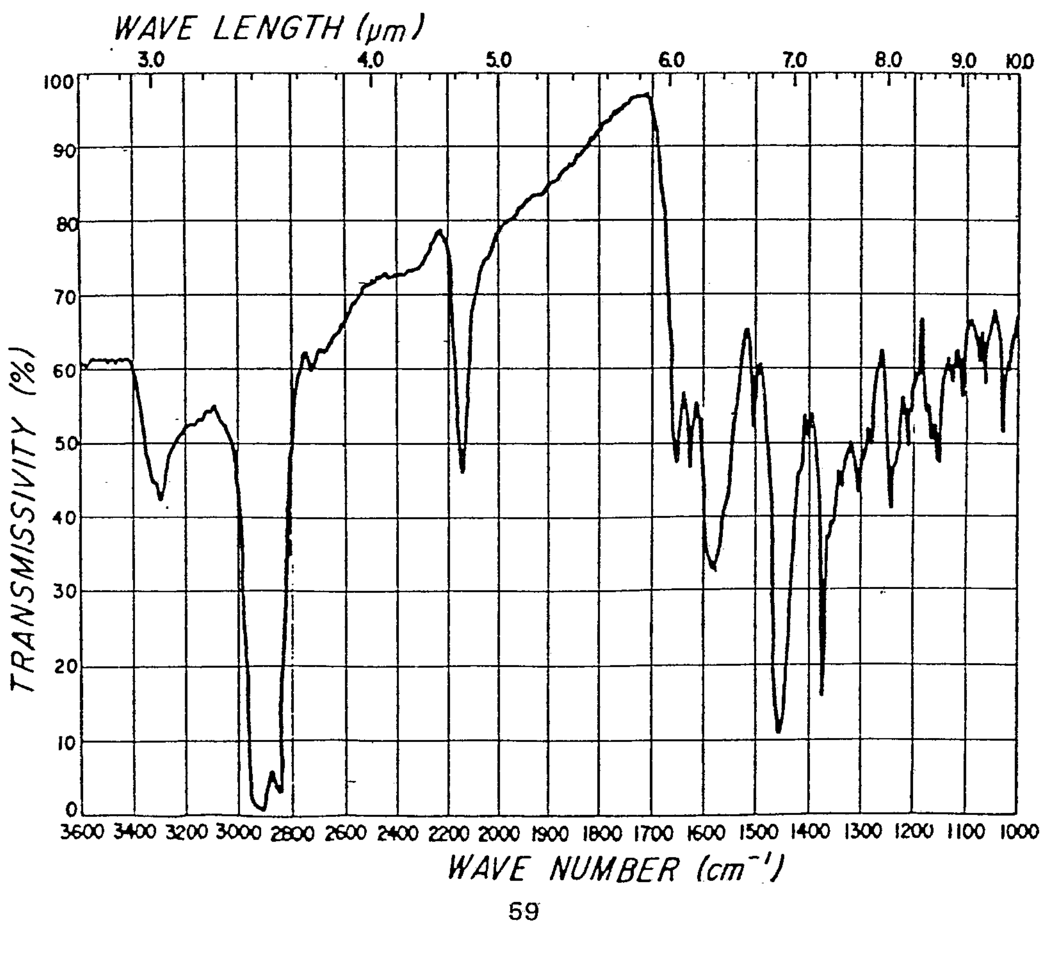

FIG. 119

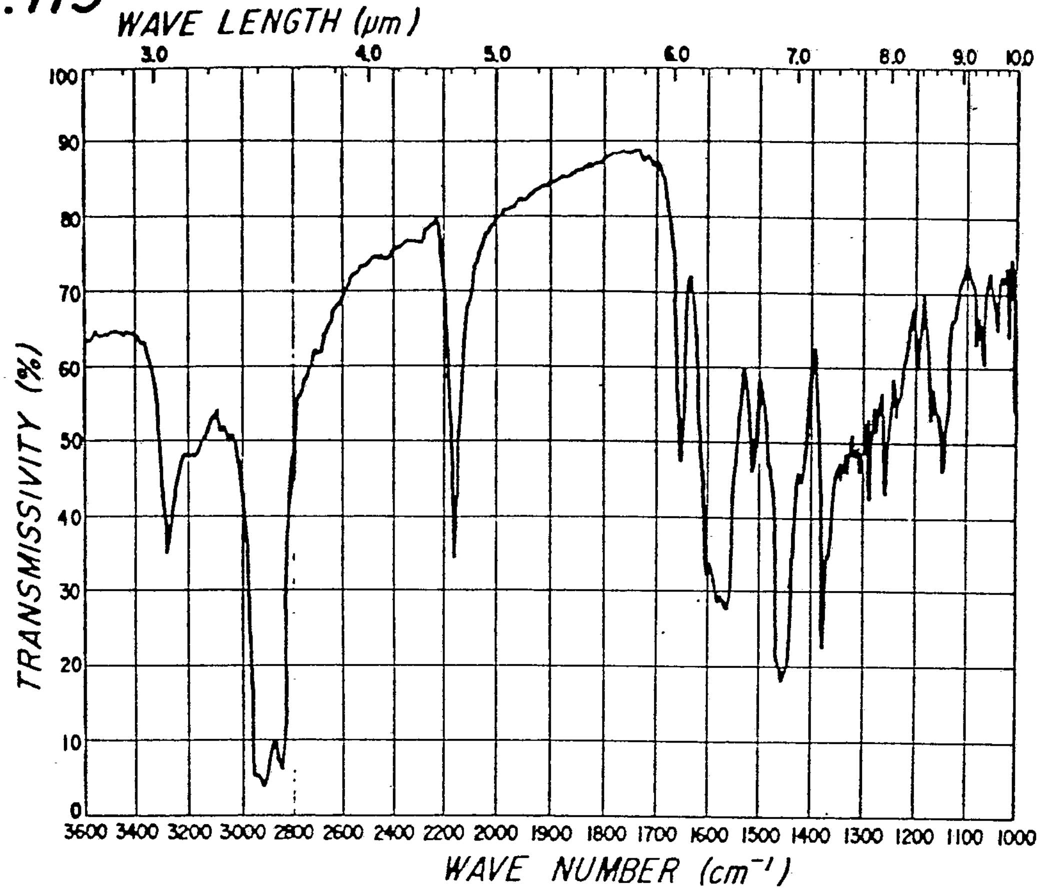


FIG. 120

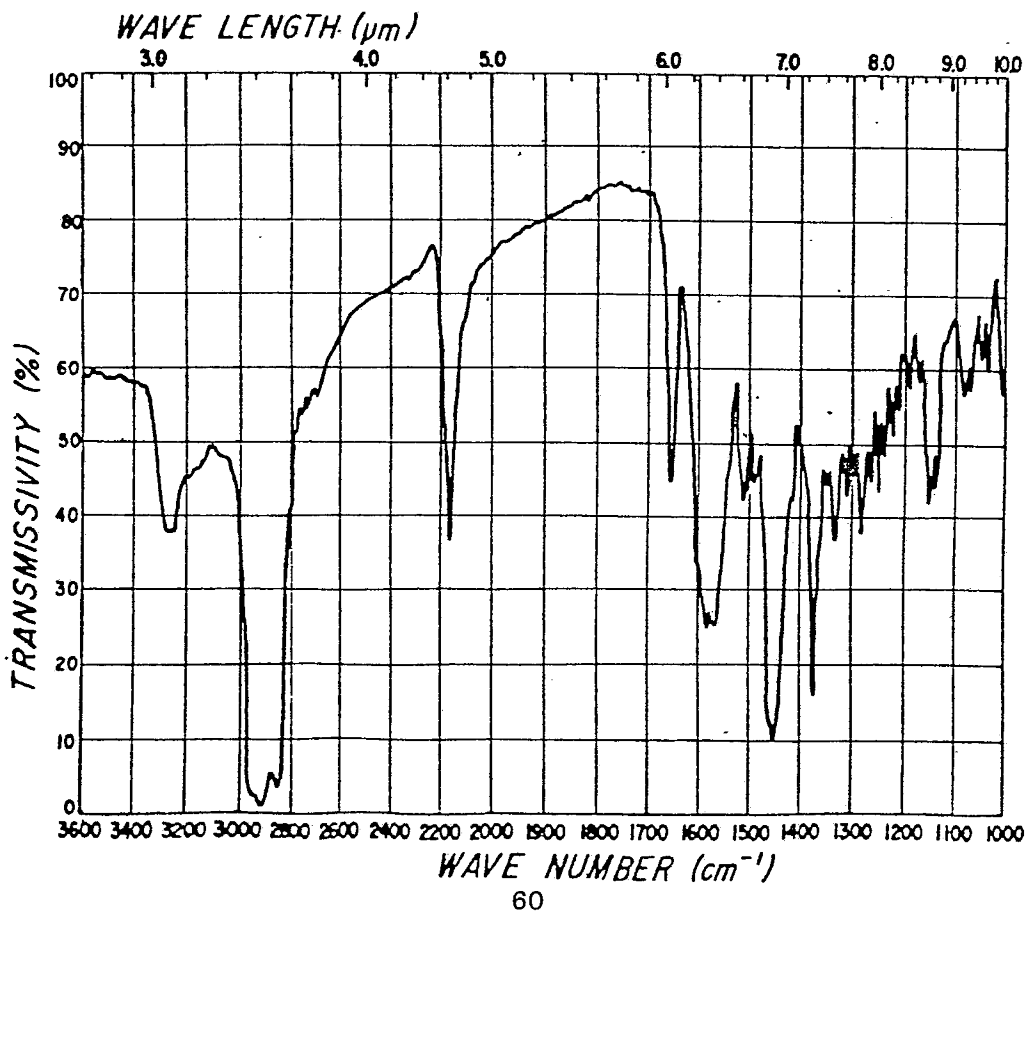

FIG. 121

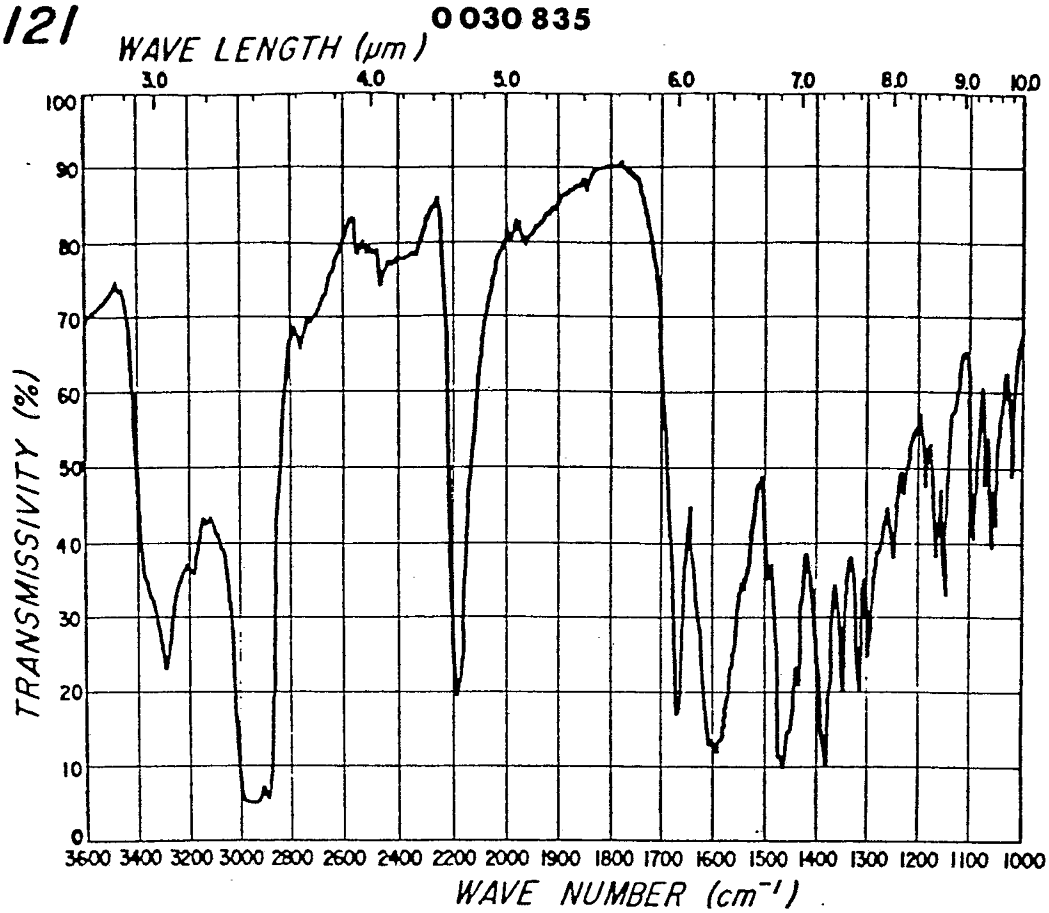


FIG. 122

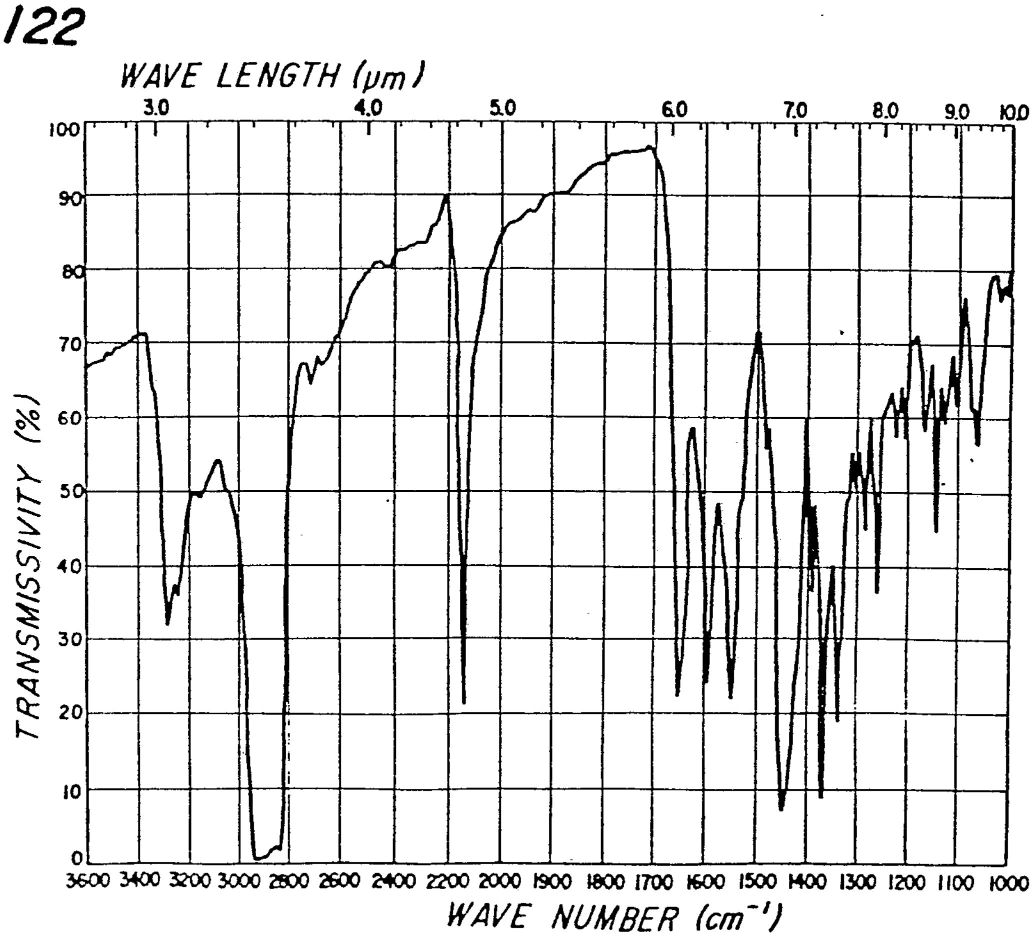

0 030 835

FIG. 123

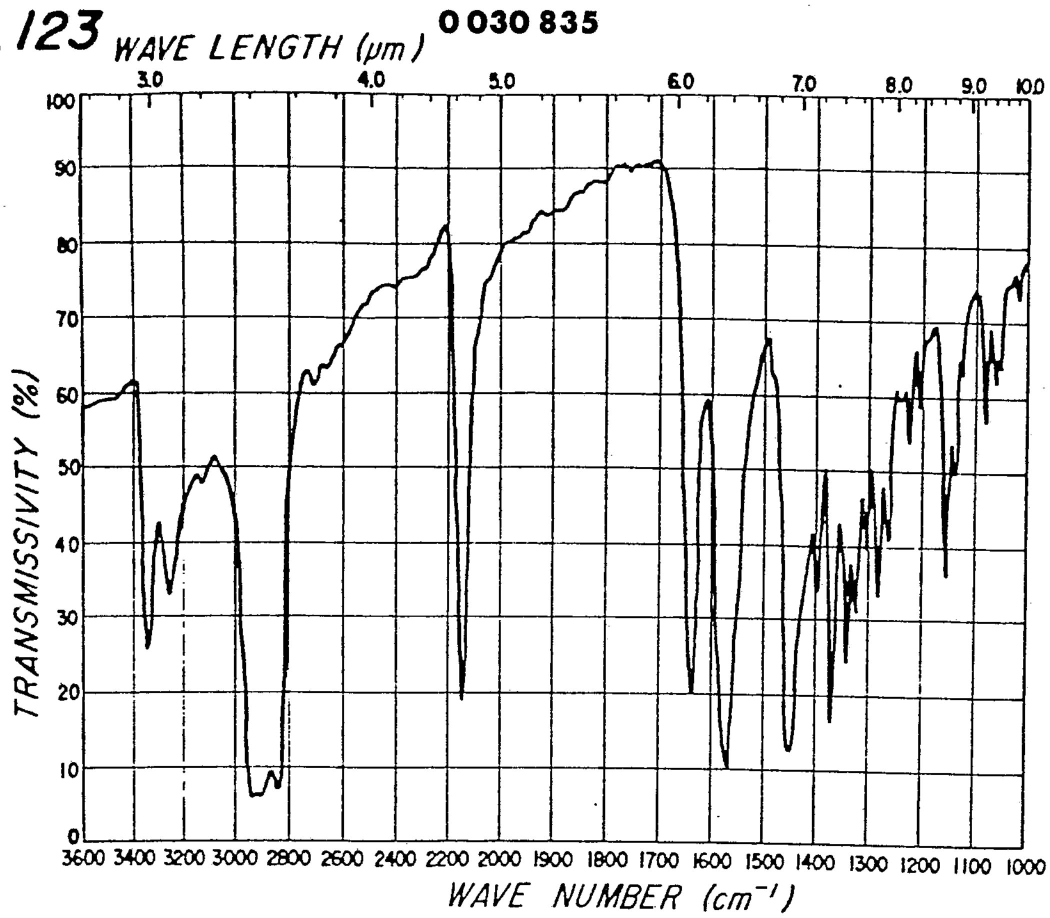


FIG. 124

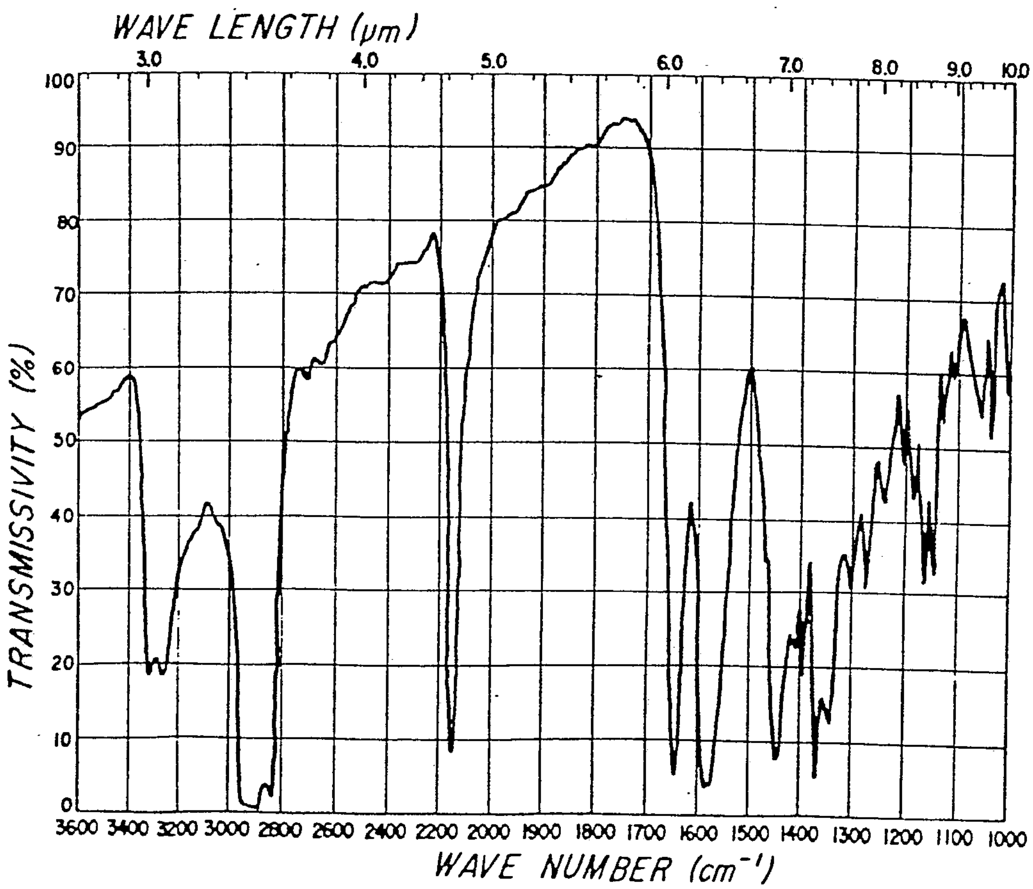

FIG. 125

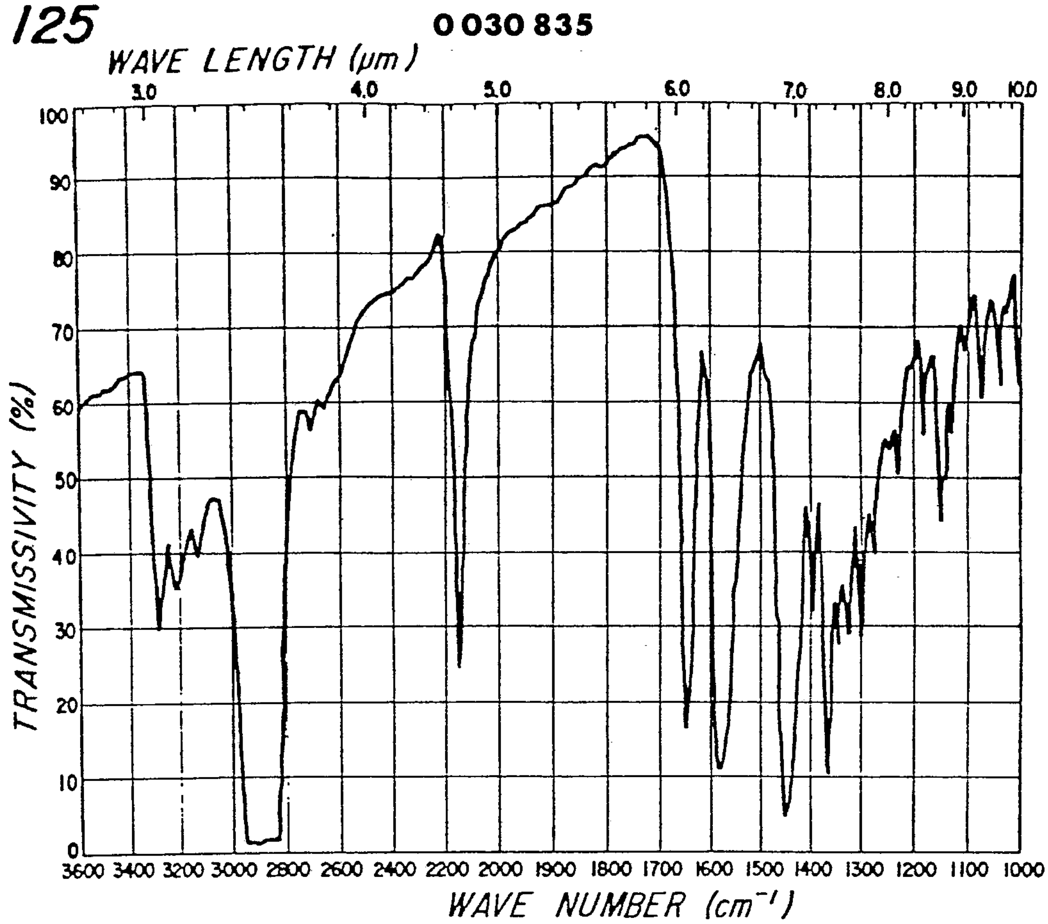


FIG. 126

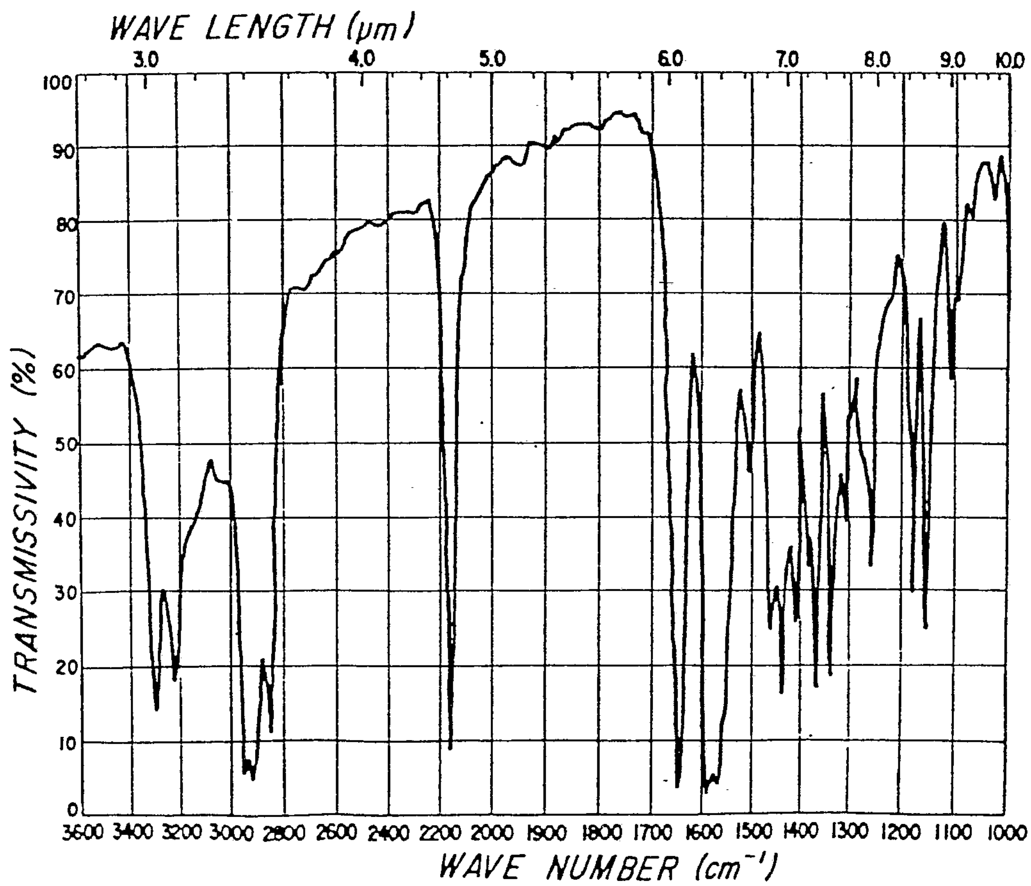

FIG. 127

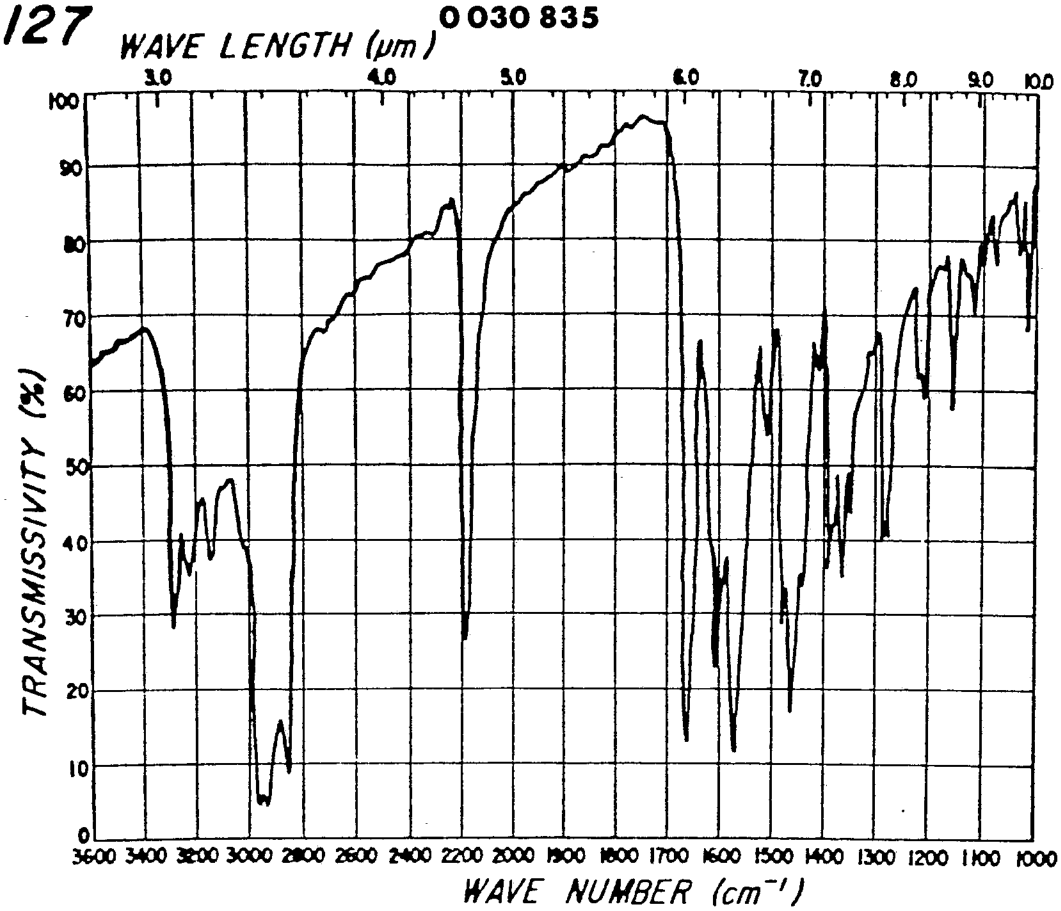


FIG. 128

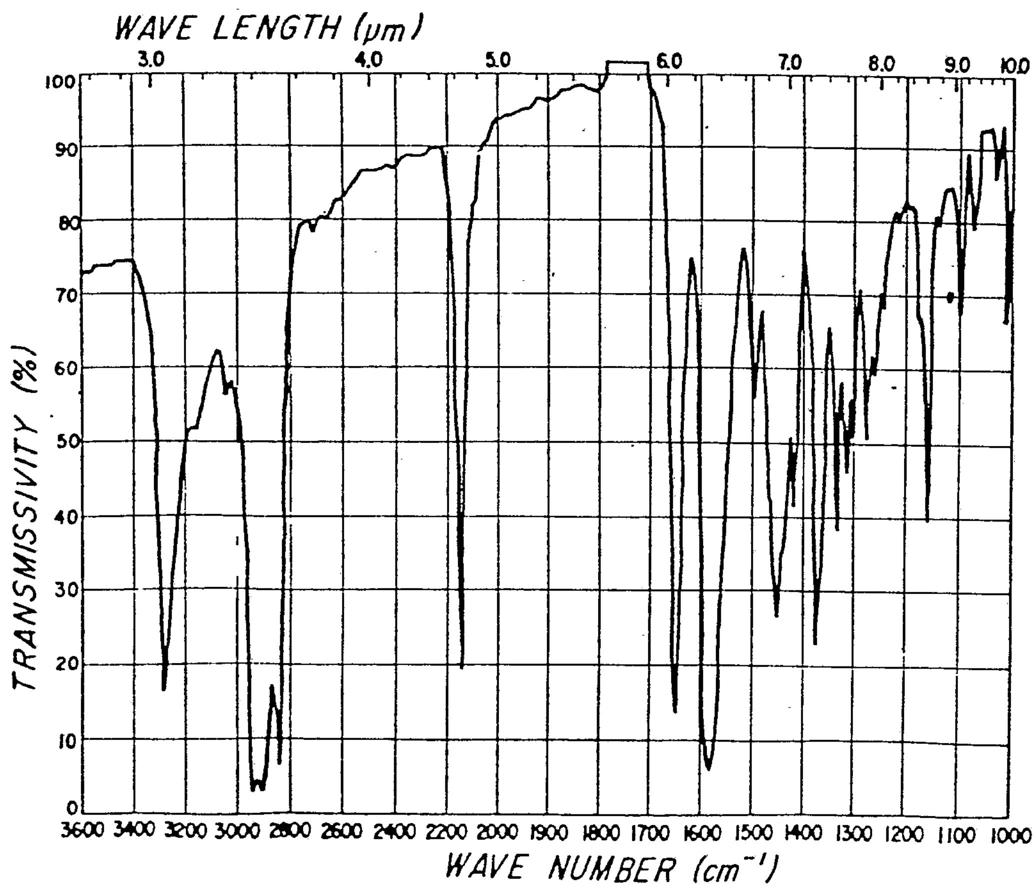

FIG. 129

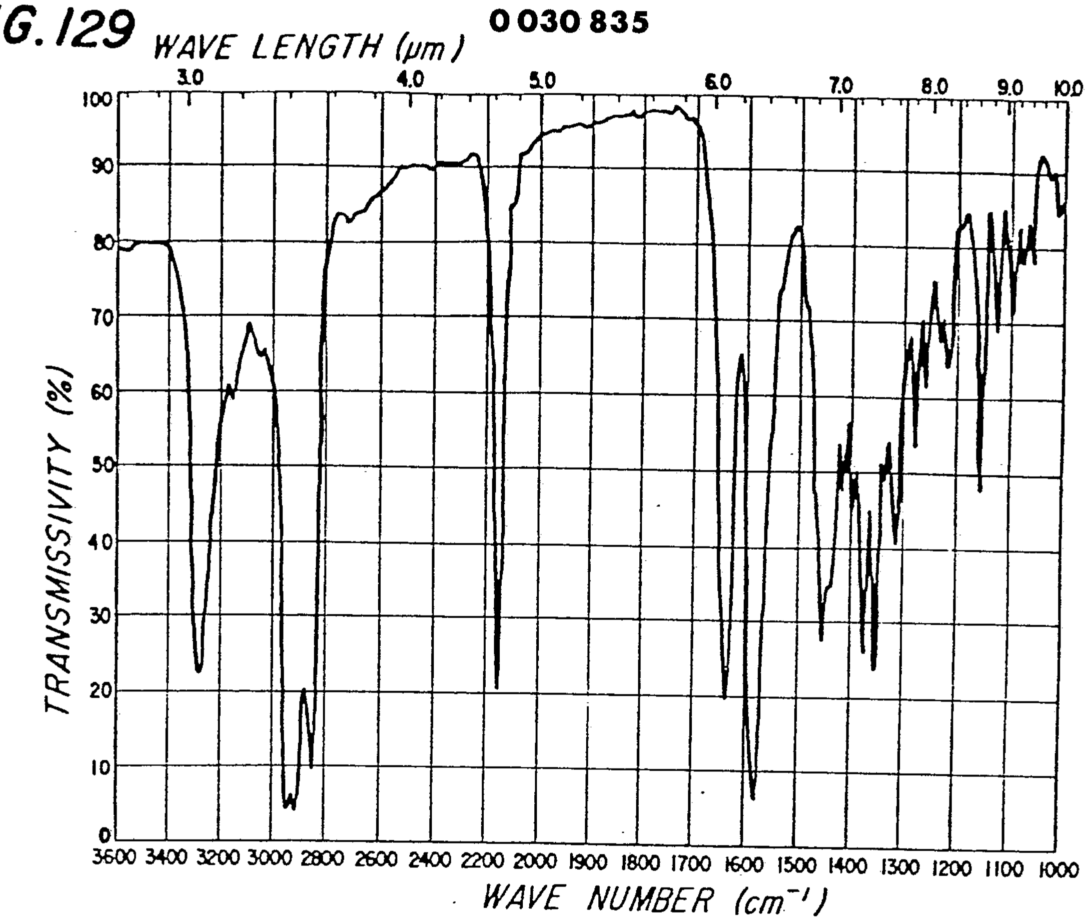


FIG. 130

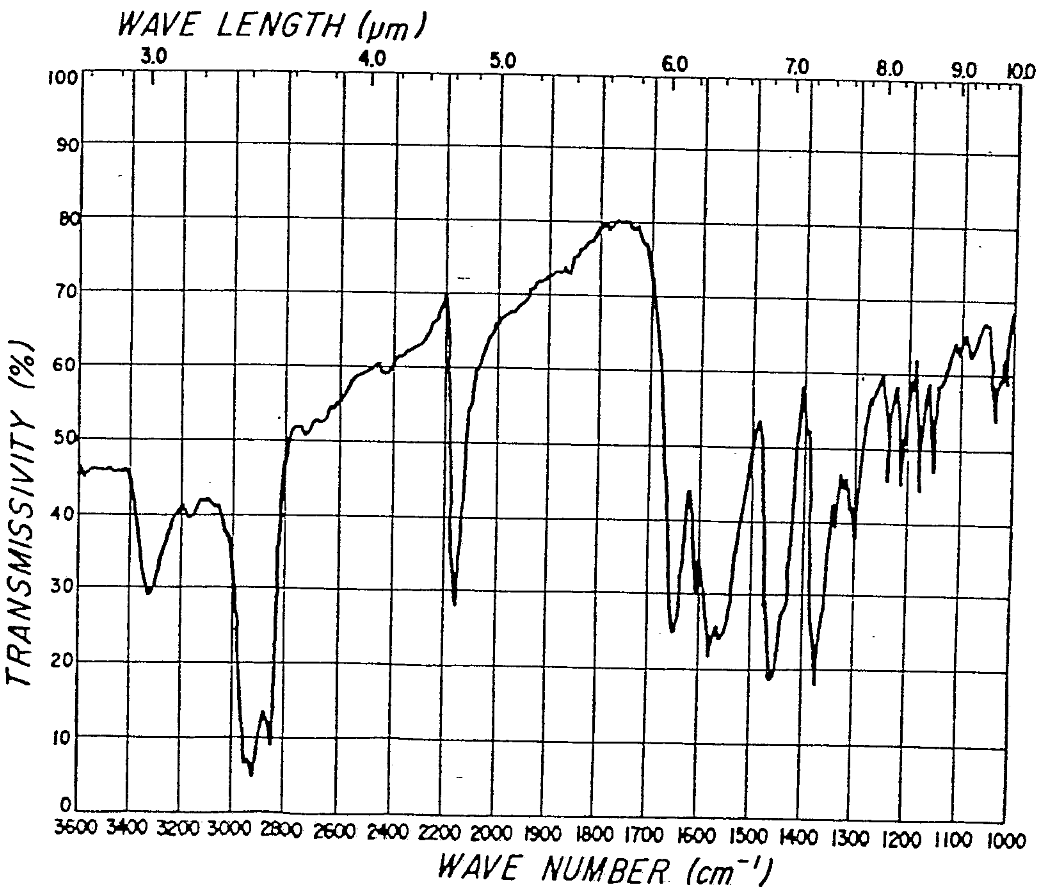

FIG. 131

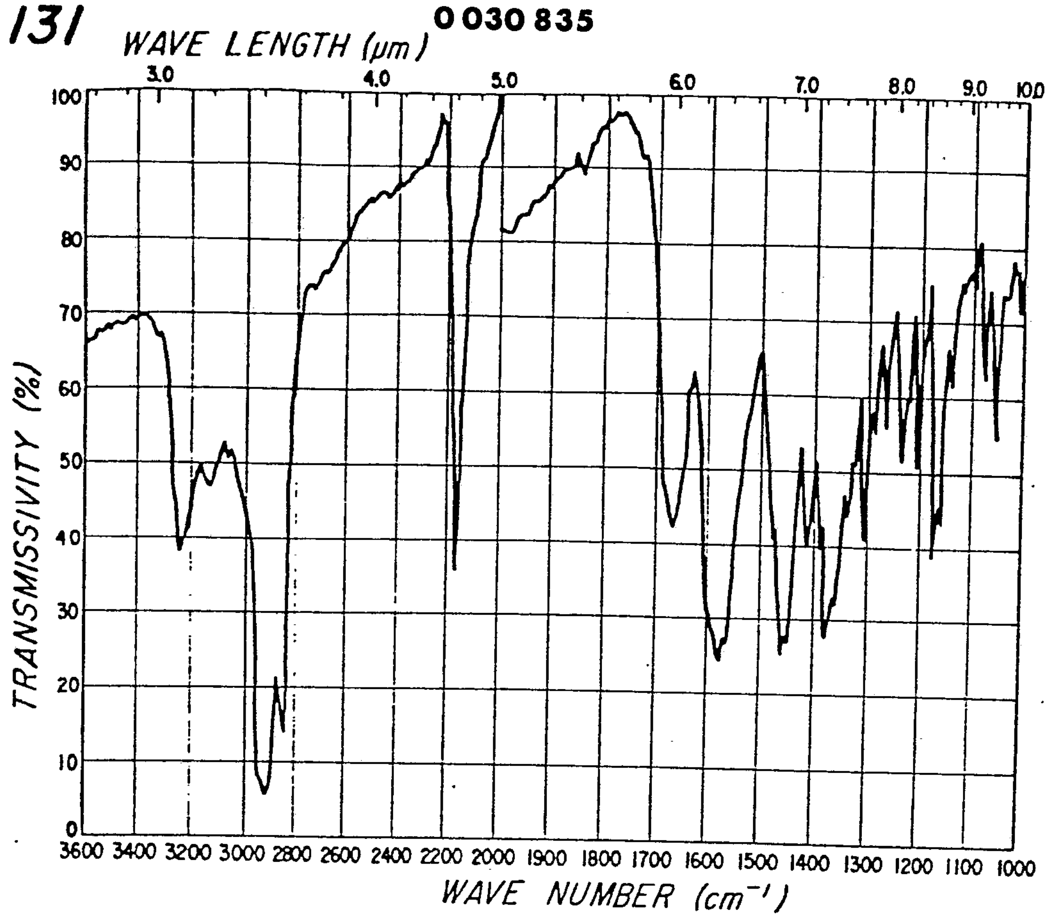


FIG. 132

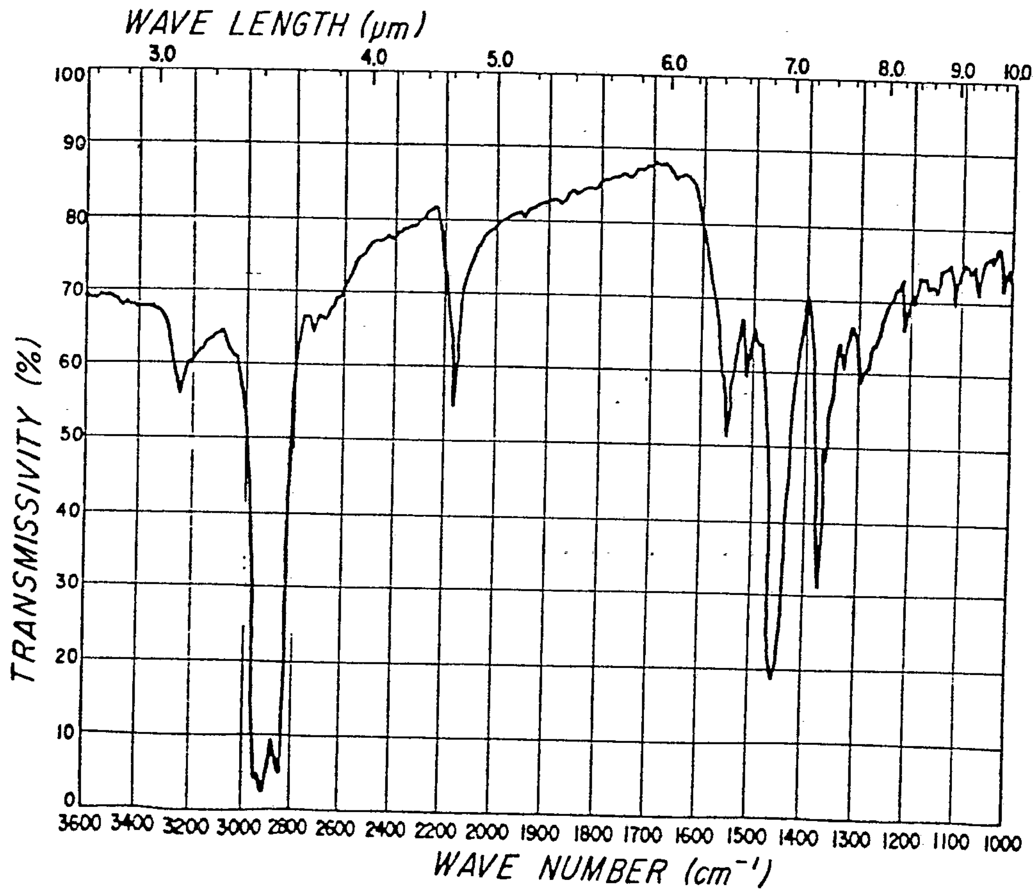

0 030 835

FIG. 133

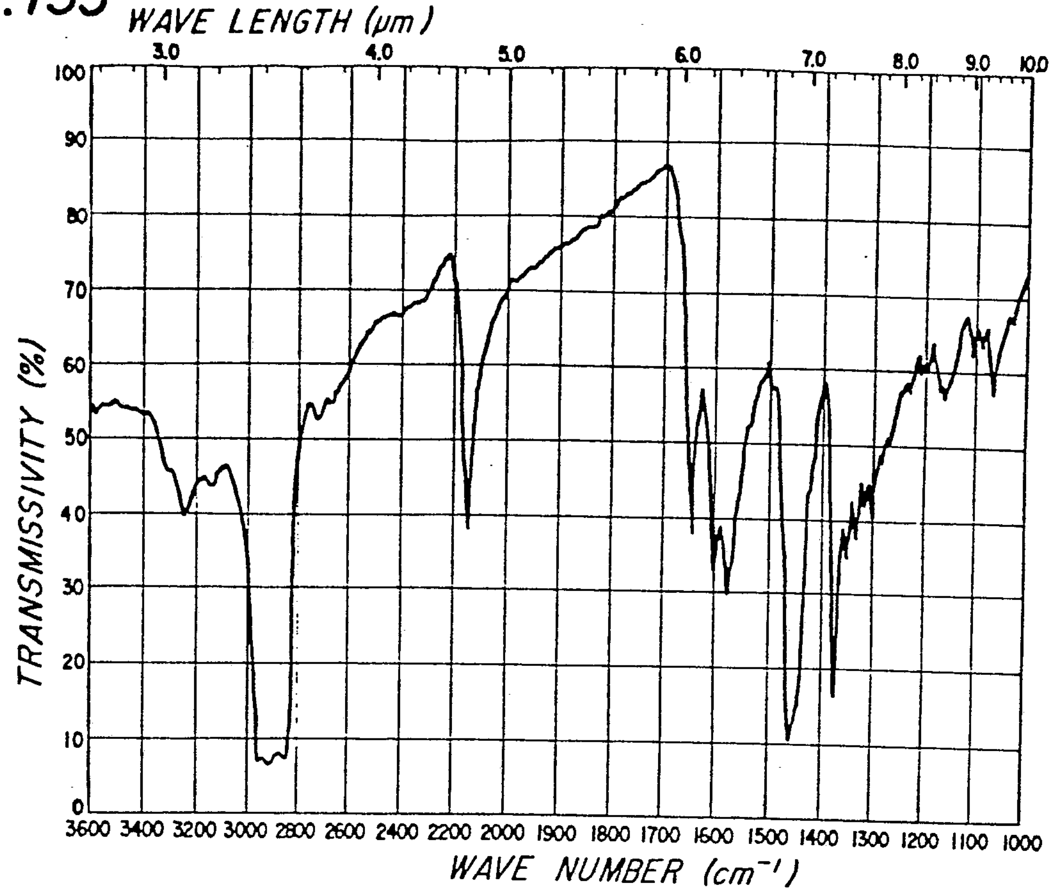


FIG. 134

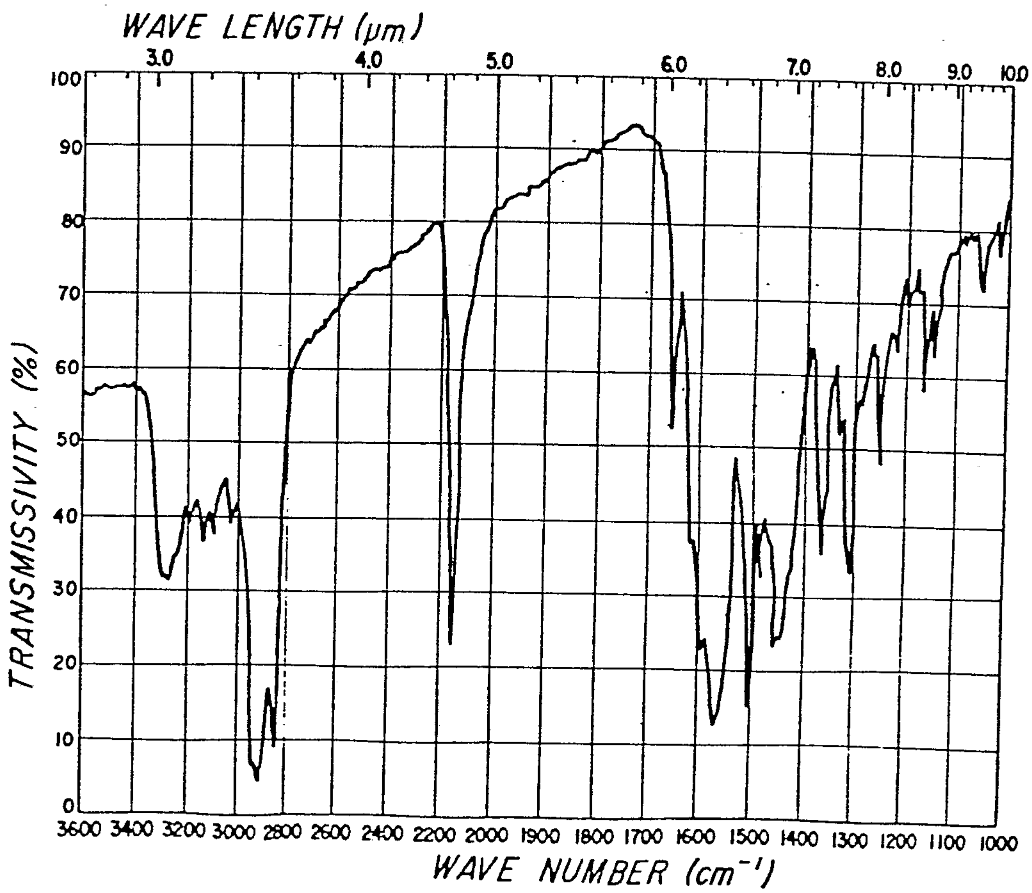

FIG. 135

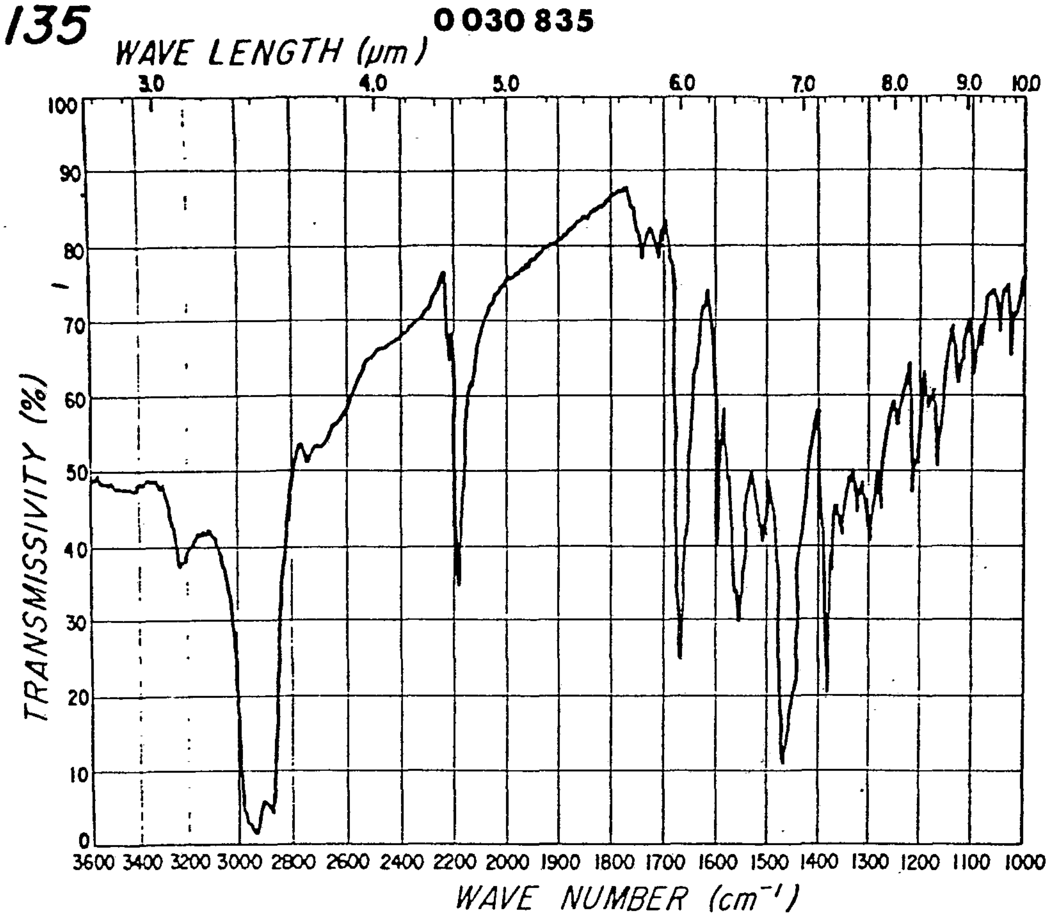


FIG. 136

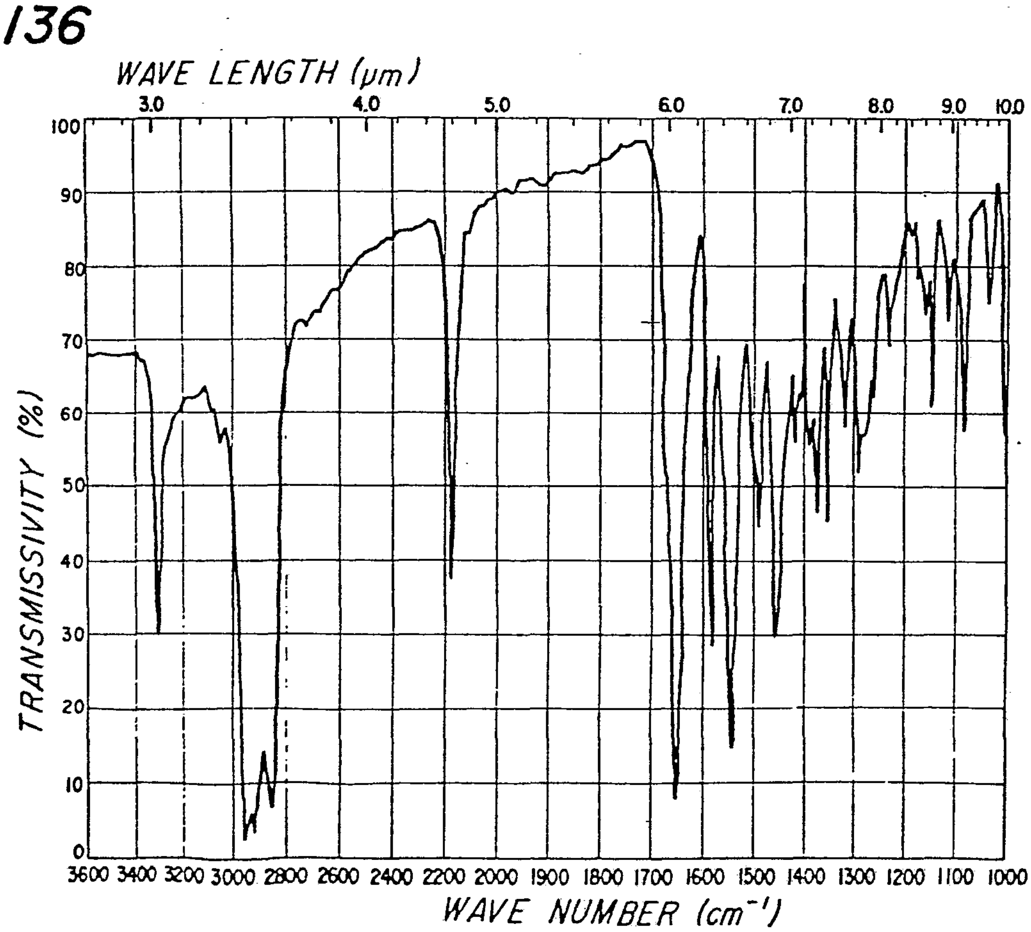

FIG. 137

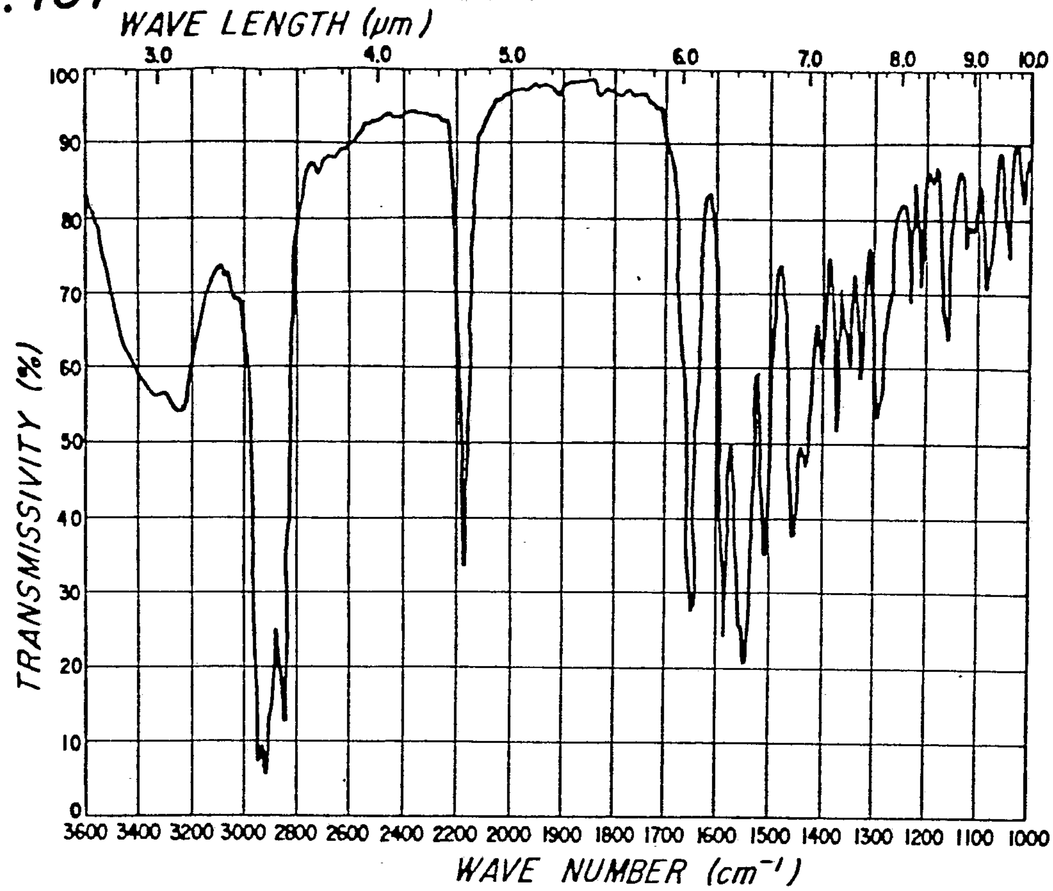


FIG. 138

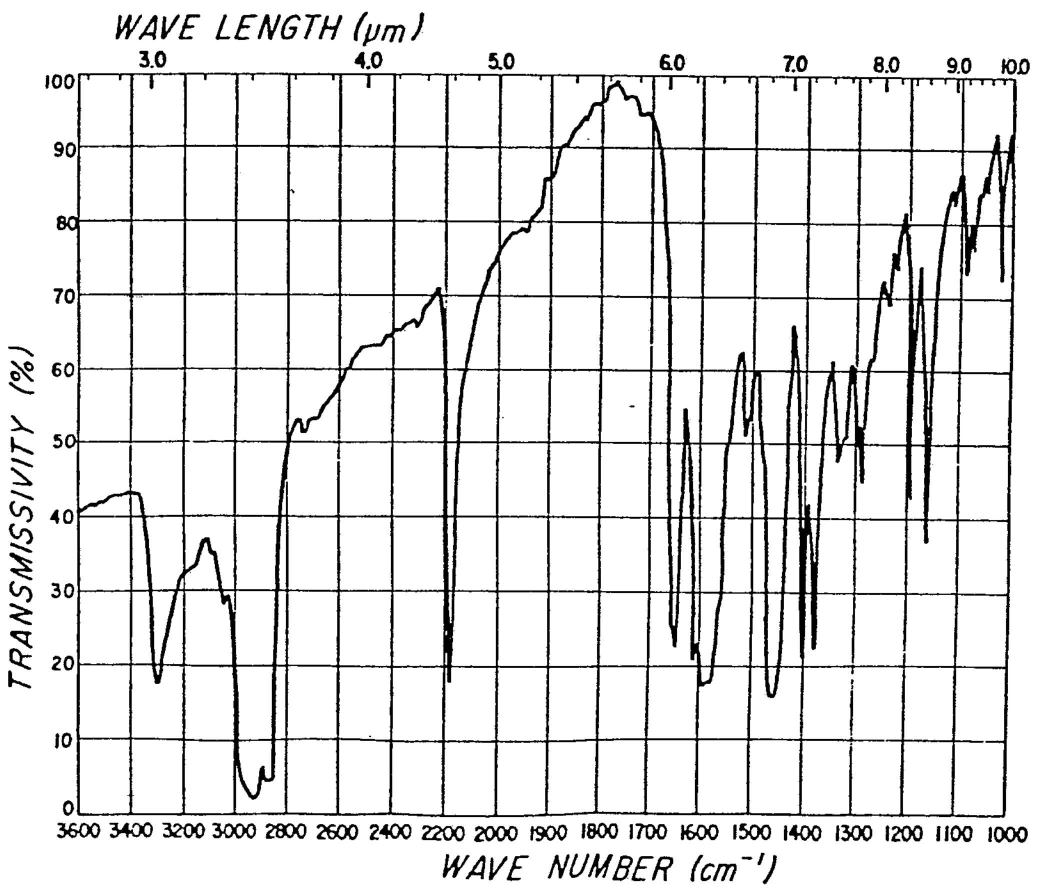

FIG. 139

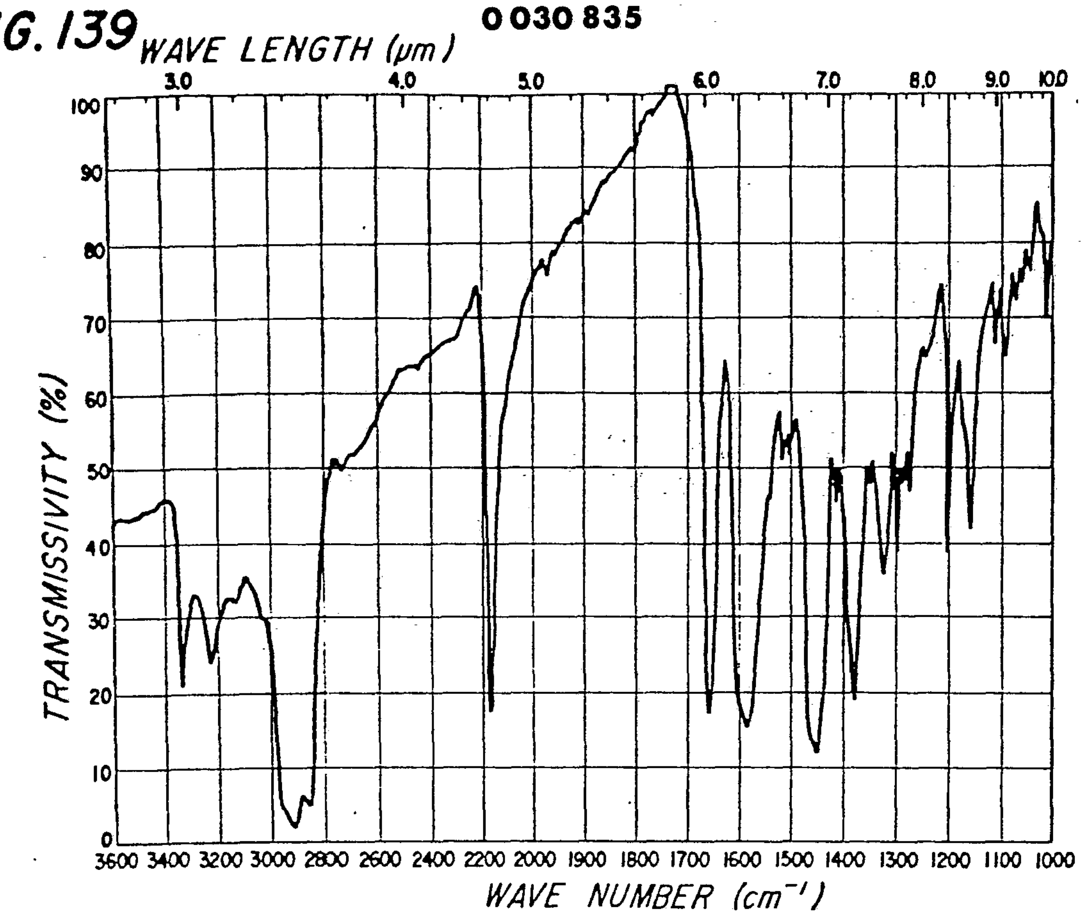


FIG. 140

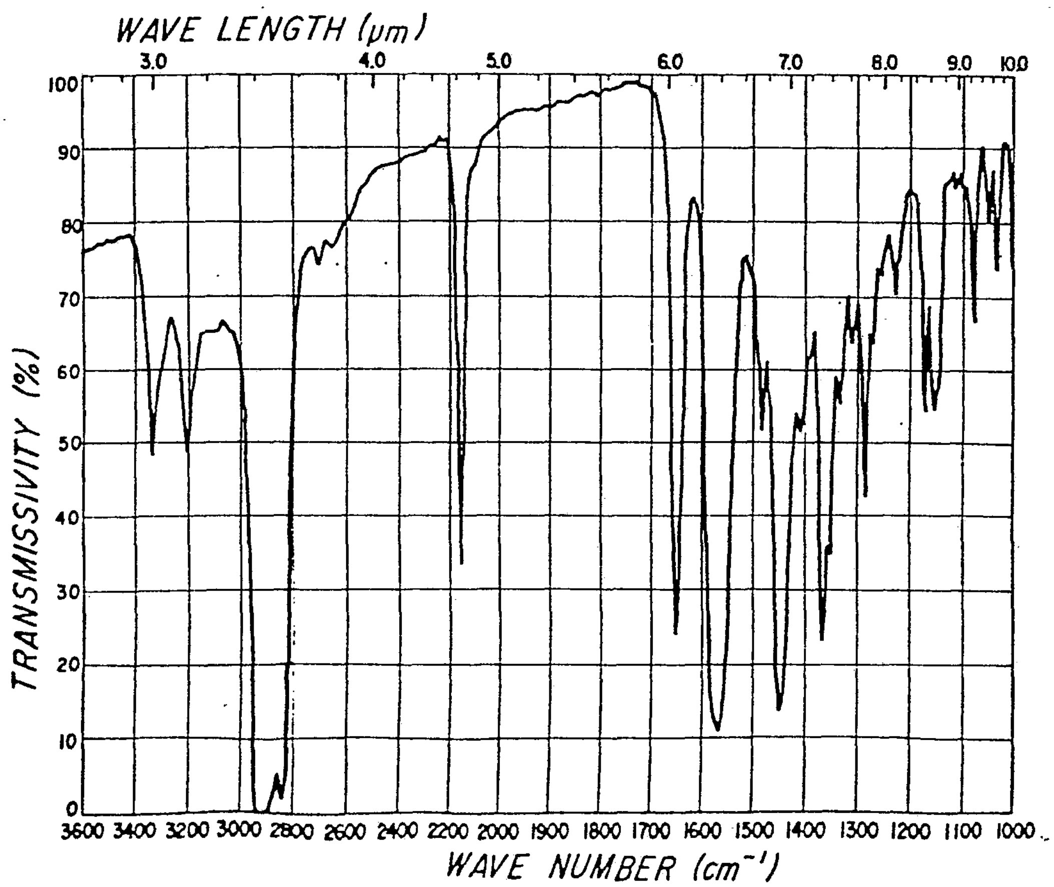

FIG. 141

0 030 835

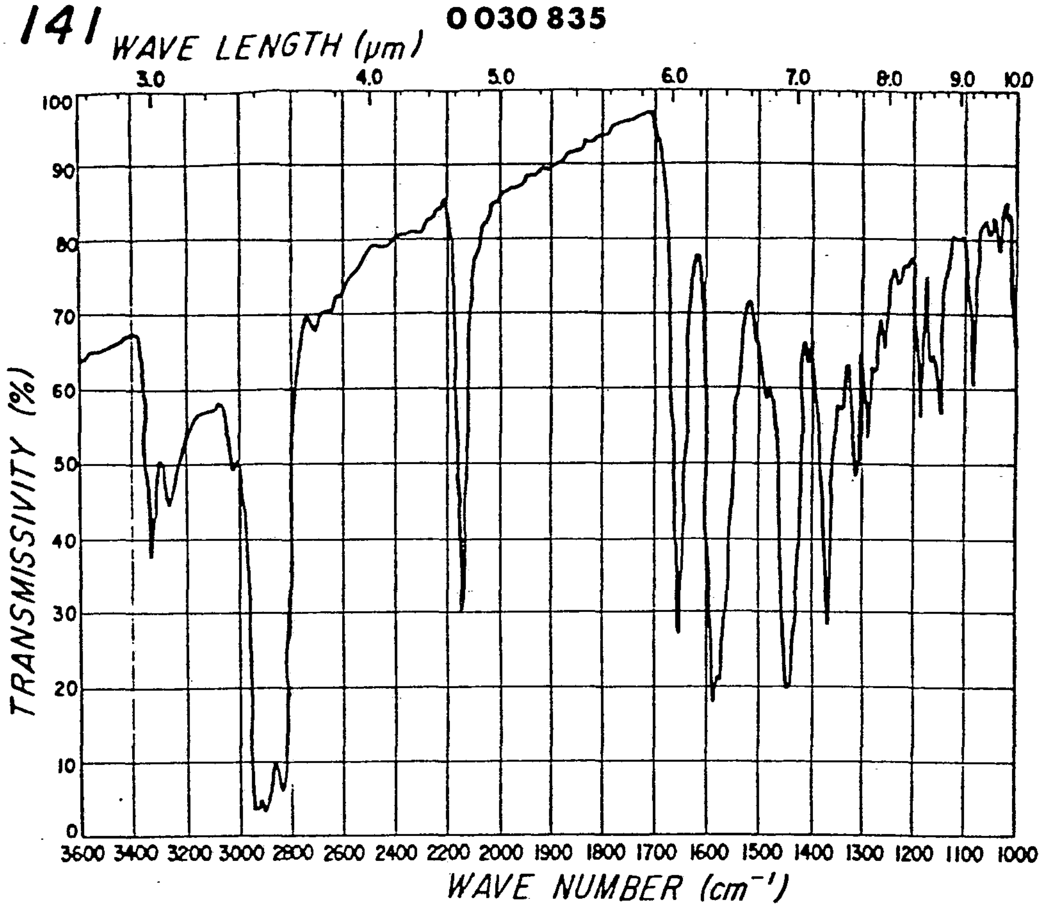


FIG. 142

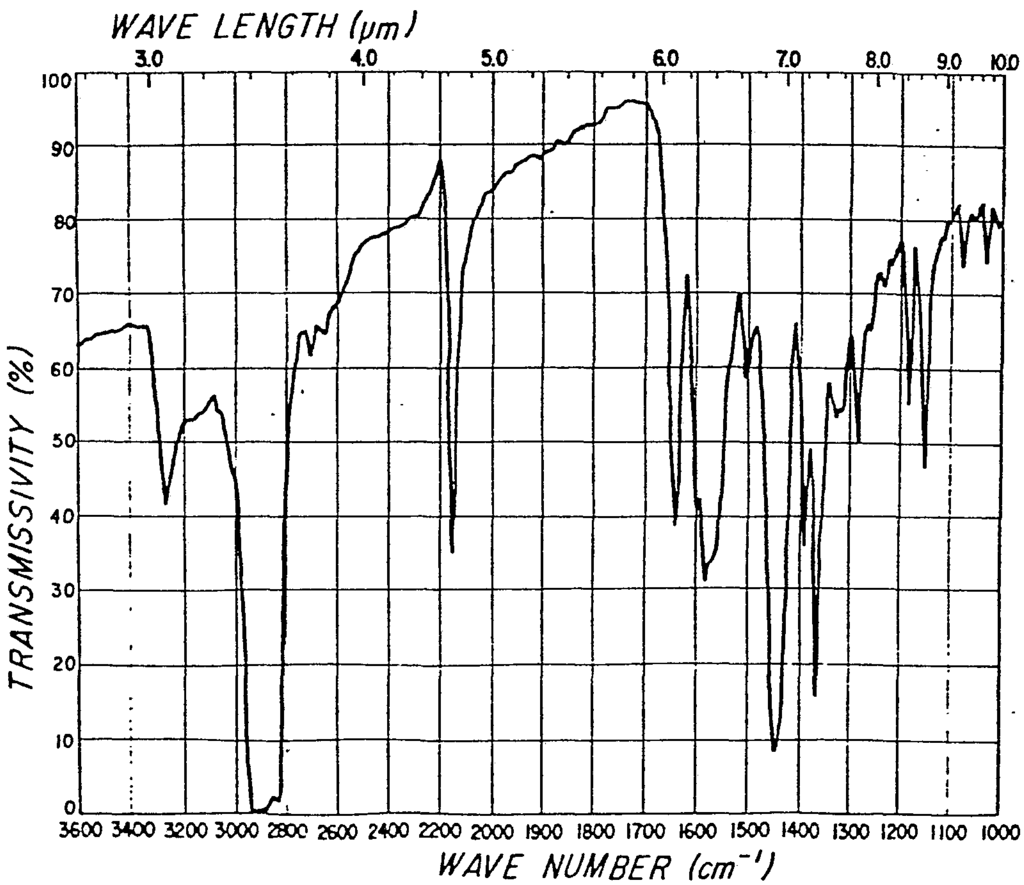

FIG. 143

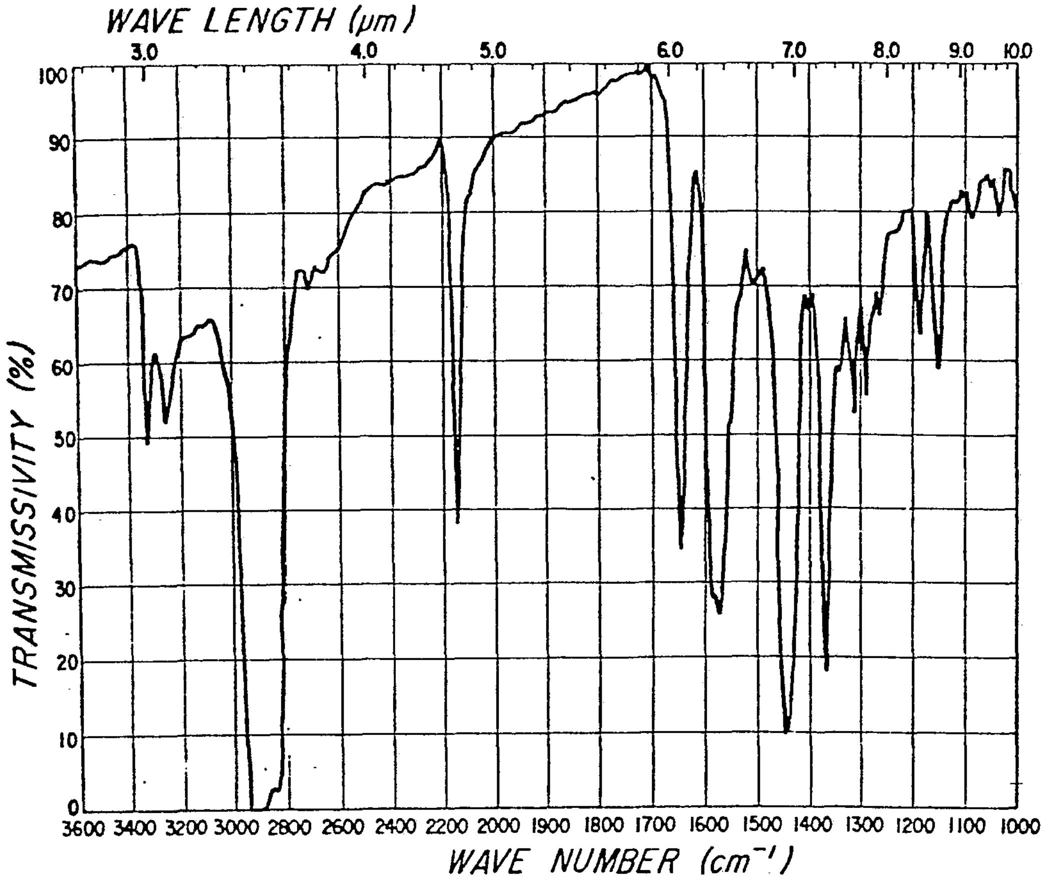
WAVE LENGTH (μm)
3.0 4.0 5.0 6.0 7.0 8.0 9.0 10.0
100 90 80 70 60 50 40 30 20 10 0
TRANSMISSIVITY (%)
3600 3400 3200 3000 2800 2600 2400 2200 2000 1900 1800 1700 1600 1500 1400 1300 1200 1100 1000
WAVE NUMBER (cm⁻¹)